(19) Europäisches Patentamt
European Patent Office
Office européen des brevets

(11) **EP 4 703 474 A1**

(12) **EUROPEAN PATENT APPLICATION**
published in accordance with Art. 153(4) EPC


(43) Date of publication:
**04.03.2026 Bulletin 2026/10**

(21) Application number: **24747371.3**

(22) Date of filing: **26.01.2024**

(51) International Patent Classification (IPC):
***C12N 15/13*** *(2006.01)* ***A61P 13/12*** *(2006.01)*
***A61K 31/5517*** *(2006.01)* ***A61K 39/395*** *(2006.01)*
***A61K 47/55*** *(2017.01)* ***A61K 47/65*** *(2017.01)*
***A61K 47/68*** *(2017.01)* ***A61P 1/02*** *(2006.01)*
***A61P 1/04*** *(2006.01)* ***A61P 1/16*** *(2006.01)*
***A61P 1/18*** *(2006.01)* ***A61P 11/00*** *(2006.01)*
***A61P 11/02*** *(2006.01)* ***A61P 11/04*** *(2006.01)*
***A61P 13/08*** *(2006.01)* ***A61P 13/10*** *(2006.01)*

(52) Cooperative Patent Classification (CPC):
**A61K 31/5517; A61K 39/395; A61K 47/55; A61K 47/65; A61K 47/68; A61P 1/02; A61P 1/04; A61P 1/16; A61P 1/18; A61P 11/00; A61P 11/02; A61P 11/04; A61P 13/08; A61P 13/10; A61P 13/12;**
(Cont.)

(86) International application number:
**PCT/JP2024/002363**

(87) International publication number:
**WO 2024/158047 (02.08.2024 Gazette 2024/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.01.2023 JP 2023010752**



(71) Applicant: **Daiichi Sankyo Company, Limited**
**Tokyo 103-8426 (JP)**

(72) Inventors:
- **SOMEYA, Shunsuke**
  **Tokyo 103-8426 (JP)**
- **YOSHIMURA, Chigusa**
  **Tokyo 103-8426 (JP)**
- **AMANO, Masato**
  **Tokyo 103-8426 (JP)**

(74) Representative: **Marks & Clerk LLP**
**15 Fetter Lane**
**London EC4A 1BW (GB)**


Remarks:
The complete document including Reference Table(s) and the Sequence Listing(s) can be downloaded from the EPO website

# (54) ANTI-LRRC15 ANTIBODY


(57) An object of the present invention is to provide a novel LRRC15 antibody or an antigen-binding fragment thereof that can be advantageously used in various therapeutic modalities, especially in antibody-drug conjugates (ADC), for example. The present invention provides an antibody that specifically binds to a novel epitope in LRRC15, for example.


Fig. 3-1

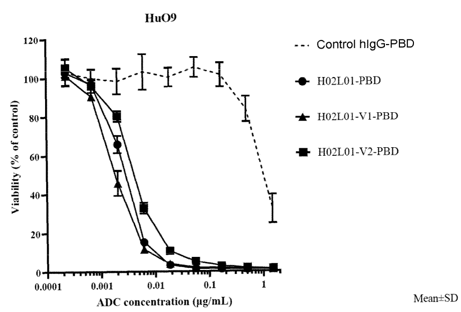




**(Cont. next page)**


EP 4 703 474 A1

(52) Cooperative Patent Classification (CPC): (Cont.)
**A61P 15/00; A61P 17/00; A61P 19/08;
A61P 25/00; A61P 35/00; A61P 43/00;
C07K 16/00; C07K 16/28; C07K 16/46;
C12N 5/10; C12N 15/62; C12N 15/74;
C12N 15/80; C12N 15/81**

## Description

### Technical Field

The present invention provides an antibody or an antigen-binding fragment thereof that specifically binds to human LRRC15, an antibody-drug conjugate (especially, antibody-pyrrolobenzodiazepine (PBD) derivative conjugate) comprising the aforementioned antibody or antigen-binding fragment, a method for producing these, and their use for treating patients with cancer.

### Background Art

Cancer is one of the leading causes of death, and the number of affected individuals is expected to increase as the population ages; however, the needs for treatment have not been sufficiently met yet.
Leucine-rich repeat-containing protein 15 (LRRC15) is a single-pass transmembrane protein that contains 15 leucine-rich repeats, whose N-terminus and C-terminus are located on the extracellular side and intracellular side, respectively. It has been reported that LRRC15 is more likely to be expressed in tumor tissues than in normal tissues, and that the high expression of LRRC15 leads to poor prognosis, and LRRC15 is considered to be one of the target molecules for cancer treatment. In addition, there is an anti-LRRC15 antibody-drug conjugate (ADC) actually administered to a human, namely an ADC obtained by conjugating a cytotoxic agent, MMAE, to an anti-LRRC15 antibody (International Publication Nos. WO 2017/095805 and WO 2017/095808). However, there are no approved anticancer agents that target LRRC15, including the above-mentioned ADC, and therefore, there is a need for antibodies with suitable qualities for targeting LRRC15 and therapeutic modalities for targeting LRRC15.

### Citation List

### Patent Literatures

Patent Literature 1: International Publication No. WO 2017/095805
Patent Literature 2: International Publication No. WO 2017/095808

### Summary of Invention

### Technical Problem

An object of the present invention is to provide a novel anti-LRRC15 antibody or an antigen-binding fragment thereof that can be advantageously used in various therapeutic modalities, especially in antibody-drug conjugates (ADC).

### Solution to Problem

The present inventors conducted diligent studies to achieve the above-described object, and as a result, surprisingly found that a novel antibody that specifically binds to a specific epitope in LRRC15 is useful in various therapeutic modalities, particularly as an antibody for ADC. Also, the present inventors succeeded in obtaining an anti-LRRC15 antibody-drug conjugate in which the anti-LRRC15 antibody is bound to a PBD derivative, and found that the anti-LRRC15 antibody-drug conjugate exhibits strong antitumor activity, leading to the completion of the present invention.
The present invention provides the following, for example.

[1] An antibody or an antigen-binding fragment of the antibody that specifically binds to an epitope at least partially present in a region selected from the group consisting of:

- a region consisting of amino acid residues 22 to 53 of SEQ ID NO: 1;
- a region consisting of amino acid residues 54 to 75 of SEQ ID NO: 1;
- a region consisting of amino acid residues 78 to 99 of SEQ ID NO: 1;
- a region consisting of amino acid residues 102 to 123 of SEQ ID NO: 1;
- a region consisting of amino acid residues 126 to 147 of SEQ ID NO: 1;
- a region consisting of amino acid residues 150 to 171 of SEQ ID NO: 1;
- a region consisting of amino acid residues 174 to 195 of SEQ ID NO: 1;

- a region consisting of amino acid residues 198 to 219 of SEQ ID NO: 1;
- a region consisting of amino acid residues 222 to 243 of SEQ ID NO: 1;
- a region consisting of amino acid residues 246 to 267 of SEQ ID NO: 1;
- a region consisting of amino acid residues 270 to 291 of SEQ ID NO: 1; and
- a region consisting of amino acid residues 294 to 315 of SEQ ID NO: 1.

[2] The antibody or an antigen-binding fragment of the antibody according to [1], wherein the antibody or an antigen-binding fragment of the antibody specifically binds to an epitope at least partially present in a region selected from the group consisting of:

- a region consisting of amino acid residues 22 to 53 of SEQ ID NO: 1;
- a region consisting of amino acid residues 54 to 75 of SEQ ID NO: 1;
- a region consisting of amino acid residues 78 to 99 of SEQ ID NO: 1;
- a region consisting of amino acid residues 102 to 123 of SEQ ID NO: 1;
- a region consisting of amino acid residues 126 to 147 of SEQ ID NO: 1;
- a region consisting of amino acid residues 150 to 171 of SEQ ID NO: 1;
- a region consisting of amino acid residues 174 to 195 of SEQ ID NO: 1; and
- a region consisting of amino acid residues 198 to 219 of SEQ ID NO: 1.

[3] The antibody or an antigen-binding fragment of the antibody according to [1] or [2], wherein the antibody or an antigen-binding fragment of the antibody specifically binds to an epitope at least partially present in a region selected from the group consisting of:

- a region consisting of amino acid residues 22 to 53 of SEQ ID NO: 1;
- a region consisting of amino acid residues 54 to 75 of SEQ ID NO: 1; and
- a region consisting of amino acid residues 78 to 99 of SEQ ID NO: 1.

[4] The antibody or an antigen-binding fragment of the antibody according to any one of [1] to [3], wherein the antibody or an antigen-binding fragment of the antibody competes for binding to human LRRC15 with at least one antibody consisting of the amino acid sequence of SEQ ID NO: 1, selected from the group consisting of:

(1) an antibody having a light chain consisting of the amino acid sequence set forth in SEQ ID NO: 66 and a heavy chain consisting of the amino acid sequence set forth in SEQ ID NO: 42;
(2) an antibody having a light chain consisting of the amino acid sequence set forth in SEQ ID NO: 66 and a heavy chain consisting of the amino acid sequence set forth in SEQ ID NO: 48;
(3) an antibody having a light chain consisting of the amino acid sequence set forth in SEQ ID NO: 66 and a heavy chain consisting of the amino acid sequence set forth in SEQ ID NO: 54; and
(4) an antibody having a light chain consisting of the amino acid sequence set forth in SEQ ID NO: 60 and a heavy chain consisting of the amino acid sequence set forth in SEQ ID NO: 36.

[5] An antibody or an antigen-binding fragment of the antibody, comprising CDRL1, CDRL2 and CDRL3, and CDRH1, CDRH2 and CDRH3 selected from the group consisting of the following (1) to (4):

(1) CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and CDRH1 consisting of the amino acid sequence set forth in SEQ ID NO: 38, CDRH2 consisting of the amino acid sequence set forth in SEQ ID NO: 39, and CDRH3 consisting of the amino acid sequence set forth in SEQ ID NO: 40;
(2) CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and CDRH1 consisting of the amino acid sequence set forth in SEQ ID NO: 44, CDRH2 consisting of the amino acid sequence set forth in SEQ ID NO: 45, and CDRH3 consisting of the amino acid sequence set forth in SEQ ID NO: 46;
(3) CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and CDRH1 consisting of the amino acid sequence set forth in SEQ ID NO: 50, CDRH2 consisting of the amino acid sequence set forth in SEQ ID NO: 51, and CDRH3 consisting of the amino acid sequence set forth in SEQ ID NO: 52; and
(4) CDRL1 consisting of the amino acid sequence set forth in SEQ ID NO: 56, CDRL2 consisting of the amino acid sequence of WAS, and CDRL3 consisting of the amino acid sequence set forth in SEQ ID NO: 58, and CDRH1 consisting of the amino acid sequence set forth in SEQ ID NO: 32, CDRH2 consisting of the amino acid sequence

set forth in SEQ ID NO: 33, and CDRH3 consisting of the amino acid sequence set forth in SEQ ID NO: 34.

[6] The antibody or an antigen-binding fragment of the antibody according to any one of [1] to [5], having an internalization ability to be taken up into a cell.
[7] The antibody or an antigen-binding fragment of the antibody according to any one of [1] to [6], wherein the antibody or an antigen-binding fragment of the antibody binds to human LRRC15 with a KD of 1 pM to 1000 nM.
[8] The antibody or an antigen-binding fragment of the antibody according to any one of [1] to [7], wherein the antibody or an antigen-binding fragment of the antibody is humanized.
[9] The antibody or an antigen-binding fragment of the antibody according to any one of [1] to [8], comprising a light chain variable region and a heavy chain variable region of one selected from the group consisting of the following (1) to (4):

(1) a light chain variable region consisting of an amino acid sequence having at least 95% or more sequence identity to the amino acid sequence set forth in SEQ ID No: 65, and a heavy chain variable region consisting of an amino acid sequence having at least 95% or more sequence identity to the amino acid sequence set forth in SEQ ID No: 41;
(2) a light chain variable region consisting of an amino acid sequence having at least 95% or more sequence identity to the amino acid sequence set forth in SEQ ID No: 65, and a heavy chain variable region consisting of an amino acid sequence having at least 95% or more sequence identity to the amino acid sequence set forth in SEQ ID No: 47;
(3) a light chain variable region consisting of an amino acid sequence having at least 95% or more sequence identity to the amino acid sequence set forth in SEQ ID No: 65, and a heavy chain variable region consisting of an amino acid sequence having at least 95% or more sequence identity to the amino acid sequence set forth in SEQ ID No: 53; and
(4) a light chain variable region consisting of an amino acid sequence having at least 95% or more sequence identity to the amino acid sequence set forth in SEQ ID No: 59, and a heavy chain variable region consisting of an amino acid sequence having at least 95% or more sequence identity to the amino acid sequence set forth in SEQ ID No: 35.

[10] The antibody or an antigen-binding fragment of the antibody according to any one of [1] to [9], comprising a light chain variable region and a heavy chain variable region of one selected from the group consisting of the following (1) to (4):

(1) a light chain variable region consisting of the amino acid sequence set forth in SEQ ID NO: 65, and a heavy chain variable region consisting of the amino acid sequence set forth in SEQ ID NO: 41;
(2) a light chain variable region consisting of the amino acid sequence set forth in SEQ ID NO: 65, and a heavy chain variable region consisting of the amino acid sequence set forth in SEQ ID NO: 47;
(3) a light chain variable region consisting of the amino acid sequence set forth in SEQ ID NO: 65, and a heavy chain variable region consisting of the amino acid sequence set forth in SEQ ID NO: 53; and
(4) a light chain variable region consisting of the amino acid sequence set forth in SEQ ID NO: 59, and a heavy chain variable region consisting of the amino acid sequence set forth in SEQ ID NO: 35.

[11] The antibody or an antigen-binding fragment of the antibody according to any one of [1] to [10], comprising a light chain and a heavy chain of one selected from the group consisting of the following (1) to (4):

(1) a light chain consisting of the amino acid sequence of SEQ ID NO: 66, and a heavy chain consisting of the amino acid sequence of SEQ ID NO: 42;
(2) a light chain consisting of the amino acid sequence of SEQ ID NO: 66, and a heavy chain consisting of the amino acid sequence of SEQ ID NO: 48;
(3) a light chain consisting of the amino acid sequence of SEQ ID NO: 66, and a heavy chain consisting of the amino acid sequence of SEQ ID NO: 54; and
(4) a light chain consisting of the amino acid sequence of SEQ ID NO: 60, and a heavy chain consisting of the amino acid sequence of SEQ ID NO: 36.

[12] The antibody or an antigen-binding fragment of the antibody according to [11], comprising the light chain consisting of the amino acid sequence of SEQ ID NO: 66, and the heavy chain consisting of the amino acid sequence of SEQ ID NO: 42.
[13] The antibody or an antigen-binding fragment of the antibody according to [11], comprising the light chain

consisting of the amino acid sequence of SEQ ID NO: 66, and the heavy chain consisting of the amino acid sequence of SEQ ID NO: 48.

[14] The antibody or an antigen-binding fragment of the antibody according to [11], comprising the light chain consisting of the amino acid sequence of SEQ ID NO: 66, and the heavy chain consisting of the amino acid sequence of SEQ ID NO: 54.

[15] The antibody or an antigen-binding fragment of the antibody according to [11], comprising the light chain consisting of the amino acid sequence of SEQ ID NO: 60, and the heavy chain consisting of the amino acid sequence of SEQ ID NO: 36.

[16] The antigen-binding fragment of the antibody according to any one of [1] to [15], wherein the antigen-binding fragment is selected from the group consisting of Fab, $F(ab')_2$, Fab', Fv, scFv, VHH, and scFv-containing fusion protein.

[17] A polynucleotide that encodes the antibody or an antigen-binding fragment of the antibody according to any one of [1] to [16].

[18] The polynucleotide according to [17], comprising:

(1) a polynucleotide that encodes a light chain variable region containing CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and a polynucleotide that encodes a heavy chain variable region containing CDRH1 consisting of the amino acid sequence set forth in SEQ ID NO: 38, CDRH2 consisting of the amino acid sequence set forth in SEQ ID NO: 39, and CDRH3 consisting of the amino acid sequence set forth in SEQ ID NO: 40;
(2) a polynucleotide that encodes a light chain variable region containing CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and a polynucleotide that encodes a heavy chain variable region containing CDRH1 consisting of the amino acid sequence set forth in SEQ ID NO: 44, CDRH2 consisting of the amino acid sequence set forth in SEQ ID NO: 45, and CDRH3 consisting of the amino acid sequence set forth in SEQ ID NO: 46;
(3) a polynucleotide that encodes a light chain variable region containing CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and a polynucleotide that encodes a heavy chain variable region containing CDRH1 consisting of the amino acid sequence set forth in SEQ ID NO: 50, CDRH2 consisting of the amino acid sequence set forth in SEQ ID NO: 51, and CDRH3 consisting of the amino acid sequence set forth in SEQ ID NO: 52; or
(4) a polynucleotide that encodes a light chain variable region containing CDRL1 consisting of the amino acid sequence set forth in SEQ ID NO: 56, CDRL2 consisting of the amino acid sequence of WAS, and CDRL3 consisting of the amino acid sequence set forth in SEQ ID NO: 58, and a polynucleotide that encodes a heavy chain variable region containing CDRH1 consisting of the amino acid sequence set forth in SEQ ID NO: 32, CDRH2 consisting of the amino acid sequence set forth in SEQ ID NO: 33, and CDRH3 consisting of the amino acid sequence set forth in SEQ ID NO: 34.

[19] The polynucleotide according to [17] or [18], comprising:

(1) a polynucleotide that encodes a light chain consisting of the amino acid sequence of SEQ ID NO: 66, and a polynucleotide that encodes a heavy chain consisting of the amino acid sequence of SEQ ID NO: 42;
(2) a polynucleotide that encodes a light chain consisting of the amino acid sequence of SEQ ID NO: 66, and a polynucleotide that encodes a heavy chain consisting of the amino acid sequence of SEQ ID NO: 48;
(3) a polynucleotide that encodes a light chain consisting of the amino acid sequence of SEQ ID NO: 66, and a polynucleotide that encodes a heavy chain consisting of the amino acid sequence of SEQ ID NO: 54; or
(4) a polynucleotide that encodes a light chain consisting of the amino acid sequence of SEQ ID NO: 60, and a polynucleotide that encodes a heavy chain consisting of the amino acid sequence of SEQ ID NO: 36.

[20] An expression vector, comprising the polynucleotide according to any one of [17] to [19].

[21] A host cell transformed with the expression vector according to [20].

[22] The host cell according to [21], wherein the host cell is a eukaryotic cell.

[23] A method for producing the antibody or an antigen-binding fragment of the antibody according to any one of [1] to [16], comprising a step of culturing the host cell according to [21] or [22], and collecting the antibody or an antigen-binding fragment of the antibody of interest from a culture obtained in the culturing step.

[24] The antibody or an antigen-binding fragment of the antibody according to any one of [1] to [16], wherein the heavy

chain or light chain has undergone one or more than one modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, pyroglutamation of N-terminal glutamine or N-terminal glutamic acid, and deletion of one or two amino acids at the carboxyl terminus.

[25] The antibody or an antigen-binding fragment of the antibody according to [24], wherein one or two amino acids are deleted at the carboxyl terminus of one or both of the heavy chains.

[26] The antibody or an antigen-binding fragment of the antibody according to [25], wherein one amino acid is deleted at the carboxyl terminus of each of the two heavy chains.

[27] The antibody or an antigen-binding fragment of the antibody according to any one of [24] to [26], wherein the proline residue at the carboxyl terminus of one or both of the heavy chains is further amidated.

[28] The antibody according to any one of [1] to [16], and [24] to [27], wherein modification of a glycan is regulated or a portion of the amino acid residue in the constant region is substituted, in order to enhance or attenuate antibody-dependent cellular cytotoxicity, to enhance or attenuate antibody-dependent cell-mediated phagocytosis, and/or to enhance or attenuate complement-dependent cytotoxicity.

[29] An antibody-drug conjugate, wherein a drug is bound to the antibody or an antigen-binding fragment of the antibody according to any one of [1] to [16], and [24] to [28].

[30] The antibody-drug conjugate according to [29], wherein the drug is an antitumor compound.

[31] An antibody-drug conjugate, represented by the formula (X):

[Chem. 1]

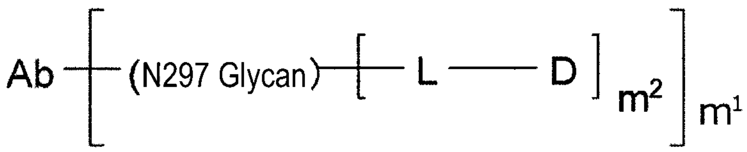

(X)

wherein $m^1$ represents an integer of 1 or 2,

$m^2$ represents an integer of 1 or 2,
Ab represents the antibody or an antigen-binding fragment of the antibody according to any one of [1] to [16] and [24] to [28],
L is a linker linking a glycan that binds to N297 (N297 glycan) of Ab to D,
the N297 glycan represents a glycan that can be remodeled,
D is one selected from the following:

[Chem. 2]

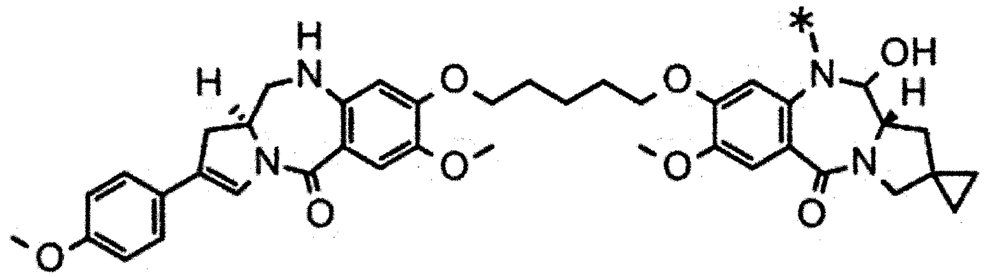

,

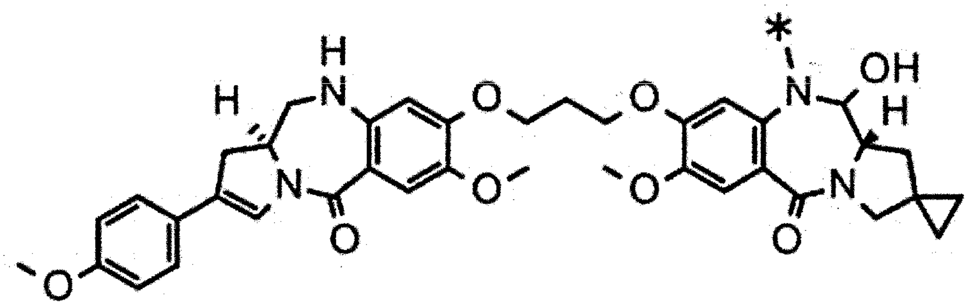

,

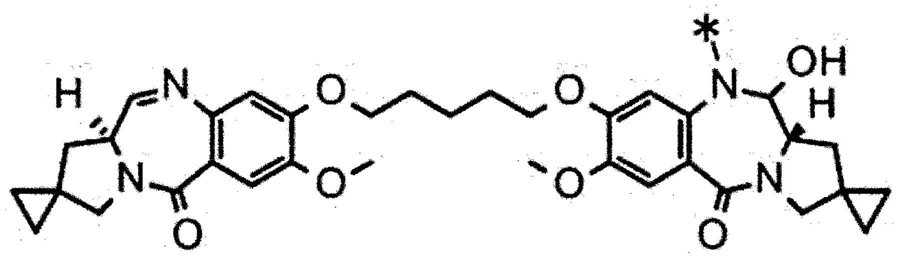

or

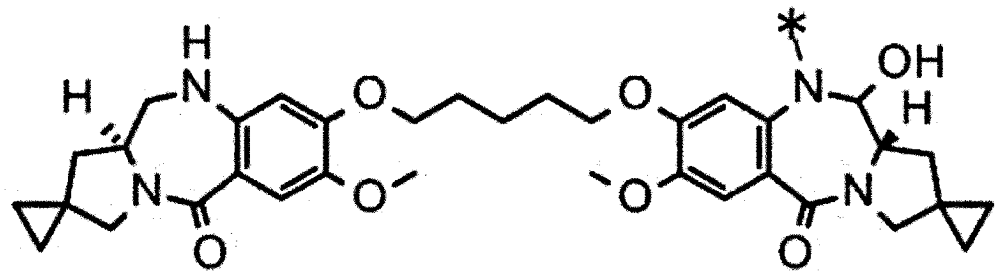

wherein * represents a bond to L.

[32]
The antibody-drug conjugate according to [31], wherein:

L is represented by -Lb-La-Lp-NH-B-$CH_2$-O(C=O)-*,
wherein * represents a bond to a nitrogen atom at the N10'-position of the D,
B is 1,4-phenyl group, 2,5-pyridyl group, 3,6-pyridyl group, 2,5-pyrimidyl group, or 2,5-thienyl group,
Lp represents one selected from the following: -GGVA-, -GG-(D-)VA-, -VA-, -GGFG-, -GGPI-, -GGVCit-, - GGVK-, and -GGPL-,

La represents one selected from the following:

$-C(=O)-CH_2CH_2-C(=O)-$,

$-C(=O)-(CH_2CH_2)_2-C(=O)-$,

$-C(=O)-CH_2CH_2-C(=O)-NH-(CH_2CH_2)_2-C(=O)-$,

$-C(=O)-CH_2CH_2-C(=O)-NH-(CH_2CH_2O)_2-CH_2-C(=O)-$,

$-C(=O)-CH_2CH_2-NH-C(=O)-(CH_2CH_2O)_4-CH_2CH_2-C(=O)-$,

$-CH_2-OC(=O)-$,

and,

$-OC(=O)-$,

and
Lb is represented by the following formula:

[Chem. 3]

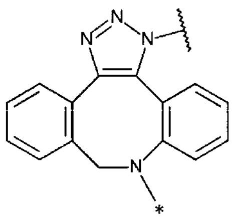

or

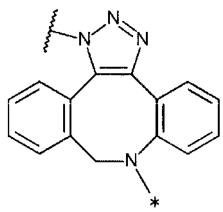

[Chem. 4]

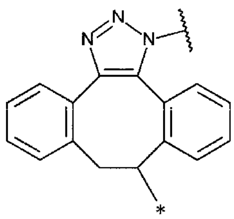

or

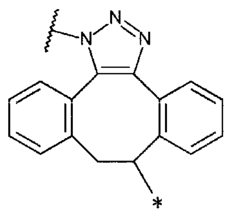

, or

[Chem. 5]

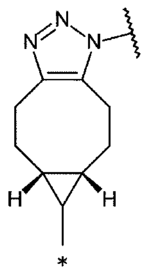

or

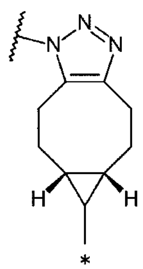

wherein in the structural formulas of Lb shown above, * represents a bond to La, and the wavy lines represent a bond to the N297 glycan.

[33] The antibody-drug conjugate according to [31] or [32], wherein L represents one selected from the group consisting of the following:

$-Z^1-C(=O)-CH_2CH_2-C(=O)-GGVA-NH-B-CH_2-OC(=O)-$,

$-Z^1-C(=O)-CH_2CH_2-C(=O)-GG-(D-)VA-NH-B-CH_2-OC(=O)-$,

$-Z^1-C(=O)-CH_2CH_2-C(=O)-VA-NH-B-CH_2-OC(=O)-$,

$-Z^1-C(=O)-(CH_2CH_2)_2-C(=O)-VA-NH-B-CH_2-OC(=O)-$,

$-Z^1-C(=O)-CH_2CH_2-C(=O)-GGPI-NH-B-CH_2-OC(=O)-$,

$-Z^1-C(=O)-CH_2CH_2-C(=O)-GGFG-NH-B-CH_2-OC(=O)-$,

$-Z^1-C(=O)-CH_2CH_2-C(=O)-GGVCit-NH-B-CH_2-OC(=O)-$,

$-Z^1-C(=O)-CH_2CH_2-C(=O)-GGVK-NH-B-CH_2-OC(=O)-$,

$-Z^1-C(=O)-CH_2CH_2-C(=O)-GGPL-NH-B-CH_2-OC(=O)-$,

$-Z^1-C(=O)-CH_2CH_2-C(=O)-NH-(CH_2CH_2)_2-C(=O)-VA-NH-B-CH_2-OC(=O)-$,

$-Z^1-C(=O)-CH_2CH_2-C(=O)-NH-(CH_2CH_2O)_2-CH_2-C(=O)-VA-NH-B-CH_2-OC(=O)-$,

$-Z^1-C(=O)-CH_2CH_2-NH-C(=O)-(CH_2CH_2O)_4-CH_2CH_2-C(=O)-VA-NH-B-CH_2-OC(=O)-$,

$-Z^2-OC(=O)-GGVA-NH-B-CH_2-OC(=O)-$,

and

$-Z^3-CH_2-OC(=O)-GGVA-NH-B-CH_2-OC(=O)-$,

wherein $Z^1$ represents the following structural formula:

[Chem. 6]

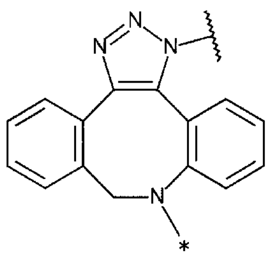

or

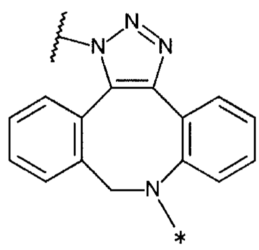

,

$Z^2$ represents the following structural formula:

[Chem. 7]

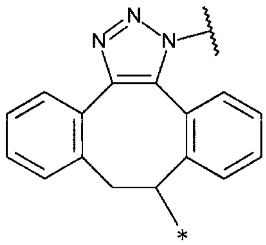

or

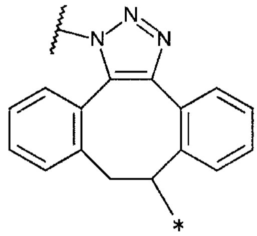

,

$Z^3$ represents the following structural formula:

[Chem. 8]

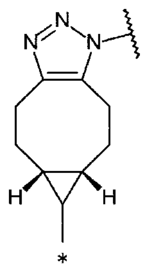

or

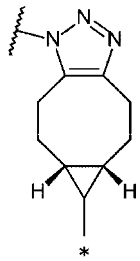

,

wherein in the structural formulas of $Z^1$, $Z^2$, and $Z^3$, * represents a bond to C(=O), O, or $CH_2$ adjacent to $Z^1$, $Z^2$, or $Z^3$, and the wavy lines represent a bond to the N297 glycan, and
B represents 1,4-phenyl group.

[34] The antibody-drug conjugate according to any one of [31] to [33], wherein L represents one selected from the group consisting of the following:

$-Z^1-C(=O)-CH_2CH_2-C(=O)-GGVA-NH-B-CH_2-OC(=O)-$,

$-Z^1-C(=O)-CH_2CH_2-C(=O)-VA-NH-B-CH_2-OC(=O)-$,

$-Z^1-C(=O)-(CH_2CH_2)_2-C(=O)-VA-NH-B-CH_2-OC(=O)-$,

$-Z^1-C(=O)-CH_2CH_2-C(=O)-GGVCit-NH-B-CH_2-OC(=O)-$,

$-Z^1-C(=O)-CH_2CH_2-C(=O)-NH-(CH_2CH_2)_2-C(=O)-VA-NH-B-CH_2-OC(=O)-$,

$-Z^1-C(=O)-CH_2CH_2-C(=O)-NH-(CH_2CH_2O)_2-CH_2-C(=O)-VA-NH-B-CH_2-OC(=O)-$,

and

$-Z^1-C(=O)-CH_2CH_2-NH-C(=O)-(CH_2CH_2O)_4-CH_2CH_2-C(=O)-VA-NH-B-CH_2-OC(=O)-$,

wherein B is 1,4-phenyl group, and $Z^1$ represents the following structural formula:

[Chem. 9]

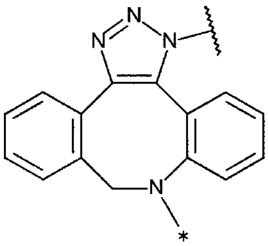

or

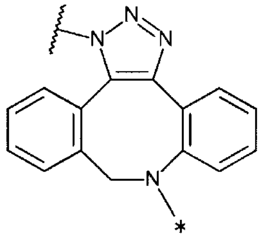

,

wherein in the structural formulas of $Z^1$, * represents a bond to C(=O) adjacent to $Z^1$, and the wavy lines represent a bond to the N297 glycan.

[35] The antibody-drug conjugate according to any one of [31] to [34], wherein the antibody is IgG1, IgG2, or IgG4.
[36] The antibody-drug conjugate according to any one of [31] to [35], wherein the N297 glycan is a remodeled glycan.
[37] The antibody-drug conjugate according to any one of [31] to [36], wherein the N297 glycan is N297-(Fuc)MSG1, N297-(Fuc)MSG2, or a mixture thereof, or N297-(Fuc)SG, having the structures represented by the following formulas:

[Chem. 10]

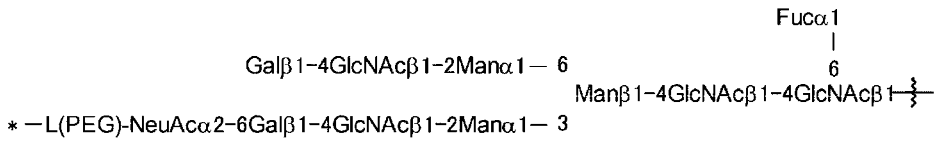

[N297-(Fuc)MSG1]

[Chem. 11]

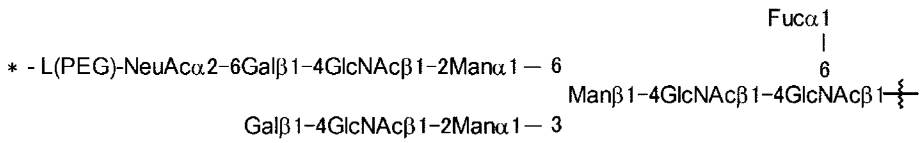

[N297-(Fuc)MSG2]

[Chem. 12]

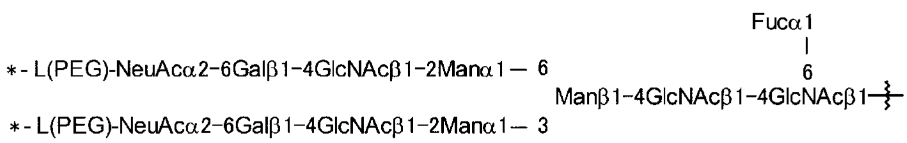

[N297-(Fuc)SG]

wherein the wavy lines represent a bond to Asn297 (N297) of the antibody,
*-L(PEG)- in the N297 glycan represents *-$(CH_2CH_2\text{-}O)n^5\text{-}CH_2CH_2\text{-}NH$-,
wherein, $n^5$ represents an integer of 2 to 5, and it is indicated that the amino group at the right end is bound via amide bond to the carboxylic acid at the 2-position of a sialic acid at the non-reducing terminus of the 1-3 chain or/and 1-6 chain of the β-Man branched chain of the N297 glycan, and * represents a bond to the nitrogen atom at position 1 or 3 on the triazole ring in each structural formula.

[38] The antibody-drug conjugate according to [37], wherein $n^5$ is 3.

[39] An antibody-drug conjugate represented by the following formula (XII):

[Chem. 13]

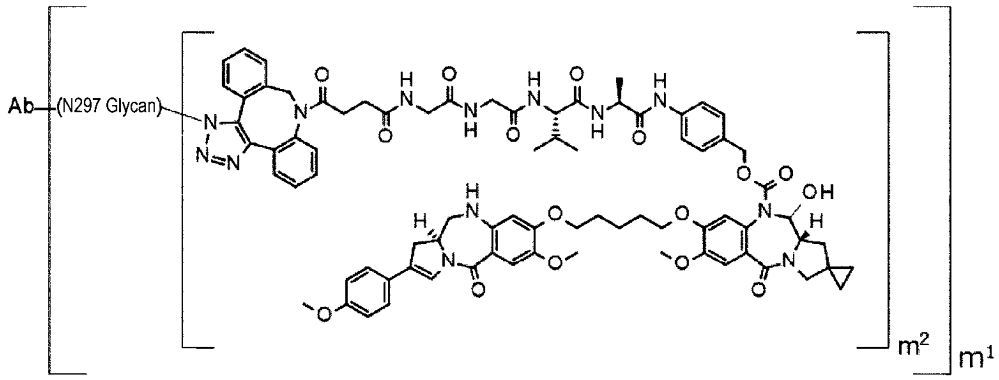

or

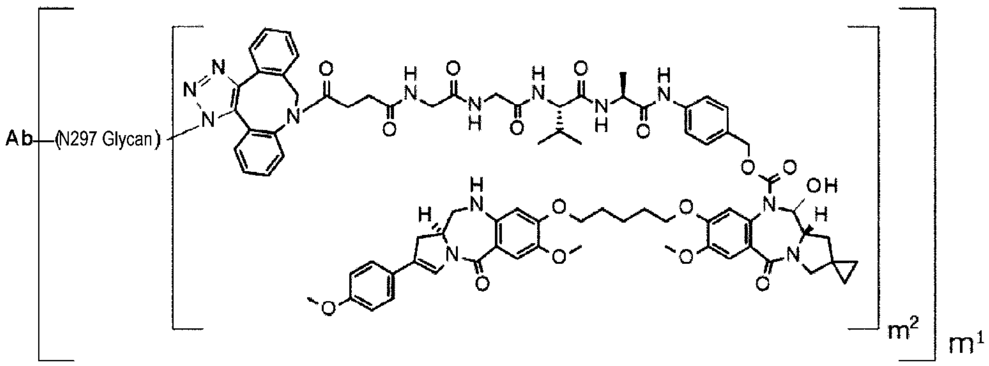

(XII)

wherein,

wherein $m^1$ represents an integer of 1 or 2 in each structural formula shown above,
$m^2$ represents an integer of 1 or 2,
Ab represents the antibody or an antigen-binding fragment of the antibody according to any one of [1] to [16] and [24] to [28],
the N297 glycan represents one of N297-(Fuc)MSG1, N297-(Fuc)MSG2, and a mixture thereof, and N297-(Fuc)SG, having the structures represented by the following formulas:

[Chem. 14]

Fucα1
|
6
Galβ1−4GlcNAcβ1−2Manα1− 6
Manβ1−4GlcNAcβ1−4GlcNAcβ1−
*−L(PEG)-NeuAcα2−6Galβ1−4GlcNAcβ1−2Manα1− 3

[N297-(Fuc)MSG1]

[Chem. 15]

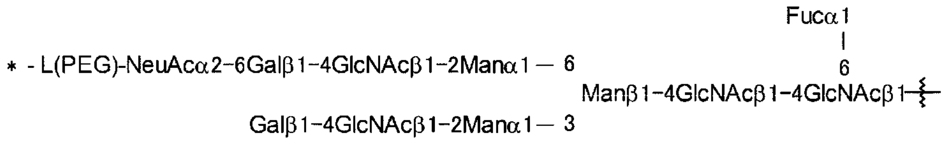

[N297-(Fuc)MSG2]

[Chem. 16]

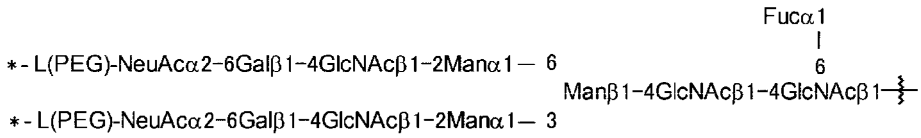

[N297-(Fuc)SG]

wherein the wavy lines represent a bond to Asn297 (N297) of the antibody, *-L(PEG)- in the N297 glycan represents *-$(CH_2CH_2\text{-}O)_3\text{-}CH_2CH_2$-NH-, wherein it is indicated that the amino group at the right end is bound via amide bond to the carboxylic acid at the 2-position of a sialic acid at the non-reducing terminus of the 1-3 chain or/and 1-6 chain of the β-Man branched chain of the N297 glycan, and * represents a bond to the nitrogen atom at the 1 or 3-position on the triazole ring in each structural formula.

[40] The antibody-drug conjugate according to [39], wherein the compound represented by the formula (XII) is represented by the following formula (XII'):

[Chem. 17]

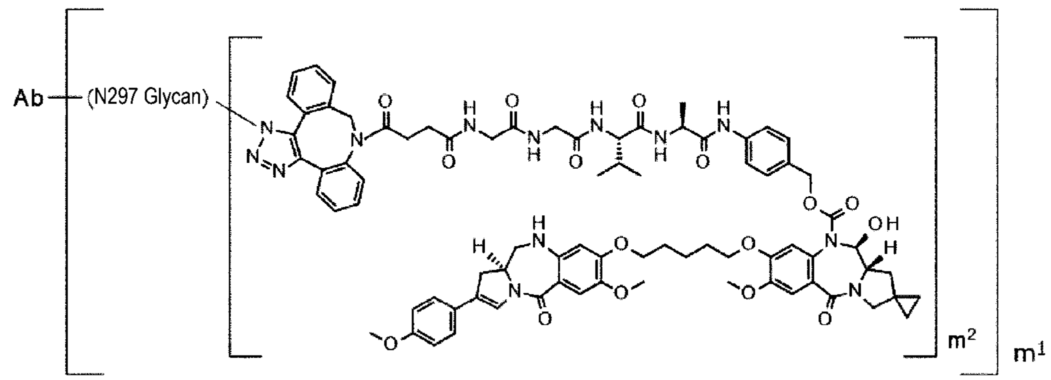

or (XII')

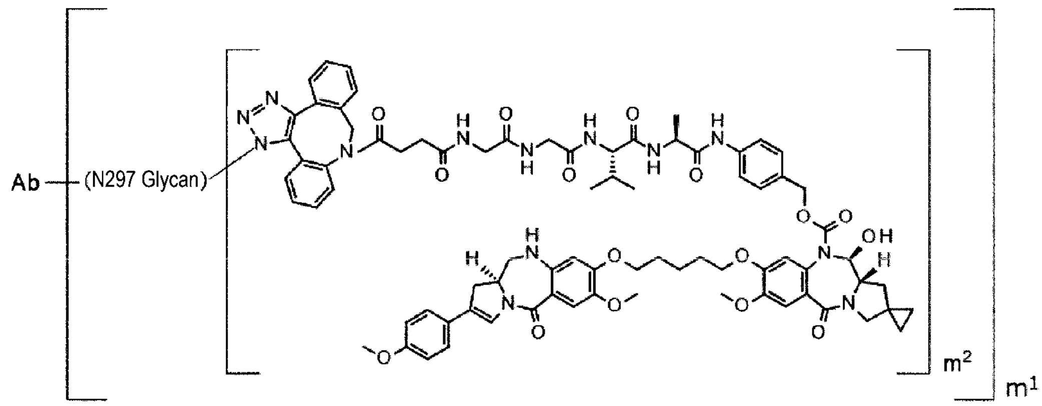

.

[41] The antibody-drug conjugate according to [40], wherein, in the structural formula of the formula (XII'), $m^1$ represents an integer of 2, $m^2$ represents an integer of 1,

the N297 glycan is N297-(Fuc)MSG1 having the structure shown in the following formula:

[Chem. 18]

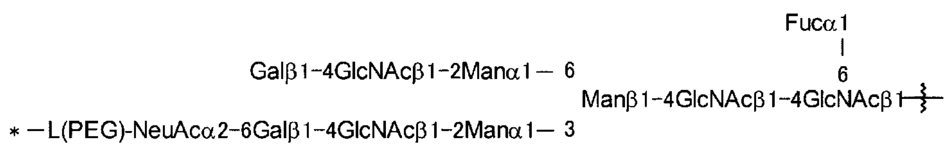

[N297-(Fuc)MSG1]

, and *-L(PEG)- in the N297 glycan represents *-$(CH_2CH_2\text{-}O)_3$-$CH_2CH_2$-NH-, wherein it is indicated that the amino group at the right end is bound via amide bond to the carboxylic acid at the 2-position of a sialic acid at the non-reducing terminus of the 1-3 chain of the β-Man branched chain of the N297 glycan, and * represents a bond to the nitrogen atom at the 1 or 3-position on the triazole ring,
Ab represents an antibody comprising CDRL1, CDRL2 and CDRL3, and CDRH1, CDRH2 and CDRH3 selected from the group consisting of:

(1) CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and

CDRH1 consisting of the amino acid sequence set forth in SEQ ID NO: 38, CDRH2 consisting of the amino acid sequence set forth in SEQ ID NO: 39, and CDRH3 consisting of the amino acid sequence set forth in SEQ ID NO: 40;
(2) CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and CDRH1 consisting of the amino acid sequence set forth in SEQ ID NO: 44, CDRH2 consisting of the amino acid sequence set forth in SEQ ID NO: 45, and CDRH3 consisting of the amino acid sequence set forth in SEQ ID NO: 46;
(3) CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and CDRH1 consisting of the amino acid sequence set forth in SEQ ID NO: 50, CDRH2 consisting of the amino acid sequence set forth in SEQ ID NO: 51, and CDRH3 consisting of the amino acid sequence set forth in SEQ ID NO: 52; and
(4) CDRL1 consisting of the amino acid sequence set forth in SEQ ID NO: 56, CDRL2 consisting of the amino acid sequence of WAS, and CDRL3 consisting of the amino acid sequence set forth in SEQ ID NO: 58, and CDRH1 consisting of the amino acid sequence set forth in SEQ ID NO: 32, CDRH2 consisting of the amino acid sequence set forth in SEQ ID NO: 33, and CDRH3 consisting of the amino acid sequence set forth in SEQ ID NO: 34.

[42] The antibody-drug conjugate according to [41], wherein Ab represents an antibody comprising of a light chain variable region and a heavy chain variable region of one selected from the group consisting of the following (1) to (4):

(1) a light chain variable region consisting of the amino acid sequence set forth in SEQ ID NO: 65, and a heavy chain variable region consisting of the amino acid sequence set forth in SEQ ID NO: 41;
(2) a light chain variable region consisting of the amino acid sequence set forth in SEQ ID NO: 65, and a heavy chain variable region consisting of the amino acid sequence set forth in SEQ ID NO: 47;
(3) a light chain variable region consisting of the amino acid sequence set forth in SEQ ID NO: 65, and a heavy chain variable region consisting of the amino acid sequence set forth in SEQ ID NO: 53; and
(4) a light chain variable region consisting of the amino acid sequence set forth in SEQ ID NO: 59, and a heavy chain variable region consisting of the amino acid sequence set forth in SEQ ID NO: 35.

[43] The antibody-drug conjugate according to [42], wherein Ab comprises a light chain and a heavy chain of one selected from the group consisting of the following (1) to (5):

(1) a light chain consisting of the amino acid sequence of SEQ ID NO: 66, and a heavy chain consisting of the amino acid sequence of SEQ ID NO: 42;
(2) a light chain consisting of the amino acid sequence of SEQ ID NO: 66, and a heavy chain consisting of the amino acid sequence of SEQ ID NO: 48;
(3) a light chain consisting of the amino acid sequence set forth in SEQ ID NO: 66, and a heavy chain consisting of the amino acid sequence set forth in SEQ ID NO: 54,
(4) a light chain consisting of the amino acid sequence set forth in SEQ ID NO: 60, and a heavy chain consisting of the amino acid sequence set forth in SEQ ID NO: 36; and
(5) the light chain (light chain a) described in one of (1) to (4), and a heavy chain consisting of the amino acid sequence obtained by deleting one or two lysines from the carboxyl terminus of the amino acid sequence of a heavy chain (heavy chain a) combined with the light chain a in (1) to (4).

[44] An antibody-drug conjugate represented by the following formula (XIII):

[Chem. 19]

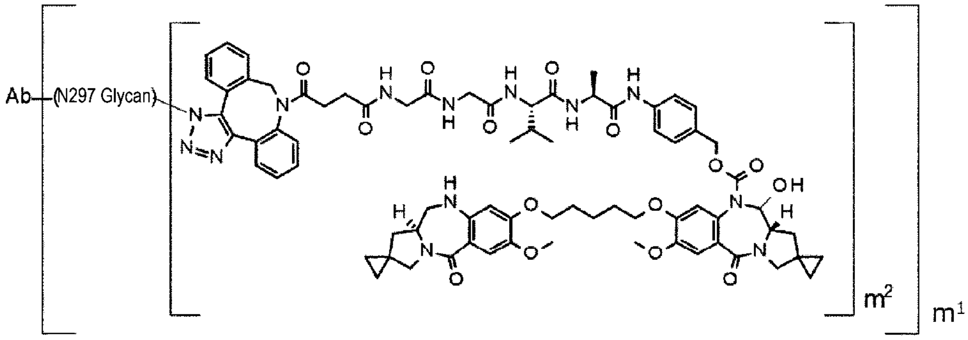

or

(XIII)

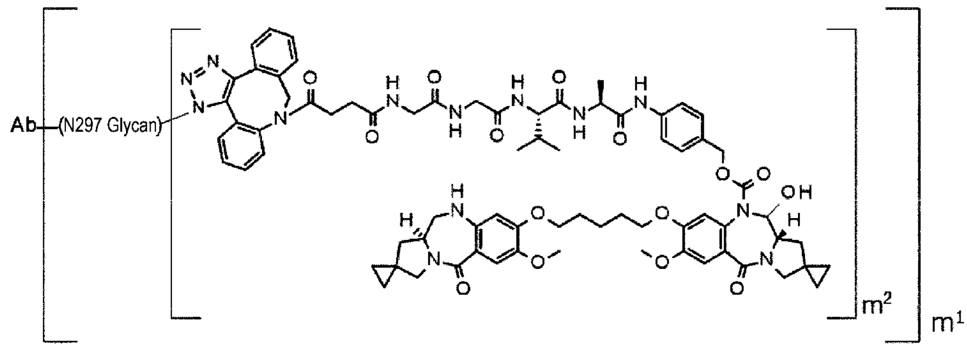

wherein,

in each structural formula shown above, $m^1$ represents an integer of 1 or 2,
$m^2$ represents an integer of 1 or 2,
Ab represents the antibody or an antigen-binding fragment of the antibody according to any one of [1] to [16] and [24] to [28], and
the N297 glycan represents one of N297-(Fuc)MSG1, N297-(Fuc)MSG2, and a mixture thereof, and N297-(Fuc)SG, having the structures represented by the following formulas:

[Chem. 20]

Fucα1
|
6
Galβ1−4GlcNAcβ1−2Manα1— 6
Manβ1−4GlcNAcβ1−4GlcNAcβ1−
∗ —L(PEG)-NeuAcα2−6Galβ1−4GlcNAcβ1−2Manα1— 3

[N297-(Fuc)MSG1]

[Chem. 21]

Fucα1
|
6
∗ - L(PEG)-NeuAcα2−6Galβ1−4GlcNAcβ1−2Manα1— 6
Manβ1−4GlcNAcβ1−4GlcNAcβ1−
Galβ1−4GlcNAcβ1−2Manα1— 3

[N297-(Fuc)MSG2]

[Chem. 22]

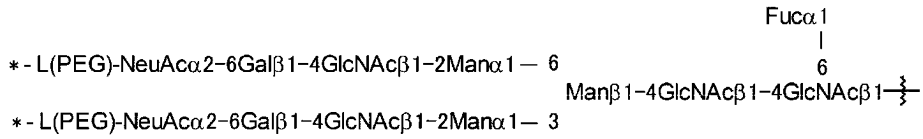

[N297-(Fuc)SG]

wherein the wavy lines represent a bond to Asn297 (N297) of the antibody, and
*-L(PEG)- in the N297 glycan represents *-$(CH_2CH_2\text{-}O)_3\text{-}CH_2CH_2\text{-}NH\text{-}$,
wherein it is indicated that the amino group at the right end is bound via amide bond to the carboxylic acid at the 2-position of a sialic acid at the non-reducing terminus of the 1-3 chain or/and 1-6 chain of the β-Man branched chain of the N297 glycan, and * represents a bond to the nitrogen atom at the 1 or 3-position on the triazole ring in each structural formula.

[45] The antibody-drug conjugate according to [44], wherein the compound represented by the formula (XIII) is represented by the following formula (XIII'):

[Chem. 23]

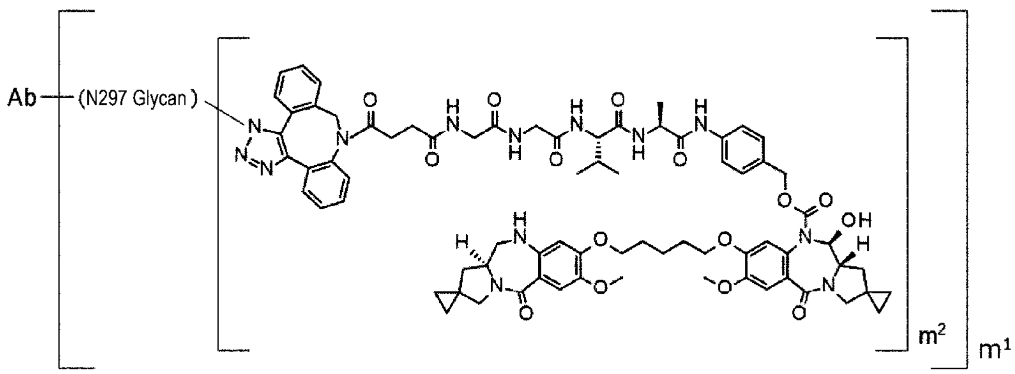

or

(XIII')

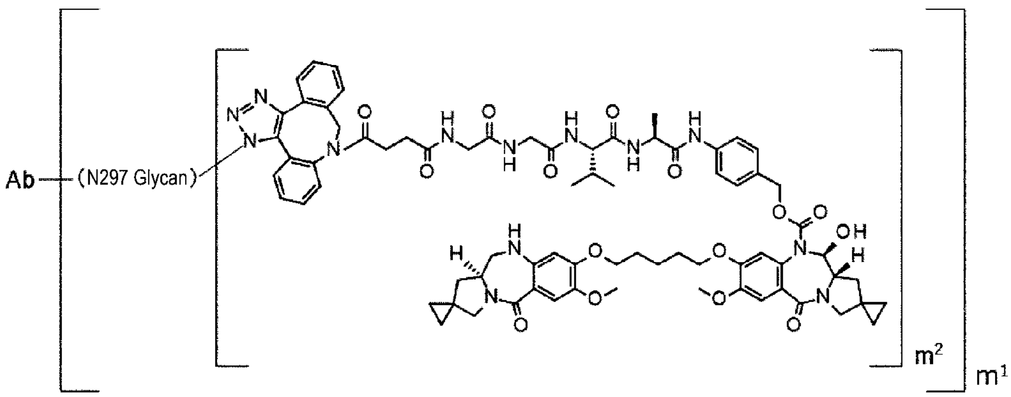

S.
[46] An antibody-drug conjugate represented by the following formula (XIV):

[Chem. 24]

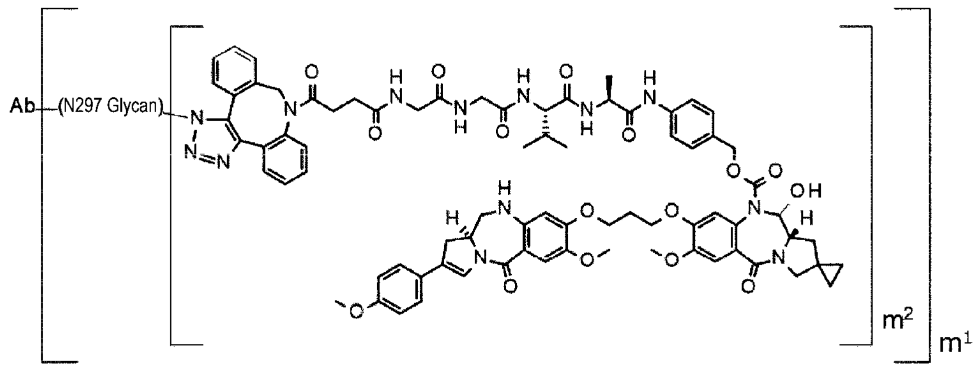

or

(XIV)

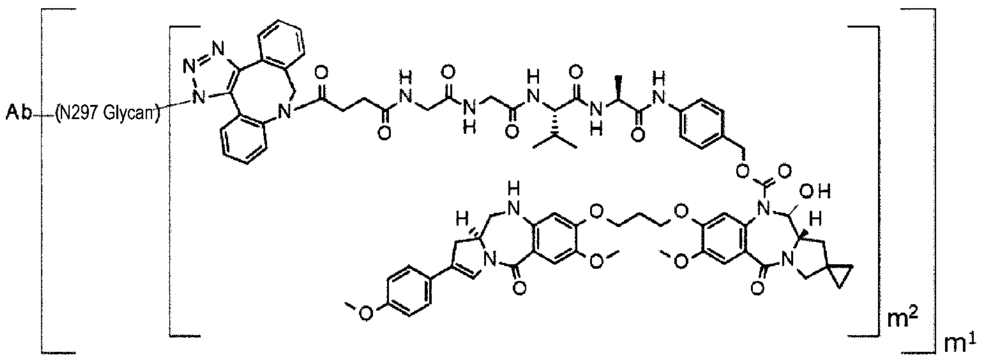

wherein,

in each structural formula shown above, $m^1$ represents an integer of 1 or 2,
$m^2$ represents an integer of 1 or 2,
Ab represents the antibody or an antigen-binding fragment of the antibody according to any one of [1] to [16] and [24] to [28], and
the N297 glycan represents one of N297-(Fuc)MSG1, N297-(Fuc)MSG2, and a mixture thereof, and N297-(Fuc) SG, having the structures represented by the following formulas:

[Chem. 25]

Fucα1
|
Galβ1-4GlcNAcβ1-2Manα1— 6 6
Manβ1-4GlcNAcβ1-4GlcNAcβ1-
∗—L(PEG)-NeuAcα2-6Galβ1-4GlcNAcβ1-2Manα1— 3

[N297-(Fuc)MSG1]

[Chem. 26]

Fucα1
|
∗ - L(PEG)-NeuAcα2-6Galβ1-4GlcNAcβ1-2Manα1— 6 6
Manβ1-4GlcNAcβ1-4GlcNAcβ1-
Galβ1-4GlcNAcβ1-2Manα1— 3

[N297-(Fuc)MSG2]

[Chem. 27]

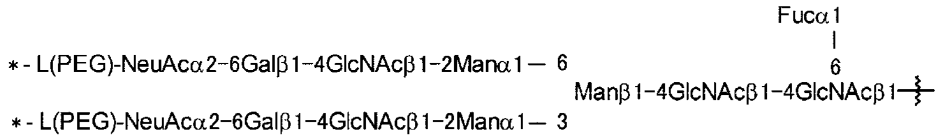

[N297-(Fuc)SG]

wherein the wavy lines represent a bond to Asn297 (N297) of the antibody, and
*-L(PEG)- in the N297 glycan represents *-$(CH_2CH_2\text{-}O)_3\text{-}CH_2CH_2\text{-}NH$-,
wherein it is indicated that the amino group at the right end is bound via amide bond to the carboxylic acid at the 2-position of a sialic acid at the non-reducing terminus of the 1-3 chain or/and 1-6 chain of the β-Man branched chain of the N297 glycan, and * represents a bond to the nitrogen atom at the 1 or 3-position on the triazole ring in each structural formula.

[47] The antibody-drug conjugate according to [46], wherein the compound represented by the formula (XIV) is represented by the following formula (XIV'):

[Chem. 28]

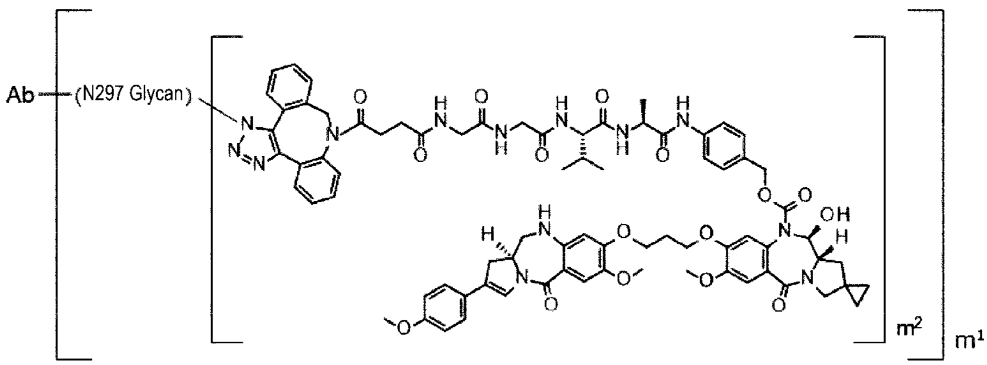

or

(XIV')

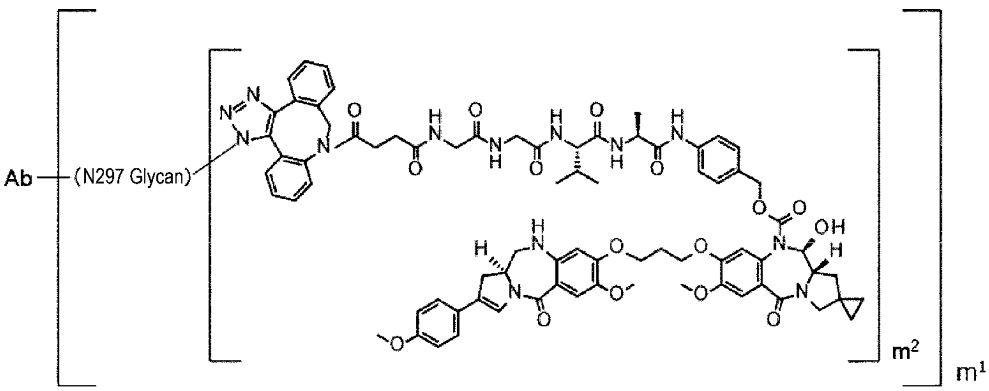

[48] An antibody-drug conjugate represented by the following formula (XV):

[Chem. 29]

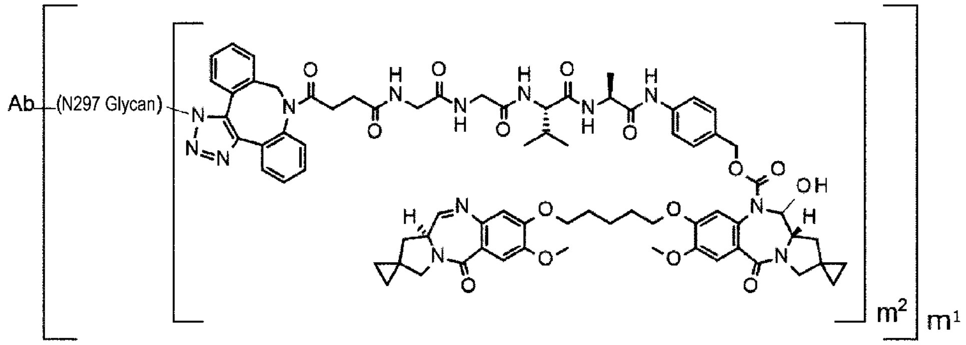

or

(XV)

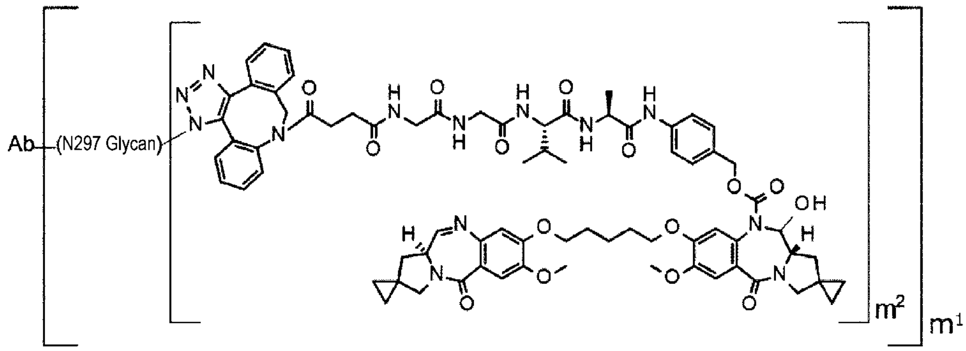

wherein,

in each structural formula shown above, $m^1$ represents an integer of 1 or 2,
$m^2$ represents an integer of 1 or 2,
Ab represents the antibody or an antigen-binding fragment of the antibody according to any one of [1] to [16] and [24] to [28], and
the N297 glycan represents one of N297-(Fuc)MSG1, N297-(Fuc)MSG2, and a mixture thereof, and N297-(Fuc)SG, having the structures represented by the following formulas:

[Chem. 30]

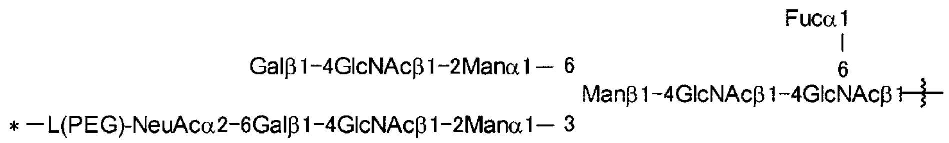

[N297-(Fuc)MSG1]

[Chem. 31]

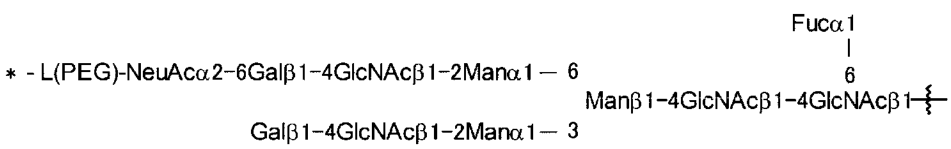

[N297-(Fuc)MSG2]

[Chem. 32]

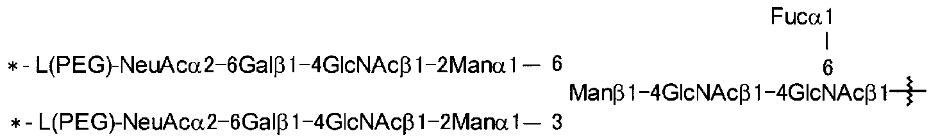

[N297-(Fuc)SG]

wherein the wavy lines represent a bond to Asn297 (N297) of the antibody, and
*-L(PEG)- in the N297 glycan represents *-$(CH_2CH_2\text{-}O)_3\text{-}CH_2CH_2\text{-}NH\text{-}$,
wherein it is indicated that the amino group at the right end is bound via amide bond to the carboxylic acid at the 2-position of a sialic acid at the non-reducing terminus of the 1-3 chain or/and 1-6 chain of the β-Man branched chain of the N297 glycan, and * represents a bond to the nitrogen atom at the 1 or 3-position on the triazole ring in each structural formula.

[49] The antibody-drug conjugate according to [48], wherein the compound represented by the formula (XV) is represented by the following formula (XV'):

[Chem. 33]

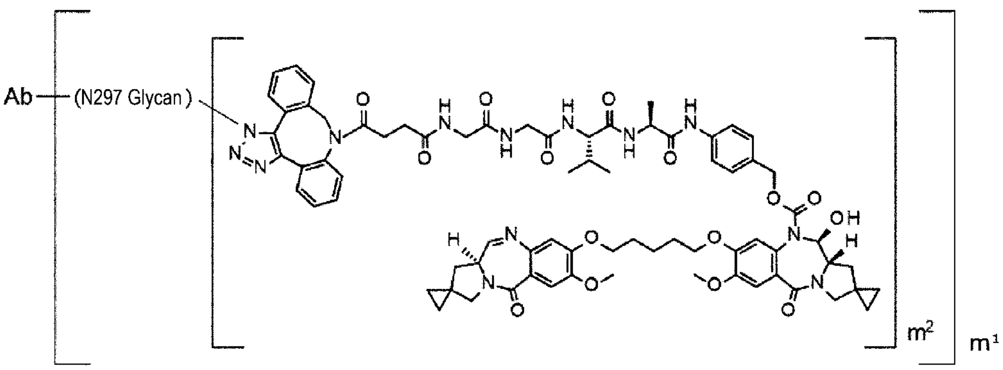

(XV')

or

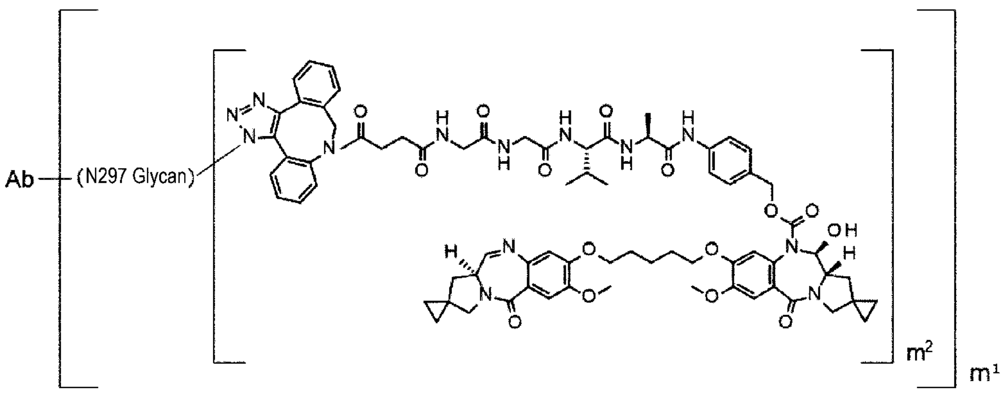

.

[50] A method for producing a glycan-remodeled antibody, comprising:

i) producing the antibody or an antigen-binding fragment of the antibody according to any one of [1] to [16] and [24] to [28],
ii) treating the antibody obtained in i) with hydrolase to produce a (Fucα1,6)GlcNAc-antibody, and
iii)-1 reacting in the presence of transglycosidase, the (Fucα1,6)GlcNAc-antibody and a glycan donner molecule

obtained by introducing a PEG linker having an azide group to the carbonyl group of the carboxylic acid at the 2-position of a sialic acid in MSG (9) or SG (10) and oxazolinating the reducing terminus, or

iii)-2 reacting in the presence of transglycosidase, the (Fucα1,6)GlcNAc-antibody and a glycan donner molecule obtained by introducing a PEG linker having an azide group to the carbonyl group of the carboxylic acid at the 2-position of a sialic acid in (MSG-)Asn or (SG-)Asn in which an α-amino group is optionally protected and to the carbonyl group of the carboxylic acid in the Asn, allowing hydrolase to act on the resulting molecule, and then oxazolinating the reducing terminus.

[51] A method for producing a glycan-remodeled antibody, comprising:

i) producing the antibody or an antigen-binding fragment of the antibody according to any one of [1] to [16] and [24] to [28],

ii) treating the antibody obtained in i) with hydrolase to produce a (Fucα1,6)GlcNAc-antibody, and iii) binding a glycan to the (Fucα1,6)GlcNAc-antibody by using two endo-β-N-acetylglucosaminidases.

[52] A method for producing the antibody-drug conjugate according to any one of [31] to [49], comprising reacting the glycan-remodeled antibody obtained by the method according to [50] or [51], with a drug linker.

[53] The antibody-drug conjugate according to any one of [31] to [49], wherein the antibody-drug conjugate is produced by the method according to [52].

[54] A pharmaceutical composition, comprising the antibody or an antigen-binding fragment of the antibody according to any one of [1] to [16], and [24] to [28], or the antibody-drug conjugate according to any one of [29] to [49] and [53].

[55] The pharmaceutical composition according to [54], wherein the pharmaceutical composition is an antitumor drug.

[56] The pharmaceutical composition according to [55], wherein a tumor expresses LRRC15 in a tumor cell and/or a cell in the stroma thereof.

[57] The pharmaceutical composition according to [55] or [56], wherein the tumor is a breast carcinoma, a head and neck carcinoma, a lung carcinoma, a pancreatic carcinoma, a colorectal carcinoma, an esophageal carcinoma, an ovarian carcinoma, a bladder carcinoma, a cholangiocarcinoma, a gastric carcinoma, a mesothelioma, an uterine carcinoma, a thyroid carcinoma, a renal carcinoma, a hepatocarcinoma, a prostate carcinoma, a bone and soft tissue sarcoma, a melanoma, or a glioma.

[58] A method for treating a tumor, comprising administering a therapeutically effective amount of the antibody or an antigen-binding fragment of the antibody according to any one of [1] to [16], and [24] to [28], the antibody-drug conjugate according to any one of [29] to [49] and [53], or the pharmaceutical composition according to any one of [54] to [57], to a subject in need of tumor treatment.

[59] The treatment method according to [58], wherein the tumor expresses LRRC15 in a tumor cell and/or a cell in the stroma thereof.

[60] The treatment method according to [58] or [59], wherein the tumor is a breast carcinoma, a head and neck carcinoma, a lung carcinoma, a pancreatic carcinoma, a colorectal carcinoma, an esophageal carcinoma, an ovarian carcinoma, a bladder carcinoma, a cholangiocarcinoma, a gastric carcinoma, a mesothelioma, an uterine carcinoma, a thyroid carcinoma, a renal carcinoma, a hepatocarcinoma, a prostate carcinoma, a bone and soft tissue sarcoma, a melanoma, or a glioma.

[61] The treatment method according to any one of [58] to [60], comprising administering to a subject a therapeutically effective amount of the antibody or an antigen-binding fragment of the antibody according to any one of [1] to [16], and [24] to [28], the antibody-drug conjugate according to any one of [29] to [49] and [53], or the pharmaceutical composition according to any one of [54] to [57] and at least one different antitumor drug concurrently, separately, or consecutively.

Advantageous Effects of Invention

The present invention provides a novel anti-LRRC15 antibody or an antigen-binding fragment thereof that can be advantageously used in various therapeutic modalities, especially in antibody-drug conjugates. In addition, the present invention provides an antibody-drug conjugate that exhibits strong antitumor activity.

Brief Description of Drawings

[Figure 1-1] Figure 1-1 shows binding activity of humanized anti-LRRC15 antibodies against humans, cynomolgus monkeys, mice and rats.

[Figure 1-2] Figure 1-2 shows binding activity of humanized anti-LRRC15 antibodies against humans, cynomolgus monkeys, mice and rats.

[Figure 2-1] Figure 2-1 shows binding activity of humanized anti-LRRC15 antibodies against endogenous LRRC15.

[Figure 2-2] Figure 2-2 shows binding activity of humanized anti-LRRC15 antibodies against endogenous LRRC15.

[Figure 3-1] Figure 3-1 shows concentration-dependent inhibitory activity against cell growth when each antibody-drug conjugate is added.

[Figure 3-2] Figure 3-2 shows concentration-dependent inhibitory activity against cell growth when each antibody-drug conjugate is added.

[Figure 3-3] Figure 3-3 shows inhibitory activity against cell growth when each antibody-drug conjugate is added.

[Figure 4] Figure 4 shows antitumor effects of the antibody-drug conjugates which were evaluated by using animal models in which an LRRC15-negative human lung carcinoma cell line EBC-1 had been transplanted into immunodeficient mice.

[Figure 5] Figure 5 shows antitumor effects of the antibody-drug conjugates which were evaluated by using animal models in which an LRRC15-negative human lung carcinoma cell line HCC827 had been transplanted into immunodeficient mice.

[Figure 6] Figure 6 shows antitumor effects of the antibody-drug conjugates which were evaluated by using animal models in which an LRRC15-positive human osteosarcoma cell line HuO9 had been transplanted into immunodeficient mice.

[Figure 7] Figure 7 shows an amino acid sequence of the full-length human LRRC15 isoform b (SEQ ID NO: 1), an amino acid sequence of the extracellular region of human LRRC15 isoform b (SEQ ID NO: 2), and an amino acid sequence of human LRRC15-His protein (SEQ ID NO: 3).

[Figure 8] Figure 8 shows an amino acid sequence of the full-length cynomolgus monkey LRRC15 (SEQ ID NO: 4), an amino acid sequence of the extracellular region of cynomolgus monkey LRRC15 (SEQ ID NO: 5), and an amino acid sequence of cynomolgus monkey LRRC15-His protein (SEQ ID NO: 6).

[Figure 9] Figure 9 shows an amino acid sequence of the full-length mouse LRRC15 (SEQ ID NO: 7), an amino acid sequence of the extracellular region of mouse LRRC15 (SEQ ID NO: 8), and an amino acid sequence of mouse LRRC15-His protein (SEQ ID NO: 9).

[Figure 10] Figure 10 shows an amino acid sequence of the full-length rat LRRC15 (SEQ ID NO: 10), an amino acid sequence of the extracellular region of rat LRRC15 (SEQ ID NO: 11), and an amino acid sequence of rat LRRC15-His protein (SEQ ID NO: 12).

[Figure 11] Figure 11 shows an amino acid sequence of the full-length human LRRC15 isoform a (SEQ ID NO: 13), a nucleotide sequence of rR024 heavy chain variable region cDNA (SEQ ID NO: 14), an amino acid sequence of rR024 heavy chain variable region cDNA (SEQ ID NO: 15), a nucleotide sequence of rR024 light chain variable region cDNA (SEQ ID NO: 16), and an amino acid sequence of rR024 light chain variable region cDNA (SEQ ID NO: 17).

[Figure 12] Figure 12 shows a DNA fragment containing a human light chain signal sequence and a DNA sequence that encodes a human κ chain constant region (SEQ ID NO: 18), and a DNA fragment containing a human heavy chain signal sequence and a DNA sequence that encodes the amino acids of a human IgG1 constant region (SEQ ID NO: 19).

[Figure 13] Figure 13 shows a chR024 CDRH1 amino acid sequence (SEQ ID NO: 20), a chR024 CDRH2 amino acid sequence (SEQ ID NO: 21), a chR024 CDRH3 amino acid sequence (SEQ ID NO: 22), a chR024 heavy chain variable region amino acid sequence (SEQ ID NO: 23), and a chR024 signal sequence and full-length heavy chain amino acid sequence (SEQ ID NO: 24).

[Figure 14] Figure 14 shows a chR024 signal sequence and full-length heavy chain nucleotide sequence (SEQ ID NO: 25).

[Figure 15] Figure 15 shows a chR024 CDRL1 amino acid sequence (SEQ ID NO: 26), a chR024 CDRL2 amino acid sequence (SEQ ID NO: 27), a chR024 CDRL3 amino acid sequence (SEQ ID NO: 28), a chR024 light chain variable region amino acid sequence (SEQ ID NO: 29), a chR024 signal sequence and full-length light chain amino acid sequence (SEQ ID NO: 30), and a chR024 signal sequence and full-length light chain nucleotide sequence (SEQ ID NO: 31).

[Figure 16] Figure 16 shows an hR024 H09 CDRH1 amino acid sequence (SEQ ID NO: 32), an hR024 H09 CDRH2 amino acid sequence (SEQ ID NO: 33), an hR024 H09 CDRH3 amino acid sequence (SEQ ID NO: 34), an hR024 H09 heavy chain variable region amino acid sequence (SEQ ID NO: 35), and an hR024 H09 signal sequence and full-length heavy chain amino acid sequence (SEQ ID NO: 36).

[Figure 17] Figure 17 shows an hR024 H09 signal sequence and full-length heavy chain nucleotide sequence (SEQ ID NO: 37).

[Figure 18] Figure 18 shows an hR024 H02 CDRH1 amino acid sequence (SEQ ID NO: 38), an hR024 H02 CDRH2 amino acid sequence (SEQ ID NO: 39), an hR024 H02 CDRH3 amino acid sequence (SEQ ID NO: 40), an hR024 H02 heavy chain variable region amino acid sequence (SEQ ID NO: 41), and an hR024 H02 signal sequence and full-length heavy chain amino acid sequence (SEQ ID NO: 42).

[Figure 19] Figure 19 shows an hR024 H02 signal sequence and full-length heavy chain nucleotide sequence (SEQ ID NO: 43).

[Figure 20] Figure 20 shows an hR024 H02-V1 CDRH1 amino acid sequence (SEQ ID NO: 44), an hR024 H02-V1 CDRH2 amino acid sequence (SEQ ID NO: 45), an hR024 H02-V1 CDRH3 amino acid sequence (SEQ ID NO: 46), an hR024 H02-V1 heavy chain variable region amino acid sequence (SEQ ID NO: 47), and an hR024 H02-V1 signal sequence and full-length heavy chain amino acid sequence (SEQ ID NO: 48).

[Figure 21] Figure 21 shows an hR024 H02-V1 signal sequence and full-length heavy chain nucleotide sequence (SEQ ID NO: 49).

[Figure 22] Figure 22 shows an hR024 H02-V2 CDRH1 amino acid sequence (SEQ ID NO: 50), an hR024 H02-V2 CDRH2 amino acid sequence (SEQ ID NO: 51), an hR024 H02-V2 CDRH3 amino acid sequence (SEQ ID NO: 52), an hR024 H02-V2 heavy chain variable region amino acid sequence (SEQ ID NO: 53), and an hR024 H02-V2 signal sequence and full-length heavy chain amino acid sequence (SEQ ID NO: 54).

[Figure 23] Figure 23 shows an hR024 H02-V2 signal sequence and full-length heavy chain nucleotide sequence (SEQ ID NO: 55).

[Figure 24] Figure 24 shows an hR024 L03 CDRL1 amino acid sequence (SEQ ID NO: 56), an hR024 L03 CDRL2 amino acid sequence (SEQ ID NO: 57), an hR024 L03 CDRL3 amino acid sequence (SEQ ID NO: 58), an hR024 L03 light chain variable region amino acid sequence (SEQ ID NO: 59), an hR024 L03 signal sequence and full-length light chain amino acid sequence (SEQ ID NO: 60), and an hR024 L03 signal sequence and full-length light chain nucleotide sequence (SEQ ID NO: 61).

[Figure 25] Figure 25 shows an hR024 L01 CDRL1 amino acid sequence (SEQ ID NO: 62), an hR024 L01 CDRL2 amino acid sequence (SEQ ID NO: 63), an hR024 L01 CDRL3 amino acid sequence (SEQ ID NO: 64), an hR024 L01 light chain variable region amino acid sequence (SEQ ID NO: 65), an hR024 L01 signal sequence and full-length light chain amino acid sequence (SEQ ID NO: 66), and an hR024 L01 signal sequence and full-length light chain nucleotide sequence (SEQ ID NO: 67).

[Figure 26] Figure 26 shows the huM25 signal sequence and full-length heavy chain amino acid sequence (SEQ ID NO: 68), and the huM25 signal sequence and full-length heavy chain nucleotide sequence (SEQ ID NO: 69).

[Figure 27] Figure 27 shows an huM25 signal sequence and full-length light chain amino acid sequence (SEQ ID NO: 70), and an huM25 signal sequence and full-length light chain nucleotide sequence (SEQ ID NO: 71).

[Figure 28] Figure 28 shows a control hIgG signal sequence and full-length heavy chain amino acid sequence (SEQ ID NO: 72), and a control hIgG signal sequence and full-length light chain amino acid sequence (SEQ ID NO: 73).

[Figure 29] Figure 29 shows the results of a pharmacokinetic study of anti-LRRC15 antibodies in mice.

[Figure 30-1] Figure 30-1 shows the results of an analytical test on the LRRC15 binding sites of the anti-LRRC15 antibodies.

[Figure 30-2] Figure 30-2 shows the results of an analytical test on the LRRC15 binding sites of the anti-LRRC15 antibodies.

[Figure 30-3] Figure 30-3 shows the results of an analytical test on the LRRC15 binding sites of the anti-LRRC15 antibodies.

[Figure 31] Figure 31 shows antitumor effects of the antibody-drug conjugates which were evaluated by using animal models in which an LRRC15-positive human osteosarcoma cell line HuO9 had been transplanted into immunodeficient mice.

[Figure 32] Figure 32 shows antitumor effects of the antibody-drug conjugates which were evaluated by using animal models in which an LRRC15-negative human lung carcinoma cell line EBC-1 had been transplanted into immunodeficient mice.

[Figure 33] Figure 33 shows antitumor effects of the antibody-drug conjugates which were evaluated by using animal models in which an LRRC15-negative breast carcinoma cell line SUM190PT had been transplanted into immunodeficient mice.

[Figure 34] Figure 34 shows antitumor effects of the antibody-drug conjugates which were evaluated by using animal models in which an LRRC15-negative breast carcinoma cell line HCC1806 had been transplanted into immunodeficient mice.

[Figure 35] Figure 35 shows antitumor effects of the antibody-drug conjugates which were evaluated by using animal models in which an LRRC15-negative head and neck carcinoma cell line Detroit 562 had been transplanted into immunodeficient mice.

[Figure 36] Figure 36 shows antitumor effects of the antibody-drug conjugates which were evaluated by using animal models in which an LRRC15-negative esophageal carcinoma cell line EC-GI-10 had been transplanted into immunodeficient mice.

[Figure 37] Figure 37 shows antitumor effects of the antibody-drug conjugates which were evaluated by using animal models in which an LRRC15-negative pancreatic carcinoma cell line BxPC-3 and mesenchymal stem cells derived

from human adipose tissues had been co-transplanted into immunodeficient mice.

[Figure 38] Figure 38 shows antitumor effects of the antibody-drug conjugates which were evaluated by using animal models in which an LRRC15-negative colorectal carcinoma cell line CMT93 had been transplanted into immunodeficient mice.

[Figure 39] Figure 39 shows antitumor effects of the antibody-drug conjugates which were evaluated by using animal models in which an LRRC15-negative gastric carcinoma cell line NCI-N87 had been transplanted into immunodeficient mice.

[Figure 40] Figure 40 shows antitumor effects of the antibody-drug conjugates which were evaluated by using animal models in which an LRRC15-negative renal carcinoma cell line Caki-1 had been transplanted into immunodeficient mice.

[Figure 41] Figure 41 shows antitumor effects of the antibody-drug conjugates which were evaluated by using animal models in which an LRRC15-positive human glioblastoma cell line U118MG had been transplanted into immunodeficient mice.

[Figure 42] Figure 42 shows antitumor effects of the antibody-drug conjugates which were evaluated by using animal models in which an LRRC15-negative fibrosarcoma cell line HT-1080 had been transplanted into immunodeficient mice.

Description of Embodiments

Hereinafter, preferred modes for carrying out the present invention will be described with reference to the drawings. Note that the embodiments described below are given merely for illustrating an example of a typical embodiment of the present invention, and the scope of the present invention is not construed as being limited by these.

As used herein, the terms of "cancer", "carcinoma" and "tumor" are used for the same meaning. As used herein, the "tumor" refers to carcinomatous cells and tissues regardless of whether they are malignant or benign, may be any precarcinomatous conditions, primary tumors, or recurrent tumors. It is not limited to tumor cells but can encompass cells surrounding tumor cells. It may include a mixture of tumor cells and normal cells or normal tissues. When the tumor tissues are mixed with normal cells or normal tissues, the volume or the number of tumor cells in respect to the normal cells or normal tissues is not particularly limited.

As used herein, the term "gene" includes not only DNA but also its mRNA, cDNA and cRNA.

As used herein, the term "polynucleotide" or "nucleotide" is used for the same meaning as nucleic acid, and includes DNA, RNA, a probe, an oligonucleotide, and a primer. As used herein, the terms "polynucleotide" and "nucleotide" can be used interchangeably unless otherwise specified.

As used herein, the term "polynucleotide" and "protein" can be used interchangeably.

As used herein, the term "cells" includes cells in an animal individual, and cultured cells.

As used herein, the term "cellular cytotoxicity" refers to causing a pathological change to cells in any form, including causing not only direct trauma but also damage to the structures and functions of any cells, such as DNA breaks, formation of basic dimers, chromosome breaks, damage to mitotic apparatus, and decrease in various enzyme activity.

As used herein, the phrase "exhibiting toxicity in a cell" refers to showing toxicity in a cell in any form, including not only direct trauma but also effects on the structures, functions and metabolism of any cells, such as DNA breaks, formation of basic dimers, chromosome breaks, damage to mitotic apparatus, decrease in various enzyme activity, and suppression of cell growth factor activity.

As used herein, the phrase "binding to the same epitope" means antibodies that bind to a common epitope. When a second antibody binds to a partial peptide or partial three-dimensional structure to which a first antibody binds, the first and second antibodies can be determined to bind to the same epitope. Alternatively, by confirming that a second antibody competes with a first antibody for the bindings to an antigen (i.e., the second antibody prevents the first antibody from binding to the antigen), the first and second antibodies can be determined to bind to the same epitope, even if a specific sequence or structure of the epitope is not determined. As used herein, the phrase "binding to the same epitope" refers to a case where the first and second antibodies are determined to bind to a common epitope by one or both of the determination methods above. When the first and second antibodies bind to the same epitope, and the first antibody has a unique effect such as antitumor activity and internalization activity, the second antibody is expected to have similar activity.

As used herein, the phrase "hybridizing under stringent conditions" refers to hybridization in a commercially available hybridization solution ExpressHyb Hybridization Solution (Clontech) at 68°C, or hybridization using a filter to which DNA has been fixed, in the presence of 0.7 to 1.0 M NaCl at 68°C followed by hybridization under conditions capable of identification by washing at 68°C with an SSC solution at 0.1 to 2-fold concentration (SSC at 1-fold concentration consists of 150 mM NaCl and 15 mM sodium citrate), or under conditions equivalent thereto.

As used herein, "one to several" or "one or several" means 1 to 10, 1 to 9, 1 to 8, 1 to 7, 1 to 7, 1 to 6, 1 to 5, 1 to 4, 1 to 3, or 1 to 2.

1. Anti-LRRC15 Antibody

In an aspect of the present invention, provided is an antibody or an antigen-binding fragment of the antibody that specifically binds to an epitope at least part of which is present in LRRNT and any of LRR1 to 11 (candidate domain for LRRC15) of SEQ ID NO: 1, preferably an antibody or an antigen-binding fragment of the antibody that specifically binds to an epitope at least part of which is present in any of LRRNT and LRR1 to 7 of SEQ ID NO: 1, and more preferably an antibody or an antigen-binding fragment of the antibody that specifically binds to an epitope at least part of which is present in LRRNT or 2 of SEQ ID NO: 1.

Leucine-rich repeat-containing protein 15 (LRRC15) is a single-pass transmembrane protein that contains 15 leucine-rich repeats, whose N-terminus and C-terminus are located on the extracellular side and intracellular side, respectively. Although the detail functions have not been revealed, LRRC15 is known to bind to an extracellular matrix containing collagen, and to have no signaling region within cells (Cancer Res (2018) 78 (14) :4059-4072). A plurality of species-specific LRRC15 are known, and examples thereof include human LRRC15 (also referred to as "hLRRC15"), cynomolgus monkey LRRC15 (also referred to as "cynoLRRC15"), mouse LRRC15 (also referred to as "mLRRC15"), and rat LRRC15 (also referred to as "ratLRRC15" or "rLRRC15"). Note that, as used herein, the term "LRRC15" can be used for the same meaning as LRRC15 protein.

The human LRRC15 is known to be present as an isoform consisting of 587 amino acid residues (SEQ ID NO: 13; NP_001128529.2) and another isoform containing 581 amino acid residues with the N-terminus truncated (SEQ ID NO: 1; NP_570843.2), both of which have the same amino acid sequence except for signal peptides. The cynomolgus monkey LRRC15 has the full-length amino acid sequence set forth in SEQ ID NO: 4 and the extracellular amino acid sequence set forth in SEQ ID NO: 5. The mouse LRRC15 has the full-length amino acid sequence set forth in SEQ ID NO: 7 and the extracellular amino acid sequence set forth in SEQ ID NO: 8. The rat LRRC15 has the full-length amino acid sequence set forth in SEQ ID NO: 10 and the extracellular amino acid sequence set forth in SEQ ID NO: 11.

In addition, a protein that consists of an amino acid sequence obtained by substituting, deleting and/or adding one or several amino acid residues in the amino acid sequence in the above LRRC15, and that has biological activity equivalent to the protein is also contained in LRRC15.

As used herein, in the structure of human LRRC15,

- the portion corresponding to amino acid residues 22 to 53 of SEQ ID NO: 1 is referred to as "LRRNT" (N-terminus in LRRC15),
- the portion corresponding to amino acid residues 54 to 75 of SEQ ID NO: 1 is referred to as "LRR1" (the first leucine-rich repeat in LRRC15),
- the portion corresponding to amino acid residues 78 to 99 of SEQ ID NO: 1 is referred to as "LRR2" (the second leucine-rich repeat in LRRC15),
- the portion corresponding to amino acid residues 102 to 123 of SEQ ID NO: 1 is referred to as "LRR3" (the third leucine-rich repeat in LRRC15),
- the portion corresponding to amino acid residues 126 to 147 of SEQ ID NO: 1 is referred to as "LRR4" (the 4th leucine-rich repeat in LRRC15),
- the portion corresponding to amino acid residues 150 to 171 of SEQ ID NO: 1 is referred to as "LRR5" (the 5th leucine-rich repeat in LRRC15),
- the portion corresponding to amino acid residues 174 to 195 of SEQ ID NO: 1 is referred to as "LRR6" (the 6th leucine-rich repeat in LRRC15),
- the portion corresponding to amino acid residues 198 to 219 of SEQ ID NO: 1 is referred to as "LRR7" (the 7th leucine-rich repeat in LRRC15),
- the portion corresponding to amino acid residues 222 to 243 of SEQ ID NO: 1 is referred to as "LRR8" (the 8th leucine-rich repeat in LRRC15),
- the portion corresponding to amino acid residues 246 to 267 of SEQ ID NO: 1 is referred to as "LRR9" (the 9th leucine-rich repeat in LRRC15),
- the portion corresponding to amino acid residues 270 to 291 of SEQ ID NO: 1 is referred to as "LRR10" (the 10th leucine-rich repeat in LRRC15),
- the portion corresponding to amino acid residues 294 to 315 of SEQ ID NO: 1 is referred to as "LRR11" (the 11th leucine-rich repeat in LRRC15),
- the portion corresponding to amino acid residues 318 to 339 of SEQ ID NO: 1 is referred to as "LRR12" (the 12th leucine-rich repeat in LRRC15),
- the portion corresponding to amino acid residues 342 to 363 of SEQ ID NO: 1 is referred to as "LRR13" (the 13th leucine-rich repeat in LRRC15),
- the portion corresponding to amino acid residues 366 to 387 of SEQ ID NO: 1 is referred to as "LRR14" (the 14th leucine-rich repeat in LRRC15),

- the portion corresponding to amino acid residues 390 to 411 of SEQ ID NO: 1 is referred to as "LRR15" (the 15th leucine-rich repeat in LRRC15), and
- the portion corresponding to amino acid residues 423 to 475 of SEQ ID NO: 1 is referred to as "LRRCT".

As used herein, the term "epitope" refers to a specific partial peptide or partial three-dimensional structure of LRRC15 to which an anti-LRRC15 antibody or an antigen-binding fragment thereof specifically binds. An epitope, which is a partial peptide or partial three-dimensional structure of LRRC15, can be determined by a method well-known to those skilled in the art, such as immunoassay. For example, first, various partial structures, partially deleted structure, or partially modified structures of an antigen are prepared. In the preparation of partial structures, partially deleted structure, or partially modified structures, a known oligonucleotide synthesis technique can be used. For example, by modifying the amino acid sequence of a specific extracellular domain to modify the three-dimensional structure of the antigen, it is possible to determine to which domain in the antigen an antibody to be tested binds. When the epitope is a partial three-dimensional structure of the antigen, it can also be determined by identifying amino acid residues of the antigen adjacent to the antibody by X-ray structural analysis.

As used herein, the "epitope at least part of which is present in LRRNT (or any of LRR1 to 11)" means that at least part of the epitope is present in LRRNT (or any of LRR1 to 11). For example, the "epitope at least part of which is present in a region selected from the group consisting of LRRNT, LRR1 and LRR2" means that at least part of the epitope is present in LRRNT, LRR1 and LRR2 (the region consisting of amino acid residues 22 to 53 of SEQ ID NO: 1, the region consisting of amino acid residues 54 to 75 of SEQ ID NO: 1, and the region consisting of amino acid residues 78 to 99 of SEQ ID NO: 1). The epitope may be a linear epitope or a conformational epitope. When the epitope is a linear epitope, all of the serially arranged amino acid residues that constitute the epitope may be present in LRRNT (or any of LRR1 to 11), or only a part of the serially arranged amino acid residues that constitute the epitope may be present in LRRNT (or any of LRR1 to 11). Similarly for the conformational epitope, all of the non-serially arranged amino acid residues that constitute the epitope may be present in LRRNT (or any of LRR1 to 11), or only a part of the non-serially arranged amino acid residues that constitute the epitope may be present in LRRNT (or any of LRR1 to 11). However, the latter embodiment is predominant due to the nature.

As used herein, "specifically" binding to a particular epitope means that the antibody or an antigen-binding fragment thereof more strongly binds to the epitope than an epitope different from the epitope (e.g., one of LRR13 to 15).

In an aspect of the present invention, the antibody or an antigen-binding fragment of the antibody of the present invention does not bind or does not specifically bind to either LRR13 or LRR14 of SEQ ID NO: 1. Therefore, in an aspect of the present invention, provided is an antibody or an antigen-binding fragment of the antibody that does not bind or does not specifically bind to either of LRR13 or LRR14 of SEQ ID NO: 1.

In an aspect of the present invention, the antibody or an antigen-binding fragment of the antibody of the present invention specifically binds to at least any of LRRNT, LRR1 or LRR2 of SEQ ID NO: 1. Therefore, in an aspect of the present invention, provided is an antibody or an antigen-binding fragment of the antibody that specifically binds to at least any of LRRNT, LRR1 or LRR2 of SEQ ID NO: 1.

The "antibody" as used herein may be derived from any species, and preferable examples thereof include a human, monkey, rat, mouse, rabbit, camel, alpaca, or llama. When derived from a species other than human, it is preferably chimerized or humanized. Thus, in an aspect of the present invention, the antibody is a human antibody, chimeric antibody, or humanized antibody, and a human antibody or humanized antibody is preferred. The antibody of the present invention may be a polyclonal antibody or a monoclonal antibody, and a monoclonal antibody is preferred. It may be a monospecific antibody or multispecific antibody (e.g., bispecific antibody and trispecific antibody).

As used herein, the phrase "antigen-binding fragment of an antibody" means a partial fragment of an antibody having binding activity to an antigen, or a fusion polypeptide containing an antigen-binding site of an antibody (e.g., light chain and/or heavy chain variable regions, or CDRs contained in the variable regions), includes Fab, $F(ab')_2$, Fv, scFv, a variable domain of heavy chain of heavy-chain antibody (VHH) antibody, a diabody, a linear antibody, an antibody or multispecific antibody formed from an antigen-binding fragment thereof, a fusion protein to which the binding properties to the antigen is imparted by linking scFv to a functional domain of a different protein. Also, the antigen-binding fragment of the antibody includes Fab', a monovalent fragment of the variable region of the antibody, obtained by treating $F(ab')_2$ under reducing conditions. However, the antigen-binding fragments are not limited to these molecules as long as they have the binding capacity to an antigen. In addition, the antigen-binding fragments include not only those obtained by treating a full-length molecule of an antibody protein with an appropriate enzyme, but also proteins produced in an appropriate host cell using a genetically engineered gene that encodes an antibody or an antigen-binding fragment thereof. In an aspect of the present invention, the antigen-binding fragment of the antibody is selected from the group consisting of Fab, $F(ab')_2$, Fab', Fv, scFv, VHH, and scFv-containing fusion protein.

In an aspect of the present invention, the antigen-binding fragment contains a functional fragment that retains asparagine (Asn297 or N297) to be modified with an N-linked glycan well stored in an Fc region of an IgG heavy chain and its surrounding amino acids, and that has the binding capacity to an antigen.

Whether or not the antibody or an antigen-binding fragment thereof of the present invention specifically binds to a specific epitope can be determined by a well-known method. For example, it can be determined by calculating the association rate constant ka, the dissociation rate constant kd, and the dissociation constant (KD; KD = kd/ka) by the SPR method disclosed in Example 4)-6-1, or by flow cytometry analysis disclosed in Example 4)-6-2.

In an aspect of the present invention, the antibody or an antigen-binding fragment thereof of the present invention has an internalization ability to be taken up into a cell. In an aspect of the present invention, the antibody or an antigen-binding fragment thereof of the present invention has an internalization ability to be taken up into a cell, and preferably into a cell that expresses LRRC15 in a tumor cell and/or a cell in the stroma thereof (e.g., cancer-associated fibroblast: CAF, such as vascular endothelial cell, vascular pericyte, and immune cell). Examples of the cell that expresses LRRC15 in a stroma can include CAF. Uptake of the antibody or an antigen-binding fragment thereof into a cell can be confirmed by, for example, (1) an assay that visualizes the antibody or an antigen-binding fragment thereof taken up into a cell with a fluorescence microscope, by using a secondary antibody (fluorescently labeled) that binds to the antibody or an antigen-binding fragment thereof (Cell Death and Differentiation, 2008, 15, 751-761), (2) an assay that measures a fluorescence intensity taken up into a cell, by using a secondary antibody (fluorescently labeled) that binds to the antibody or an antigen-binding fragment thereof (Molecular Biology of the Cell Vol. 15, 5268-5282, December 2004), (3) an assay in which an antibody-drug conjugate in which a compound with cellular cytotoxicity is bound to the antibody or an antigen-binding fragment thereof via a linker is used to evaluate the growth inhibitory activity against the cell line, or (4) a Mab-ZAP assay in which an immunotoxin that binds to the antibody or an antigen-binding fragment thereof is used, and upon uptake into a cell, the toxin is released to inhibit cell growth (Bio Techniques 28: 162-165, January 2000). As the immunotoxin, a recombinant complex protein of a catalytic domain of diphtheria toxin and protein G may also be used. For example, in an aspect of the present invention, in a Mab-ZAP assay of (4), the situation where the viability (expressed as a relative rate to the cell viability upon addition of a control antibody taken as 100%) of LRRC15-expressing cells to which the antibody and a saporin-labeled anti-IgG antibody are administered is preferably 70% or less, and more preferably 60% or less, means that the antibody has internalization activity.

In an aspect of the present invention, the antibody or an antigen-binding fragment thereof can target LRRC15-positive cells present in tumor tissues or its surrounding tissues, that is, has properties capable of recognizing these cells, binding to these cells, and/or being taken up and internalized into these cells. Accordingly, in an aspect of the present invention, the antibody or an antigen-binding fragment thereof of the present invention can be an antibody-drug conjugate in which it has been bound to a compound with cellular cytotoxicity via a linker.

The binding activity to LRRC15-positive cells present in tumor tissues of the antibody or an antigen-binding fragment thereof, or its surrounding tissues can be confirmed, for example, using flow cytometry.

Since the antibody-drug conjugate mentioned above has been bound to a compound that exerts cellular cytotoxicity, the antibody itself may have cellular cytotoxicity, but it is not essential. For the purpose of exerting a cytotoxic compound specifically and/or selectively in target cells, it is important and preferred that the antibody has properties of being internalized and transferred into the cells.

In another aspect of the present invention, provided is an antibody or an antigen-binding fragment of the antibody that competes for binding to human LRRC15 with at least one antibody consisting of the amino acid sequence of SEQ ID NO: 1, selected from the group consisting of:

(1) an antibody having a light chain consisting of the amino acid sequence set forth in SEQ ID NO: 66 and a heavy chain consisting of the amino acid sequence set forth in SEQ ID NO: 42;
(2) an antibody having a light chain consisting of the amino acid sequence set forth in SEQ ID NO: 66 and a heavy chain consisting of the amino acid sequence set forth in SEQ ID NO: 48;
(3) an antibody having a light chain consisting of the amino acid sequence set forth in SEQ ID NO: 66 and a heavy chain consisting of the amino acid sequence set forth in SEQ ID NO: 54; and
(4) an antibody having a light chain consisting of the amino acid sequence set forth in SEQ ID NO: 60 and a heavy chain consisting of the amino acid sequence set forth in SEQ ID NO: 36.

In an aspect of the present invention, when, for example, the binding activity (e.g., the dissociation constant, and a signal value in flow cytometry or ELISA) of human LRRC15 consisting of the amino acid sequence of SEQ ID NO: 1 to at least one reference antibody selected from the group consisting of the above (1) to (4) is determined in an assay system and a test antibody or an antigen-binding fragment thereof is added to the assay system, in a case where the binding activity of the reference antibody is significantly decreased, it is determined that the test antibody or an antigen-binding fragment thereof competes with at least one reference antibody selected from the group consisting of the above (1) to (4) for binding to human LRRC15 consisting of the amino acid sequence of SEQ ID NO: 1. Specifically, for example, when the binding activity of at least one reference antibody selected from the group consisting of the above (1) to (4) is decreased by at least 30% or more, for example, at least 30%, 40%, 50%, 60%, 70%, 80%, 90%, 95%, or even more, or decreased to a percentage within any of the above values at a concentration of the test antibody 10 times higher than that of the reference

antibody, the test antibody is considered to compete with the reference antibody. In another aspect of the present invention, when, for example, the binding activity of human LRRC15 consisting of the amino acid sequence of SEQ ID NO: 1 to the test antibody or an antigen-binding fragment thereof in an assay system, and at least one reference antibody selected from the group consisting of the above (1) to (4) is added to the assay system, in a case where the binding activity of the test antibody or an antigen-binding fragment thereof is significantly decreased, it is determined that the test antibody or an antigen-binding fragment thereof competes with at least one reference antibody selected from the group consisting of the above (1) to (4) for binding to human LRRC15 consisting of the amino acid sequence of SEQ ID NO: 1. Specifically, for example, when the binding activity of the test antibody is decreased by at least 30% or more, for example, at least 30%, 40%, 50%, 60%, 70%, 80%, 90%, 95%, or even more, or decreased to a percentage within any of the above values at a concentration of the reference antibody 10 times higher than that of the test antibody, the test antibody is considered to compete with the reference antibody. When the test antibody or an antigen-binding fragment thereof competes with at least one antibody selected from the group consisting of the above (1) to (4) for binding to human LRRC15 consisting of the amino acid sequence of SEQ ID NO: 1, the test antibody or an antigen-binding fragment thereof is highly likely to recognize an epitope that is identical or overlaps with at least one antibody selected from the group consisting of the above (1) to (4), and is therefore, highly expected to have antigen binding capacity, biological activity, and/or internalization activity equivalent to at least one antibody selected from the group consisting of the above (1) to (4).

In an aspect of the present invention, provided is an antibody that binds to human LRRC15 at a KD of 1 pM to 1000000 nM, preferably 1 pM to 100000 nM, more preferably 1 pM to 10000 nM, further preferably 1 pM to 5000 nM, particularly preferably 10 pM to 1000 nM, 10 pM to 500 nM, 10 pM to 400 nM, 10 pM to 300 nM, 100 pM to 300 nM, 100 pM to 250 nM, 100 pM to 200 nM, 100 pM to 150 nM, 100 pM to 100 nM, or 100 pM to 80 nM, or antigen-binding fragment of the antibody. In an aspect of the present invention, the human LRRC15 is human LRRC15 containing the amino acid sequence of SEQ ID NO: 1, or human LRRC15 consisting of the amino acid sequence of SEQ ID NO: 1. KD is measured by using, for example, surface plasmon resonance (SPR), bilayer interferometry (BLI), or isothermal titration calorimetry (ITC), and in an aspect of the present invention, it is measured by SPR.

In a further aspect of the present invention, provided is an antibody or an antigen-binding fragment of the antibody containing CDRL1, CDRL2 and CDRL3, and CDRH1, CDRH2 and CDRH3 selected from the group consisting of the following (1) to (4):

(1) CDRL1 consisting of the amino acid sequence set forth in SEQ ID NO: 62, CDRL2 consisting of the amino acid sequence of WAS (the amino acid sequence set forth in SEQ ID NO: 63), and CDRL3 consisting of the amino acid sequence set forth in SEQ ID NO: 64, and CDRH1 consisting of the amino acid sequence set forth in SEQ ID NO: 38, CDRH2 consisting of the amino acid sequence set forth in SEQ ID NO: 39, and CDRH3 consisting of the amino acid sequence set forth in SEQ ID NO: 40;
(2) CDRL1 consisting of the amino acid sequence set forth in SEQ ID NO: 62, CDRL2 consisting of the amino acid sequence of WAS (the amino acid sequence set forth in SEQ ID NO: 63), and CDRL3 consisting of the amino acid sequence set forth in SEQ ID NO: 64, and CDRH1 consisting of the amino acid sequence set forth in SEQ ID NO: 44, CDRH2 consisting of the amino acid sequence set forth in SEQ ID NO: 45, and CDRH3 consisting of the amino acid sequence set forth in SEQ ID NO: 46;
(3) CDRL1 consisting of the amino acid sequence set forth in SEQ ID NO: 62, CDRL2 consisting of the amino acid sequence of WAS (the amino acid sequence set forth in SEQ ID NO: 63), and CDRL3 consisting of the amino acid sequence set forth in SEQ ID NO: 64, and CDRH1 consisting of the amino acid sequence set forth in SEQ ID NO: 50, CDRH2 consisting of the amino acid sequence set forth in SEQ ID NO: 51, and CDRH3 consisting of the amino acid sequence set forth in SEQ ID NO: 52; and
(4) CDRL1 consisting of the amino acid sequence set forth in SEQ ID NO: 56, CDRL2 consisting of the amino acid sequence of WAS (the amino acid sequence set forth in SEQ ID NO: 57), and CDRL3 consisting of the amino acid sequence set forth in SEQ ID NO: 58, and CDRH1 consisting of the amino acid sequence set forth in SEQ ID NO: 32, CDRH2 consisting of the amino acid sequence set forth in SEQ ID NO: 33, and CDRH3 consisting of the amino acid sequence set forth in SEQ ID NO: 34.

As used herein, the term "CDR" means complementarity-determining regions. It is known that there are 3 CDRs each in the heavy and light chains of an antibody molecule. CDRs, also referred to hypervariable regions, are located in the variable regions of the heavy and light chains of an antibody, are sites with particularly high variation of the primary structure. CDRs are separated into three locations each in the primary structure of the polypeptide chain of each of heavy and light chains. In the present specification, regarding the CDRs of the antibody, the CDRs of the heavy chain are expressed as CDRH1, CDRH2, and CDRH3 in the order from the amino terminus to the carboxyl terminus of the heavy chain amino acid sequence, the CDRs of the light chain are expressed as CDRL1, CDRL2, and CDRL3 in the order from the amino terminus to the carboxyl terminus of the light chain amino acid sequence. These sites are close to each other in the three-dimensional structure, and determine the specificity to an antigen to bind. Accordingly, the antibody containing

the set of CDRs specified in the above (1) to (4) has high probability of specifically recognizing an intended epitope of human LRRC15, expecting its utility. The CDR sequences are defined according to the schemes of IMGT (Lefranc et al., 2003, Dev Comparat Immunol 27:55-77), Kabat (Kabat et al., 1991, Sequences of Proteins of Immunological Interest, 5th Ed. Public Health Service, National Institutes of Health, Bethesda, Md.), and Chothia (Al-Lazikani et al., 1997, J. Mol. Biol 273:927-948), and in this application, the CDR sequences are specified by IMGT.

In an aspect of the present invention, provided is an antibody or an antigen-binding fragment of the antibody containing a light chain variable region and a heavy chain variable region of one selected from the group consisting of the following (1) to (4):

(1) a light chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID No: 65, and a heavy chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID No: 41,
(2) a light chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID No: 65, and a heavy chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID No: 47,
(3) a light chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID No: 65, and a heavy chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID No: 53, and
(4) a light chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID No: 59, and a heavy chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID No: 35.

In a further aspect of the present invention, provided is an antibody or an antigen-binding fragment of the antibody containing a light chain variable region and a heavy chain variable region of one selected from the group consisting of the following (1) to (4):

(1) a light chain variable region consisting of an amino acid sequence having 100% sequence identity to the CDR sequence in the amino acid sequence set forth in SEQ ID No: 65 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region, and a heavy chain variable region consisting of an amino acid sequence having 100% sequence identity to the CDR sequence in the amino acid sequence set forth in SEQ ID No: 41 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region,
(2) a light chain variable region consisting of an amino acid sequence having 100% sequence identity to the CDR sequence in the amino acid sequence set forth in SEQ ID No: 65 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region, and a heavy chain variable region consisting of an amino acid sequence having 100% sequence identity to the CDR sequence in the amino acid sequence set forth in SEQ ID No: 47 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region,
(3) a light chain variable region consisting of an amino acid sequence having 100% sequence identity to the CDR sequence in the amino acid sequence set forth in SEQ ID No: 65 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region, and a heavy

chain variable region consisting of an amino acid sequence having 100% sequence identity to the CDR sequence in the amino acid sequence set forth in SEQ ID No: 53 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region, and

(4) a light chain variable region consisting of an amino acid sequence having 100% sequence identity to the CDR sequence in the amino acid sequence set forth in SEQ ID No: 59 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region, and a heavy chain variable region consisting of an amino acid sequence having 100% sequence identity to the CDR sequence in the amino acid sequence set forth in SEQ ID No: 35 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region.

In the present invention, the identity between amino acid sequences can be evaluated according to any known methods, and in an aspect of the present invention, the identity between two amino acid sequences can be determined by aligning the sequences using the default parameters of, for example, ClustalW version 2, (Larkin MA, Blackshields G, Brown NP, Chenna R, McGettigan PA, McWilliam H, Valentin F, Wallace IM, Wilm A, Lopez R, Thompson JD, Gibson TJ and Higgins DG (2007), "Clustal W and Clustal X version 2.0", Bioinformatics. 23(21):2947-2948).

In a further aspect of the present invention, provided is an antibody or an antigen-binding fragment of the antibody containing a light chain and a heavy chain of one selected from the group consisting of the following (1) to (4):

(1) a light chain consisting of the amino acid sequence of SEQ ID NO: 66, and a heavy chain consisting of the amino acid sequence of SEQ ID NO: 42;

(2) a light chain consisting of the amino acid sequence of SEQ ID NO: 66, and a heavy chain consisting of the amino acid sequence of SEQ ID NO: 48;

(3) a light chain consisting of the amino acid sequence of SEQ ID NO: 66, and a heavy chain consisting of the amino acid sequence of SEQ ID NO: 54; and

(4) a light chain consisting of the amino acid sequence of SEQ ID NO: 60, and a heavy chain consisting of the amino acid sequence of SEQ ID NO: 36.

The anti-LRRC15 of the present invention can be obtained by immunizing an animal with an antigenic polypeptide, and collecting and purifying an antibody produced in the living body, using a method usually performed in the art, and LRRC15 that retains the three-dimensional structure is preferably used as an antigen. Examples of such a method can include the DNA immunization method.

The origin of the antigen is not limited to humans, and animals may be immunized with an antigen derived from a non-human animal such as a cynomolgus monkey, a rat, or a mouse. In this case, the cross-reactivity of antibodies that bind to the obtained heterologous antigens with human antigens can be tested to screen for an antibody applicable to a human disease.

In addition, antibody-producing cells that produce antibodies against an antigen are fused with myeloma cells according to a known method (e.g., Kohler and Milstein, Nature (1975) 256, 495-497; and Kennet, R. ed., Monoclonal Antibodies, 365-367, Plenum Press, N.Y. (1980)) to establish hybridomas, from which monoclonal antibodies can be obtained.

Hereinafter, a method for obtaining an antibody against LRRC15 will be specifically explained.

(1) Preparation of Antigen

The antigen can be obtained by introducing a gene that encodes an antigenic protein into a host by genetic engineering, and express the gene in the host cell. Specifically, the antigen can be obtained by, for example, preparing a vector capable of expressing an antigenic gene, introducing the vector into a host cell to express the gene, and purifying the expressed antigen. Antibodies can also be obtained by immunizing an animal with the antigen-expressing cells by the above genetic engineering or with a cell line that expresses the antigen.

Antibodies can also be obtained without using an antigenic protein, by incorporating cDNA of the antigenic protein into an expression vector to administer it to an animal to be immunized, expressing the antigenic protein in the body of the animal to be immunized, and producing an antibody against the antigenic protein.

The LRRC15 protein used in the production of the antibody of the present invention can be directly purified from LRRC15-expressing cells of humans, non-human mammals (e.g., rat, mouse, and monkey) for use, or can be used by preparing cell membrane fractions of the cells. LRRC15 can be synthesized in vitro or produced in a host cell by genetic engineering. In genetic engineering, specifically, the protein can be obtained by incorporating LRRC15 cDNA into an

expression-capable vector, and then synthesizing in a solution containing an enzyme, a substrate and energy substances necessary for transcription and translation, or by transforming host cells of other prokaryotes or eukaryote to express LRRC15 in the host cells. In addition, LRRC15-expressing cells by the above genetic engineering or a cell line that expresses LRRC15 can be directly administered to animals to be immunized. Also, the expression vector in which LRRC15 cDNA is incorporated is directly administered to animals to be immunized to express LRRC15 in the body of the animals to be immunized.

Further, LRRC15 includes proteins that consist of the amino acid sequence obtained by substituting, deleting and/or adding one or several amino acids in the amino acid sequence of the above LRRC15, and that have biological activity equivalent to that of the protein.

(2) Production of Anti-LRRC15 Monoclonal Antibody

The anti-LRRC15 antibody used in the present invention is not particularly limited, and for example, an antibody having the amino acid sequence shown in the sequence listing of the present application can be preferably used. As the anti-LRRC15 antibody used in the present invention, those having the following characteristics are desirable.

(1) An antibody having the following characteristics;

(a) specifically binding to LRRC15, and/or
(b) having the activity to be internalized into LRRC15-expressing cells by binding to LRRC15,

(2) The antibody described in the above (1), in which LRRC15 is human LRRC15.

The method for obtaining an antibody against the LRRC15 of the present invention is not particularly limited as long as an anti-LRRC15 antibody can be obtained, and LRRC15 that retains the high-dimensional structure is preferably used as an antigen.

One preferred example of the method for obtaining an antibody can include the DNA immunization method. The DNA immunization method is an approach in which an antigen-expressing plasmid is a gene transferred into an animal individual such as a mouse and rat, and the antigen is expressed in the individual, thereby inducing immunity against the antigen. Examples of the gene transfer approach include a method of directly injecting plasmid into muscle, a method of intravenously injecting an introduction reagent such as liposome or polyethyleneimine, a method using a viral vector, an approach of injecting gold particle with plasmid attached with a gene gun, and a hydrodynamic method in which a large amount of plasmid solution is rapidly injected intravenously. For the gene transfer method by intramuscular injection of expression plasmid, as an approach to enhance the expression level, there is known a technique called in vivo electroporation in which the plasmid is intramuscularly injected followed by the electroporation at the same site (Aihara H, Miyazaki J. Nat Biotechnol. 1998 Sep; 16(9):867-70 or Mir LM, Bureau MF, Gehl J, Rangara R, Rouy D, Caillaud JM, Delaere P, Branellec D, Schwartz B, Scherman D. Proc Natl Acad Sci USA. 1999 Apr 13; 96(8):4262-7.). This approach can further enhance the expression level by treating muscles with hyaluronidase before intramuscular injection of plasmid (McMahon JM1, Signori E, Wells KE, Fazio VM, Wells DJ. Gene Ther. 2001 Aug; 8(16): 1264-70). The preparation of hybridomas can be performed by a known method, and for example, it can be performed using Hybrimune Hybridoma Production System (Cyto Pulse Sciences, Inc.).

Specific examples for obtaining a monoclonal antibody can include the following:

(a) LRRC15 cDNA can be incorporated into an expression vector (e.g., pcDNA3.1: Thermo Fisher Scientific, Inc.), the vector can be directly administered to an animal to be immunized (e.g., rat or mouse) by a method such as electroporation or a gene gun to express LRRC15 in the animal body, and thereby inducing an immunoreaction. The administration of the vector by electroporation or the like may be performed once or several times if necessary for increasing the antibody titer, and preferably several times;
(b) tissues (e.g., lymph nodes) containing antibody-producing cells are collected from the above-mentioned animal in which the immunoreaction has been induced;
(c) preparation of myeloma cells (hereinafter, referred to as myeloma) (e.g., mouse myeloma SP2/0-ag14 cells);
(d) cell fusion of antibody-producing cells with myeloma;
(e) screening for hybridomas producing the antibody of interest;
(f) division into single cell clones (cloning);
(g) in some cases, culturing hybridomas or raising animals with the hybridomas transplanted to produce a large amount of monoclonal antibodies; and/or
(h) examination of the bioactivity (internalization activity) and binding specificity of the thus produced monoclonal antibodies, or verification of their properties as the labeling reagent.

Examples of the method for measuring antibody titers used herein can include, but are not limited to, flow cytometry or Cell-ELISA method.

Examples of the hybridoma strain thus established can include anti-LRRC15 antibody-producing hybridoma rR024. Note that, as used herein, an antibody produced by the anti-LRRC15 antibody-producing hybridoma rR024 is described as "rR024 antibody" or simply as "rR024".

The light chain variable region of the rR024 antibody consists of the amino acid sequence shown in SEQ ID NO: 17. The amino acid sequence of the light chain variable region of the rR024 antibody is encoded by the nucleotide sequence shown in SEQ ID NO: 16. The light chain variable region of the rR024 antibody have CDRL1 consisting of the amino acid sequence shown in SEQ ID No: 26, CDRL2 consisting of the amino acid sequence of WAS (the amino acid sequence shown in SEQ ID No: 27), and CDRL3 consisting of the amino acid sequence shown in SEQ ID No: 28. The heavy chain variable region of the rR024 antibody consists of the amino acid sequence shown in SEQ ID NO: 15. The amino acid sequence of the heavy chain variable region of the rR024 antibody is encoded by the nucleotide sequence shown in SEQ ID NO: 14. The heavy chain variable region of the rR024 antibody have CDRH1 consisting of the amino acid sequence shown in SEQ ID No: 20, CDRH2 consisting of the amino acid sequence shown in SEQ ID No: 21, and CDRH3 consisting of the amino acid sequence shown in SEQ ID No: 22.

Further, even when monoclonal antibodies are separately and independently obtained by performing (a) through (h) in "(2) Production of Anti-LRRC15 Monoclonal Antibody" again, or when monoclonal antibodies are separately obtained by another method, antibodies having internalization activity equivalent to that of the rR024 antibody can be obtained. One example of such an antibody can include an antibody that binds to the same epitope as the rR024 antibody. When a newly prepared monoclonal antibody binds to a partial peptide or partial three-dimensional structure to which the rR024 antibody binds, the monoclonal antibody can be determined to bind to the same epitope as the rR024 antibody. Also, by confirming that the monoclonal antibody competes with the rR024 antibody for the bindings to LRRC15 (i.e., the monoclonal antibody prevents the rR024 antibody from binding to LRRC15), the monoclonal antibody can be determined to bind to the same epitope as the rR024 antibody, even if a specific sequence or structure of the epitope has not been determined. When the epitopes are confirmed to be the same, the monoclonal antibody is highly expected to have antigen binding capacity, biological activity, and/or internalization activity equivalent to the rR024 antibody.

(3) Other Antibodies

The antibody of the present invention includes, in addition to the above monoclonal antibodies against LRRC15, recombinant antibodies artificially modified for the purpose of decreasing heterologous antigenicity to humans, such as chimeric antibodies, humanized antibodies, or human antibodies. These antibodies can be produced by a known method.

Examples of the chimeric antibody can include an antibody in which the variable region and the constant region of the antibody are heterologous to each other, such as a chimeric antibody in which the variable region of mouse- or rat-derived antibody is joined to a human-derived constant region (see, Proc. Natl. Acad. Sci. U.S.A., 81, 6851-6855, (1984)).

Examples of the chimeric antibody derived from a rat anti-human LRRC15 antibody include an antibody consisting of a light chain containing the light chain variable region of the rat anti-human LRRC15 antibody described herein (e.g., rR024 antibody) and a human-derived constant region, and a heavy chain containing the heavy chain variable region of the above rat anti-human LRRC15 antibody and a human-derived constant region.

Another example of the chimeric antibody derived from a rat anti-human LRRC15 antibody includes an antibody consisting of a light chain containing a light chain variable region obtained by substituting one to several amino acid residues, 1 to 3 amino acid residues, 1 to 2 amino acid residues, preferably one amino acid residue in the light chain variable region of the rat anti-human LRRC15 antibody described herein (e.g., rR024 antibody) with another amino acid residue or other amino acid residues, and a heavy chain containing a heavy chain variable region obtained by substituting one to several amino acid residues, 1 to 3 amino acid residues, 1 to 2 amino acid residues, preferably one amino acid residue in the heavy chain variable region of the above rat anti-human LRRC15 antibody with another amino acid residue or other amino acid residues, and the antibody may have any human-derived constant region.

Another example of the chimeric antibody derived from a rat anti-human LRRC15 antibody includes an antibody consisting of a light chain containing a light chain variable region obtained by substituting 1 to 2 amino acid residues, preferably one amino acid residue in any 1 to 3 CDRs in the light chain variable region of the rat anti-human LRRC15 antibody described herein (e.g., rR024 antibody) with another amino acid residue or other amino acid residues, and a heavy chain containing a heavy chain variable region obtained by substituting by substituting 1 to 2 amino acid residues, preferably one amino acid residue in any 1 to 3 CDRs in the heavy chain variable region of the above rat anti-human LRRC15 antibody with another amino acid residue or other amino acid residues, and the antibody may have any human-derived constant region.

Examples of the chimeric antibody derived from the rR024 antibody include an antibody consisting of a light chain containing the light chain variable region consisting of the amino acid sequence shown in SEQ ID No: 29, and a heavy chain containing the heavy chain variable region consisting of the amino acid sequence shown in SEQ ID NO: 23, and the

antibody may have any human-derived constant region.

Another example of the chimeric antibody derived from the rR024 antibody includes an antibody consisting of a light chain containing a light chain variable region obtained by substituting one to several amino acid residues, 1 to 3 amino acid residues, 1 to 2 amino acid residues, preferably one amino acid residue in the light chain variable region consisting of the amino acid sequence shown in SEQ ID No: 29 with another amino acid residue or other amino acid residues, and a heavy chain containing a heavy chain variable region obtained by substituting one to several amino acid residues, 1 to 3 amino acid residues, 1 to 2 amino acid residues, preferably one amino acid residue in the heavy chain variable region consisting of the amino acid sequence shown in SEQ ID No: 23 with another amino acid residue or other amino acid residues, and the antibody may have any human-derived constant region.

Another example of the chimeric antibody derived from the rR024 antibody includes an antibody consisting of a light chain containing a light chain variable region obtained by substituting 1 or 2 amino acid residues (preferably one amino acid residue) in any 1 to 3 CDRs in the light chain variable region consisting of the amino acid sequence shown in SEQ ID NO: 29 with another amino acid residue or other amino acid residues, and a heavy chain containing a heavy chain variable region obtained by substituting 1 or 2 amino acid residues (preferably one amino acid residue) in any 1 to 3 CDRs in the heavy chain variable region consisting of the amino acid sequence shown in SEQ ID NO: 23 with another amino acid residue or other amino acid residues, and the antibody may have any human-derived constant region.

Specific examples of the chimeric antibody derived from the rR024 antibody include an antibody consisting of a light chain consisting of the full-length light chain amino acid sequence shown in SEQ ID No: 30, and a heavy chain consisting of the full-length heavy chain amino acid sequence shown in SEQ ID No: 24, and the chimeric anti-human LRRC15 antibody is referred to herein as "chimeric R024 antibody", "chR024 antibody", or "chR024". The full-length light chain amino acid sequence of the chR024 antibody is encoded by the nucleotide sequence shown in SEQ ID NO: 31, and the full-length heavy chain amino acid sequence of the chR024 antibody is encoded by the nucleotide sequence shown in SEQ ID NO: 25.

The light chain variable region amino acid sequence of the chR024 antibody is identical to the light chain variable region amino acid sequence of the rR024 antibody, and consists of the amino acid sequence shown in SEQ ID NO: 29. The light chain of the chR024 antibody have CDRL1 consisting of the amino acid sequence shown in SEQ ID No: 26, CDRL2 consisting of the amino acid sequence of WAS (the amino acid sequence shown in SEQ ID No: 27), and CDRL3 consisting of the amino acid sequence shown in SEQ ID No: 28, each of which is identical to CDRL1, CDRL2, and CDRL3 of the light chain of rR024, respectively.

The heavy chain variable region amino acid sequence of the chR024 antibody consists of the amino acid sequence shown in SEQ ID NO: 23. The heavy chain of the chR024 antibody has CDRH1 consisting of the amino acid sequence shown in SEQ ID No: 20, CDRH2 consisting of the amino acid sequence shown in SEQ ID No: 21, and CDRH3 consisting of the amino acid sequence shown in SEQ ID No: 22.

Examples of the humanized antibody can include an antibody obtained by incorporating only the complementarity-determining regions (CDRs) into a human-derived antibody (see, Nature (1986) 321, p.522-525), an antibody obtained by grafting the amino acid residues of some frameworks, in addition to the CDR sequences, into a human antibody by a CDR grafting method (WO 90/07861), and further, an antibody obtained by modifying the amino acid sequences of some CDRs while retaining the binding capacity to an antigen.

As used herein, the humanized antibody derived from the rR024 antibody or the chR024 antibody is not limited to a specific humanized antibody, and can be any humanized antibody as long as it carries all of the 6 CDR sequences each proper to either the rR024 antibody or the chR024 antibody, and preferably has internalization activity. As long as the humanized antibody retains the binding activity to an antigen and preferably has internalization activity, some amino acid sequences of some CDRs may further be modified. The humanized antibody derived from the rR024 antibody or the chR024 antibody is referred to herein as "hR024".

Illustrative examples of the humanized antibody of the chR024 antibody can include any combination of a light chain containing the light chain variable region having the amino acid sequence described in one selected from the group consisting of:

(1) the amino acid sequence set forth in SEQ ID NO: 29;
(2) an amino acid sequence having at least 95% or more sequence identity to the amino acid sequence of the above (1) (preferably an amino acid sequence having 100% sequence identity to the CDR sequences in the amino acid sequence of the above (1), and having at least 95% or more sequence identity to the framework regions in the amino acid sequence of the above (1)); and
(3) an amino acid sequence obtained by deleting, substituting, or adding one or several amino acids in the amino acid sequence of the above (1), and

a heavy chain containing the heavy chain variable region having the amino acid sequence described in one selected from the group consisting of:

(4) the amino acid sequence set forth in SEQ ID NO: 23;

(5) an amino acid sequence having at least 95% or more sequence identity to the amino acid sequence of the above (4) (preferably an amino acid sequence having 100% sequence identity to the CDR sequences in the amino acid sequence of the above (4), and having at least 95% or more sequence identity to the framework regions in the amino acid sequence of the above (4)); and

(6) an amino acid sequence obtained by deleting, substituting, or adding one or several amino acids in the amino acid sequence of the above (4).

In addition, an antibody can be used in which one of the heavy chain and light chain is humanized, and the other is the light chain or the heavy chain of a rat antibody and a chimeric antibody. Examples thereof can include any combination of a light chain containing the light chain variable region described in one selected from the group consisting of:

(1) a light chain variable region consisting of the amino acid sequence set forth in SEQ ID NO: 59 or 65;

(2) a light chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more sequence identity to the amino acid sequence of the above (1) (preferably a light chain variable region consisting of an amino acid sequence having 100% sequence identity to the CDR sequences in the amino acid sequence of the above (1), and having at least 80% or more, preferably 85%, 86%, 87%, 88%, 89%, 90%, 91%, 92%, 93%, or 94% or more, more preferably 95%, 96%, 97%, or 98% or more, and most preferably 99% or more sequence identity to the sequence in the same framework region); and

(3) a light chain variable region consisting of an amino acid sequence obtained by deleting, substituting, or adding one or several amino acids in the amino acid sequence of the above (1),
and

a heavy chain containing the heavy chain variable region described in one selected from the group consisting of:

(4) a heavy chain variable region consisting of the amino acid sequence set forth in SEQ ID NO: 35, 41, 47, or 53;

(5) a heavy chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more sequence identity to the amino acid sequence of the above (4) (preferably a heavy chain variable region consisting of an amino acid sequence having 100% sequence identity to the CDR sequences in the amino acid sequence of the above (1), and having at least 80% or more, preferably 85%, 86%, 87%, 88%, 89%, 90%, 91%, 92%, 93%, or 94% or more, more preferably 95%, 96%, 97%, or 98% or more, and most preferably 99% or more sequence identity to the sequence in the same framework region); and

(6) a heavy chain variable region consisting of an amino acid sequence obtained by deleting, substituting, or adding one or several amino acids in the amino acid sequence of the above (4).

In addition, the substitution of an amino acid herein is preferably conservative amino acid substitution. The conservative amino acid substitution is substitution that occurs in an amino acid group related to amino acid side chains. Preferred amino acid groups are as follows: acidic group = aspartic acid and glutamic acid; basic group = lysine, arginine and histidine; non-polar group = alanine, valine, leucine, isoleucine, proline, phenylalanine, methionine, and tryptophan; and uncharged polar family = glycine, asparagine, glutamine, cysteine, serine, threonine, and tyrosine. Other preferred amino acid groups are as follows: aliphatic hydroxy group = serine and threonine; amide-containing group = asparagine and glutamine; aliphatic group = alanine, valine, leucine, and isoleucine; and aromatic group = phenylalanine, tryptophan, and tyrosine. Such amino acid substitution is preferably carried out to such a degree that the properties of the substance having the original amino acid sequence are not decreased.

Examples of the antibody having a preferable combination of the light chain and heavy chain described above include:

an antibody consisting of a light chain having the light chain variable region amino acid sequence shown in SEQ ID NO: 65 (also referred to herein as hR024 L01 light chain variable region amino acid sequence), and a heavy chain having the heavy chain variable region amino acid sequence shown in SEQ ID NO: 41 (also referred to herein as hR024 H02 heavy chain variable region amino acid sequence);

an antibody consisting of a light chain having the light chain variable region amino acid sequence shown in SEQ ID NO: 65 (also referred to herein as hR024 L01 light chain variable region amino acid sequence), and a heavy chain having the heavy chain variable region amino acid sequence shown in SEQ ID NO: 47 (also referred to herein as hR024 H02-V1 heavy chain variable region amino acid sequence);

an antibody consisting of a light chain having the light chain variable region amino acid sequence shown in SEQ ID NO:

65 (also referred to herein as hR024 L01 light chain variable region amino acid sequence), and a heavy chain having the heavy chain variable region amino acid sequence shown in SEQ ID NO: 53 (also referred to herein as hR024 H02-V2 heavy chain variable region amino acid sequence); or

an antibody consisting of a light chain having the light chain variable region amino acid sequence shown in SEQ ID NO: 59 (also referred to herein as hR024 L03 light chain variable region amino acid sequence), and a heavy chain having the heavy chain variable region amino acid sequence shown in SEQ ID NO: 35 (also referred to herein as hR024 H09 heavy chain variable region amino acid sequence).

Another examples of the antibody having a preferable combination of the light chain and heavy chain described above include: an antibody consisting of:

a light chain consisting of the amino acid sequences 21 to 240 of the full-length light chain amino acid sequence shown in SEQ ID NO: 66 (also referred to herein as hR024 L01 full-length light chain amino acid sequence); or

a light chain consisting of the amino acid sequences 21 to 240 of the full-length light chain amino acid sequence shown in SEQ ID NO: 60 (also referred to herein as hR024 L03 full-length light chain amino acid sequence); and

a heavy chain consisting of the amino acid sequences 20 to 472 of the full-length heavy chain amino acid sequence shown in SEQ ID NO: 42 (also referred to herein as hR024 H02 full-length heavy chain amino acid sequence);

a heavy chain consisting of the amino acid sequences 20 to 472 of the full-length heavy chain amino acid sequence shown in SEQ ID NO: 48 (also referred to herein as hR024 H02-V1 full-length heavy chain amino acid sequence);

a heavy chain consisting of the amino acid sequences 20 to 472 of the full-length heavy chain amino acid sequence shown in SEQ ID NO: 54 (also referred to herein as hR024 H02-V2 full-length heavy chain amino acid sequence); or

a heavy chain consisting of the amino acid sequences 20 to 472 of the full-length heavy chain amino acid sequence shown in SEQ ID NO: 36 (also referred to herein as hR024 H09 full-length heavy chain amino acid sequence).

More preferred examples of the antibody include:

an antibody consisting of a light chain consisting of the amino acid sequences 21 to 240 of the full-length light chain amino acid sequence shown in SEQ ID NO: 66, and a heavy chain consisting of the amino acid sequences 20 to 472 of the full-length heavy chain amino acid sequence shown in SEQ ID NO: 42 (also referred to herein as "H02L01 antibody" or "H02L01");

an antibody consisting of a light chain consisting of the amino acid sequences 21 to 240 of the full-length light chain amino acid sequence shown in SEQ ID NO: 66, and a heavy chain consisting of the amino acid sequences 20 to 472 of the full-length heavy chain amino acid sequence shown in SEQ ID NO: 48 (also referred to herein as "H02L01-V1 antibody" or "H02L01-V1");

an antibody consisting of a light chain consisting of the amino acid sequences 21 to 240 of the full-length light chain amino acid sequence shown in SEQ ID NO: 66, and a heavy chain consisting of the amino acid sequences 20 to 472 of the full-length heavy chain amino acid sequence shown in SEQ ID NO: 54 (also referred to herein as "H02L01-V2 antibody" or "H02L01-V2"); or

an antibody consisting of a light chain consisting of the amino acid sequences 21 to 240 of the full-length light chain amino acid sequence shown in SEQ ID NO: 60, and a heavy chain consisting of the amino acid sequences 20 to 472 of the full-length heavy chain amino acid sequence shown in SEQ ID NO: 36 (also referred to herein as "H09L03 antibody" or "H09L03").

By combining a heavy chain and a light chain having amino acid sequences showing high sequence identity to the above heavy chain amino acid sequence and/or the light chain amino acid sequence, antibodies having biological activity equivalent to each of the above antibodies can be obtained. Such identity is generally identity of 80% or more, preferably identity of 90%, 91%, 92%, 93%, or 94% or more, more preferably identity of 95%, 96%, 97%, or 98% or more, and most preferably identity of 99% or more. In addition, by combining a heavy chain and a light chain having amino acid sequences obtained by substituting, deleting, or adding one or several amino acid residues in the above heavy chain amino acid sequence and/or the light chain amino acid sequence, antibodies having biological activity equivalent to each of the above antibodies can also be obtained.

Note that, in the hR024 L01 full-length light chain amino acid sequence shown in SEQ ID NO: 66, the amino acid sequence consisting of the amino acid residues 1 to 20 is a signal sequence, the amino acid sequence consisting of the amino acid residues 21 to 134 is a variable region, and the amino acid sequence consisting of the amino acid residues 135 to 240 is a constant region.

In the hR024 L03 full-length light chain amino acid sequence shown in SEQ ID NO: 60, the amino acid sequence consisting of the amino acid residues 1 to 20 is a signal sequence, the amino acid sequence consisting of the amino acid residues 21 to 134 is a variable region, and the amino acid sequence consisting of the amino acid residues 135 to 240 is a

constant region.

In the hR024 H02 full-length heavy chain amino acid sequence shown in SEQ ID NO: 42, the amino acid sequence consisting of the amino acid residues 1 to 19 is a signal sequence, the amino acid sequence consisting of the amino acid residues 20 to 142 is a variable region, and the amino acid sequence consisting of the amino acid residues 143 to 472 is a constant region.

In the hR024 H02-V1 full-length heavy chain amino acid sequence shown in SEQ ID NO: 48, the amino acid sequence consisting of the amino acid residues 1 to 19 is a signal sequence, the amino acid sequence consisting of the amino acid residues 20 to 142 is a variable region, and the amino acid sequence consisting of the amino acid residues 143 to 472 is a constant region.

In the hR024 H02-V2 full-length heavy chain amino acid sequence shown in SEQ ID NO: 54, the amino acid sequence consisting of the amino acid residues 1 to 19 is a signal sequence, the amino acid sequence consisting of the amino acid residues 20 to 142 is a variable region, and the amino acid sequence consisting of the amino acid residues 143 to 472 is a constant region.

In the hR024 H09 full-length heavy chain amino acid sequence shown in SEQ ID NO: 36, the amino acid sequence consisting of the amino acid residues 1 to 19 is a signal sequence, the amino acid sequence consisting of the amino acid residues 20 to 142 is a variable region, and the amino acid sequence consisting of the amino acid residues 143 to 472 is a constant region.

Examples of the antibody of the present invention can include a human antibody that binds to LRRC15. The human antibody means an antibody having only amino acid sequences derived from a human chromosome. The anti-LRRC15 human antibody can be obtained by a method using a human antibody-producing mouse that has a human chromosome fragment containing the genes of a heavy chain and light chain of a human antibody (see, Tomizuka, K. et al., Nature Genetics (1997) 16, p. 133-143; Kuroiwa, Y. et al., Nucl. Acids Res. (1998) 26, p. 3447-3448; Yoshida, H. et al., Animal Cell Technology: Basic and Applied Aspects vol. 10, p. 69-73 (Kitagawa, Y., Matsuda, T. and Iijima, S. eds.), Kluwer Academic Publishers, 1999; Tomizuka, K. et al., Proc. Natl. Acad. Sci. USA (2000) 97, p. 722-727, and the like).

Such a human antibody-producing mouse can be produced by, for example, destroying the gene loci for the heavy chain and light chain of endogenous immunoglobulin in a mouse, instead, introducing the gene loci for the heavy chain and light chain of human immunoglobulin therein via a yeast artificial chromosome (YAC) vector or the like.

In addition, this antibody can also be obtained as follows: a eukaryotic cell is transformed with cDNA that encodes each of the heavy chain and light chain of such a human antibody, preferably with a vector containing the cDNA, through a gene recombinant technique, and the transformed cell that produces a gene recombinant human monoclonal antibody is cultured, and the antibody is obtained from the culture supernatant.

Here, as the host, for example, eukaryotic cells, preferably mammalian cells such as CHO cells, lymphocytes, and myelomas can be used.

Further, the antibody of the present invention can be obtained by a method of obtaining a human antibody derived from phage display screened from a human antibody library (see, Wormstone, I. M. et al., Investigative Ophthalmology & Visual Science. (2002) 43(7), p. 2301-2308; Carmen, S. et al., Briefings in Functional Genomics and Proteomics (2002), 1(2), p. 189-203; Siriwardena, D. et al., Ophthalmology (2002) 109(3), p. 427-431, and the like).

For example, a phage display method (Nature Biotechnology (2005), 23, (9), p. 1105-1116) can be used in which the variable region of a human antibody is expressed on a phage surface as a single chain antibody (scFv), and phages that bind to the antigen are selected.

Analysis of phage genes that bind to the antigen can determine the DNA sequence that encodes the variable region of the human antibody that binds to the antigen.

Once the DNA sequence of scFv that binds to the antigen has been clarified, the human antibody can be obtained by producing an expression vector having the sequence and introducing the expression vector into an appropriate host for expression (WO 92/01047, WO 92/20791, WO 93/06213, WO 93/11236, WO 93/19172, WO 95/01438, WO 95/15388, Annu. Rev. Immunol (1994) 12, p. 433-455, Nature Biotechnology (2005) 23(9), p. 1105-1116).

When a newly prepared human antibody binds to a partial peptide or partial three-dimensional structure of LRRC15 to which one of the rat anti-human LRRC15 antibody, chimeric anti-human LRRC15 antibody, or humanized anti-human LRRC15 antibody described herein (e.g., rR024 antibody, chR024 antibody, H02L01 antibody, H02L01-V1 antibody, or H02L01-V2 antibody) binds, the human antibody can be determined to bind to the same epitope as the rat anti-human LRRC15 antibody, chimeric anti-human LRRC15 antibody, or humanized anti-human LRRC15 antibody. Alternatively, by confirming that the human antibody competes with the rat anti-human LRRC15 antibody, chimeric anti-human LRRC15 antibody, or humanized anti-human LRRC15 antibody described herein (e.g., rR024 antibody, chR024 antibody, H02L01 antibody, H02L01-V1 antibody, H02L01-V2 antibody, or H09L03 antibody) for the bindings to LRRC15 (e.g., the human antibody prevents the rR024 antibody, chR024 antibody, H02L01 antibody, H02L01-V1 antibody, H02L01-V2 antibody, or H09L03 antibody from binding to LRRC15, preferably at least LRRNT, LRR1, or LRR2 of LRRC15), the human antibody can be determined to bind to the same epitope as the rat anti-human LRRC15 antibody, chimeric anti-human LRRC15 antibody, or humanized anti-human LRRC15 antibody described herein, even if a specific

sequence or structure of the epitope has not been determined. As used herein, when it is determined that it "binds to the same epitope" by at least either one of the determination methods, it can be said that the newly prepared human antibody "binds to the same epitope" as the rat anti-human LRRC15 antibody, chimeric anti-human LRRC15 antibody, or humanized anti-human LRRC15 antibody. When the epitopes are confirmed to be the same, the human antibody is highly expected to have biological activity equivalent to the rat anti-human LRRC15 antibody, chimeric anti-human LRRC15 antibody, or humanized anti-human LRRC15 antibody (e.g., rR024 antibody, chR024 antibody, H02L01 antibody, H02L01-V1 antibody, H02L01-V2 antibody, or H09L03 antibody).

The chimeric antibody, humanized antibody, or human antibody obtained by the above methods can be evaluated for their binding activity to antigens and screened for preferred antibodies by a known method or the like.

The antibody of the present invention includes modified variants of the antibody. The modified variant refers to a variant obtained by subjecting the antibody of the present invention to chemical or biological modification. Examples of the chemically modified variant include variants including a linkage of a chemical moiety to an amino acid skeleton, and variants with chemical modification of an N-linked or O-linked carbohydrate chain. Examples of the biologically modified variant include variants obtained by post-translational modification (e.g., N-linked or O-linked glycosylation, N- or C-terminal processing, deamidation, isomerization of aspartic acid, oxidation of methionine, or pyroglutamation of N-terminal glutamic acid), and variants obtained by adding a methionine residue to the N terminus by expressing them in a prokaryotic host cell. Further, an antibody labeled so as to enable the detection or isolation of the antibody of the present invention or an antigen, for example, an enzyme-labeled antibody, a fluorescence-labeled antibody, and an affinity-labeled antibody are also included in the meaning of the modified variant. Such a modified variant of the antibody of the present invention is useful for improving the stability and blood retention of the antibody, reducing the antigenicity thereof, detecting or isolating an antibody or an antigen, and the like.

Further, by regulating the modification of a glycan which is bound to the antibody of the present invention (e.g., glycosylation, defucosylation), the antibody-dependent cellular cytotoxicity can be enhanced or attenuated, antibody-dependent cell-mediated phagocytosis can be enhanced or attenuated, or complement-dependent cytotoxicity can be enhanced or attenuated. Known techniques for regulating the modification of a glycan of antibodies include, but are not limited to, WO 1999/54342, WO 2000/61739, WO 2002/31140, WO 2007/133855, and the like. The antibody of the present invention also includes an antibody in which the modification of a glycan has been regulated.

When the antibody gene is isolated and then introduced into an appropriate host to prepare an antibody, a combination of an appropriate host and an expression vector can be used. Specific examples of the antibody gene can include a combination of a gene that encodes the heavy chain sequence and a gene that encodes the light chain sequence described herein. When a host cell is transformed, the heavy chain sequence gene and the light chain sequence gene may be inserted into the same expression vector, or may be inserted into separate expression vectors.

When eukaryotic cells are used as the hosts, for example, animal cells, plant cells, and eukaryotic microorganisms can be used. In particular, examples of the animal cells can include mammalian cells, such as monkey cells COS cells (Gluzman, Y. Cell (1981) 23, p. 175-182, ATCC CRL-1650), mouse fibroblast NIH3T3 (ATCC No. CRL-1658), a dihydrofolate reductase-deficient strain (Urlaub, G. and Chasin, L. A. Proc. Natl. Acad. Sci. U.S.A. (1980) 77, p. 4126-4220) of Chinese hamster ovary cells (CHO cells, ATCC CCL-61), FreeStyle 293F cells (Invitrogen), and Expi293 cells (Thermo Fisher Scientific, Inc.).

When prokaryotic cells are used, examples thereof can include Escherichia coli, and Bacillus subtilis.

Antibodies can be obtained by introducing an antibody gene of interest into these cells by transformation, and culturing the transformed cells in vitro. The yields may vary depending on the antibody sequence in the culture, and hence, antibodies that are easily produced as medicine can be selected from antibodies with equivalent binding activity, using the yield as an indicator. Accordingly, the antibody of the present invention includes an antibody obtained by a method for producing the antibody, comprising a step of culturing the transformed host, and collecting the antibody or an antigen-binding fragment of the antibody of interest from a culture obtained in the culturing step.

Note that, when antibodies are produced in cultured mammalian cells, it is known that the lysine residue at the carboxyl terminus of the heavy chain of the antibody is deleted (Journal of Chromatography A, 705: 129-134 (1995)), and further, that two amino acid residues, glycine and lysine, at the heavy chain carboxyl terminus again are deleted, and the proline residue located at the carboxyl terminus is newly amidated (Analytical Biochemistry, 360: 75-83 (2007)). However, such deletion and modification of the heavy chain sequences do not affect the antigen binding capacity or effector functions (e.g., complement activation, antibody-dependent cellular cytotoxicity, and antibody-dependent cell-mediated phagocytosis). Therefore, the antibody of the present invention also includes an antibody subjected to the modification and an antigen-binding fragment of the antibody, and hence, also encompasses deletion variants obtained by deleting one or two amino acids at the heavy chain carboxyl terminus, and a newly generated deletion variants obtained by amidating the carboxyl terminus (e.g., deletion variants obtained by amidating the proline residue at the carboxyl terminal site). However, the deletion variants at the carboxyl terminus of the heavy chain of the antibody according to the present invention are not limited to the above type as long as the antigen binding capacity or effector function is maintained. The two heavy chain constituting the antibody according to the present invention may be any one of heavy chains selected from the group

consisting of the full-length and the above deletion variants, or a combination of any two of them. The ratio of the amount of each deletion variant can be affected by the type of cultured mammalian cells which produce the antibody according to the present invention and the culture conditions; however, examples thereof can include an antibody obtained by deleting one amino acid residue at the carboxyl terminus in both of the two heavy chains, as a main component of the antibody according to the present invention.

Examples of the isotype of the antibody of the present invention can include IgG (IgG1, IgG2, IgG3, IgG4), IgD, IgM, and preferred examples thereof include IgG1, IgG2, and IgG4.

When IgG1 is used as the isotype of the antibody of the present invention, the effector function can be adjusted by substituting some amino acid residues in the constant region. As an aspect, the antibody-dependent cellular cytotoxicity can be enhanced or attenuated, antibody-dependent cell-mediated phagocytosis can be enhanced or attenuated, or complement-dependent cytotoxicity can be enhanced or attenuated. Examples of an aspect of the mutant of IgG1 in which the effector function has been reduced or attenuated can include a variant obtained by substituting leucine at the 234-position and leucine at the 235-position in the amino acid sequence of the constant region of wild-type human IgG1, specified by EU index (Proc. Natl. Acad. Sci. U.S.A., Vol. 63, No. 1 (May 15, 1969), pp.78-85), with alanine and alanine, respectively, or a variant obtained by substituting leucine at the 234-position, leucine at the 235-position, and proline at the 329-position in the amino acid sequence of the constant region of wild-type human IgG1, specified by EU index, with alanine, alanine, and alanine, respectively.

Examples of the biological activity of the antibody can generally include antigen binding activity, activity to internalize into cells that express an antigen by binding to the antigen, activity to neutralize antigen activity, activity to inhibit the antigen activity, activity to enhance the antigen activity, antibody-dependent cellular cytotoxicity (ADCC) activity, complement-dependent cytotoxicity (CDC) activity, and antibody-dependent cell-mediated phagocytosis (ADCP) activity. The functions that the antibody according to the phagocytosis is binding activity to LRRC15, and preferably activity to internalize into LRRC15-expressing cells by binding to LRRC15. Further, the antibody of the present invention may have ADCC activity, CDC activity and/or ADCP activity in combination, in addition to cellular internalization activity.

The obtained antibody can be purified to homogeneity. The separation and purification of the antibody can be performed using methods for separation and purification of normal proteins. For example, antibodies can be separated and purified by appropriately selecting, but not limited to, column chromatography, filter filtration, ultrafiltration, salting-out, dialysis, preparative polyacrylamide gel electrophoresis, isoelectric focusing electrophoresis, and the like, and combining thereof (Strategies for Protein Purification and Characterization: A Laboratory Course Manual, Daniel R. Marshak et al. eds., Cold Spring Harbor Laboratory Press (1996); Antibodies: A Laboratory Manual. Ed Harlow and David Lane, Cold Spring Harbor Laboratory (1988)).

Examples of the chromatography can include affinity chromatography, ion exchange chromatography, hydrophobic chromatography, gel filtration chromatography, reversed-phase chromatography, and adsorption chromatography.

These chromatographies can be carried out using liquid chromatography such as HPLC and FPLC.

Examples of the column for use in affinity chromatography can include a protein A column and a protein G column. Examples of the column using the protein A column can include Hyper D, POROS, Sepharose F. F. (Pharmacia).

The antibody can also be purified by using binding activity to an antigen, with a carrier to which the antigen has been immobilized.

The present invention also relates to a polynucleotide that encodes the antibody or an antigen-binding fragment of the antibody of the present invention. The polynucleotide of the present invention is a polynucleotide containing the polynucleotide described in one of the following (a) to (f):

(a) a polynucleotide that encodes a heavy chain amino acid sequence of the LRRC15 antibody, a heavy chain amino acid sequence containing CDRH1 to CDRH3 of any one antibody of the LRRC15 antibodies, or a heavy chain amino acid sequence containing the amino acid sequence of the heavy chain variable region of the LRRC15 antibody of the present invention;

(b) a combination of a polynucleotide that encodes a heavy amino acid sequence and a polynucleotide that encodes a light chain amino acid sequence of the LRRC15 antibody of the present invention;

(c) a combination of a polynucleotide that encodes a heavy amino acid sequence containing CDRH1 to CDRH3 and a polynucleotide that encodes a light chain amino acid sequence containing CDRL1 to CDRL3 of any one antibody of the LRRC15 antibodies;

(d) a combination of a polynucleotide that encodes a heavy amino acid sequence containing the amino acid sequence of the heavy chain variable region and a polynucleotide that encodes a light chain amino acid sequence containing the amino acid sequence of the light chain variable region of the LRRC15 antibody of the present invention;

(e) a polynucleotide that hybridizes with a nucleotide consisting of a polynucleotide complementary to one of the polynucleotide described in (a) to (d) under stringent conditions, and that encodes the amino acid sequence of the antibody that binds to LRRC15; and

(f) a polynucleotide that encodes an amino acid sequence obtained by substituting, deleting, adding, or inserting 1 to 50, 1 to 45, 1 to 40, 1 to 35, 1 to 30, 1 to 25, 1 to 20, 1 to 15, 1 to 10, 1 to 8, 1 to 6, 1 to 5, 1 to 4, 1 to 3, 1 or 2, or one amino acid in the amino acid sequence of a polypeptide encoded by one of the polynucleotides (a) to (d), and that encodes the amino acid sequence of an antibody that binds to LRRC15.

Examples of the above polynucleotide include polynucleotides containing:

(1) a polynucleotide that encodes a light chain variable region containing CDRL1 consisting of the amino acid sequence set forth in SEQ ID NO: 62, CDRL2 consisting of the amino acid sequence of WAS (the amino acid sequence set forth in SEQ ID NO: 63), and CDRL3 consisting of the amino acid sequence set forth in SEQ ID NO: 64, and a polynucleotide that encodes a heavy chain variable region containing CDRH1 consisting of the amino acid sequence set forth in SEQ ID NO: 38, CDRH2 consisting of the amino acid sequence set forth in SEQ ID NO: 39, and CDRH3 consisting of the amino acid sequence set forth in SEQ ID NO: 40,
(2) a polynucleotide that encodes a light chain variable region containing CDRL1 consisting of the amino acid sequence set forth in SEQ ID NO: 62, CDRL2 consisting of the amino acid sequence of WAS (the amino acid sequence set forth in SEQ ID NO: 63), and CDRL3 consisting of the amino acid sequence set forth in SEQ ID NO: 64, and a polynucleotide that encodes a heavy chain variable region containing CDRH1 consisting of the amino acid sequence set forth in SEQ ID NO: 44, CDRH2 consisting of the amino acid sequence set forth in SEQ ID NO: 45, and CDRH3 consisting of the amino acid sequence set forth in SEQ ID NO: 46,
(3) a polynucleotide that encodes a light chain variable region containing CDRL1 consisting of the amino acid sequence set forth in SEQ ID NO: 62, CDRL2 consisting of the amino acid sequence of WAS (the amino acid sequence set forth in SEQ ID NO: 63), and CDRL3 consisting of the amino acid sequence set forth in SEQ ID NO: 64, and a polynucleotide that encodes a heavy chain variable region containing CDRH1 consisting of the amino acid sequence set forth in SEQ ID NO: 50, CDRH2 consisting of the amino acid sequence set forth in SEQ ID NO: 51, and CDRH3 consisting of the amino acid sequence set forth in SEQ ID NO: 52, or
(4) a polynucleotide that encodes a light chain variable region containing CDRL1 consisting of the amino acid sequence set forth in SEQ ID NO: 56, CDRL2 consisting of the amino acid sequence of WAS (the amino acid sequence set forth in SEQ ID NO: 57), and CDRL3 consisting of the amino acid sequence set forth in SEQ ID NO: 58, and a polynucleotide that encodes a heavy chain variable region containing CDRH1 consisting of the amino acid sequence set forth in SEQ ID NO: 32, CDRH2 consisting of the amino acid sequence set forth in SEQ ID NO: 33, and CDRH3 consisting of the amino acid sequence set forth in SEQ ID NO: 34.

In a further aspect of the present invention,

(1) a polynucleotide that encodes a light chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID NO: 65, and a polynucleotide that encodes a heavy chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID NO: 41,
(2) a polynucleotide that encodes a light chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID NO: 65, and a polynucleotide that encodes a heavy chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID NO: 47,
(3) a polynucleotide that encodes a light chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID NO: 65, and a polynucleotide that encodes a heavy chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID NO: 53, or
(4) a polynucleotide that encodes a light chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence

set forth in SEQ ID NO: 59, and a polynucleotide that encodes a heavy chain variable region consisting of an amino acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the amino acid sequence set forth in SEQ ID NO: 35.

In a further aspect of the present invention, provided is a polynucleotide containing the following (1) to (4):

(1) a polynucleotide that encodes a light chain variable region consisting of an amino acid sequence having 100% sequence identity to the CDR sequence in the amino acid sequence set forth in SEQ ID NO: 65 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region, and a polynucleotide that encodes a heavy chain variable region consisting of an amino acid sequence having 100% sequence identity to the CDR sequence in the amino acid sequence set forth in SEQ ID NO: 41 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region,
(2) a polynucleotide that encodes a light chain variable region consisting of an amino acid sequence having 100% sequence identity to the CDR sequence in the amino acid sequence set forth in SEQ ID NO: 65 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region, and a polynucleotide that encodes a heavy chain variable region consisting of an amino acid sequence having 100% sequence identity to the CDR sequence in the amino acid sequence set forth in SEQ ID NO: 47 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region,
(3) a polynucleotide that encodes a light chain variable region consisting of an amino acid sequence having 100% sequence identity to the CDR sequence in the amino acid sequence set forth in SEQ ID NO: 65 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region, and a polynucleotide that encodes a heavy chain variable region consisting of an amino acid sequence having 100% sequence identity to the CDR sequence in the amino acid sequence set forth in SEQ ID NO: 53 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region, or
(4) a polynucleotide that encodes a light chain variable region consisting of an amino acid sequence having 100% sequence identity to the CDR sequence in the amino acid sequence set forth in SEQ ID No: 59 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region, and a polynucleotide that encodes a heavy chain variable region consisting of an amino acid sequence having 100% sequence identity to the CDR sequence in the amino acid sequence set forth in SEQ ID No: 35 and having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the sequence in the same framework region.

In a further aspect of the present invention,

(1) a polynucleotide that encodes a light chain consisting of the amino acid sequence of SEQ ID NO: 66, and a polynucleotide that encodes a heavy chain consisting of the amino acid sequence of SEQ ID NO: 42;
(2) a polynucleotide that encodes a light chain consisting of the amino acid sequence of SEQ ID NO: 66, and a polynucleotide that encodes a heavy chain consisting of the amino acid sequence of SEQ ID NO: 48;
(3) a polynucleotide that encodes a light chain consisting of the amino acid sequence of SEQ ID NO: 66, and a polynucleotide that encodes a heavy chain consisting of the amino acid sequence of SEQ ID NO: 54; or
(4) a polynucleotide that encodes a light chain consisting of the amino acid sequence of SEQ ID NO: 60, and a polynucleotide that encodes a heavy chain consisting of the amino acid sequence of SEQ ID NO: 36.

In a further aspect of the present invention,

(1) a polynucleotide consisting of a nucleic acid sequence having at least 80% or more, preferably 85%, 86%, 87%,

88%, 89%, 90%, 91%, 92%, 93%, or 94% or more, more preferably 95%, 96%, 97%, or 98% or more, and most preferably 99% or more or 100% sequence identity to the nucleic acid sequence in SEQ ID NO: 67, and a polynucleotide consisting of a nucleic acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the nucleic acid sequence in SEQ ID NO: 43,
(2) a polynucleotide consisting of a nucleic acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the nucleic acid sequence in SEQ ID NO: 67, and a polynucleotide consisting of a nucleic acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the nucleic acid sequence in SEQ ID NO: 49,
(3) a polynucleotide consisting of a nucleic acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the nucleic acid sequence in SEQ ID NO: 67, and a polynucleotide consisting of a nucleic acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the nucleic acid sequence in SEQ ID NO: 55, or (4) a polynucleotide consisting of a nucleic acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the nucleic acid sequence in SEQ ID NO: 61, and a polynucleotide consisting of a nucleic acid sequence having at least 80% or more, preferably 85%, 86%, 87%, 88%, 89%, 90%, 91%, 92%, 93%, or 94% or more, more preferably 95%, 96%, 97%, or 98% or more, and most preferably 99% or more or 100% sequence identity to the nucleic acid sequence in SEQ ID NO: 37.

The present invention includes a polynucleotide that encodes the antibody or an antigen-binding fragment thereof of the present invention, or a modified variant thereof, an expression vector containing the polynucleotide, and a host cell transformed with the polynucleotide or the vector.

In an aspect of the present invention, the host cell is a eukaryotic cell or a prokaryotic cell. Examples of the eukaryotic cells include animal cells, plant cells, and eukaryotic microorganisms. In particular, examples of the animal cells can include mammalian cells, such as monkey cells COS cells (Gluzman, Y. Cell (1981) 23, p.175-182, ATCC CRL-1650), mouse fibroblast NIH3T3 (ATCC No. CRL-1658), a dihydrofolate reductase-deficient strain (Urlaub, G. and Chasin, L. A. Proc. Natl. Acad. Sci. U.S.A. (1980) 77, p.4126-4220) of Chinese hamster ovary cells (CHO cells, ATCC CCL-61), FreeStyle 293F cells (Invitrogen), and Expi293 cells (Thermo Fischer Scientific, Inc.).

In addition, the present invention also include a method for producing an antibody or an antigen-binding fragment of the antibody, including culturing the host cell, and collecting the antibody or an antigen-binding fragment of the antibody of interest from a culture obtained in the culturing step.

Figures 7 to 25 show the amino acid sequences or nucleotide sequences of the antibody of the present invention, and the amino acid sequences of the proteins or nucleotide sequences of the nucleic acids used in the present invention.

The antibody of the present invention is expected to be effectively usable in any medical modalities. Hereinafter, as an example thereof, embodiments for an antibody-drug conjugate in which a drug is bound to the antibody or an antigen-binding fragment of the antibody of the present invention will be described in detail. However, that does not mean that the antibody or an antigen-binding fragment of the antibody of the present invention exhibits effectiveness only in such medical modalities.

## 2. Anti-LRRC15 Antibody-Drug Conjugate

In an aspect of the present invention, provided is an antibody-drug conjugate in which a drug is bound to the antibody or an antigen-binding fragment of the antibody described above in detail.

### <Drug>

The anti-LRRC15 antibody obtained in the above "1. Anti-LRRC15 Antibody" can be made into an antibody-drug conjugate by binding a drug thereto via a linker structure moiety. The drug is not particularly limited as long as it has a substituent or partial structure that can be bound to a linker structure. The antibody-drug conjugate of the present invention can be used in various applications according to the drugs to be bound. Examples of such drugs can include pharmaceutically active compounds, such as compounds that exert toxicity within cells. In an aspect of the present invention, examples of the drug used in the antibody-drug conjugate can include antitumor compounds, chemotherapeutic agents, molecular target drugs, immunostimulant agents, immunosuppressant agents, toxins, photosensitizer, antibacterial

agents, antiviral agents, diagnostic agents, proteins, peptides, amino acids, nucleic acids, antigens, vitamins, and hormones. In a further aspect of the present invention, examples of the drug used in the antibody-drug conjugate can include substances having antitumor activity (antitumor compound), substances having an effect against hematologic diseases, substances having an effect against autoimmune diseases, antiinflammatory substances, antibacterial substances, antifungal substances, antiparasitic substances, antiviral substances, and antianesthetic substances.

<Antitumor Compound>

An example in which an antitumor compound is used as a compound to be bound to the antibody-drug conjugate of the present invention will be described below. The antitumor compound is not particularly limited as long as it is a compound having an antitumor effect and has a substituent or partial structure that can be bound to a linker structure. The antitumor compound exhibits an antitumor effect when a part or all of the linker is cleaved in a tumor cell and the antitumor compound moiety is released. When the linker is cleaved at the binding part with the drug, the antitumor compound is released in its original structure, and thereby its original antitumor effect is exhibited.

One example of the antitumor compound used in the present invention can include any one selected from the following:

[Chem. 34]

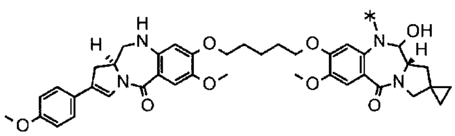

,

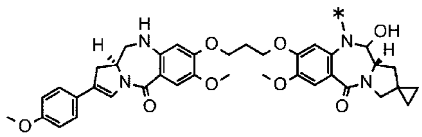

,

(XI)

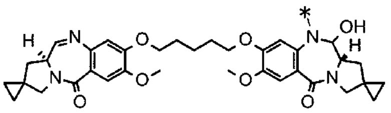

or

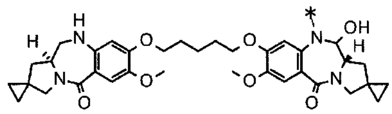

wherein * represents a direct or indirect bond to an antibody or an antigen-binding fragment of the antibody. The PBD derivatives of the present invention have an asymmetric carbon at the 11'-position, and hence optical isomers are present. As used herein, these isomers and mixtures of these isomers are all represented by a single formula. Accordingly, the above PBD derivatives of the present invention each contain optical isomers and mixtures of the optical isomers at any ratio. The absolute conformation of the above PBD derivatives of the present invention at the 11'-position can be determined by X-ray crystal structure analysis or NMR, such as a Mosher method, of crystalline products or intermediates, or derivative thereof. In this case, the absolute conformation can be determined by using a crystalline product or intermediate derived with a reagent having an asymmetric center with a known conformation. Stereoisomers can be obtained by isolating the synthesized compound according to the present invention using a conventional optical resolution method or separation method as desired.

The antibody-drug conjugate of the present invention, a free drug or production intermediate thereof may contain stereoisomers or optical isomers derived from an asymmetric carbon atom, geometric isomers, tautomers, or optical isomers such as d-form, l-form, and atropisomers, and these isomers, optical isomers and mixtures thereof are all included in the present invention.

In an aspect of the present invention, the antitumor compound used in the present invention is one selected from the following:

[Chem. 35]

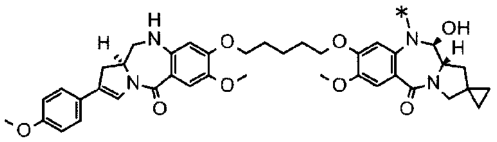

,

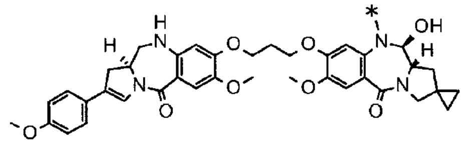

,

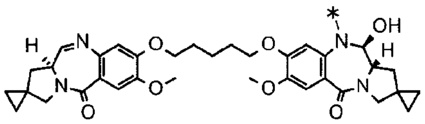

or

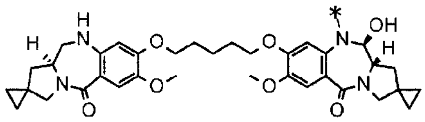

wherein * represents a direct or indirect bond to an antibody or an antigen-binding fragment of the antibody.

The antibody-drug conjugate of the present invention, a free drug or production intermediate thereof may contain stereoisomers or optical isomers derived from an asymmetric carbon atom, geometric isomers, tautomers, or optical isomers such as d-form, l-form, and atropisomers, and these isomers, optical isomers and mixtures thereof are all included in the present invention.

An example of the antibody-drug conjugate of the present invention in a case where the antitumor compound illustrated above is used as a compound to be bound to the antibody-drug conjugate of the present invention can include an antibody-drug conjugate represented in the following formula (X):

[Chem. 36]

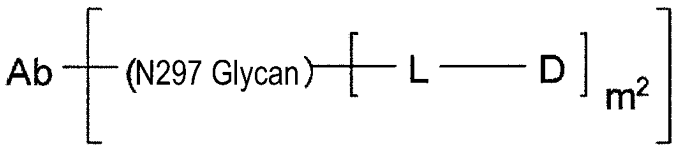

(X)

wherein $m^1$ represents an integer of 1 or 2,

$m^2$ represents an integer of 1 or 2,

Ab represents one of the antibody or an antigen-binding fragment of the antibody described above, such as the antibody or an antigen-binding fragment of the antibody described in any one of [1] to [16] and [24] to [28] of the paragraph [0007] herein,

L is a linker linking a glycan that binds to N297 (N297 glycan) of Ab to D,

the N297 glycan represents a glycan that can be remodeled,

D is one selected from the following:

[Chem. 37]

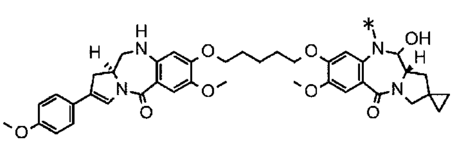

,

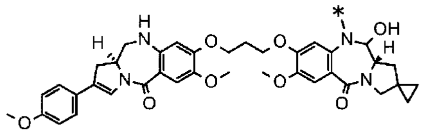

,

(XI)

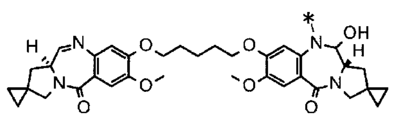

or

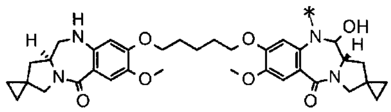

wherein * represents a bond to L.

<Linker Structure>

A linker structure that binds an antitumor compound to an antibody in the antibody-drug conjugate of the present invention will be described below. Note that the linker structure is hereinafter abbreviated as "L", and this means not only a partial structure "L" in the above formula (X) but also a linker structure in an antibody-drug conjugate not having the structural formula of the formula (X).

In an aspect of the present invention, the linker L is represented by the following formula:

$-Lb-La-Lp-NH-B-CH_2-O(C=O)-*.$

Wherein * represents a bond to the above antitumor compound (in particular, the nitrogen atom at the N10'-position of the antitumor compound represented by D in the formula (X)), and Lb represents a spacer that binds La to an antibody or an antigen-binding fragment thereof (in particular, the "N297 glycan" in the formula (X)).

B represents a phenyl group or heteroaryl group, preferably 1,4-phenyl group, 2,5-pyridyl group, 3,6-pyridyl group, 2,5-pyrimidyl group, or 2,5-thienyl group, and more preferably 1,4-phenyl group.

Lp represents a linker consisting of an amino acid sequence cleavable in a living body or a target cell. Lp is cleaved by, for example, the action of an enzyme such as esterase and peptidase. In an aspect of the present invention, Lp is a peptide residue composed of 2 to 7 (preferably, 2 to 4) amino acids. In other words, Lp is composed of an oligopeptide residue in which 2 to 7 amino acids are peptide-bound. In a further aspect of the present invention, Lp is bound to the carbonyl group of La in Lb-La- at the N-terminus, forms an amide bond with the amino group (-NH-) of the -NH-B-$CH_2$-O(C=O) part of the linker at the C-terminus, and the bond between the C-terminus of Lp and -NH- is cleaved by the enzyme such as esterase.

The amino acids constituting Lp are not particularly limited, and examples thereof include L- or D-amino acids, preferably L-amino acids. In addition to $\alpha$-amino acids, they may be amino acids with structures of, for example, $\beta$-alanine, $\varepsilon$-aminocaproic acid, or $\gamma$-aminobutyric acid, and may further be non-natural amino acids such as N-methylated amino acids.

Examples of the amino acids constituting LP can include, but are not particularly limited to, glycine (Gly; G), valine (Val; V), alanine (Ala; A), phenylalanine (Phe; F), glutamic acid (Glu; E), isoleucine (Ile; I), proline (Pro; P), citrulline (Cit), leucine (Leu; L), serine (Ser; S), lysine (Lys; K), and aspartic acid (Asp; D), and preferably glycine (Gly; G), valine (Val; V), alanine (Ala; A), and citrulline (Cit). These amino acids can appear multiple times, and Lp may have an amino acid sequence containing optionally selected amino acids. Also, drug release patterns may be controlled depending on the type of amino acids.

Specific examples of the Linker Lp can include - GGVA-, -GG-(D-)VA-, -VA-, -GGFG-, -GGPI-, -GGVCit-, -GGVK-, -GG(D-)PI-, -GGPL-, -EGGVA, -PI-, -GGF-, DGGF-, (D-)D-GGF-, -EGGF-, -SGGF-, -KGGF-, -DGGFG-, -GGFGG-, - DDGGFG-, -KDGGFG-, and -GGFGGGF-. The above "(D-)V", "(D-)P", "(D-)D" here mean D-valine, D-proline, and D-aspartic acid, respectively. The Linker Lp is one selected from the group consisting of -GGVA-, -GG-(D-)VA-, -VA-, -GGFG-, -GGPI-, -GGVCit-, -GGVK-, - GG(D-)PI-, and -GGPL-, more preferably one selected from the group consisting of -GGVA-, -GG-(D-)VA-, -VA-, - GGFG-, -GGPI-, -GGVCit-, -GGVK-, and -GGPL-, and further preferably one selected from the group consisting of - GGVA-, -GGVCit-, and -VA-.

La represents, but is not particularly limited to, one selected from the group consisting of:

$-C(=O)-CH_2CH_2-C(=O)-,$

$-C(=O)-(CH_2CH_2)_2-C(=O)-,$

$-C(=O)-CH_2CH_2-C(=O)-NH-(CH_2CH_2)_2-C(=O)-,$

$-C(=O)-CH_2CH_2-C(=O)-NH-(CH_2CH_2O)_2-CH_2-C(=O)-,$

$-C(=O)-CH_2CH_2-NH-C(=O)-(CH_2CH_2O)_4-CH_2CH_2-C(=O)-,$

$-CH_2-OC(=O)-,$

and,

$-OC(=O)-,$

and preferably

$-C(=O)-CH_2CH_2-C(=O)-$,

or

$-C(=O)-(CH_2CH_2)_2-C(=O)-$.

The spacer Lb is not particularly limited, and examples thereof include spacers represented by the following formulas.

[Chem. 38]

(Lb−1)

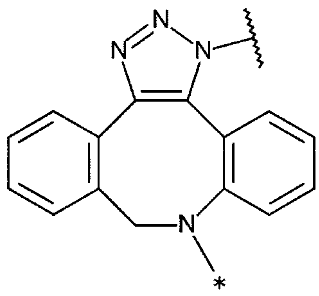

or

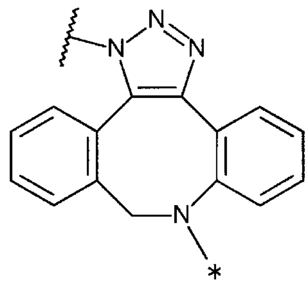

[Chem. 39]

(Lb−2)

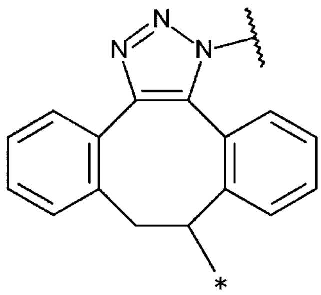

or

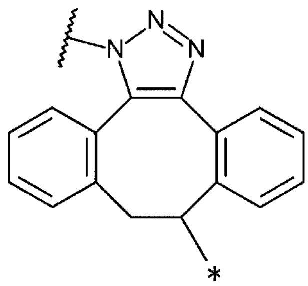

[Chem. 40]

(Lb−3)

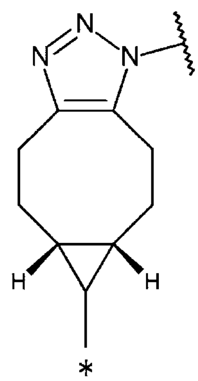

or

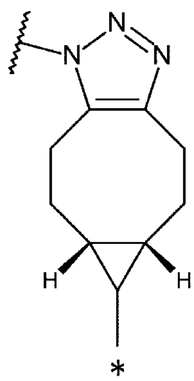

In each of the structural formulas of Lb shown above, * represents a bond to La (in particular -(C=O), O, or $-CH_2$ at the left end of La), and the wavy line represents a bond to an antibody or an antigen-binding fragment thereof (in particular, the "N297 glycan" in the formula (X)).

In each of the structural formulas of Lb (Lb-1, Lb-2, and Lb-3) shown above, the triazole ring moiety formed through click reaction between an azide group and a cyclooctynyl group has a geometric isomeric structure and exists as either one of the two structures or a mixture thereof in one Lb. There may be multiple (1 to 4) "-L-D"s in one molecule of the antibody-drug conjugate of the present invention, and each Lb (Lb-1, Lb-2, or Lb-3) in L in each "-L-D" is composed of either

one of the two structures or a mixture of both.

In an aspect of the present invention, L represents one selected from the group consisting of the following:

$-Z^1-C(=O)-CH_2CH_2-C(=O)-GGVA-NH-B-CH_2-OC(=O)-$,

$-Z^1-C(=O)-CH_2CH_2-C(=O)-GG-(D-)VA-NH-B-CH_2-OC(=O)-$,

$-Z^1-C(=O)-CH_2CH_2-C(=O)-VA-NH-B-CH_2-OC(=O)-$,

$-Z^1-C(=O)-(CH_2CH_2)_2-C(=O)-VA-NH-B-CH_2-OC(=O)-$,

$-Z^1-C(=O)-CH_2CH_2-C(=O)-GGPI-NH-B-CH_2-OC(=O)-$,

$-Z^1-C(=O)-CH_2CH_2-C(=O)-GGFG-NH-B-CH_2-OC(=O)-$,

$-Z^1-C(=O)-CH_2CH_2-C(=O)-GGVCit-NH-B-CH_2-OC(=O)-$,

$-Z^1-C(=O)-CH_2CH_2-C(=O)-GGVK-NH-B-CH_2-OC(=O)-$,

$-Z^1-C(=O)-CH_2CH_2-C(=O)-GGPL-NH-B-CH_2-OC(=O)-$,

$-Z^1-C(=O)-CH_2CH_2-C(=O)-NH-(CH_2CH_2)_2-C(=O)-VA-NH-B-CH_2-OC(=O)-$,

$-Z^1-C(=O)-CH_2CH_2-C(=O)-NH-(CH_2CH_2O)_2-CH_2-C(=O)-VA-NH-B-CH_2-OC(=O)-$,

$-Z^1-C(=O)-CH_2CH_2-NH-C(=O)-(CH_2CH_2O)_4-CH_2CH_2-C(=O)-VA-NH-B-CH_2-OC(=O)-$,

$-Z^2-OC(=O)-GGVA-NH-B-CH_2-OC(=O)-$,

and

$-Z^3-CH_2-OC(=O)-GGVA-NH-B-CH_2-OC(=O)-$,

wherein $Z^1$ represents the following structural formula:

[Chem. 41]

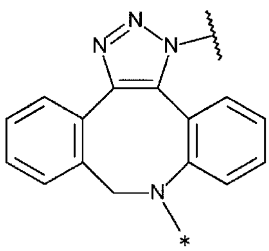

or

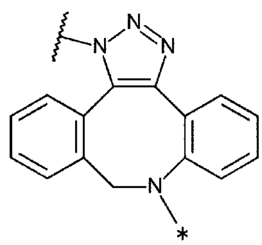

$Z^2$ represents the following structural formula:

[Chem. 42]

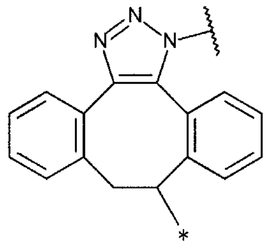

or

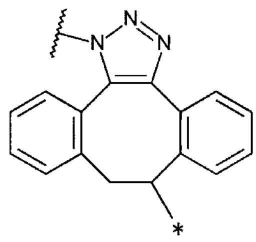

$Z^3$ represents the following structural formula:

[Chem. 43]

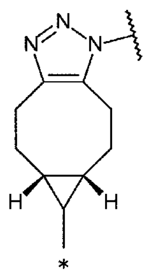

or

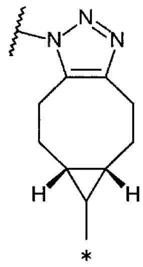

wherein in the structural formulas of $Z^1$, $Z^2$, and $Z^3$, * represents a bond to C(=O), O, or $CH_2$ adjacent to $Z^1$, $Z^2$, or $Z^3$, and the wavy lines represent a bond to an antibody or an antigen-binding fragment thereof (in particular, the "N297 glycan" in the formula (X)), and
B represents a 1,4-phenyl group.

In a further aspect of the present invention, L represents one selected from the group consisting of the following:

$-Z^1-C(=O)-CH_2CH_2-C(=O)-GGVA-NH-B-CH_2-OC(=O)-$,

$-Z^1-C(=O)-CH_2CH_2-C(=O)-VA-NH-B-CH_2-OC(=O)-$,

$-Z^1-C(=O)-(CH_2CH_2)_2-C(=O)-VA-NH-B-CH_2-OC(=O)-$,

$-Z^1-C(=O)-CH_2CH_2-C(=O)-GGVCit-NH-B-CH_2-OC(=O)-$,

$-Z^1-C(=O)-CH_2CH_2-C(=O)-NH-(CH_2CH_2)_2-C(=O)-VA-NH-B-CH_2-OC (=O) -$,

$-Z^1-C(=O)-CH_2CH_2-C(=O)-NH-(CH_2CH_2O)_2-CH_2-C(=O)-VA-NH-B-CH_2-OC(=O)-$,

and

$-Z^1-C(=O)-CH_2CH_2-NH-C(=O)-(CH_2CH_2O)_4-CH_2CH_2-C(=O)-VA-NH-B-CH_2-OC(=O)-$

wherein B is a 1,4-phenyl group, and $Z^1$ represents the following structural formula:

[Chem. 44]

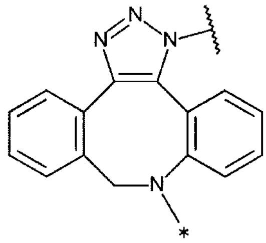

or

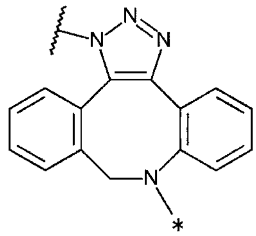

wherein in the structural formula of $Z^1$, * represents a bond to C(=O) adjacent to $Z^1$, and the wavy lines represent a bond to an antibody or an antigen-binding fragment thereof (in particular, the "N297 glycan" in the formula (X)).

The antibody-drug conjugate of the present invention is considered to exert the antitumor activity through a process in which the majority of the antibody-drug conjugate is transferred into tumor cells, and a linker portion (e.g., Lp) is then cleaved and activated by an enzyme or the like, which releases a drug portion (e.g., D) (hereinafter, referred to as a free drug). Accordingly, the antibody-drug conjugate of the present invention is preferred to be stable outside the tumor cells.

<Glycan Remodeling>

Recently, a method has been reported in which a heterogeneous glycoprotein of an antibody is remodeled through enzymatic reaction or the like to homogeneously introduce a glycan having a functional group (ACS Chemical Biology 2012, 7, 110, ACS Medicinal Chemistry Letters 2016, 7, 1005). An attempt using the glycan remodeling technique has also been made to site-specifically introduce a drug to synthesize a homogeneous ADC (Bioconjugate Chemistry 2015, 26, 2233, Angew. Chem. Int. Ed. 2016, 55, 2361-2367, US2016361436).

Accordingly, in an aspect of the present invention, a glycan in the antibody or an antigen-binding fragment thereof of the present invention, in particular, in the antibody or an antigen-binding fragment thereof incorporated into the antibody-drug conjugate (in particular, the "N297 glycan" in the antibody-drug conjugate represented by the above formula (X)) is a remodeled glycan.

The glycan remodeling of the present invention is performed through a process in which, first, using hydrolase, heterogeneous glycans added to a protein (e.g., antibody) are cleaved while only GlcNAc at the terminus remained to prepare a homogeneous protein moiety with GlcNAc added (hereinafter, referred to as "acceptor"). Second, an arbitrary glycan separately prepared (hereinafter, referred to as "donner") is provided, and the acceptor and the donner are linked together using glycosyltransferase. Accordingly, a homogeneous glycoprotein having an arbitrary glycan structure can be synthesized.

As used herein, the "glycan" means a structural unit in which two or more monosaccharides are bound together via glycosidic bonds. Specific monosaccharides and glycans may be abbreviated as, for example, "GlcNAc-", and "MSG-". When these abbreviations are used in a structural formula, the structural formula is shown assuming that an oxygen atom or nitrogen atom involved in the formation of the glycosidic bond with another structural unit at the reducing terminus is not included in the abbreviations indicating the glycan, unless otherwise specifically defined.

In the present invention, a monosaccharide as a basic unit of a glycan is described for convenience with the carbon atom, in the ring structure, that is bound to the oxygen atom constituting the ring and directly bound to a hydroxyl group (or an oxygen atom involved in a glycosidic bond) as the 1-position (the 2-position only for sialic acids), unless otherwise specified. The names of compounds in Examples are provided in view of the chemical structure as a whole, and this rule may not necessarily apply.

When a glycan is described as a symbol (e.g., GLY, SG, MSG, or GlcNAc) in the present invention, the symbol is to include the carbon at the reducing terminus, but not include N or O involved in an N- or O-glycosidic bond, unless otherwise defined.

In the present invention, the partial structure where a glycan is linked to a side chain of an amino acid is indicated such that the side chain portion is in parentheses, for example, as "(SG-)Asn", unless otherwise specifically specified.

The glycan of the antibody or an antigen-binding fragment of the antibody of the present invention (in particular, Ab in the formula (X)) is an N-linked glycan or O-linked glycan, and preferably an N-linked glycan. The N-linked glycan and O-linked glycan are bound to an amino acid side chain of the antibody by an N-glycosidic bond and O-glycosidic bond, respectively.

IgG has a well-conserved N-linked glycan at the 297th asparagine residue (hereinafter, referred to as "Asn297 or

N297") in the Fc region of its heavy chain and is known to contribute to the activity, kinetics and the like of the antibody molecule (Biotechnol. Prog., 2012, 28, 608-622, Sanglier-Cianferani, S., Anal. Chem., 2013, 85, 715-736).

The amino acid sequence in the constant region of IgG is well conserved, and each amino acid is identified by Eu numbering (Eu INDEX) in Edelman et al., (Proc. Natl. Acad. Sci. U.S.A., Vol. 63, No. 1 (May 15, 1969), pp. 78-85). For example, Asn297, to which an N-linked glycan in the Fc region is added, corresponds to the 297-position in Eu INDEX numbering, and each amino acid is uniquely identified by Eu INDEX numbering even if the actual position of the amino acid is varied due to fragmentation of the molecule or deletion of a region.

In the antibody-drug conjugate of the present invention, the antibody or an antigen-binding fragment thereof more preferably binds to L via a glycan that binds to a side chain of Asn297 thereof (hereinafter, referred to as "N297 glycan"), the antibody or an antigen-binding fragment thereof further more preferably binds to L via the N297 glycan, and the N297 glycan is a remodeled glycan. Note that an antibody having the remodeled glycan is referred to as a glycan-remodeled antibody.

SGP stands for sialyl glycopeptide and is a representative N-linked complex glycan. SGP can be isolated and purified from hen egg yolk according to the method described in WO 2011/0278681, for example. Also, purified products of SGP are commercially available (Tokyo Chemical Industry Co., Ltd.; FUSHIMI Pharmaceutical Co., Ltd.) and can be purchased. Disialooctasaccharide (Tokyo Chemical Industry Co., Ltd.) formed of only a glycan lacking one GlcNAc at the reducing terminus in the glycan portion of SG (hereinafter, "SG(10)") or the like is commercially available.

In the present invention, the description is made such that a glycan structure in which a sialic acid at the non-reducing terminus is deleted only in either one of branched chains of β-Man in SG(10) is referred to as MSG(9), a structure having a sialic acid only in the 1-3 branched chain of the glycan as MSG1, and a structure having a sialic acid only in the 1-6 branched chain of the glycan as MSG2.

In an aspect of the present invention, the remodeled glycan is N297-(Fuc)MSG1, N297-(Fuc)MSG2, or a mixture of N297-(Fuc)MSG1 and N297-(Fuc)MSG2, or N297-(Fuc)SG, preferably N297-(Fuc)MSG1, N297-(Fuc)MSG2, or N297-(Fuc)SG, and more preferably N297-(Fuc)MSG1 or N297-(Fuc)MSG2.

N297-(Fuc)MSG1 is represented by the following structural formula or sequence formula:

[Chem. 45]

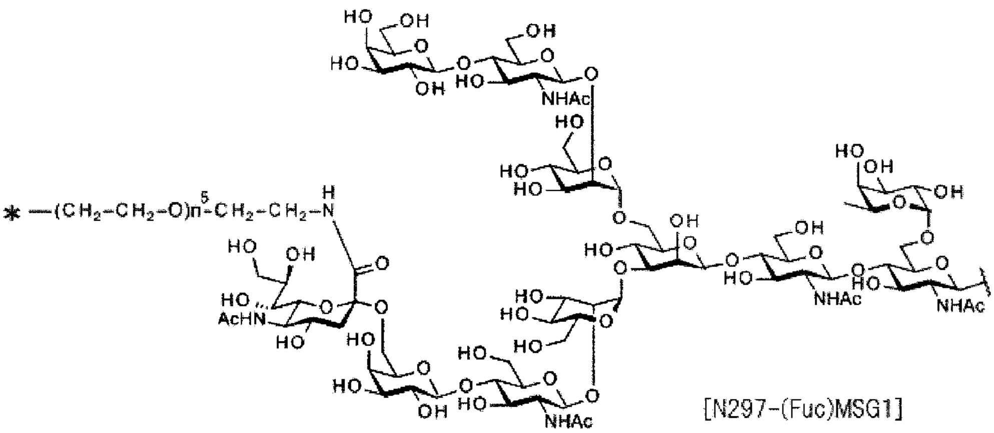

[N297-(Fuc)MSG1]

[Chem. 46]

Fucα1
|
6

Galβ1-4GlcNAcβ1-2Manα1— 6
Manβ1-4GlcNAcβ1-4GlcNAcβ1—
* —L(PEG)-NeuAcα2-6Galβ1-4GlcNAcβ1-2Manα1— 3

[N297-(Fuc)MSG1]

wherein the wavy lines represent a bond to Asn297 of the antibody,
L (PEG) represents $-(CH_2CH_2-O)n^5-CH_2CH_2-NH-$,
it is indicated that the amino group at the right end is bound via amide bond to the carboxylic acid at the 2-position of a sialic acid at the non-reducing terminus of the 1-3 branched chain of β-Man of the N297 glycan, and

* represents a bond to the linker L, in particular, a nitrogen atom at the 1- or 3-position on the 1,2,3-triazole ring of Lb in the linker L,

wherein $n^5$ is an integer from 2 to 10, preferably from 2 to 5, more preferably from 2 to 4, further more preferably 3 or 4, and the most preferably 3.

N297-(Fuc)MSG2 is represented by the following structural formula or sequence formula:

[Chem. 47]

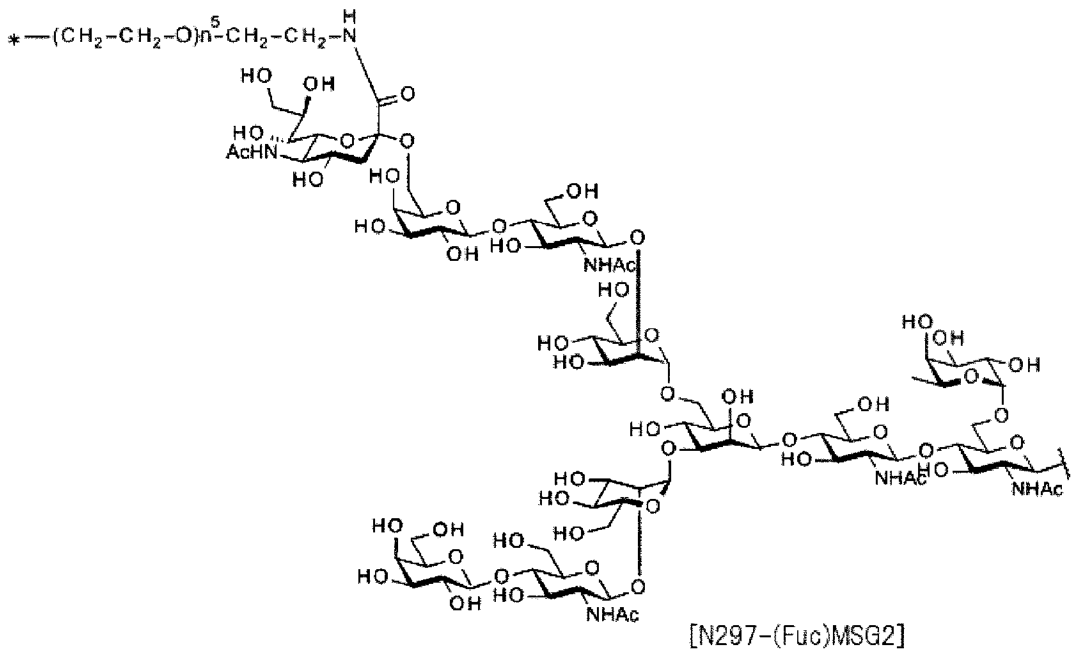

[N297-(Fuc)MSG2]

[Chem. 48]

Fucα1
|
6
* - L(PEG)-NeuAcα2-6Galβ1-4GlcNAcβ1-2Manα1 — 6
Manβ1-4GlcNAcβ1-4GlcNAcβ1-
Galβ1-4GlcNAcβ1-2Manα1 — 3

[N297-(Fuc)MSG2]

wherein the wavy lines represent a bond to Asn297 of the antibody,

L (PEG) represents $-(CH_2CH_2\text{-}O)n^5\text{-}CH_2CH_2\text{-}NH\text{-}$,

it is indicated that the amino group at the right end is bound via amide bond to the carboxylic acid at the 2-position of a sialic acid at the non-reducing terminus of the 1-6 branched chain of β-Man of the N297 glycan, and

* represents a bond to the linker L, in particular, a nitrogen atom at the 1- or 3-position on the 1,2,3-triazole ring of Lb in the linker L,

wherein $n^5$ is an integer from 2 to 10, preferably from 2 to 5, more preferably from 2 to 4, further more preferably 3 or 4, and the most preferably 3.

N297-(Fuc)SG is represented by the following structural formula or sequence formula:

[Chem. 49]

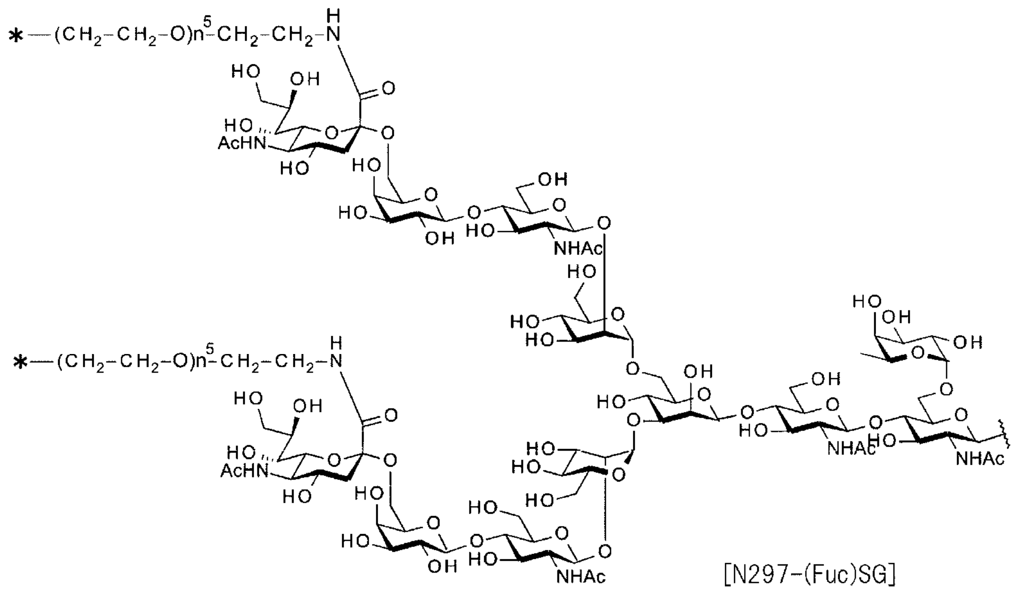

[N297-(Fuc)SG]

[Chem. 50]

Fucα1
|
6

*- L(PEG)-NeuAcα2-6Galβ1-4GlcNAcβ1-2Manα1 — 6
Manβ1-4GlcNAcβ1-4GlcNAcβ1—
*- L(PEG)-NeuAcα2-6Galβ1-4GlcNAcβ1-2Manα1 — 3

[N297-(Fuc)SG]

wherein the wavy lines represent a bond to Asn297 of the antibody,
L(PEG) represents $-(CH_2CH_2-O)n^5-CH_2CH_2-NH-$,
it is indicated that the amino groups at the right ends are bound via amide bond to the carboxylic acids at the 2-positions of sialic acids at the non-reducing termini of the 1-3 and 1-6 branched chains of β-Man of the N297 glycan, and
* represents a bond to the linker L, in particular, a nitrogen atom at the 1 or 3-position on the 1,2,3-triazole ring of Lb in the linker L,
wherein $n^5$ is an integer from 2 to 10, preferably from 2 to 5, more preferably from 2 to 4, further more preferably 3 or 4, and the most preferably 3.

When the N297 glycan of the antibody in the antibody-drug conjugate of the present invention is N297-(Fuc)SG, the heavy chain of the antibody forms a dimer, and therefore, the antibody-drug conjugate is a molecule to which 4 linkers L and 4 drugs D are bound (the above $m^2$=2). In other words, the number of the drugs D bound per heavy chain in the antibody-drug conjugate is 2. On the other hand, when the N297 glycan of the antibody in the antibody-drug conjugate of the present invention is N297-(Fuc)MSG1 or N297-(Fuc)MSG2, or a mixture thereof, the heavy chain of the antibody forms a dimer, and therefore, the antibody-drug conjugate is a molecule to which 2 linkers L and 2 drugs D are bound (the above $m^2$=1). In other words, the number of the drug D bound per heavy chain in the antibody-drug conjugate is 1.

<Preparation of Glycan-Remodeled Antibody>

The glycan-remodeled antibody of the present invention can be produced by a method shown in the following formula in accordance with the methods described in International Publication Nos. 2013/120066, 2022/050300, or 2023/167238, for example.

[Chem. 51]

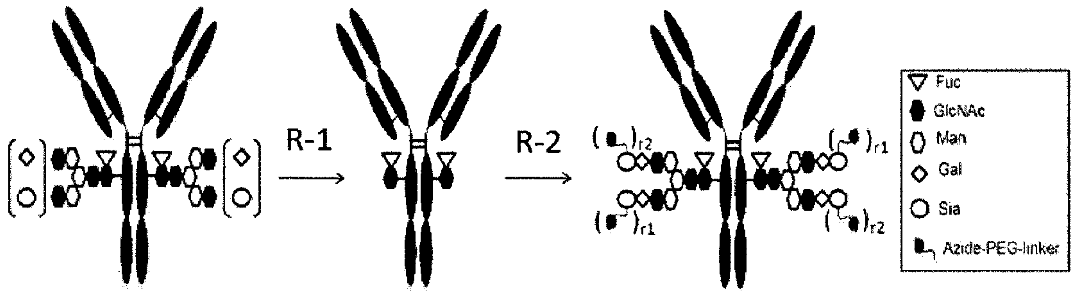


(r1,r2) = any of (1,0), (0,1), (1,1)

Step R-1: Hydrolysis of Glycosidic Bond between GlcNAcβ1-4GlcNAc in Chitobiose structure at Reducing Terminus

This step is a step of preparing a glycan-cleaved antibody by cleaving the N-linked glycan (N297-linked glycan) bound to asparagine at the 297-position of the amino acid sequence of the antibody heavy chain of an antibody of interest through a known enzymatic reaction.

For example, an antibody of interest (20 mg/ml) in a buffer solution (50 mM phosphate buffer solution or the like) is subjected to hydrolysis reaction of the glycosidic bond between GlcNAcβ1 and 4GlcNAc in the chitobiose structure at the reducing terminus using hydrolase such as the enzyme EndoS at 0°C to 40°C. The reaction time ranges from 10 minutes to 72 hours, and preferably from 1 to 6 hours. The wild-type enzyme EndoS is used in an amount of 0.1 to 10 mg, and preferably 0.1 to 3 mg, per 100 mg of the antibody. After completion of the reaction, a (Fucα1,6)GlcNAc antibody can be produced in which the glycan between GlcNAcβ1 and 4GlcNAc has been hydrolyzed by performing affinity chromatography purification and/or hydroxyapatite column purification to be described later.

In addition to this, for example, there is a step of producing a glycan-cleaved antibody by cleaving the glycosidic bond between GlcNAcβ1-4GlcNAc in the chitobiose structure at the reducing terminus of the N-linked glycan (N297-linked glycan) bound to asparagine at the 297-position of the amino acid sequence of the antibody, in which an antibody of interest (10 mg/mL) in a buffer solution (phosphate buffer solution or the like) can be subjected to hydrolysis reaction of the glycosidic bond between GlcNAcβ1 and 4GlcNAc in the chitobiose structure at the reducing terminus using hydrolase such as a wild-type enzyme EndoS at 0°C to 40°C. The reaction time ranges from 10 minutes to 72 hours, and preferably from 1 to 6 hours. The wild-type enzyme EndoS is used in an amount of 0.1 to 10 mg, and preferably 0.1 to 3 mg, per 100 mg of the antibody, and after completion of the reaction, a (Fucα1,6)GlcNAc antibody can be produced by purifying through affinity chromatography (HiTrap rProtein A FF (5 ml) (manufactured by GE Healthcare Inc.)) and/or hydroxyapatite column (Bio-Scale Mini CHT Type I cartridge (5 ml) (manufactured by BIO-RAD LABORATORIES, INC.)). Various hydrolases can be used including Endo-Si WT, Endo-A, Endo-D, Endo-E, Endo-F3, Endo-H, Endo-S, Endo-S2, Endo-Si.

Step R-2: Transglycosylation Reaction

This step is a step of producing a glycan-remodeled antibody by binding the above-described (Fucα1,6)GlcNAc antibody to MSG- (MSG1-, MSG2-) or SG-type glycan oxazoline body (hereinafter, "azide glycan oxazoline body") having a PEG linker containing an azide group through enzymatic reaction.

For example, the transglycosylation reaction is performed by reacting the above-described glycan-cleaved antibody with an azide glycan oxazoline body in a buffer solution (phosphate buffer solution or the like) in the presence of glycosyltransferase such as EndoS (D233Q/Q303L) in a catalytic amount at 0°C to 40°C. The reaction time ranges from 10 minutes to 72 hours, and preferably from 1 to 6 hours. The enzyme EndoS (D233Q/Q303L) is used in an amount of 1 to 10 mg, and preferably 1 to 3 mg, per 100 mg of the antibody, and the azide glycan oxazoline body is used in an amount of 2 equivalents to an excess equivalent, and preferably 2 to 20 equivalents.

After completion of the reaction, a purified glycan-remodeled antibody can be obtained by performing affinity chromatography purification and hydroxyapatite column purification.

Accordingly, in an aspect of the present invention, provided is a method for producing a glycan-remodeled antibody, including the following steps:

i) producing one of the antibody or an antigen-binding fragment of the antibody of the present invention, such as the antibody or an antigen-binding fragment of the antibody described in any one of [1] to [16] and [24] to [28] of the paragraph [0007] herein,

ii) treating the antibody obtained in i) with hydrolase to produce a (Fucα1,6)GlcNAc-antibody, and

iii)-1 reacting in the presence of glycosyltransferase, the (Fucα1,6)GlcNAc-antibody and a glycan donner molecule obtained by introducing a PEG linker having an azide group to the carbonyl group of the carboxylic acid at the 2-position of a sialic acid in MSG (9) or SG (10) and oxazolinating the reducing terminus, or

iii)-2 reacting in the presence of glycosyltransferase, the (Fucα1,6)GlcNAc-antibody and a glycan donner molecule obtained by introducing a PEG linker having an azide group to the carbonyl group of the carboxylic acid at the 2-position of a sialic acid in (MSG-)Asn or (SG-)Asn in which an α-amino group is optionally protected and to the carbonyl group of the carboxylic acid in the Asn, allowing hydrolase to act on the resulting molecule, and then oxazolinating the reducing terminus.

The azide glycan oxazoline body can be prepared according to a known method or by applying a known method, and for example, according to the method described in Example 5. Using a reaction known in the organic synthesis science field (condensation reaction or the like), $N_3$-$(CH_2CH_2$-$O)n_5$ -$CH_2CH_2$-$NH_2$, which is a PEG linker containing an azide group, ($N_3$-L(PEG)), can be introduced into MSG1. In other words, a carboxylic acid at the 2-position of a sialic acid and an amino group at the right end of $N_3$-$(CH_2CH_2$-$O)n_5$-$CH_2CH_2$-$NH_2$ are subjected to condensation reaction to form an amide bond.

Examples of the condensing agent when using condensation reaction include, but are not limited to, N,N'-dicyclohexylcarbodiimide (DCC), 1-ethyl-3-(3-dimethylaminopropyl)carbodiimide (EDCI), carbonyldiimidazole (CDI), 2-(2H-benzotriazol-2-yl)-4-(1,1,3,3-tetramethylbutyl)phenol (BOP), 1H-benzotriazole-1-yloxytripyrrolidinophosphonium hexafluorophosphate (PyBOP), and O-(7-azabenzotriazol-1-yl)-N,N,N',N'-tetramethyluronium hexafluorophosphate (HATU), and examples of the solvent used in reaction include, but are not limited to, dichloromethane, DMF, THF, ethyl acetate, and a mixed solvent thereof.

The reaction temperature usually ranges from -20°C to 100°C or the boiling point of the solvent, and preferably from -5°C to 50°C. Also, an organic base such as triethylamine, diisopropylethylamine, N-methylmorpholine and 4-dimethylaminopyridine, or an inorganic base such as potassium carbonate, sodium carbonate, potassium hydrogen carbonate, and sodium hydrogen carbonate can be added, as necessary. Further, 1-hydroxybenzotriazole, N-hydroxysuccinimide or the like can be added as a reaction accelerator.

Note that MSG1 can be obtained by hydrolyzing a separated and purified (MSG1-)Asn with hydrolase such as EndoM.

Oxazolination can be performed from GlcNAc at the reducing terminus of MSG1 according to a known document (J. Org Chem., 2009, 74(5), 2210-2212, Helv. Chim. Acta, 2012, 95, 1928-1936.).

As the enzyme used in the hydrolysis reaction of the N297 glycan, EndoS or mutant enzyme that retains its hydrolysis activity.

The (Fucα1,6)GlcNAc-antibody obtained through the above hydrolysis can be used as a glycan acceptor molecule and reacted with an MSG- (MSG1-, MSG2-) or SG-type glycan donner molecule using glycosyltransferase such as EndoS D233Q or EndoS D233Q/Q303L mutant (International Publication No. 2017/010559, and the like) to obtain an antibody having an MSG- (MSG1-, MSG2-) or SG-type N297 glycan composed of the above structure.

When the number $m^2$ of the drug bound per antibody heavy chain in the antibody-drug conjugate is 1, a glycan donner molecule having MSG, MSG1 or MSG2 as the glycan is adopted. Such a glycan can be adopted by separating (MSG1-)Asn or (MSG2-)Asn from commercially available monosialo-Asn free (1S2G/1G2S-10NC-Asn, manufactured by GlyTech, Inc.; hereinafter, referred to as "(MSG-)Asn") as a raw material, in accordance with the method described in Examples herein, or can be adopted as a mixture without separation.

When the number $m^2$ of the drug bound per antibody heavy chain in the antibody-drug conjugate is 2, a glycan donner molecule having SG(10) as the glycan is adopted for this transglycosylation reaction. As such an SG(10) glycan, for example, one obtained through hydrolysis or the like from SGP may be used, or a commercially available SG(10) glycan such as disialooctasaccharide (Tokyo Chemical Industry Co., Ltd.) may be used.

The MSG- (MSG1-, MSG2-) or SG-type glycan contained in the donner molecule has a PEG linker ($N_3$-L(PEG)) containing an azide group at the 2-position of its sialic acid. Introduction of a PEG linker ($N_3$-L(PEG)) containing an azide group at the 2-position of its sialic acid can be carried out by reacting MSG (MSG(9)), MSG1 or MSG2, or disialooctasaccharide (SG(10)) with $N_3$-$(CH_2CH_2$-$O)n_5$ -$CH_2CH_2$-$NH_2$, which is a PEG linker ($N_3$-L(PEG)) containing an azide group, ($n_5$ is an integer from 2 to 10, preferably from 2 to 5, more preferably from 2 to 4, further more preferably 3 or 4, and the most preferably 3) through a reaction known in the organic synthesis science field (condensation reaction or the like). In other words, a carboxylic acid at the 2-position of a sialic acid and an amino group at the right end of $N_3$-$(CH_2CH_2$-$O)n_3$-$CH_2CH_2$-$NH_2$ are subjected to condensation reaction to form an amide bond.

The MSG- (MSG1-, MSG2-) or SG-type glycan can also be obtained by, for example, introducing a PEG linker ($N_3$-$(CH_2CH_2$-$O)n_3$-$CH_2CH_2$-$NH_2$) containing an azide group into the carboxylic acid at the 2-position of a sialic acid, which is a raw material, such as (MSG1-)Asn, (MSG2-)Asn, or (SG-)Asn (GlyTech, Inc.) in which an α-amino group is optionally protected or modified, and the carboxylic acid in the Asn, through condensation reaction, allowing hydrolase to act on the

resulting molecule such as EndoM and EndoRp. Examples of the protecting group for the α-amino group include, but are not limited to, an acetyl (Ac) group, t-butoxycarbonyl (Boc) group, a benzoyl (Bz) group, a benzyl (Bzl) group, carbobenzoxy (Cbz) group, and 9-fluorenylmethoxycarbonyl (Fmoc) group. The protecting group for the α-amino group is preferably a Fmoc group.

Examples of the modifying group for the α-amino group can include a hydroxyacetyl group, or a modifying group with the PEG structure that improves water solubility.

In (MSG1-)Asn, (MSG2-)Asn, or (SG-)Asn, the α-amino group is preferably protected by the above-described protecting group. When the α-amino group is protected by a protecting group (e.g., Fmoc group), the protecting group can be removed after introducing a PEG linker having an azide group and before allowing hydrolase to act on the resulting molecule, as necessary.

As GlcNAc at the reducing terminus of MSG- (MSG1-, MSG2-) or SG-type glycan contained in a molecule, it is preferred to use one activated by such an oxazolination with, for example, 2-chloro-1,3-dimethyl-1H-benzimidazol-3-ium-chloride treatment.

Various enzymes used for transglycosylation reaction (glycosyltransferase) can be adopted as long as they have activity to transfer a complex-type glycan to the N297 glycan, and a preferred enzyme is EndoS D233Q, a modified body that suppresses hydrolysis reaction by substituting ASP at the 233-position of EndoS with Gln. The transglycosylation reaction using EndoS D233Q is described in International Publication No. 2013/120066, and the like. In addition, a modified enzyme such as EndoS D233Q/Q303L (International Publication No. 2017/010559), which has further been mutated to EndoS D233Q can be used.

Step R-3: Transglycosylation Reaction Other Than R-2 (One-Pot Method)

This is a step of producing a glycan-remodeled antibody of an aspect in which a glycan-remodeled antibody is produced other than the step R-2, through enzymatic reaction on the (Fucα1,6)GlcNAc antibody obtained in the above step R-1. This step is a step of producing a glycan-remodeled antibody through transglycosylation reaction on the (Fucα1,6)GlcNAc-antibody obtained in the above step by using two endo-β-N-acetylglucosaminidases. Simultaneous use of two enzymes allows a glycan donner molecule whose reducing terminus is not activated to directly undergo transglycosylation to the N297 glycan of the antibody. Regarding the two endo-β-N-acetylglucosaminidases to be used, one endo-β-N-acetylglucosaminidase that uses the N297-linked glycan of an Fc region-containing molecule (e.g., antibody) as a substrate (enzyme A) and another endo-β-N-acetylglucosaminidase that uses a complex-type glycan of the glycan donner molecule as a substrate but does not use the N297-linked glycan as a substrate (enzyme B) coexisted to bind the glycan of the donner molecule to a core GlcNAc residue of the glycan-cleaved IgG or Fc region-containing molecule, which is an acceptor molecule. The enzyme A generally contains a domain with high affinity to the Fc-containing molecule, a domain with high affinity to the glycan, and a transglycosylation reaction catalytic domain, and typically has both hydrolytic activity and transglycosylation activity using the N297-linked glycan of the Fc-containing molecule (e.g., antibody) as a substrate. Since what is important in the present invention is the activity of transferring a glycan in a glycan donner molecule (especially a glycan activated by a chemical or enzymological approach) into an acceptor molecule, the hydrolytic activity may be attenuated or disappeared. Rather, an enzyme that retains high hydrolytic activity is likely to hydrolyze even the glycan transferred to the core GlcNAc of the acceptor molecule as a substrate through the transglycosylation activity, and hence, it is preferred that the hydrolytic activity of the enzyme A in the present invention be relatively reduced. In an aspect of the present invention, the enzyme A has an activity capable of acting on the glycan of the Fc-containing molecule (especially, transglycosylation activity using the N297-linked glycan of the Fc-containing molecule as a substrate). Also, in another aspect of the present invention, the enzyme A has an activity capable of acting on the glycan of the Fc-containing molecule, and retains or has lost the hydrolytic activity. The transglycosylation activity of the enzyme A refers to an activity of binding, via glycosidic bonds, the reducing terminus of the above glycan donner molecule (glycan-containing molecule having GlcNAc whose reducing terminus is activated or GlcNAc whose reducing terminus is not activated) to an acceptor molecule containing an Fc moiety that only has core GlcNAc (to which core fucose may or may not be added) at N297. In an aspect, the enzyme A is a mutant enzyme of Endo-S, EndoS-2, Endo-Si, Endo-Sd, Endo-Se, or Endo-Sz, and having hydrolytic activity lower than that of the corresponding wild-type enzyme. Specific examples thereof can include Endo-S D233Q, Endo-S D233Q/Q303L, Endo-S D233Q/E350A, Endo-S D233Q/E350Q, Endo-S D233Q/E350D, Endo-S D233Q/E350N, Endo-S D233Q/D405A, Endo-Si D241Q, Endo-Si D241Q/Q311L, Endo-Si D241Q/E360Q, Endo-Si D241M, Endo-Si D241M/Q311L, Endo-Si D241M/E360Q, Endo-Si T190Q, Endo-Si T190/D241Q, Endo-Si T190Q/D241M, Endo-S2 D184M, Endo-S2 T138Q, Endo-S2 D184Q, Endo-S2 D184Q/Q250L, Endo-S2 D184Q/E289Q, Endo-S2 D184M/Q250L, Endo-S2 D184M/E289Q, Endo-S2 D182Q, Endo-S2 D226Q, Endo-S2 T227Q, Endo-S2 T228Q.

Also, the enzyme B used herein shows a high transglycosylation efficiency in the one-pot method when the enzyme B has the activity of transglycosylation from a glycan donner to an acceptor having GlcNAc. In other words, the enzyme B can be selected from endo-β-N-acetylglucosaminidases that uses as a substrate a complex-type glycan of the

glycan donner molecule whose reducing terminus is not activated but does not use the N297-linked glycan as a substrate, using the activity of transglycosylation to an acceptor having GlcNAc as the index. Accordingly, in an aspect of the present invention, the enzyme-B is an enzyme having the activity of transglycosylation from SGP to a GlcNAc derivative.

The activating action on the glycan donner molecule by the enzyme-B is to act on the glycan donner molecule whose reducing terminus is not activated and which contains GlcNAc to generate an activated intermediate in which the reducing terminus becomes available for transglycosylation. However, when the hydrolytic activity by the enzyme-B is high, the activated intermediate may react with $H_2O$ molecules present in the reaction system, not reacting with the acceptor molecule via the enzyme-A, leading to generation of a large number of free glycans. Therefore, it is preferred that the enzyme-B in the present invention have relatively reduced hydrolytic activity, compared to the action of glycan activation.

Examples of the enzyme-B of the present invention include Endo-M (enzyme derived from Mucor hiemalis) (see Yamamoto K, et al., Biochem Biophys Res Commun. 1994, 203, 244-252, and Umekawa M, et al., J. Biol Chem. 2021, 285, 511-521), Endo-CC (enzyme derived from Coprinopsis cinerea) (see Eshima Y, et al., PLoS One. 2015, 10, e0132859), Endo-Om (enzyme derived from Ogataea minuta) (see Murakami S, et al., Glycobiology. 2013, 23, 736-744), Endo-Rp (enzyme derived from Rhizomucor pusillus) (WO2018101454), or mutant enzymes thereof (preferably mutant enzymes having reduced hydrolytic activity compared to the wild type). In an aspect of the present invention, the enzyme-B is a mutant enzyme of Endo-M, Endo-Om, Endo-CC, or Endo-Rp, and having hydrolytic activity lower than that of the corresponding wild-type enzyme. Particularly preferred examples thereof can include, but are not limited to, enzymes selected from the group consisting of Endo-Rp N172Q, Endo-Rp N172H, Endo-Rp N172A, Endo-Rp N172C, Endo-Rp N172D, Endo-Rp N172E, Endo-Rp N172G, Endo-Rp N172I, Endo-Rp N172L, Endo-Rp N172M, Endo-Rp N172P, Endo-Rp N172S, Endo-Rp N172T, Endo-Rp N172V, Endo-Rp W278F/S216V, Endo-Rp W278F/N246D, Endo-Rp W278F/D276N, Endo-Rp W278F/A310D, Endo-Rp W278F/N172D/F307Y, Endo-Rp W278F/N172D/F307H, Endo-Rp W278F/N172D/A310D, Endo-Rp W214F/F307Y/L306I, Endo-M N175Q, Endo-M N175Q/Y217F, Endo-CC N180H, and Endo-Om N194Q. Also, the enzyme-B may have further mutations added, in addition to the above-mentioned particular mutations. For example, referring to the Patent Literature (International Publication No. 2022/050300) or the like, the enzyme-B may have 1 to several amino acid residues that have been substituted, deleted, inserted, and/or added to the extent that the enzyme activity is not affected. Further, examples of the amino acid sequence of the enzyme-B include amino acid sequences having at least 80% or more, preferably 85% or more, more preferably 90% or more, and even more preferably 95%, 96%, 97%, 98%, or 99% or more homology or identity to amino acid residues other than the particular mutated amino acid residues, referring to the Patent Literature (International Publication No. 2022/050300) or the like.

([$N_3$-PEG(3)]$_2$-SG)-Asn-PEG(3)-$N_3$, ([$N_3$-PEG(3)]-MSG1-)Asn-PEG(3)-$N_3$, [$N_3$-PEG(3)]-MSG2-Asn-PEG(3)-$N_3$, or the like can be used as the glycan donner.

The transglycosylation reaction can be performed by reacting an antibody with a glycan donner such as ([$N_3$-PEG(3)]-MSG1-)Asn-PEG(3)-$N_3$ in a buffer solution (Tris buffer solution or the like) in the presence of the enzymes A and B. The reaction temperature can be appropriately selected depending on the optimum temperature of the enzyme to be used, and can be appropriately selected between 4°C and 50°C, but is preferably from 15°C to 45°C, more preferably from 20°C to 40°C, and even more preferably from 25°C to 40°C. The reaction time can be appropriately selected between 10 minutes and 96 hours, preferably from 0.5 to 80 hours, more preferably from 2 to 70 hours, even more preferably from 4 to 60 hours, particularly preferably from 6 to 48 hours, and the most preferably from 8 to 30 hours.

After completion of the reaction, a purification method (affinity chromatography, hydroxyapatite column, or the like) or an ultrafiltration method (ultrafiltration membrane) suitable for the reaction scale, or a method of bringing into contact with a cation exchange chromatography medium or multi-mode chromatography medium under the acidic conditions is selected, referring to the Patent Literature (International Publication No. 2023/167238) or the like, to obtain a glycan-remodeled antibody.

In the preparation of the above-described glycan-remodeled antibody, the concentration of, concentration measurement of, and buffer exchange to an aqueous antibody solution can be carried out according to the following common operations A to C.

Common Operation A: Concentration of Aqueous Antibody Solution

In a container Amicon Ultra (30,000 to 50,000 MWCO, Millipore Co.), a solution of an antibody or an antibody-drug conjugate, which will be described later, was placed and subjected to centrifugation (2000 G to 4000G) with a centrifuge (Allegra X-15R, Beckman Coulter, Inc.) to concentrate the solution of an antibody or antibody-drug conjugate.

Common Operation B: Measurement of Antibody Concentration

Measurement was performed by using a UV measuring device (DeNovix (registered trademark) DS-11 Spectrophotometer, DeNovix Inc.) according to the method specified by the manufacturer.

At that time, a different 280 nm absorption coefficient was used for each antibody (1.3 mL $mg^{-1}$ $cm^{-1}$ to 1.8 mL $mg^{-1}$ $cm^{-1}$).

Common Operation C-1: Buffer Exchange for Antibody

A buffer solution such as 50 mM phosphate buffer solution (pH 6.0) (hereinafter, PB6.0) or 5 mM ethylenediaminetetraacetic acid-phosphate buffered saline (hereinafter, PBS 5 mM EDTA) was added to a solution of an antibody, and the solution was concentrated using Amicon Ultra (common operation A). After the operation was performed several times, the antibody concentration was measured (common operation B) and adjusted with a buffer solution.

Common Operation C-2: Buffer Exchange for Antibody

Using a buffer solution such as PBS 5 mM EDTA or PB6.0, a NAP-25 column (Cat. 17085202, Cytiva, Inc.) was equilibrated with a Sephadex (trademark) G-25 carrier. 2.5 mL aqueous solution of an antibody was loaded on each NAP-25 column, and a fraction (3.5 mL) eluted with 3.5 mL buffer solution was then separated and collected. The antibody concentration was measured (common operation B) and appropriately adjusted by, for example, adding a buffer solution.

<Conjugation>

In an aspect of the present invention, provided are a method for producing an antibody-drug conjugate comprising reacting a glycan-remodeled antibody obtained by the above-described method with a drug linker, and an antibody-drug conjugate produced by the method. Hereinafter, a method for producing an antibody-drug conjugate will be described, in which the above-described glycan-remodeled antibody was bound to a production intermediate (2) shown below through SPAAC (strain-promoted alkyne azide cycloaddition: J. AM. CHEM. SOC. 2004, 126, 15046-15047) reaction.

[Chem. 52]

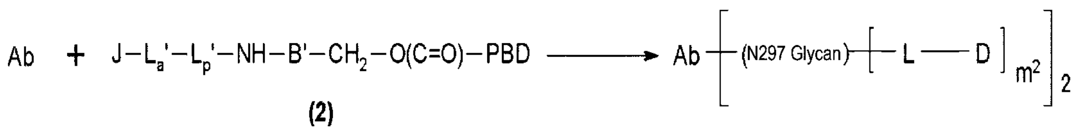

(2)

wherein Ab represents a glycan-remodeled antibody,
La', Lp', and B' are synonymous with La, Lp, and B, and
J represents any of the following structural formulas,

[Chem. 53]

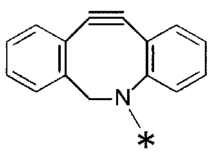

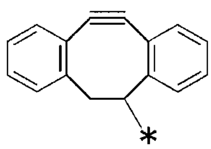

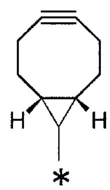

wherein the asterisks represent a bond to La'.

J-La'-Lp'-NH-B'-CH2-O(C=O)-PBD can be prepared according to a known method or by applying a known method, and for example, can be synthesized by the method described in Example 5 or the like.

SPAAC reaction is allowed to proceed by mixing a buffer solution (sodium acetate solution, sodium phosphate, sodium borate solution, or the like, or a mixture thereof) of the antibody Ab, and a solution obtained by dissolving a compound (2) in an appropriate solvent (dimethylsulfoxide (DMSO), dimethylformamide (DMF), dimethylacetamide (DMA), N-methyl-2-pyridone (NMP), propylene glycol (PG), or the like, or a mixture thereof).

The compound (2) can be used in an amount of 2 moles to an excess amount of moles, and preferably 1 mole to 30 moles per mole of the antibody, and the ratio of the organic solvent is preferably 1 to 200% v/v to the buffer solution of the

antibody. The reaction temperature ranges from 0 to 37°C, and preferably from 10 to 25°C, and the reaction time ranges from 1 to 150 hours, and preferably from 6 to 100 hours. The pH during the reaction is preferably 5 to 9.

Antibody-drug conjugate compounds (ADCs) can be identified by measuring the buffer exchange, purification, antibody concentration, and average number of drug bound per antibody molecule (hereinafter, referred to as drug to antibody ratio (DAR)) of each antibody-drug conjugate by the common operations A through C described above and common operations D through F described later.

Common Operation D: Purification of Antibody-Drug Conjugate

A NAP-25 column was equilibrated with any buffer solution of commercially available acetate buffer solutions (10 mM, pH 5.5; hereinafter, referred to as ABS) containing Sorbitol (5%). On this NAP-25 column, an aqueous reaction solution of an antibody-drug conjugate (about 1.5 to 2.5 mL) was loaded and eluted with the buffer solution in an amount specified by the manufacturer to separate and collect an antibody fraction. This fraction separated and collected was again loaded on the NAP-25 column, and a gel filtration purification operation to elute with a buffer solution was repeated twice or three times in total to obtain an antibody-drug conjugate in which an unbound drug linker, dimethylsulfoxide, and propylene glycol have been removed. The concentration of the antibody-drug conjugate solution was adjusted through common operations A and C.

Common Operation E: Measurement of Antibody Concentration of Antibody-Drug Conjugate

The concentration C' (mg/mL) of the antibody-drug conjugate was calculated using the formula (I) which can be derived from the Lambert-Beer's law, absorbance A280 = molar absorption coefficient ε280 (L·mol-1 cm-1) × molar concentration C (mol·L-1) × cell optical path length l (cm).

[Math 1]

$$C'(mg\cdot mL^{-1}) = MW(g\cdot mol^{-1})\cdot C(mol\cdot L^{-1}) = \frac{A_{280}\cdot MW(g\cdot mol^{-1})}{\varepsilon_{280}(L\cdot mol^{-1}\cdot cm^{-1})\cdot l(cm)} \quad \text{Expression (I)}$$

Each value used in the calculation of C' (mg/mL) was obtained as follows.

Absorbance A280: a measured value of UV absorbance of an aqueous solution of an antibody-drug conjugate at 280 nm

Measured using Lamba 25 (Perkin Elmer)

MW ($g\cdot mol^{-1}$): a calculated estimated value of the molecular weight of an antibody determined from the amino acid sequence of the antibody (used as an approximate value of the molar mass of an antibody-drug conjugate).

Optical path length l (cm): 1 cm

Molar absorption coefficient ε280 of an antibody-drug conjugate: calculated using the following expression (II)

[Math 2]

$$\varepsilon_{280} = \varepsilon_{Ab,280} + \varepsilon_{DL,280} \times DAR \quad \text{Expression (II)}$$

$\varepsilon_{Ab,\ 280}$: a molar absorption coefficient of an antibody at 280 nm

$\varepsilon_{DL,\ 280}$: a molar absorption coefficient of a drug linker at 280 nm

DAR: calculated in the common operation F (see below)

For $\varepsilon_{Ab,\ 280}$, a value calculated by using a known calculation method (Protein Science, 1995, vol. 4, 2411-2423) was used (Table 1). For the absorption coefficient of huM25 used in the reference example, a value calculated by using SEQ ID NOs: 68 and 70 was used. For $\varepsilon_{DL,\ 280}$, a measured molar absorption coefficient of a drug linker (280 nm) was used.

[Table 1]

Table 1: Molar absorption coefficients, molecular weight of antibodies

| | ε Ab, 280 (whole) | Light chain | Heavy chain | Molecular weight |
|---|---|---|---|---|
| H02L01 | 238300 | 31650 | 82968 | 147600 |
| H02L01-V1 | 235320 | 36182 | 81478 | 147530 |
| H02L01-V2 | 235320 | 36182 | 81478 | 147586 |
| Control hIgG | 230280 | 29192 | 85948 | 145908 |

(continued)

| Table 1: Molar absorption coefficients, molecular weight of antibodies | | | | |
|---|---|---|---|---|
| | ε Ab, 280 (whole) | Light chain | Heavy chain | Molecular weight |
| huM25 | 234400 | 31712 | 85488 | 145734 |

Common Operation F: Measurement of DAR per Antibody Molecule in Antibody-Drug Conjugate

DAR can be determined through high performance liquid chromatography (HPLC) analysis, or reversed-phase chromatography (hereinafter, RPC) according to the method shown below.

(F-1. Preparation of Sample for HPLC Analysis (Reduction of Antibody-Drug Conjugate))

A solution of an antibody-drug conjugate (about 1 mg/mL, 60 μL) is mixed with an aqueous solution of dithiothreitol (DTT) (100 mM, 15 μL). The mixture is incubated at 37°C for 30 minutes to prepare a sample in which the disulfide bond between the L chain and H chain of the antibody-drug conjugate has been cleaved, and the sample is used for HPLC analysis.

(F-2. HPLC Analysis)

HPLC analysis is carried out under the following measurement conditions.
HPLC system: Agilent 1290 HPLC system (Agilent Technologies, Inc.)
Detector: ultraviolet absorption spectrometer (measurement wavelength: 280 nm, 329 nm)
Column: BEH Phenyl (2.1 × 50 mm, 1.7 μm, Waters Acquity)
Column temperature: 75°C
Mobile phase A: 0.1% trifluoroacetic acid (TFA), 15% isopropyl alcohol aqueous solution
Mobile phase B: 0.075% TFA, 15% isopropyl alcohol acetonitrile solution
Gradient program: 14%-36% (0 to 15 minutes), 36%-80% (15 to 17 minutes), 80%-14% (17 to 17.1 minutes), 14%-14% (17.1 to 23 minutes)
Amount of sample infused: 10 μL

(F-3. Data Analysis)

(F-3-1) Since an L-chain with a drug bound (L chain to which one drug is bound; $L_1$) and an H chain with a drug bound (H chain to which one drug is bound; $H_1$, H chain to which two drugs are bound; $H_2$, H chain to which three drugs are bound; $H_3$) have hydrophobicity and retention time increased in proportion of the number of drugs bound, compared to an L chain ($L_0$) and H chain ($H_0$) to which no drug is bound, $L_0$, $L_1$, $H_0$, $H_1$, $H_2$, and the like are eluted in this order. Therefore, the detection peak can be assigned to any of $L_0$, $L_1$, $H_0$, $H_1$, $H_2$, and $H_3$ through comparison of the retention time to $L_0$ and $H_0$. Also, whether a drug is bound can be confirmed by the absorption at a wavelength of 329 nm, which is characteristic of the drug.

(F-3-2) Since the drug linker absorbs UV, peak area values are corrected by using the following expression with the molar absorption coefficients of the light chain (L chain), heavy chain (H chain), and drug linker according to the number of drug linkers bound. wherein i and j represent integers, and integers of 0 or 1 for i and 0 to 3 for j are used herein, but not limited to these, and any integer (the number of drugs bound) can be applied.

[Math 3]

$$\text{Corrected value of L-chain peak area } (A_{Li}) \ (i = 0, 1) = \text{Peak area} \times \frac{\text{Molar absorption coefficient of L-chain}}{\text{Molar absorption coefficient of L-chain} + \text{Number of conjugated drug} \times \text{Molar absorption coefficient of drug linker}}$$

$$\text{Corrected value of H-chain peak area } (A_{Hj}) \ (j = 0, 1, 2, 3) = \text{Peak area} \times \frac{\text{Molar absorption coefficient of H-chain}}{\text{Molar absorption coefficient of H-chain} + \text{Number of conjugated drug} \times \text{Molar absorption coefficient of drug linker}}$$

Here, for the molar absorption coefficients (280 nm) of the L chain and H chain in each antibody, the values listed in Table 1 were used.

(F-3-3) The peak area ratio (%) of each chain to the total of corrected peak area values is calculated according to the following expressions.

[Math 4]

$$\text{Li peak area ratio } (i = 0, 1) = \frac{A_{Li}}{A_{L0} + A_{L1}} \times 100$$

$$\text{Hj peak area ratio } (j = 0, 1, 2, 3) = \frac{A_{Hj}}{A_{H0} + A_{H1} + A_{H2} + A_{H3}} \times 100$$

(F-3-4) DAR per antibody molecule in the antibody-drug conjugate is calculated according to the following expression.

$$DAR = (L_0 \text{ peak area ratio} \times 0 + L_1 \text{ peak area ratio} \times 1) \times 2 + (H_0 \text{ peak area ratio} \times 0 + H_1 \text{ peak area ratio} \times 1 + H_2 \text{ peak area ratio} \times 2 + H_3 \text{ peak area ratio} \times 3) \times 2$$ [Math 5]

3. Medicament

In an aspect of the present invention, provided is a pharmaceutical composition that contains the anti-LRRC15 antibody or an antigen-binding fragment of the antibody of the present invention described in the section "1. Anti-LRRC15 Antibody" described above and Examples, or an antibody-drug conjugate containing the antibody or antigen-binding fragment. In particular, the anti-LRRC15 antibody of the present invention described in the section "1. Anti-LRRC15 Antibody" described above and Examples and an antigen-binding fragment of the antibody binds to LRRC15 on the surface of target cells and typically has internalization activity on the target cells, and therefore, they can be used singly or in combinations with other drugs as a therapeutic agent (e.g., antitumor drug) for epithelial and mesenchymal cancers, such as a breast carcinoma, a head and neck carcinoma, a lung carcinoma (e.g., small-cell lung carcinoma or non-small cell lung carcinoma), a pancreatic carcinoma, a colorectal carcinoma, an esophageal carcinoma, an ovarian carcinoma, a bladder carcinoma, a cholangiocarcinoma, a gastric carcinoma, a mesothelioma, an uterine carcinoma, a thyroid carcinoma, a renal carcinoma, a hepatocarcinoma, a prostate carcinoma, a bone and soft tissue sarcoma, a melanoma, or a glioma, as a medicament.

Also, the anti-LRRC15 antibody or antigen-binding fragment of the antibody, or the antibody-drug conjugate of the present invention can be used for detecting cells that express LRRC15. Further, the anti-LRRC15 antibody or antigen-binding fragment of the antibody, or the antibody-drug conjugate of the present invention can be used for screening LRRC15 antigens.

Furthermore, when the anti-LRRC15 antibody of the present invention and an antigen-binding fragment of the antibody have internalization activity, they can be suitably used as the antibody used for antibody-drug conjugates.

Among the anti-LRRC15 antibody-drug conjugates of the present invention described in the above section "2.

Anti-LRRC15 Antibody-Drug Conjugate" and Examples, those in which a drug having antitumor activity such as cellular cytotoxicity is used as the drug are typically conjugates of an anti-LRRC15 antibody having internalization activity and/or an antigen-binding fragment of the antibody with a drug having antitumor activity such as cellular cytotoxicity, and exhibit cellular cytotoxicity against carcinoma-associated fibroblasts and carcinoma cells expressing LRRC15, and therefore, they can be used as a medicament, in particular as a therapeutic agent and/or prophylactic agent for cancer (e.g., antitumor drug).

The anti-LRRC15 antibody-drug conjugate of the present invention may become an hydrate by absorbing moisture or allowing adhesion of adsorbed water when being left in the atmosphere or subjected to recrystallization or purification operations. Such water-containing compounds or pharmacologically acceptable salts are also included in the present invention.

The anti-LRRC15 antibody-drug conjugate of the present invention can form a pharmacologically acceptable acid addition salt as desired, when having a basic group such as an amino group. Examples of such acid addition salts can include hydrohalides such as hydrofluoride, hydrochloride, hydrobromide, and hydroiodide; inorganic acid salts such as nitrate, perchlorate, sulfate, and phosphate; lower alkanesulfonates such as methanesulfonate, trifluoromethanesulfonate, and ethanesulfonate; arylsulfonates such as benzenesulfonate, and p-toluenesulfonate; organic acid salts such as formate, acetate, trifluoroacetate, malate, fumarate, succinate, citrate, tartrate, oxalate, and maleate; and amino acid salts such as ornithinate, glutamate, and aspartate.

The anti-LRRC15 antibody-drug conjugate of the present invention can form a pharmacologically acceptable base addition salt as desired, when having an acidic group such as a carboxy group. Examples of such base addition salts can include alkali metal salts such as sodium salt, potassium salt, and lithium salt; alkali earth metal salts such as calcium salt, and magnesium salt; inorganic salts such as ammonium salt; and organic amine salts such as dibenzylamine salt, morpholine salt, phenylglycine alkyl ester salt, ethylenediamine salt, N-methylglucamine salt, diethylamine salt, triethylamine salt, cyclohexylamine salt, dicyclohexylamine salt, N,N'-dibenzylethylenediamine salt, diethanolamine salt, N-benzyl-N-(2-phenylethoxy)amine salt, piperazine salt, tetramethylammonium salt, and tris(hydroxymethyl)aminomethane salt.

The present invention may also encompass an anti-LRRC15 antibody-drug conjugate in which one or more of the atoms constituting the antibody-drug conjugate are substituted with an isotope of the atoms. Isotopes are of two types: radioactive isotopes and stable isotopes, and examples thereof can include isotopes of hydrogen (2H and 3H), isotopes of carbon (11C, 13C, and 14C), isotopes of nitrogen (3N and 15N), isotopes of oxygen (150, 170, and 180), and isotope of fluorine (18F). Compositions containing isotope-labeled antibody-drug conjugates are useful, for example, as a therapeutic agent, a prophylactic agent, a reagent for research, an assay reagent, a diagnostic agent, a diagnostic reagent for in vivo imaging, and the like. Isotope-labeled antibody-drug conjugates and mixtures of isotope-labeled antibody-drug conjugates at any ratio are also encompassed in the present invention. Isotope-labeled antibody-drug conjugates can be produced by a method known in the art, for example, by using an isotope-labeled raw material in place of the raw material in the above-described production method of the present invention.

Cytocidal activity in vitro can be measured using a known method, or by a method in which a known method is applied, and for example, it can be determined by measuring growth inhibitory activity of cells. For example, it can be determined by culturing a carcinoma cell line that overexpresses LRRC15, adding an anti-LRRC15 antibody or an antigen-binding fragment thereof, an anti-LRRC15 antibody-drug conjugate, or the like to the culture system at various concentrations, and measuring inhibitory activity against focus formation, colony formation, and spheroid growth. Here, by using, for example, cancer cell lines derived from osteosarcoma and melanoma, and the like, cell growth inhibitory activity against osteosarcoma and melanoma can be examined.

Therapeutic effects on cancer in vivo using laboratory animals can be measured using a known method, or by a method in which a known method is applied, and for example, it can be determined by applying an anti-LRRC15 antibody or an antigen-binding fragment thereof, an anti-LRRC15 antibody-drug conjugate, or the like to immunodeficient mice transplanted with a tumor cell line that highly express LRRC15, and measuring changes in cancer cells. As an alternative, despite a tumor cell line not expressing LRRC15, for example, when the tumor cell line is transplanted into immunodeficient mice and LRRC15 is highly expressed in cells (e.g., fibroblasts) in the formed tumor stroma, the effects can be determined by administering an anti-LRRC15 antibody-drug conjugate and the like to the mice and measuring changes in cancer cells. Here, by using, for example, animal models in which cells derived from osteosarcoma, glioblastoma, non-small cell lung carcinoma, breast carcinoma, head and neck carcinoma, or pancreatic carcinoma are transplanted into immunodeficient mice, therapeutic effects on osteosarcoma, glioblastoma, non-small cell lung carcinoma, breast carcinoma, head and neck carcinoma, or pancreatic carcinoma can be measured.

Types of cancers to which the anti-LRRC15 antibody or an antigen-binding fragment thereof, or the anti-LRRC15 antibody-drug conjugate of the present invention is applied is not particularly limited as long as it is a cancer which expresses LRRC15 in cancer cells to be treated, and/or a cancer in which cells in the cancer stroma express LRRC15, and examples thereof can include breast carcinoma, head and neck carcinoma, lung carcinoma (e.g., small-cell lung carcinoma or non-small cell lung carcinoma), pancreatic carcinoma, colorectal carcinoma, esophageal carcinoma,

ovarian carcinoma, bladder carcinoma, cholangiocarcinoma, gastric carcinoma, mesothelioma, uterine carcinoma, thyroid carcinoma, renal carcinoma, hepatocarcinoma, prostate carcinoma, bone and soft tissue sarcoma, melanoma, and glioma. More preferred examples of cancers can include breast carcinoma, head and neck carcinoma, non-small cell lung carcinoma, pancreatic carcinoma, colorectal carcinoma, esophageal carcinoma, ovarian carcinoma, bladder carcinoma, osteosarcoma, chondrosarcoma, myofibroblastic sarcoma, leiomyosarcoma, melanoma, and glioblastoma.

The anti-LRRC15 antibody or antigen-binding fragment thereof, or the anti-LRRC15 antibody-drug conjugate of the present invention can be preferably administered to mammals, and more preferably administered to humans.

Substances used in pharmaceutical compositions containing the anti-LRRC15 antibody or antigen-binding fragment thereof, or the anti-LRRC15 antibody-drug conjugate of the present invention can be appropriately selected and applied from formulation additives or the like commonly used in the art at dosage or administration concentrations.

The anti-LRRC15 antibody or antigen-binding fragment thereof, or the anti-LRRC15 antibody-drug conjugate of the present invention can be administered as a pharmaceutical composition containing one or more pharmaceutically suitable components, such as, pharmaceutically acceptable carriers and/or pharmaceutically acceptable excipients. For example, the above pharmaceutical composition typically contains one or more pharmaceutically acceptable carriers (e.g., sterilized solution (including water and oil (petroleum, or oil of animal-, plant-, or synthetic origin (such as peanut oil, soybean oil, mineral oil, and sesame oil)))). Water is a more typical carrier when the above pharmaceutical composition is intravenously administered. A saline solution, an aqueous dextrose solution, and an aqueous glycerol solution can also be used as a liquid carrier, in particular, for an injection solution. Suitable pharmaceutically acceptable excipients are known in the art. The above composition may also contain a trace amount of a wetting agent, an emulsifying agent, or a pH buffering agent, if desired. Examples of the suitable pharmaceutically acceptable excipients are described in "Remington's Pharmaceutical Sciences" by E. W. Martin. The formulations correspond to the administration mode.

Various delivery systems are known, and can be used to administer the anti-LRRC15 antibody or antigen-binding fragment thereof, or the anti-LRRC15 antibody-drug conjugate of the present invention. Examples of introduction method include, but not limited to, intradermal, intramuscular, intraperitoneal, intravenous, and subcutaneous routes. The administration may be made by, for example, infusion or bolus injection. In a specific preferred embodiment, the administration of the anti-LRRC15 antibody or antigen-binding fragment thereof, or the anti-LRRC15 antibody-drug conjugate described above is made by infusion. Parenteral administration is a preferred administration route.

In a representative embodiment, the above pharmaceutical composition is prescribed as a pharmaceutical composition suitable for intravenous administration to humans, according to conventional procedures. The composition for intravenous administration is typically a solution in a sterile and isotonic aqueous buffer solution. If necessary, the above medicament may also contain a solubilizer and local anesthetic to alleviate pain at an injection site (e.g., lignocaine). Generally, the above components are supplied either separately or by mixing together in a unit dosage form (e.g., as a dried lyophilized powder or anhydrous concentrate in a sealed container as an ampule, sachet, or the like indicating the amount of active agent). When the above medicament is to be administered by infusion, it may be administered with an infusion bottle containing sterile water or saline solution of pharmaceutical grade. When the above medicament is administered by injection, an ampule of sterile water or saline solution for injection may be provided so that the components described above are mixed with each other before administration, for example.

The pharmaceutical composition of the present invention may be a pharmaceutical composition that only contains the anti-LRRC15 antibody or an antigen-binding fragment thereof, or the antibody-drug conjugate of the present invention, or may be a pharmaceutical composition that further contains at least one other cancer therapeutic agent. The anti-LRRC15 antibody or antigen-binding fragment thereof, or the anti-LRRC15 antibody-drug conjugate of the present invention can be administered in combination with other cancer therapeutic agents, and thereby anti-cancer effects can be enhanced. Other anti-cancer agents used for such purposes may be administered to an individual concurrently with, separately from, or consecutively with the anti-LRRC15 antibody or an antigen-binding fragment thereof, or the antibody-drug conjugate, or may be administered while changing the administration intervals for each. Examples of such cancer therapeutic agents can include antibody-drug conjugates including Enhertz, Kadcyla, Padoseb, and Trodelvy; microtubule inhibitors including Taxol, Abraxane, and Halaven; PARP inhibitors including Olaparib and Talazoparib; angiogenesis inhibitors including Avastin and Cyramza; metabolic antagonists including Gemzar, Alimta, and 5-FU; anthracycline drugs including doxorubicin and Doxil; tyrosine kinase inhibitors including imatinib, Stivarga, and Tagrisso; CDK4/6 inhibitors including Ibrance; HSP90 inhibitors including Jeselhy; MEK inhibitors including MEK162; and immune checkpoint inhibitors including Keytruda, Tecentriq, and Yervoy, but are not limited thereto as long as they have antitumor activity.

The pharmaceutical composition can be formulated into a lyophilized formulation or a liquid formulation as a formulation having selected composition and necessary purity. When formulated as a lyophilized formulation, it may be a formulation containing suitable formulation additives used in the art. For a liquid formulation as well, it may be formulated as a liquid formulation containing various formulation additives used in the art.

4. Treatment Method

In an aspect of the present invention, provided is a method for treating a tumor, including administering a therapeutically effective amount of the anti-LRRC15 antibody or an antigen-binding fragment of the antibody of the present invention described in the section "1. Anti-LRRC15 Antibody" described above and Examples, an antibody-drug conjugate containing the antibody or antigen-binding fragment, or a pharmaceutical composition that contains the antibody or antigen-binding fragment, or the antibody-drug conjugate, to a subject in need of tumor treatment. In addition, in a further aspect of the present invention, provided is a method for treating a tumor, including administering to a subject a therapeutically effective amount of the anti-LRRC15 antibody or an antigen-binding fragment of the antibody of the present invention described in the section "1. Anti-LRRC15 Antibody" described above and Examples, an antibody-drug conjugate containing the antibody or antigen-binding fragment, or a pharmaceutical composition that contains the antibody or antigen-binding fragment, or the antibody-drug conjugate and at least one different antitumor drug concurrently, separately, or consecutively.

As used herein, the "therapeutically effective amount" of an antibody or the like means an effective amount for treating a tumor, for example, an amount of an antibody or antigen-binding fragment, or an antibody-drug conjugate sufficient to achieve a desired therapeutic effect when administered to a subject in a desired dosage volume. The therapeutically effective amount can be determined in consideration of the type and progression of the tumor to be treated, and characteristics of the individual to be treated, such as body weight, age, sex, health condition, and tolerance to the antibody or the like. Accordingly, when determining the therapeutically effective amount of an antibody or the like, it can also be set based on the affinity between the antibody or the like and the antigen. The antibody of the present invention or the like may be administered to a human at a dosage of about 0.001 to 100 mg/kg once or multiple times at intervals of once a day to every 180 days, for example.

All of the descriptions in the above section "3. Medicament" apply to a pharmaceutical composition that can be used in the treatment method of the present invention, a tumor o be treated, a delivery method of an antibody or the like, and at least one other antitumor drug or the like that can be used in combination with the antibody of the present invention or the like.

Examples

The present invention will be specifically described with reference to Examples shown below; however, the present invention is not limited to these. In addition, these should not be construed as limitation in any sense. Note that each operation regarding genetic engineering in the following Examples was carried out by the method described in "Molecular Cloning" (Sambrook, J., Fritsch, E.F., and Maniatis, T., published by Cold SpringHarbor Laboratory Press in 1989) or methods described in other laboratory manuals used by those skilled in the art, or when commercially available reagents or kits are used, according to the instructions of the commercially available products, unless otherwise explicitly specified. Also, reagents, solvents and starting materials not specifically described herein are readily available from commercially available sources.

<Example 1: Acquisition of Rat Anti-Human LRRC15 Antibody>

1)-1.1 Construction of Human, Mouse, Rat and Cynomolgus Monkey LRRC15 Expression Vectors

Human LRRC15 expression vector pcDNA3.1-hLRRC15 was prepared by incorporating a nucleotide sequence that encodes a human LRRC15 protein into a mammalian expression vector (GenScript Inc.). Similarly, cynomolgus monkey, mouse, and rat LRRC15 expression vectors pcDNA3.1-cynoLRRC15, pcDNA3.1-mLRRC15, and pcDNA3.1-ratLRRC15 were prepared. The amino acid sequences of each protein are shown in SEQ ID NOs: 1, 4, 7, and 10.

Human LRRC15 extracellular domain expression vector pDisplay-hLRRC15 ECD was prepared by incorporating a nucleotide sequence that encodes an extracellular domain of the human LRRC15 protein into a mammalian expression vector (GenScript Inc.). Similarly, cynomolgus monkey, mouse, and rat LRRC15 extracellular domain expression vectors pDisplay-cynoLRRC15 ECD, pDisplay-mLRRC15 ECD, and pDisplay-ratLRRC15 ECD were prepared. The amino acid sequences of the extracellular domains of each protein are shown in SEQ ID NOs: 2, 5, 8, and 11.

Large-scale preparation of plasmid DNA was carried out using EndoFree Plasmid Giga Kit (QIAGEN N.V.).

1)-1.2 Preparation of human, cynomolgus monkey, mouse and rat LRRC15-His Protein

A recombinant protein with His-tag added at the C-terminus of the extracellular domain of the human LRRC15 protein was expressed in a mammalian cell and purified by Ni affinity and SEC, or Ni affinity, SEC and ion exchange chromatography to prepare a human LRRC15-His protein. Similarly, cynomolgus monkey, mouse, and rat LRRC15-His

proteins were prepared. The amino acid sequences of each protein are shown in SEQ ID NOs: 3, 6, 9, and 12.

1)-2 Immunization

Immunization was carried out using female WKY/Izm rats (Japan SLC, Inc.). First, rat's lower legs were pretreated with hyaluronidase (Sigma Aldrich Co. LLC), and then, the plasmid DNA that had been prepared in large-scale in Example 1)-1 was intramuscularly injected into the same sites. Subsequently, ECM830 (BTX, Inc.) was used to perform in vivo electroporation at the same sites. After repeating the same in vivo electroporation, lymph nodes or spleens of the rats were collected and used for hybridoma production.

1)-3 Hybridoma Production

Lymph node or spleen cells were electrofused with mouse myeloma SP2/0-ag14 cells (ATCC, No. CRL-1581) using LF301 Cell Fusion Unit (BEX Co., Ltd.), and then the cells were suspended in ClonaCell-HY Selection Medium D (StemCell Technologies Inc.), diluted, and cultured under conditions of 37°C, 5% $CO_2$. Each hybridoma colony that emerged was collected as a monoclone, and suspended in ClonaCell-HY Selection Medium E (StemCell Technologies Inc.) to culture under conditions of 37°C and 5% $CO_2$. After the cells had grown to a sufficient extent, frozen stocks of each hybridoma cell were prepared, and resulting hybridoma culture supernatant was used for screening of anti-LRRC15 antibody-producing hybridomas.

1)-4 Screening of Antibody-Producing Hybridomas by Cell-ELISA Method

1)-4-1 Preparation of Antigenic Gene-Expressing Cells for Cell-ELISA

HEK293α (a stable expressing cell line derived from HEK293 that expresses integrin αv and integrin β3) was prepared at $5 \times 10^5$ cells/mL in a 10% FBS-containing DMEM medium. HEK293α into which pcDNA3.1-hLRRC15 had been introduced, or HEK293α without vector introduced, as a negative control, was dispensed onto a 96-well plate (Corning Incorporated) according to a transfection procedure using Lipofectamine 2000 (Thermo Fisher Scientific, Inc.), and then cultured overnight under conditions of 37°C, 5% $CO_2$. The resulting transfected cells were used in Cell-ELISA while remaining adherent.

1)-4-2 Cell-ELISA

After removing the culture supernatant of HEK293α prepared in Example 1)-4-1, the hybridoma culture supernatant was added to each of HEK293α with pcDNA3.1-hLRRC15 introduced or HEK293α without introduction of pcDNA3.1-hLRRC15, and the cells were left to stand at 4°C for 30 minutes. After washing the cells in the wells once with 5% FBS-containing PBS, an anti-rat IgG-Peroxidase antibody in goat (Jackson ImmunoResearch Inc.) diluted with 5% FBS-containing PBS was added thereto and left to stand at 4°C for 30 minutes. After washing the cells in the wells three times with 5% FBS-containing PBS, Super AquaBlue coloring solution (Thermo Fisher Scientific, Inc.) was added thereto and left to stand for 20 minutes, and then the absorbance of the cells was measured at 405 nm using a plate reader (ENVISION: PerkinElmer, Inc.). Hybridomas that produce culture supernatants showing higher absorbance in HEK293α with the pcDNA3.1-hLRRC15 expression vector introduced compared to the negative control HEK293α were selected as antibody-producing hybridomas that bind to human LRRC15.

1)-5 Isotype Determination of Rat Monoclonal Antibody

Clone rR024, which was suggested to strongly and specifically bind to human LRRC15, was selected from the rat anti-LRRC15 antibody-producing hybridomas selected in Example 1)-4, and the isotype of the antibody was identified. The heavy chain subclass and light chain type of the antibody were determined using a RAT MONOCLONAL ANTIBODY ISOTYPING TEST KIT (Antagen pharmaceuticals, Inc.). As a result, it was confirmed that the subclass of rR024 was confirmed to be IgG2b and the light chain was a κ chain.

<Example 2: Determination of Nucleotide Sequence of cDNA That Encodes Variable Region of Rat Anti-LRRC15 Antibody>

2)-1 Amplification and Sequencing of Gene Fragments of rR024 Heavy Chain Variable Region And Light Chain Variable Region

2)-1-1 Preparation of total RNA from rR024-Producing Hybridomas

In order to amplify cDNA containing the variable region of rR024, total RNA was prepared from rR024-producing hybridomas (TRIZOL-treated 1 x $10^7$ cells), using an RNA purification kit, Direct-zol RNA Miniprep Kit (ZYMO RESEARCH Inc.).

2)-1-2 Amplification and Determination of Nucleotide Sequence of cDNA Containing Heavy Chain Variable Region of rR024 by 5'-RACE PCR

Amplification of cDNA containing the heavy chain variable region was carried out using about 1 μg of the total RNA prepared in Example 2)-1-1 and SMARTer (registered trademark) RACE 5'/3' Kit (Clontech Laboratories, Inc.). As primers for amplifying cDNA of the variable region of the heavy chain gene of rR024 by 5'-RACE PCR, Universal Primer A Mix (UPM: included in SMARTer (registered trademark) RACE 5'/3' Kit) and a primer designed from the sequence of a known rat heavy chain constant region were used. Next, sequence analysis was carried out using the PCR fragment containing the heavy chain variable region amplified by 5'-RACE PCR. The nucleotide sequence of the determined cDNA that encodes the heavy chain variable region of rR024 is shown in SEQ ID NO: 14, and the amino acid sequence is shown in SEQ ID NO: 15.

2)-1-3 Amplification and Determination of Nucleotide Sequence of cDNA Containing Light Chain Variable Region of rR024 by 5'-RACE PCR

It was carried out by the same method as in Example 2)-1-2. Except that, as primers for amplifying cDNA of the variable region of the light chain gene of rR024 by 5'-RACE PCR, Universal Primer A Mix (UPM: included in SMARTer (registered trademark) RACE cDNA Amplification Kit) and a primer designed from the sequence of a known rat light chain constant region were used. The nucleotide sequence of the determined cDNA that encodes the light chain variable region of rR024 is shown in SEQ ID NO: 16, and the amino acid sequence is shown in SEQ ID NO: 17.

<Example 3: Preparation of Human Chimerized Anti-LRRC15 Antibody>

3)-1 Construction of Expression Vector of Human Chimerized Anti-LRRC15 chR024

3)-1-1 Construction of Chimerized and Humanized Light Chain Expression Vector pCMA-LK

About 5.4 kb fragment obtained by digesting plasmid pcDNA3.3-TOPO/LacZ (Invitrogen) with restriction enzymes XbaI and PmeI was joined to a DNA fragment containing a DNA sequence that encodes the human light chain signal sequence and human κ chain constant region shown in SEQ ID NO: 18 using an In-Fusion HD PCR cloning kit (Clontech Laboratories, Inc.) to prepare pcDNA3.3/LK. pCMA-LK was constructed by removing the neomycin expression unit from pcDNA3.3/LK.

3)-1-2 Construction of Chimerized and Humanized IgG1 Type Heavy Chain Expression Vector pCMA-G1

A DNA fragment containing a nucleotide sequence that encodes the human heavy chain signal sequence and the amino acid of the human IgG1 constant region shown in SEQ ID NO: 19 was synthesized (Eurofins Genomics Inc.). This DNA fragment was cleaved with restriction enzymes XbaI and PmeI, and a 1.1 kb DNA fragment was excised by agarose gel electrophoresis and purified using Wizard SV Gel and PCR Clean-Up System (Promega). About 3.4 kb fragment obtained by digesting pCMA-LK with restriction enzymes XbaI and PmeI was joined to the 1.1 kb DNA fragment using Ligation High (TOYOBO Co., Ltd.) to construct pCMA-G1.

3)-1-3 Construction of chR024 Heavy Chain Expression Vector

A DNA sequence containing the variable region of chR024 heavy chain was synthesized using the artificial gene synthesis service of GeneArt, Inc., and the synthesized DNA fragment was inserted into pCMA-G1 by joining them using the restriction enzyme site of pCMA-G1 with the In-Fusion HD PCR cloning kit (Clontech Laboratories, Inc.), thereby constructing a chR024 heavy chain expression vector. A DNA sequence (58 to 1416) that encodes the chR024 heavy chain is shown in SEQ ID NO: 25. Note that, in the sequence, the 1- to 57-positions are signal sequences.

3)-1-4 Construction of chR024 Light Chain Expression Vector

A DNA sequence containing the variable region of chR024 light chain was synthesized using the artificial gene synthesis service of GeneArt, Inc., and the synthesized DNA fragment was inserted into pCMA-LK by joining them using the restriction enzyme site of pCMA-LK with the In-Fusion HD PCR cloning kit (Clontech Laboratories, Inc.), thereby constructing a chR024 light chain expression vector. A DNA sequence (61 to 720) that encodes the chR024 light chain is shown in SEQ ID NO: 31. Note that, in the sequence, the 1- to 60-positions are signal sequences.

3)-2 Production and Purification of Human Chimerized Anti-LRRC15 Antibody chR024

3)-2-1 Production of chR024

Expi293F cells (Thermo Fisher Scientific, Inc.) were passage-cultured according to the manual. Expi293F cells were diluted with an Expi293 Expression Medium (gibco) and prepared at 2.5 x $10^6$ cells/ml. 0.012 mg heavy chain expression vector, 0.018 mg light chain expression vector, and 0.1 mg PEI MAX (Polysciences, Inc.) were added to a 0.85 ml Opti-Pro SFM medium (gibco), gently stirred, and then left for 5 minutes before being added to 30 ml of Expi293F cells. Culture supernatants obtained by culturing in an 8% $CO_2$ incubator at 37°C for 6 days with shaking at 135 rpm were filtered with Steritop (Millipore, #S2GPT02R).

3)-2-2 Purification of chR024

The culture supernatant obtained in Example 3)-2-1 was purified by a one-step process of rProtein A affinity chromatography. The culture supernatant was applied to PBS-equilibrated MonoSpin L ProA (GL Sciences Inc.), and then the column was washed twice with 10 ml PBS. Next, elution was performed with 0.1 M sodium chloride and 0.1 M arginine buffer solution (pH 3.8) to collect fractions containing the antibody. The fractions were subjected to buffer replacement with HBSor (25 mM histidine/5% sorbitol, pH 6.0) by ultrafiltration (Merck KGaA, Amicon Ultra-15 50K).

The binding evaluation of the prepared human chimerized anti-LRRC15 antibody chR024 was performed by a method similar to that described in International Publication No. 2018/212136, which confirmed that the antibody has binding activity to LRRC15.

<Example 4: Preparation of Humanized Anti-LRRC15 Antibody>

4)-1 Design of Humanized Anti-LRRC15 Antibody hR024

4)-1-1 Molecular Modeling of Variable Region of chR024

Molecular modeling of the variable region of chR024 was performed by using a method known as homology modeling (Methods in Enzymology, 203, 121-153, (1991)). It was performed with a commercially available protein three-dimensional structure analysis program Discovery Studio (Dassault Systems Inc.) using a structure that has high sequence identity to the variable regions of the heavy and light chains of chR024 and is registered in Protein Data Bank, as a template.

4)-1-2 Design of Humanized Amino Acid Sequence for chR024

chR024 was humanized by CDR grafting (Proc. Natl. Acad. Sci. USA 86, 10029-10033 (1989)). The consensus sequences of human gamma chain subgroup 3 and kappa chain subgroup 1 defined in KABAT et al. (Sequences of Proteins of Immunological Interest,5th Ed.Public Health Service National Institutes of Health,Bethesda,MD. (1991)) were selected as heavy chain and light chain acceptors, respectively, because they have high identity to the framework regions of chR024. The donor residues to be transferred onto the acceptors were selected by analyzing three-dimensional models with reference to criteria provided by Queen et al. (Proc. Natl. Acad. Sci. USA 86, 10029-10033 (1989)) or the like.

4)-2 Humanization of chR024 Heavy Chain

Four designed heavy chains were named hR024 H02, hR024 H02-V1, hR024H02-V2, and hR024 H09. The full-length heavy chain amino acid sequence of hR024 H02 is set forth in SEQ ID NO: 42 (the heavy chain variable region: positions of 20 to 142, the heavy chain constant region: positions of 143 to 472). The nucleotide sequence that encodes the amino acid sequence of SEQ ID NO: 42 is set forth in SEQ ID NO: 43 (the heavy chain variable region: positions of 58 to 426, the heavy chain constant region: positions of 427 to 1416). The full-length heavy chain amino acid sequence of hR024

H02-V1 is set forth in SEQ ID NO: 48 (the heavy chain variable region: positions of 20 to 142, the heavy chain constant region: positions of 143 to 472). The nucleotide sequence that encodes the amino acid sequence of SEQ ID NO: 48 is set forth in SEQ ID NO: 49 (the heavy chain variable region: positions of 58 to 426, the heavy chain constant region: positions of 427 to 1416). The full-length heavy chain amino acid sequence of hR024 H02-V2 is set forth in SEQ ID NO: 54 (the heavy chain variable region: positions of 20 to 142, the heavy chain constant region: positions of 143 to 472). The nucleotide sequence that encodes the amino acid sequence of SEQ ID NO: 54 is set forth in SEQ ID NO: 55 (the heavy chain variable region: positions of 58 to 426, the heavy chain constant region: positions of 427 to 1416). The full-length heavy chain amino acid sequence of hR024 H09 is set forth in SEQ ID NO: 36 (the heavy chain variable region: positions of 20 to 142, the heavy chain constant region: positions of 143 to 472). The nucleotide sequence that encodes the amino acid sequence of SEQ ID NO: 36 is set forth in SEQ ID NO: 37 (the heavy chain variable region: positions of 58 to 426, the heavy chain constant region: positions of 427 to 1416).

4)-3 Humanization of chR024 Light Chain

Two designed light chains were named hR024 L01, and hR024 L03. The full-length light chain amino acid sequence of hR024 L01 is set forth in SEQ ID NO: 66. The nucleotide sequence that encodes the amino acid sequence of SEQ ID NO: 66 is set forth in SEQ ID NO: 67. The full-length light chain amino acid sequence of hR024 L03 is set forth in SEQ ID NO: 60. The nucleotide sequence that encodes the amino acid sequence of SEQ ID NO: 60 is set forth in SEQ ID NO: 61.

4)-4 Design of hR024 by Combination of Heavy Chain and Light Chain

An antibody consisting of hR024 H02 and hR024 L01 is referred to as "H02L01 antibody" or "H02L01". An antibody consisting of hR024 H02-V1 and hR024 L01 is referred to as "H02L01-V1 antibody" or "H02L01-V1". An antibody consisting of hR024 H02-V2 and hR024 L01 is referred to as "H02L01-V2 antibody" or "H02L01-V2". An antibody consisting of hR024 H09 and hR024 L03 is referred to as "H09L03 antibody" or "H09L03".

4)-5 Expression of Humanized Anti-LRRC15 Antibody hR024

4)-5-1 Construction of hR024 Heavy Chain Expression Vector

4)-5-1-1 Construction of hR024 H02 Type Heavy Chain Expression Vector

A DNA fragment shown in SEQ ID NOs: 36 to 443 of the nucleotide sequence of the humanized hR024-H02 type heavy chain shown in SEQ ID NO: 43 was synthesized (GeneArt, Inc.). A humanized hR024-H02 type heavy chain expression vector was constructed by inserting the synthesized DNA fragment into the site where pCMA-G1 was cleaved with the restriction enzyme BlpI, with the In-Fusion HD PCR cloning kit (Clontech Laboratories, Inc.).

4)-5-1-2 Construction of hR024 H02-V1 Type Heavy Chain Expression Vector

A DNA fragment was synthesized (GeneArt, Inc.) by adding CCAGCCTCCGGACTCTAGAGCCACC to the 5' end and TGAGTTTAAACGGGGGAGGCTAACT to the 3' end of the sequence of SEQ ID NO: 49, which contains the full length nucleotide sequence of the hR024 H02-V1 type heavy chain at positions 58 to 1416. A hR024-H02-V1 type heavy chain expression vector was constructed by inserting the synthesized DNA fragment into the site where pCMA-LK was cleaved with the restriction enzymes XbaI and PmeI, with the In-Fusion HD PCR cloning kit (Clontech Laboratories, Inc.).

4)-5-1-3 Construction of hR024 H02-V2 Type Heavy Chain Expression Vector

A hR024 H02-V2 type heavy chain expression vector was constructed by the same method as in Example 4)-5-1-2. A sequence fragment contained in the hR024 H02-V2 type heavy chain expression vector and containing a heavy chain nucleotide sequence at positions 58 to 1416 is shown in SEQ ID NO: 55.

4)-5-1-4 Construction of hR024 H09 Type Heavy Chain Expression Vector

A hR024 H09 type heavy chain expression vector was constructed by the same method as in Example 4)-5-1-2. A sequence fragment contained in the hR024 H09 type heavy chain expression vector and containing a heavy chain nucleotide sequence at positions 58 to 1416 is shown in SEQ ID NO: 37.

4)-5-2 Construction of hR024 Light Chain Expression Vector

4)-5-2-1 Construction of hR024 L01 Type Light Chain Expression Vector

A DNA fragment containing a DNA sequence that encodes the variable region of the humanized hR024-L01 type light chain shown in SEQ ID NOs: 37 to 420 of the nucleotide sequence of the humanized hR024-L01 type light chain shown in SEQ ID NO: 67 was synthesized (GeneArt, Inc.). A humanized hR024-L01 type light chain expression vector was constructed by inserting the synthesized DNA fragment into the site where pCMA-LK was cleaved with the restriction enzyme BsiWI, with the In-Fusion HD PCR cloning kit (Clontech Laboratories, Inc.).

4)-5-2-2 Construction of hR024 L03 Type Light Chain Expression Vector

A DNA fragment containing a DNA sequence that encodes the variable region of the humanized hR024-L03 type light chain shown in SEQ ID NOs: 37 to 420 of the nucleotide sequence of the humanized hR024-L03 type light chain shown in SEQ ID NO: 61 was synthesized (GeneArt, Inc.). A humanized hR024-L03 type light chain expression vector was constructed by inserting the synthesized DNA fragment into the site where pCMA-LK was cleaved with the restriction enzyme BsiWI, with the In-Fusion HD PCR cloning kit (Clontech Laboratories, Inc.).

4)-5-3 Preparation of hR024

4)-5-3-1 Production of H02L01, H02L01-V1, and H02L01-V2

H02L01, H02L01-V1, and H02L01-V2 were produced by combination of the heavy and light chains shown in Example 4)-4. In other words, Freestyle 293F cells (Thermo Fisher Scientific, Inc.) were passage-cultured according to the manual. Logarithmic growth phase FreeStyle 293F cells were diluted with a FreeStyle 293 expression medium (Thermo Fisher Scientific, Inc.) to adjust to 2.0 x $10^6$ cells/mL, and 1.2 L was seeded into a 5 L Optimum Growth Flask (Thomson Instrument Company). 3.6 mg polyethyleneimine (Polysciences, Inc.) was added to 40 mL Opti-Pro SFM medium (Thermo Fisher Scientific, Inc.) Next, 600 μg heavy chain expression vector and 600 μg light chain expression vector were added to 40 mL of Opti-Pro SFM medium. The mixture solution of the expression vector/Opti-Pro SFM was added to the mixture solution of polyethyleneimine/Opti-Pro SFM, gently stirred, and then left to stand for 5 minutes before being added to the FreeStyle 293F cells. After culturing in an 8% $CO_2$ incubator at 37°C for 4 hours with shaking at 105 rpm, 960 mL Balan CD HEK293 medium (FUJIFILM Wako Pure Chemical Corporation) containing 1% GlutaMAX (Thermo Fisher Scientific, Inc.) and 240 mL Balan CD HEK293 Feed medium (FUJIFILM Wako Pure Chemical Corporation) containing 1% GlutaMAX were added. Further, the culture supernatant obtained by culturing at 37°C, 8% $CO_2$ and 105 rpm for 6 days was passed through a depth filter (pore size: 5 μm, GE Healthcare Inc.), and then filtered through a capsule cartridge filter (pore size: 0.45 μm, Advantec Toyo Kaisha, Ltd.).

4)-5-3-2 Production of H09L03

Production was made by the following method by combination of the heavy and light chains shown in Example 4)-4. In other words, Freestyle 293F cells (Thermo Fisher Scientific, Inc.) were passage-cultured according to the manual. 1.2 x $10^9$ FreeStyle 293F cells (Thermo Fisher Scientific, Inc.) in the logarithmic growth phase were seeded in 3 L Fernbach Erlenmeyer Flask (Corning Incorporated), and diluted with a FreeStyle 293 expression medium (Thermo Fisher Scientific, Inc.) to adjust to 2.0 x $10^6$ cells/ml. 0.3 mg heavy chain expression vector, 0.3 mg light chain expression vector, and 1.8 mg polyethyleneimine (Polysciences, Inc.) were added to 40 ml Opti-Pro SFM medium (Thermo Fisher Scientific, Inc.), gently stirred, and then left to stand for another 5 minutes before being added to the FreeStyle 293F cells. After culturing in an 8% $CO_2$ incubator at 37°C, for 4 hours with shaking at 90 rpm, 600 ml EX-CELL VPRO medium (SAFC Biosciences, Inc.), 18 ml GlutaMAX I (GIBCO), and 30 ml Yeastolate Ultrafiltrate (GIBCO) were added thereto, and the culture supernatant obtained by culturing in an 8% $CO_2$ incubator at 37°C for 6 days with shaking at 90 rpm was filtered through a Disposable Capsule Filter (Advantec Toyo Kaisha, Ltd.).

4)-5-3-3 Two-Step Purification of H02L01, H02L01-V1, H02L01-V2, and H09L03

The culture supernatants obtained in Examples 4)-5-3-1 and 4)-5-3-2 were purified in a two-step process of rProtein A affinity chromatography and ceramic hydroxyapatite. The culture supernatant was applied to a column loaded with PBS-equilibrated MabSelectSuRe (GE healthcare Bioscience BioProcess Corp.), and then the column was washed with PBS in an amount of 2 times or more of the column volume. Next, an antibody was eluted with 2M arginine hydrochloride solution (pH 4.0). Fractions containing the antibody were subjected to buffer replacement with PBS by

dialysis (Thermo Fisher Scientific, Inc., Slide-A-Lyzer Dialysis Cassette), diluted 5-fold with 5 mM sodium phosphate/50 mM MES/pH 7.0 buffer, and then applied to a ceramic hydroxyapatite column (Bio-Scale CHT Type-1 Hydroxyapatite Column, Japan Bio-Rad Laboratories, KK) equilibrated with 5 mM sodium phosphate/50 mM MES/30 mM NaCl/pH 7.0 buffer. Linear concentration gradient elution was performed with sodium chloride to collect fractions containing the antibody. The fractions were subjected to buffer replacement with HBSor (25 mM histidine/5% sorbitol, pH 6.0) by dialysis (Thermo Fisher Scientific, Inc., Slide-A-Lyzer Dialysis Cassette). The antibody was concentrated using a Centrifugal UF Filter Device VIVASPIN20 (fractionated molecular weight UF10K, Sartorius AG), and finally filtered through a Minisart-Plus filter (Sartorius AG) to obtain a purified sample.

4)-6 Binding Evaluation of Humanized Anti-LRRC15 Antibody hR024

4)-6-1 SPR

The dissociation constant of the humanized anti-LRRC15 antibody prepared in Example 4)-5 with the human LRRC15 antigen was measured by the SPR method with Biacore T200 (Cytiva, Inc.). The humanized anti-LRRC15 antibody was captured as a ligand using an immobilized anti-human IgG (Fc) antibody with a Human Antibody Capture Kit (Cytiva, Inc.), and the antigen was measured as an analyte by the capture method. The antigen prepared in Example 1)-1.2 was used. HBS-EP+ (Cytiva, Inc.) was used as the running buffer solution, and H02L01-V1 prepared in Example 4)-5-3-3 was diluted to 0.5 μg/mL with the running buffer solution to capture by feeding at 10 μL/min for 30 sec. The antigen was prepared in a concentration series of 16 μg/mL to a common ratio of 2 in the running buffer solution, and the binding phase was measured by feeding at 30 μL/min for 240 sec by a multi-cycle kinetic method, and then the dissociation phase was measured by feeding the running buffer solution at 30 μL/min for 600 sec. In any of these, 3M magnesium chloride (Cytiva, Inc.) was fed at 20 μL/mL for 30 sec for each measurement to regenerate the sensor chip. The resulting sensorgram was subjected to kinetic analysis (fitting with a 1:1 binding model) to calculate the dissociation constant (KD). The result is shown in Table 2.

[Table 2]

| mAb | *KD* (nM) |
|---|---|
| H02L01-V1 | 59. 9 |

4)-6-2 Flow Cytometry

4)-6-2-1 Preparation of Antigenic Gene-Expressing Cells for Flow Cytometry Analysis

HEK293α was prepared at $5 \times 10^5$ cells/mL in a 10% FBS-containing DMEM medium. HEK293α into which pDisplay-hLRRC15 ECD, pDisplay-cynoLRRC15 ECD, pDisplay-mLRRC15 ECD, or pDisplay-ratLRRC15 ECD had been introduced, or HEK293α with an empty vector introduced, as a negative control, was seeded in a flask, according to a transfection procedure using Lipofectamine 2000 (Thermo Fisher Scientific, Inc.), and then cultured overnight under conditions of 37°C, 5% $CO_2$. HEK293α into which each vector had been introduced was treated with Cell Dissociation Buffer (Thermo Fisher Scientific, Inc.), and the cells were washed with 5% FBS-containing PBS, and then suspended in 5% FBS-containing PBS. The resulting cell suspension was used for flow cytometry analysis.

4)-6-2-2 Binding Analysis against human, cynomolgus monkey, mouse, and rat LRRC15

The binding activity of humanized anti-LRRC15 antibodies against humans, cynomolgus monkeys, mice and rats was evaluated by the flow cytometry method. The four humanized anti-LRRC15 antibodies prepared in Example 4)-5 (clone numbers of H02L01, H02L01-V1, H02L01-V2, and H09L03) or human IgG control (BioLegend, Inc.) were diluted with 5% FBS-containing PBS and mixed with the cell suspension prepared in Example 4)-6-2-1 to be a final concentration of 10 μg/mL, and then left to stand at 4°C for 30 minutes. After washing with 5% FBS-containing PBS, an APC-labeled anti-human IgG antibody (Jackson ImmunoResearch Inc.) diluted with 5% FBS-containing PBS was added thereto and left to stand at 4°C for 30 minutes. After washing with 5% FBS-containing PBS, the detection was performed with a flow cytometer SA3800 (Sony Corporation). Data Analysis was performed with FlowJo (TreeStar, Inc.). The results thereof are shown in Figure 1. In the histograms in Figure 1, the abscissas indicate the fluorescence intensity of APC, which represents the amount of antibody bound, and the ordinates indicate the cell count; the histograms with half-tone dots indicate cases where a human IgG control is used, and the white-solid histograms indicate cases where a humanized anti-LRRC15 antibody is used. As shown in Figure 1, the four humanized anti-LRRC15 antibodies are confirmed not to bind to HEK293α

with a negative control empty vector introduced, but bind to HEK293α with pDisplay-hLRRC15 ECD, pDisplay-cynoLRRC15 ECD, pDisplay-mLRRC15 ECD, and pDisplay-ratLRRC15 ECD introduced.

4)-6-2-3 Confirmation of Binding to Endogenous LRRC15

In order to confirm that the humanized anti-LRRC15 antibody prepared in Example 4)-5 binds to endogenous LRRC15, cell lines were searched from a known database and the expression of LRRC15 on the cell membrane surface was evaluated by the flow cytometry method. After culturing a human melanoma cell line Hs294T (ATCC) and a mouse fibroblast cell line Balb/3T3 (JCRB) under conditions of 37°C, 5% $CO_2$, the cell suspension was prepared. The binding activity of the four humanized anti-LRRC15 antibodies prepared in Example 4)-5 (clone numbers of H02L01, H02L01-V1, H02L01-V2, and H09L03) or human IgG control (BioLegend, Inc.) to each cell line was analyzed by flow cytometry by the same method as in Example 4)-6-2-2. The results thereof are shown in Figure 2. In the histograms in Figure 2, the abscissas indicate the fluorescence intensity of APC, which represents the amount of antibody bound, and the ordinates indicate the cell count; the histograms with half-tone dots indicate cases where a human IgG control is used, and the white-solid histograms indicate cases where a humanized anti-LRRC15 antibody is used. As a result, the four humanized anti-LRRC15 antibodies were confirmed to bind to human LRRC15 and mouse LRRC15 expressed by the cell lines.

<Reference Example 1: Preparation of Anti-LRRC15 Antibody huM25>

The anti-LRRC15 antibody huM25 used in Examples was prepared with reference to the amino acid sequences of the full-length heavy chain and the full-length light chain of huM25 described in International Publication No. 2017/095805 (SEQ ID NO: 18 and SEQ ID NO: 19, respectively, in International Publication No. 2017/095805).

Reference Example 1)-1 Anti-LRRC15 Antibody huM25

Reference Example 1)-1-1 Construction of Expression Vector for Heavy Chain of Anti-LRRC15 Antibody huM25

A DNA fragment containing the variable region of the heavy chain of huM25 shown in nucleotide numbers of 36 to 434 of the nucleotide sequence of the heavy chain of huM25 shown in SEQ ID NO: 69 was synthesized (GeneArt, Inc.). A huM25 heavy chain expression vector was constructed by the same method as in Example 3)-1-3. The amino acid sequence of the heavy chain of huM25 expressed by the huM25 heavy chain expression vector is shown in SEQ ID NO: 68. In the amino acid sequence shown in SEQ ID NO: 68, the amino acid sequence consisting of the amino acid residues 1 to 19 is a signal sequence.

Reference Example 1)-1-2 Construction of Expression Vector for Light Chain of Anti-LRRC15 Antibody huM25

A DNA fragment containing a DNA sequence that encodes the variable region of the light chain of huM25 shown in SEQ ID NOs: 37 to 402 of the nucleotide sequence of the light chain of huM25 shown in SEQ ID NO: 71 was synthesized (GeneArt, Inc.). A huM25 light chain expression vector was constructed by the same method as in Example 3)-1-4. The amino acid sequence of the light chain of huM25 expressed by the huM25 light chain expression vector is shown in SEQ ID NO: 70. In the amino acid sequence shown in SEQ ID NO: 70, the amino acid sequence consisting of the amino acid residues 1 to 20 is a signal sequence.

Reference Example 1)-2 Preparation of Anti-LRRC15 Antibody huM25

Reference Example 1)-2-1 Production of Anti-LRRC15 Antibody huM25

Freestyle 293F cells (Thermo Fisher Scientific, Inc.) were passage-cultured in a spinner flask with a BCP-type animal cell culture device at 37°C, 8% $CO_2$. Subsequent culture was carried out with WAVE BIREACTOR (GE Healthcare Inc.) according to the manual. Logarithmic growth phase FreeStyle 293F cells were diluted with a FreeStyle293 expression medium (Thermo Fisher Scientific, Inc.) to adjust to 2.0 to 2.4 x $10^6$ cells/mL, and 2.5 L was transferred into a 10 L culture WAVE bag (Cytiva, Inc.). 7.5 mg polyethyleneimine (Polysciences, Inc.) was added to 80 mL Opti-Pro SFM medium (Thermo Fisher Scientific, Inc.) Next, 1.25 mg huM25 heavy chain expression vector and 1.25 mg huM25 light chain expression vector were added to 80 mL Opti-Pro SFM medium. The mixture solution of the expression vector/Opti-Pro SFM was added to the mixture solution of polyethyleneimine/Opti-Pro SFM, gently stirred, left to stand for 5 minutes, and then the entire amount was added to the 10 L culture WAVE bag in which FreeStyle 293F cells were cultured. After 4 hours of culturing at 37°C, 8% $CO_2$ while agitating, 2.5 L of EX-CELL VPRO medium (SAFC Biosciences, Inc.) and 125 mL of 43.4 g/L BD Recharge CD (BD Biosciences) were added thereto, and the culture supernatant obtained

by culturing at 37°C, 8% $CO_2$ for 6 days while agitating was passed through a depth filter (pore size: 5 μm, GE Healthcare Inc.), and then filtered through a capsule cartridge filter (pore size: 0.45 μm, Advantec Toyo Kaisha, Ltd.).

Reference Example 1)-2-2 Purification of Anti-LRRC15 Antibody huM25

The anti-LRRC15 antibody huM25 was purified from the culture supernatant obtained in Reference Example 1)-2-1 by the same method as in Example 4)-5-3-3.

[Reference Example 2: Preparation of Control hIgG Antibody]

A control hIgG antibody is a humanized hIgG monoclonal antibody that does not bind to mammalian cells, and was prepared with reference to the amino acid sequences of SEQ ID NOs: 72 and 73.

<Example 5: Antibody-Drug Conjugate>

[Example 5-1: H02L01 Antibody-[MSG1-$N_3$]$_2$]

[Chem. 54]

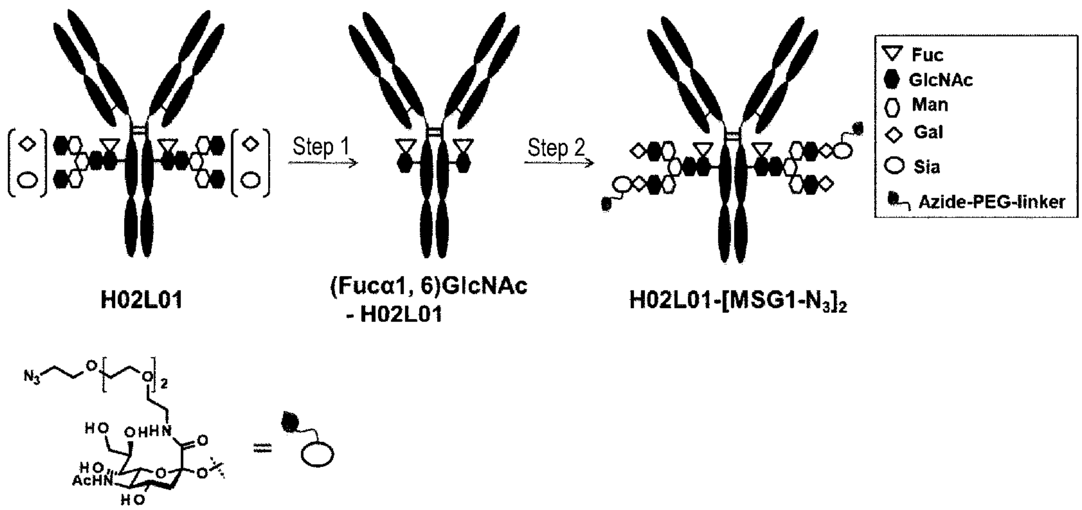


Step 1: Preparation of (Fucα1,6)GlcNAc-H02L01

The H02L01 solution prepared in Example 4)-5-3-3 was subjected to buffer exchange with PB6.0 according to the common operation C-2 and was prepared at a concentration of 17.3 mg/mL (amount of solution of 6.00 mL). To this solution, 0.06990 mL EndoS solution (phosphate buffer saline; hereinafter, PBS, 7.52 mg/mL) was added and the mixture was incubate at 37°C for 2 hours. After confirming that the glycan had been cleaved using an Agilent 2100 bioanalyzer, the mixture was purified by affinity chromatography and a hydroxyapatite column according to the following method.

(1) Purification

Purification device: AKTA avant (manufactured by GE Healthcare Inc.)
Column: GSTrap (trademark) HP (5 mL) (Cytiva, Inc.) and Bio-Scale Mini CHT Type I Cartridge (5 mL) (manufactured by Bio-Rad Laboratories, Inc.)
Column volume (CV): 10.54 mL
Flow rate: 5 mL/min (1.25 mL/min when charging)

A GSTrap (trademark) HP (upper) and a CHT column (lower) were connected, and a reaction solution was added

to the GSTrap (trademark). Solution A (5 mM phosphate buffer solution, 50 mM 2-morpholinoethanesulfonic acid (MES) solution (pH 6.8)) was run at 1.25 mL/min for 4 CV, and further 3 CV at 5 mL/min. Next, solution B (5 mM phosphate buffer solution, 50 mM 2-morpholinoethanesulfonic acid (MES) solution (pH 6.8), 0.55 to 0.60 M sodium chloride solution) was run for 5 CV to elute an antibody fraction. The resulting antibody solution was subjected to buffer solution exchange by the common operation C-1 to obtain 19.4 mg/mL (Fucα1,6)GlcNAc-H02L01 antibody solution (PB 6.0) (5.14 mL).

Step 2: Preparation of H02L01-[MSG1-$N_3$]$_2$

To 19.4 mg/mL (Fucα1,6)GlcNAc-H02L01 antibody solution (50 mM PB 6.0) (5.14 mL) obtained in the above step 1, a glycan oxazoline (3aR,5R,6S,7R,7aR)-3a,6,7,7a-Tetrahydro-7-hydroxy-5-(hydroxymethyl)-2-methyl-5H-pyrano [3,2-d]oxazol-6-yl O-[N5-acetyl-N1-[2-[2-[2-(2-azidoethoxy)ethoxy]ethoxy]ethyl]-α-neuraminamidosyl]-(2→6)-O-β-D-galactopyranosyl-(1→4)-O-2-(acetylamino) -2-deoxy-β-D-glucopyranosyl-(1→2)-O-α-D-mannopyranosyl-(1→3)-O-[O-β-D-galactopyranosyl-(1→4)-O-2-(acetylamino)-2-deoxy-β-D-glucopyranosyl-(1→2)-α-D-mannopyranosyl-(1→6)]-β-D-mannopyranoside (International Publication No. 2019/065964, hereinafter, [N3-PEG(3)]-MSG1-Ox) solution (PB6.0) (50.0 mg/mL, 0.310 mL) and a GST-EndoS D233Q/Q303L solution (International Publication No. 2017/010559) (PBS) (4.80 mg/mL, 0.415 mL) were added and the mixture was incubated at 30°C for 3 hours. After confirming the transfer of the glycan using an Agilent 2100 bioanalyzer, antibody fractions were eluted with GST, CHT column (AKTA) in the same manner as in the step 1. The fraction of interest was subjected to buffer exchange by the common operation C-1 to obtain an H02L01-[MSG1-$N_3$]$_2$ solution (ABS) (10.2 mg/mL, 9.40 mL).

[Example 5-2: H02L01-V1-[MSG1-$N_3$]$_2$]

[Chem. 55]

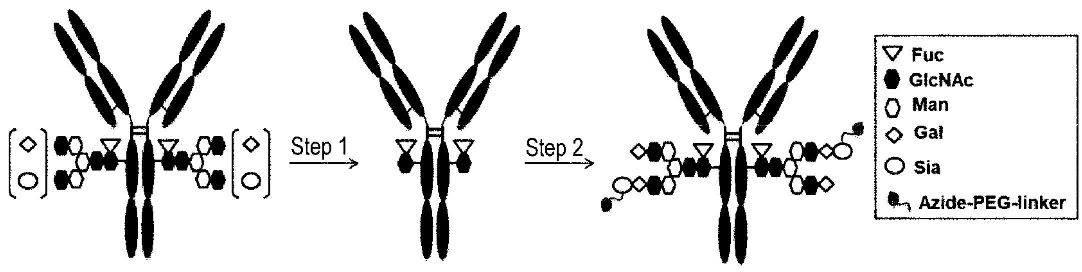


H02L01-V1 (Fucα1, 6)GlcNAc - H02L01-V1 H02L01-V1-[MSG1-$N_3$]$_2$

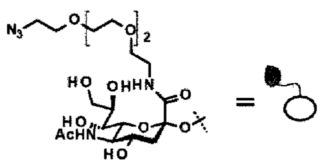

Step 1: Preparation of (Fucα1,6)GlcNAc-H02L01-V1 Antibody

The H02L01-V1 antibody solution prepared in Example 4)-5-3-3 was subjected to buffer exchange with PB6.0 according to the common operation C-2 and was prepared at a concentration of 16.5 mg/mL (6.00 mL). The same operation as in step 1 of Example 5-1 was performed to obtain a (Fucα1,6)GlcNAc-H02L01-V1 solution (PB6.0) (20.6 mg/mL, 4.50 mL).

Step 2: Preparation of H02L01-V1-[MSG1-$N_3$]$_2$

The same operation as in step 2 of Example 5-1 was performed by using the (Fucα1,6)GlcNAc-H02L01-V1 solution (PB6.0) (20.6 mg/mL, 4.50 mL) obtained in the above step

1 to obtain a H02L01-V1-[MSG1-$N_3$]$_2$ (ABS) (10.2 mg/mL, 8.86 mL).

[Example 5-3: H02L01-V2-[MSG1-$N_3$]$_2$]

[Chem. 56]

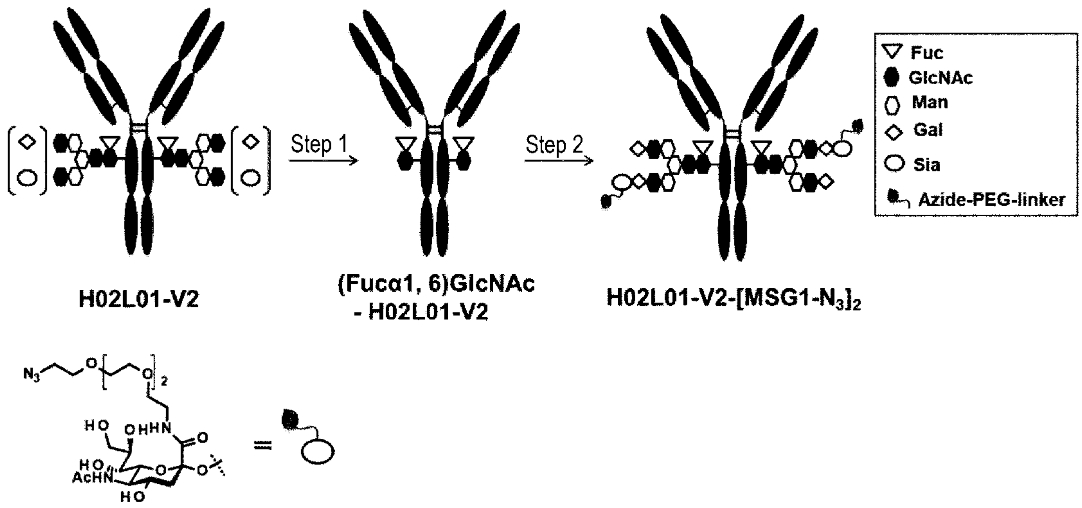


Step 1: Preparation of (Fucα1,6)GlcNAc-H02L01-V2 Antibody

The H02L01-V2 antibody solution prepared in Example 4)-5-3-3 was subjected to buffer exchange with PB6.0 according to the common operation C-2 and was prepared at a concentration of 17.7 mg/mL (1.20 mL). The same operation as in step 1 of Example 5-1 was performed to obtain a (Fucα1,6)GlcNAc-H02L01-V2 solution (PB6.0) (18.5 mg/mL, 1.00 mL).

Step 2: Preparation of H02L01-V2-[MSG1-$N_3$]$_2$

The same operation as in step 2 of Example 5-1 was performed by using the (Fucα1,6)GlcNAc-H02L01-V2 solution (PB6.0) (18.5 mg/mL, 1.00 mL) obtained in the above step 1 to obtain a H02L01-V2-[MSG1-$N_3$]$_2$ (ABS) (10.9 mg/mL, 1.50 mL).

[Reference Example 3: Control hIgG-[MSG1-$N_3$]$_2$]

[Chem. 57]

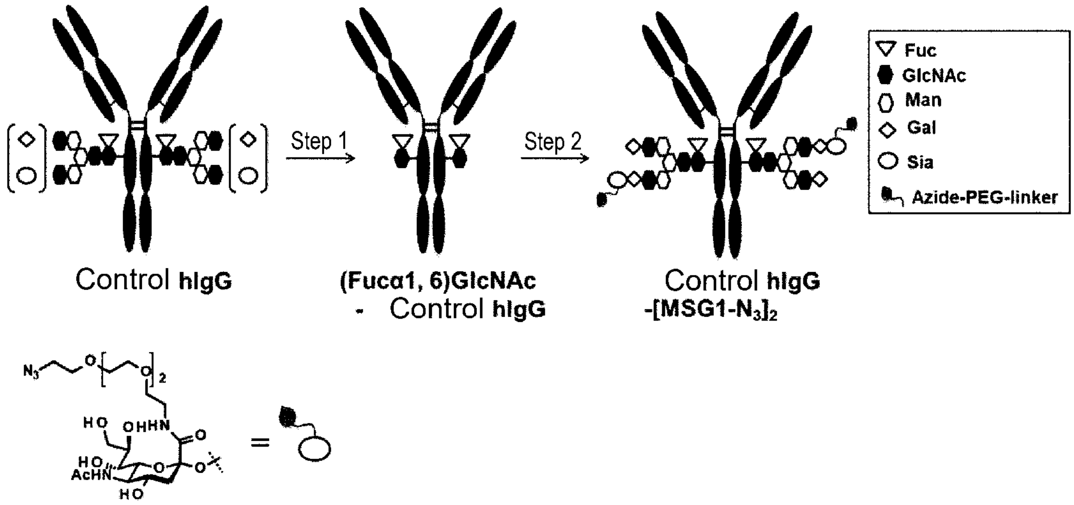


Step 1: Preparation of (Fucα1,6)GlcNAc-Control hIgG Antibody

The control hIgG antibody solution prepared in Reference Example 2 was subjected to buffer exchange with PB6.0 according to the common operation C-1 and was prepared at a concentration of 12.0 mg/mL (4.80 mL). The same operation as in step 1 of Example 5-1 was performed to obtain a (Fucα1,6)GlcNAc-control hIgG solution (PB6.0) (14.6 mg/mL, 3.50 mL).

Step 2: Preparation of Control hIgG-[MSG1-$N_3$]$_2$

The same operation as in step 2 of Example 5-1 was performed by using the (Fucα1,6)GlcNAc-control hIgG solution (PB6.0) (14.6 mg/mL, 3.50 mL) obtained in the above step 1 to obtain a control hIgG-[MSG1-$N_3$]$_2$ (ABS) (10.3 mg/mL, 4.50 mL).

[Example 5-4]

[Chem. 58]

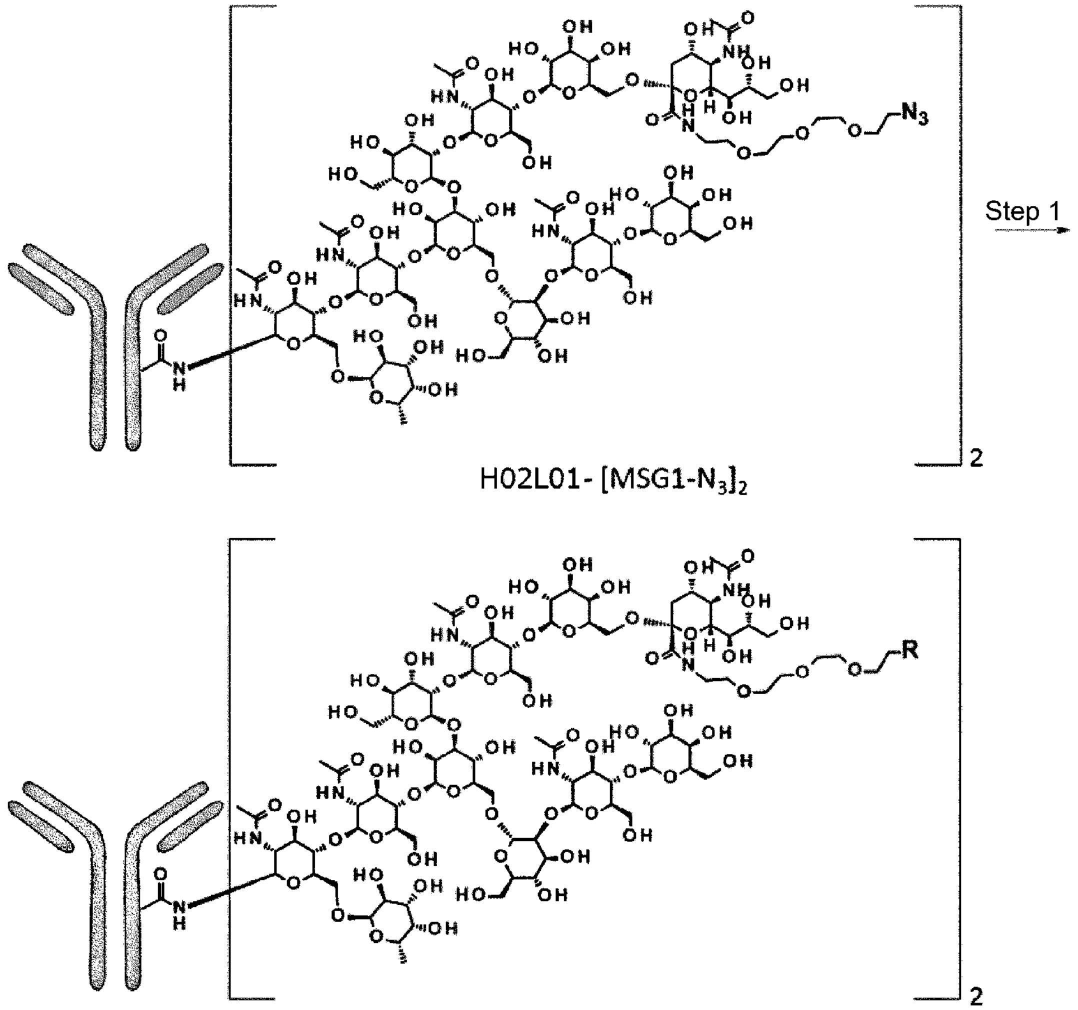


H02L01- $[MSG1\text{-}N_3]_2$

[Chem. 59]

R =

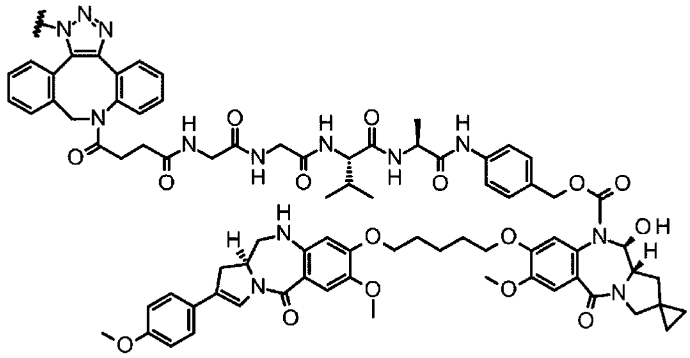

or

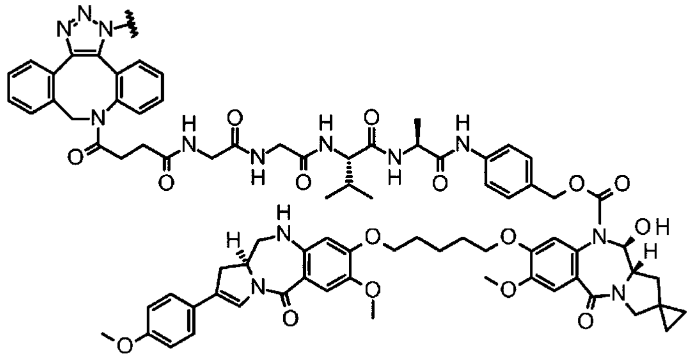

(The compound obtained in step 1 of Example 5-1 can be obtained as a mixture of triazole geometric isomer shown by R.)

To the H02L01-[MSG1-$N_3$]$_2$ (10.2 mg/mL, 10.9 mL) (ABS) obtained in step 2 of Example 5-1, 1,2-propanediol (10.4 mL), and a solution of N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepine-8 -yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (International Publication No. 2020/196474, hereinafter referred to as "Linker 1") in 10 mM dimethylsulfoxide (0.448 mL; 6 equivalents per antibody molecule) were added at room temperature, and the reaction was carried out at room temperature for 48 hours using a tube rotator (MTR-103, AS ONE Corporation).

Purification operation: the above solution was purified by the common operation D to obtain 45.5 mL solution of a compound of interest.

Characteristic evaluation: the following characteristic values were obtained by the common operations E and F. The molar absorption coefficient of a linker 1 (280 nm) was 23155.

Antibody concentration: 1.96 mg/mL, antibody yield: 89.2 mg (81%), DAR: 1.9

[Example 5-5]

[Chem. 60]

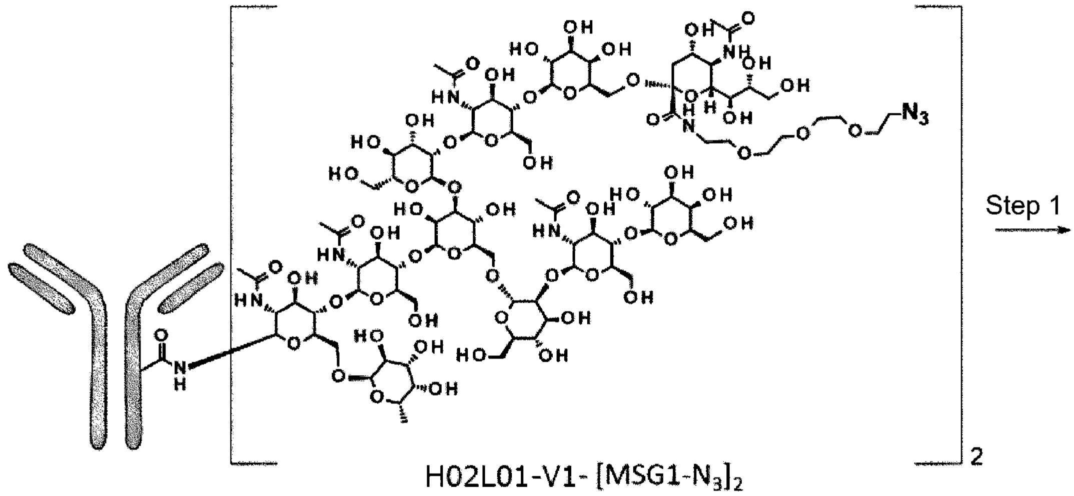


H02L01-V1- [MSG1-$N_3$]$_2$

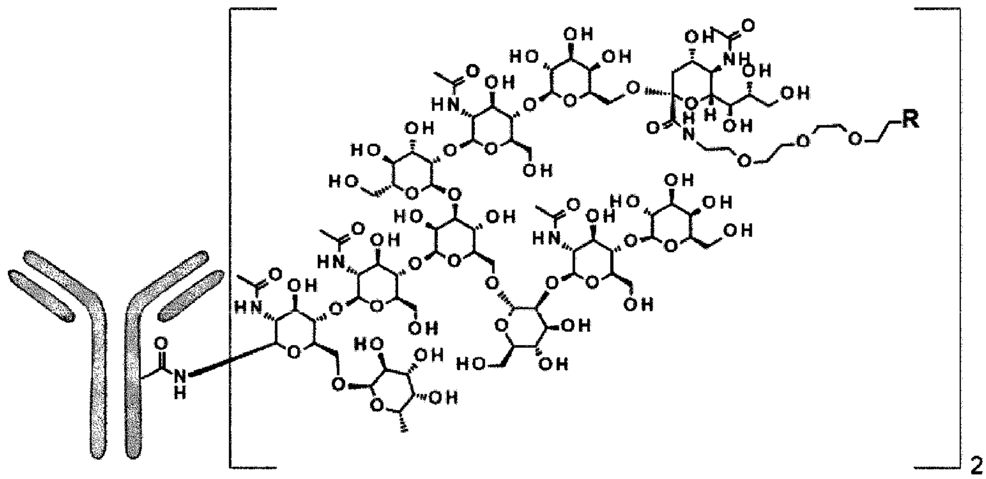

[Chem. 61]

R =

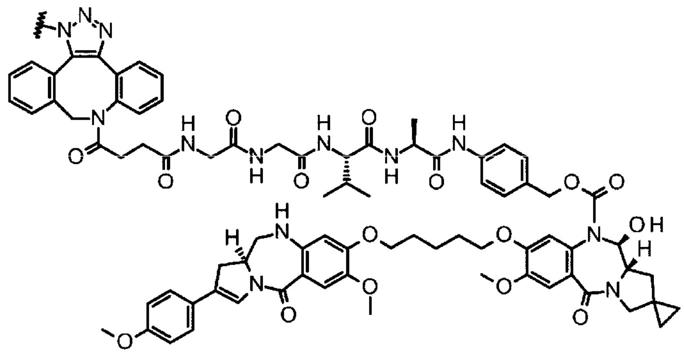

or

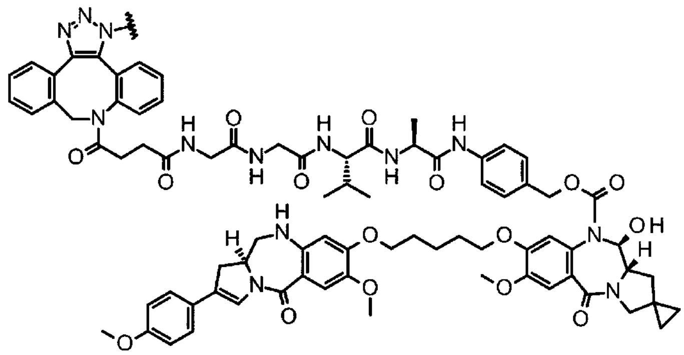

(The compound obtained in step 1 of Example 5-2 can be obtained as a mixture of triazole geometric isomer shown by R.)

To the H02L01-V1-[MSG1-$N_3$]$_2$ (10.0 mg/mL, 14.6 mL) (ABS) obtained in step 2 of Example 5-2, 1,2-propanediol (14.0 mL), and a solution of the Linker 1 in 10 mM dimethylsulfoxide (0.595 mL; 6 equivalents per antibody molecule) were added at room temperature, and the reaction was carried out at room temperature for 48 hours using a tube rotator (MTR-103, AS ONE Corporation).
Purification operation: the above solution was purified by the common operation D to obtain 59.5 mL solution of a compound of interest.
Characteristic evaluation: the following characteristic values were obtained by the common operations E and F.
Antibody concentration: 2.09 mg/mL, antibody yield: 124 mg (85%), DAR: 1.9

[Example 5-6]

[Chem. 62]

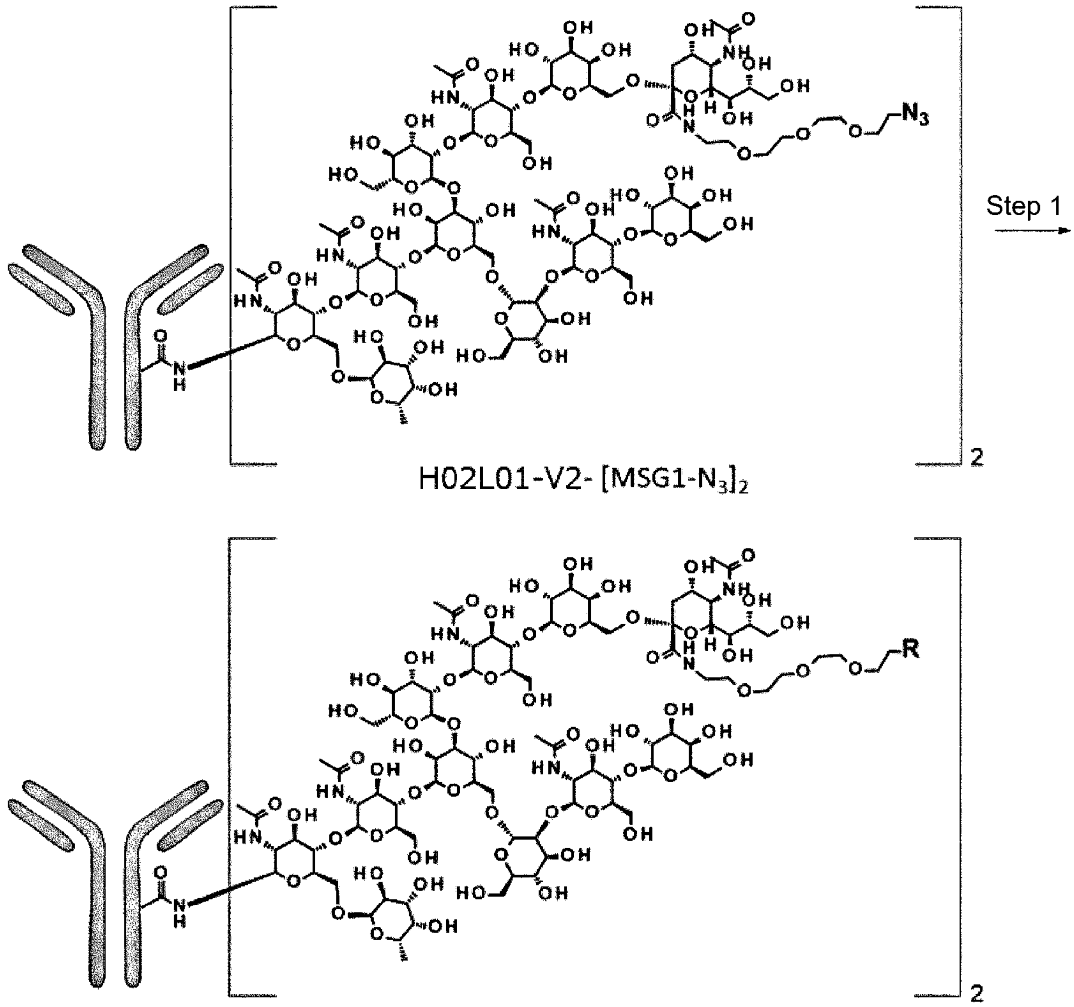


H02L01-V2- [MSG1-$N_3$]$_2$

[Chem. 63]

R =

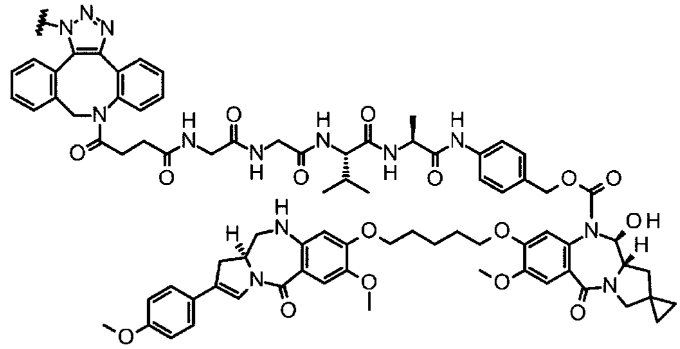

or

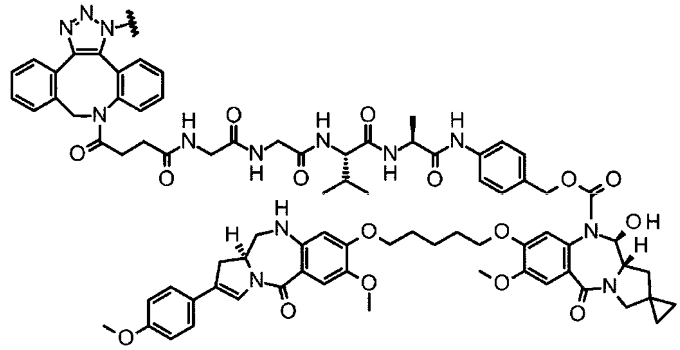

(The compound obtained in step 1 of Example 5-3 can be obtained as a mixture of triazole geometric isomer shown by R.)

To the H02L01-V2-[MSG1-$N_3$]$_2$ (10.9 mg/mL, 1.00 mL) (ABS) obtained in step 2 of Example 5-3, 1,2-propanediol (0.956 mL), and a solution of the Linker 1 in 10 mM dimethylsulfoxide (0.0445 mL; 6 equivalents per antibody molecule) were added at room temperature, and the reaction was carried out at room temperature for 48 hours using a tube rotator (MTR-103, AS ONE Corporation).

Purification operation: the above solution was purified by the common operation D to obtain 5.00 mL solution of a compound of interest.

Characteristic evaluation: the following characteristic values were obtained by the common operations E and F.

Antibody concentration: 1.78 mg/mL, antibody yield: 8.90 mg (81%), DAR: 1.9

[Reference Example 4: Control hIgG-ADC (Control hIgG-PBD)]

[Chem. 64]

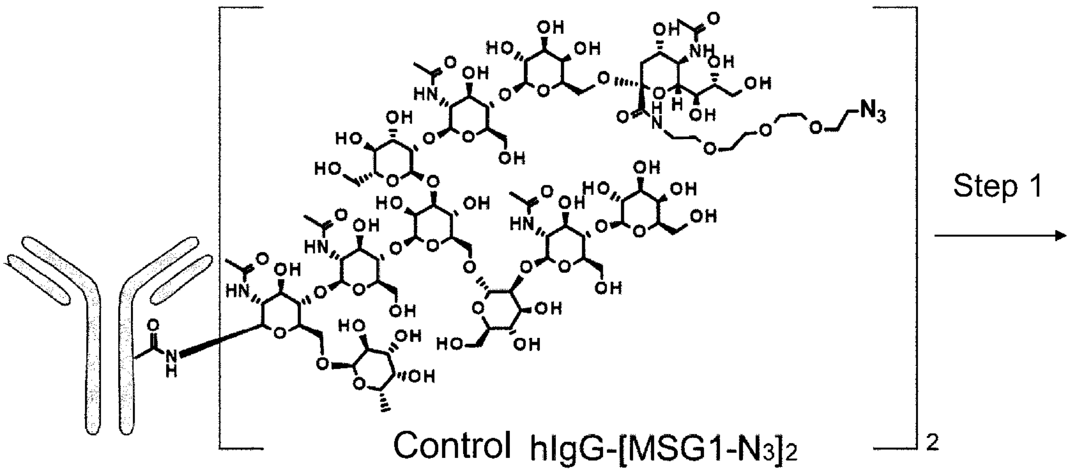


Control hIgG-[MSG1-$N_3$]$_2$

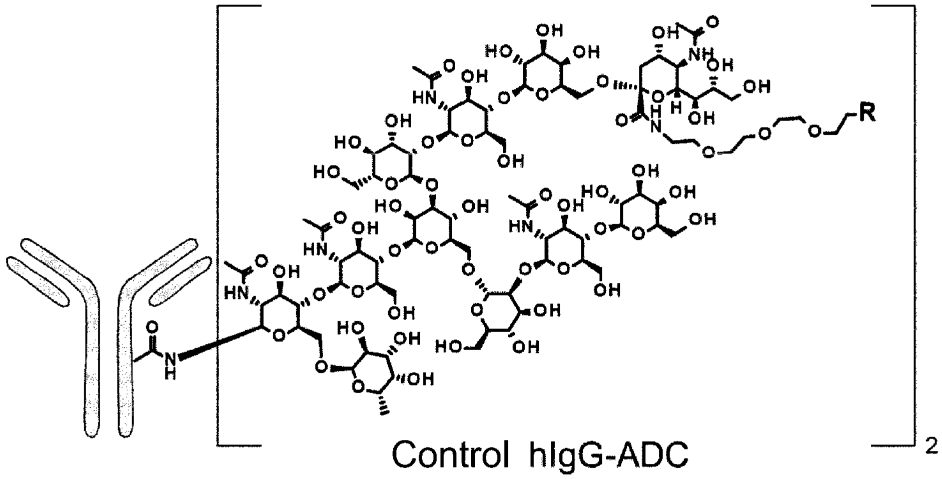

Control hIgG-ADC

[Chem. 65]

R = 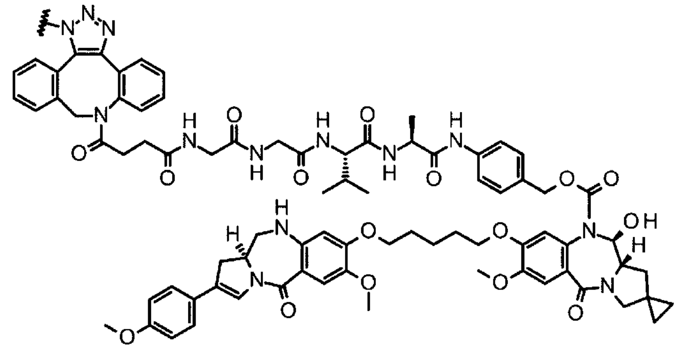

or

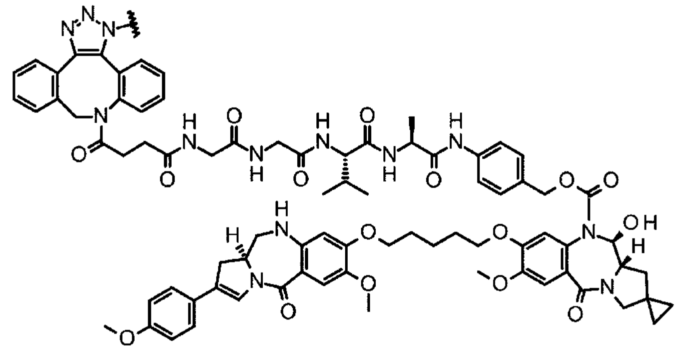

(The compound obtained in step 1 of Reference Example 3 can be obtained as a mixture of triazole geometric isomer shown by R.)

To the control hIgG-[MSG1-$N_3$]$_2$ (10.3 mg/mL, 1.50 mL) (ABS) obtained in step 2 of Reference Example 3, 1,2-propanediol (1.44 mL), and a solution of the Linker 1 in 10 mM dimethylsulfoxide (0.064 mL; 6 equivalents per antibody molecule) were added at room temperature, and the reaction was carried out at room temperature for 67 hours using a tube rotator (MTR-103, AS ONE Corporation).

Purification operation: the above solution was purified by the common operation D to obtain 7.00 mL solution of a compound of interest.

Characteristic evaluation: the following characteristic values were obtained by the common operations E and F.

Antibody concentration: 1.70 mg/mL, antibody yield: 11.9 mg (77%), DAR: 1.8

Reference Example 5 Preparation of huM25-vcMMAE-E2:

Reference Example 5-1: Preparation of Antibody Conjugate

The huM25 solution prepared in Reference Example 1)-2-2 (antibody) was subjected to buffer exchange with PBS 5 mM EDTA using NAP-25 (the common operation C-2) and was prepared at a concentration of 10.01 mg/mL (5.35 mL). To an aqueous solution of the antibody, an aqueous solution of 10 mM Tris(2-carboxyethyl)phosphine hydrochloride (TCEP) (0.037 mL, 1 equivalent per antibody molecule) was added at room temperature, and the mixture was adjusted with an aqueous solution of 1M dipotassium hydrogen phosphate (0.535 mL) to be pH 7.70, followed by stirring at 37°C for 2 hours. A solution of 10 mM vcMMAE (CAS number: 646502-53-6) in DMSO (0.088 mL, 2.2 equivalents per antibody molecule) was added to the reaction solution, which was then stirred at room temperature for 1 hour. An aqueous solution of N-acetyl-L-cysteine (NAC) (0.008 mL, 2.2 equivalents per antibody molecule) was added thereto, and the mixture was stirred at room temperature for 20 minutes to terminate the reaction.

Purification operation: the above reaction solution was purified using NAP-25 (the common operation D) to obtain 14.0 mL solution of an antibody conjugate (3.39 mg/mL, 47 mg).

The resulting antibody conjugate was subjected to Hydrophobic Interaction Chromatography (hereinafter, HIC) analysis, and it was confirmed that the following distribution was obtained.

DAR0 fraction = 46%,
DAR2 fraction = 42%,
DAR4 fraction = 11%,
DAR6 fraction = 1%.

HIC Analysis Conditions

HPLC: LC-20AD
Column: TSKgel Butyl-NPR, 4.6 x 100 mm, 2.5 mm (Tosoh Corporation, Part No. 0042168, Column No. 97C00072D)
Temperature: 30°C
Detection: 280 nm
Flow rate: 0.8 mL/min
Solvent: Solution A 1.5 mol/L-ammonium sulfate, 25 mM-phosphate buffer solution, pH 7.0
Solution B 25% v/v-isopropanol, 25 mM-phosphate buffer solution, pH 7.0
Gradient conditions: (B%, min) = (15,0) - (90,20) - (10,20.01) - (10,30)

Reference Example 5-2 Preparation of huM25-vcMMAE: HIC Purification

Purification device: AKTA avant (manufactured by GE Healthcare Inc.)
Column: HiTrap Butyl HP (5 mL, GE Healthcare Japan)
Column volume (CV): 5 mL
Flow rate: 5 mL/min (2.5 mL/min when charging)
Solution A: 1.5 mol/L-ammonium sulfate, 25 mM-phosphate buffer solution, pH 7.0
Solution B: 25% v/v-isopropanol, 25 mM-phosphate buffer solution, pH 7.0
HiTrap Butyl HP was equilibrated with solution A/solution B (85/15).
Sample preparation: the conjugate solution obtained by the above operation was replaced with solution A (the common operation C-1).
Purification: the sample solution prepared above (the amount of sample loaded was about 10 mg was charged onto the column at a flow rate of 2.5 mL/min. Solution A/solution B (85/15) was run for 2 CV at a flow rate of 5 mL/min. Next, solution A/solution B was run for 20 CV under gradient conditions (15/85 -> 20/80). In addition, solution A/solution B (0/100) was run for 5 CV, and a fraction of huM25-vcMMAE (DAR2) was collected. The resulting aqueous fraction solution was replaced with ABS solution using an Amicon (the common operation C-1).

The above conjugation and HIC purification were repeated to obtain 12.3 mL of an ABS solution of huM25-vcMMAE (1.32 mg/mL, 1.32 mg/mL, DAR: 2.1). (The fact that the DAR2 fraction had been purified was confirmed by the above-described HIC analysis method. The concentration and the exact DAR value were calculated by the common operations E and F. The molar absorption coefficient (280 nm) of vcMMAE was 2804, and the antibody physical properties were as shown in Table 1.)

Reference Example 6 Preparation of huM25-ADC (huM25-PBD) :

[Reference Example 6-1: huM25-[MSG1-$N_3$]$_2$]

[Chem. 66]

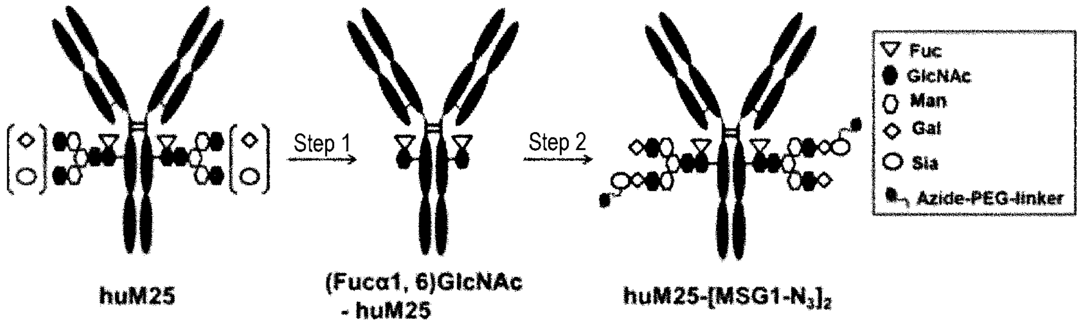


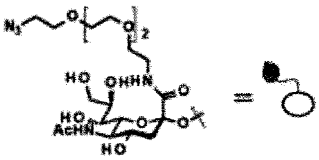

Step 1: Preparation of (Fucα1,6)GlcNAc-huM25 Antibody

The huM25 antibody solution prepared in Reference Example 1)-2 was subjected to buffer exchange with PB6.0 according to the common operation C-2 and was prepared at a concentration of 18.1 mg/mL (1.00 mL). The same operation as in step 1 of Example 5-1 was performed to obtain a (Fucα1,6)GlcNAc-huM25 solution (PB6.0) (16.8 mg/mL, 0.800 mL).

Step 2: Preparation of huM25-[MSG1-$N_3$]$_2$

The same operation as in step 2 of Example 5-1 was performed by using the (Fucα1,6)GlcNAc-huM25 solution (PB6.0) (16.8 mg/mL, 0.800 mL) obtained in the above step 1 to obtain a huM25-[MSG1-$N_3$]$_2$ (ABS) (15.9 mg/mL, 1.50 mL) .

[Reference Example 6-2]

[Chem. 67]

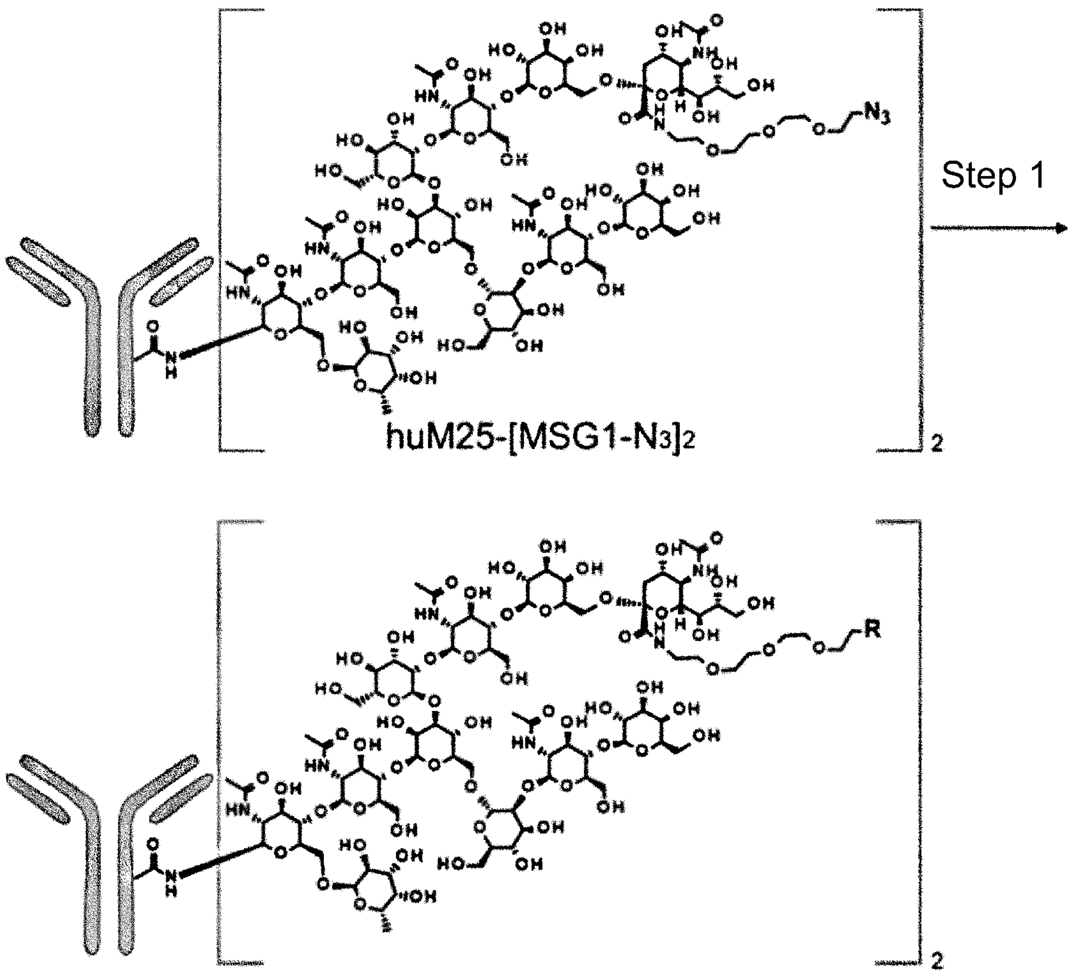


huM25-[MSG1-$N_3$]$_2$

huM25-ADC

[Chem. 68]

R = 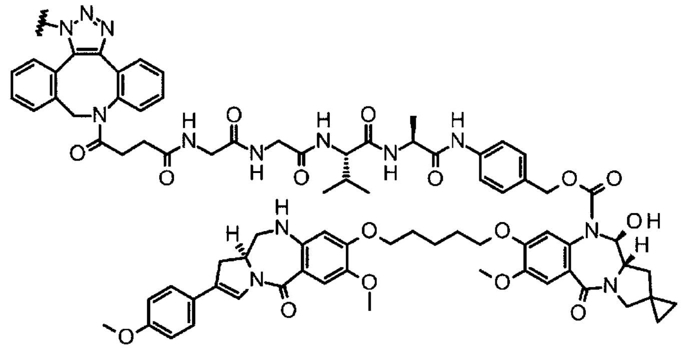

or

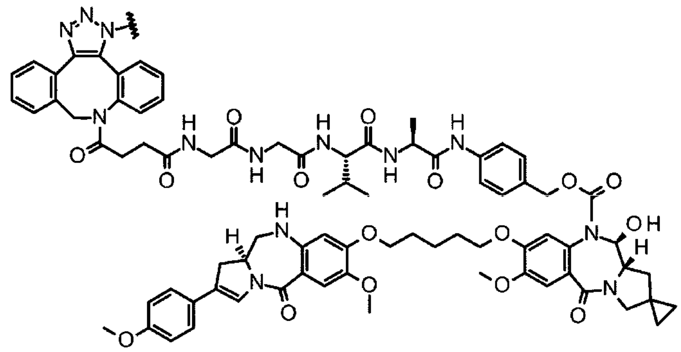

(The compound obtained in step 1 of Reference Example 6-1 can be obtained as a mixture of triazole geometric isomer shown by R.)

To the huM25-[MSG1-$N_3$]$_2$ (10.6 mg/mL, 1.00 mL) (ABS) obtained in step 2 of Reference Example 6-1, 1,2-propanediol (0.956 mL), and a solution of the Linker 1 in 10 mM dimethylsulfoxide (0.0435 mL; 6 equivalents per antibody molecule) were added at room temperature, and the reaction was carried out at room temperature for 48 hours using a tube rotator (MTR-103, AS ONE Corporation).

Purification operation: the above solution was purified by the common operation D to obtain 5.00 mL solution of a compound of interest.

Characteristic evaluation: the following characteristic values were obtained by the common operations E and F.

Antibody concentration: 1.76 mg/mL, antibody yield: 8.79 mg (83%), DAR: 1.9

<Example 6: in vitro Activity Evaluation of Antibody-Drug Conjugate>

6)-1 in vitro Evaluation of Cell Growth Inhibitory Activity of Antibody-Drug Conjugate Against Cell Lines

LRRC15-positive human osteosarcoma cell line HuO9 (JCRB) was seeded in 10% FBS-containing RPMI medium at 5 x $10^3$ cells/50 μL/well, LRRC15-positive human melanoma cell line Hs294T was seeded in 10% FBS and 20 ng/mL TGFβ1-containing RPMI medium at 5 x $10^3$ cells/50 μL/well, or, as a negative control, LRRC15-negative HEK293α was seeded in 10% FBS-containing RPMI medium at 2 x $10^3$ cells/50 μL/well in a 96-well plate, and cultured overnight under conditions of 37°C and 5% $CO_2$. The next day, the three types of hR024-drug conjugates (clone names of H02L01-PBD, H02L01-V1-PBD, and H02L01-V2-PBD) prepared in Example 5 and the control hIgG-PBD were diluted at a common ratio of 3 to a final concentration of 0.0002 (μg/mL) from 1.5 (μg/mL) and added. After 6 days of culture, the number of viable cells was measured by quantification of ATP using CellTiter-Glo 2.0 Assay (Promega). Figure 3 shows concentration-dependent inhibitory activity against cell growth when each antibody-drug conjugate is added. As a result, it was indicated that the three hR024-drug conjugates inhibited tumor cell proliferation when added at a lower concentration against LRRC15-positive HuO9 and Hs294T, compared to the control hIgG-PBD, and had high antitumor activity. On the other hand, in the negative control HEK293α, the three hR024-drug conjugates exhibits growth inhibitory activity equivalent to that of the control hIgG-PBD, indicating that the growth inhibitory activity of the three hR024-drug conjugates

is dependent on LRRC15 expression.

<Example 7: in vivo Antitumor Effect of Antibody-Drug Conjugate>

The antitumor effects of the antibody-drug conjugates were evaluated by using animal models in which cells of the human tumor cell line had been transplanted into immunodeficient mice. 4 to 6 Week-old SCID mice (CB17/Icr-Prkdc [scid]/CrlCrlj, Jackson Laboratory Japan, Inc.) were habituated for 3 days or more under SPF conditions before experimental use. The mice were fed with sterilized solid feed (FR-2, Funabashi Farms Co., Ltd.) and given sterilized tap water (prepared by adding a solution of 5 to 15 ppm sodium hypochlorite). The major and minor diameters of the transplanted tumors were measured twice a week with an electronic digital caliper (CD-15CX, Mitutoyo Corporation), and the estimated tumor volume was calculated using the following formula.

Estimated tumor volume ($mm^3$) = 1/2 $\times$ major diameter (mm) $\times$ [minor diameter (mm)]$^2$

All antibody-drug conjugates were diluted with ABS and administered into the tail vein at doses shown in each Example. In addition, ABS (vehicle) or control hIgG-PBD prepared in Reference Example 4 was administered as the control group, in a similar manner. 5 to 6 Mice were used per group in the experiment.

7)-1 Antitumor Effect (EBC-1)

LRRC15-negative human lung carcinoma cell line EBC-1 (JCRB) was suspended in PBS mixed with 50% Matrigel (Corning Incorporated), 1.0 $\times$ $10^6$ cells were subcutaneously transplanted into the right flank of female SCID mice (Day 0), and random grouping was performed on Day 9. From publicly known information, it is known that in this tumor model, carcinoma cells are LRRC15 negative, and cells in the tumor stroma are LRRC15 positive (International Publication No. 2017/095805). On the day of grouping, a vehicle, the antibody-drug conjugate H02L01-PBD, H02L01-V1-PBD, or H02L01-V2-PBD prepared in Example 5 was administered into the tail vein at a dose of 0.4 mg or 0.2 mg/kg. The results are shown in Figure 4. The abscissa indicates the number of days, and the ordinate indicates estimated tumor volume, and the error bars indicate SEM values. As a result, it was indicated that the three hR024-drug conjugates have antitumor activity higher than that of the vehicle group.

7)-2 Antitumor Effect (HCC827)

LRRC15-negative human lung carcinoma cell line HCC827 (ATCC) was suspended in PBS mixed with 50% Matrigel (Corning Incorporated), 1 μg/mL FGF2 (Medical & Biological Laboratories Co., Ltd.), and 100 μg/mL heparin (Sigma Aldrich Co. LLC), and 2.5 $\times$ $10^6$ cells were subcutaneously transplanted into the right flank of female SCID mice (Day 0), and random grouping was performed on Day 10. From publicly known information, it is known that in this tumor model, carcinoma cells are LRRC15 negative, and cells in the tumor stroma are LRRC15 positive (International Publication No. 2017/095805). On the day of grouping, the vehicle, the antibody-drug conjugates H02L01-PBD and H02L01-V1-PBD prepared in Example 5, or the control hIgG-PBD prepared in Reference Example 4 was administered at a dose of 0.4 mg/kg, and the huM25-MMAE prepared in Reference Example 5 was administered at a dose of 0.4 mg/kg or 3.6 mg/kg into the tail vein. The results are shown in Figure 5. The abscissa indicates the number of days, and the ordinate indicates estimated tumor volume, and the error bars indicate SEM values. As a result, it was indicated that the two hR024-drug conjugates have antitumor activity higher than that of the vehicle, the control hIgG-PBD, and huM25-MMAE.

7)-3 Antitumor Effect (HuO9)

Based on publicly known information, LRRC15-positive human osteosarcoma cell line HuO9 (JCRB) was suspended in Matrigel (Corning Incorporated), 5.0 $\times$ $10^6$ cells were subcutaneously transplanted into the right flank of female SCID mice (Day 0), and random grouping was performed on Day 10. On the day of grouping, the vehicle, the antibody-drug conjugates H02L01-PBD, H02L01-V1-PBD, and H02L01-V2-PBD prepared in Example 5, or the control hIgG-PBD prepared in Reference Example 4 was administered at a dose of 0.4 mg/kg, and the huM25-MMAE prepared in Reference Example 5 was administered at a dose of 0.4 mg/kg or 3.6 mg/kg into the tail vein. The results are shown in Figure 6. The abscissa indicates the number of days, and the ordinate indicates estimated tumor volume, and the error bars indicate SEM values. As a result, it was indicated that the three hR024-drug conjugates have antitumor activity higher than that of the vehicle, the control hIgG-PBD, and huM25-MMAE, in particular, HO2L01-V1-PBD and H02L01-V2-PBD were shown to lead to tumor regression.

<Example 8: Pharmacokinetic Study of Anti-LRRC15 Antibody in Mice>

A pharmacokinetic study was carried out using six female Balb/c mice per group for the anti-LRRC15 antibodies hR024 H02-V1 and hR024 H02-V2 prepared in Example 4, and the anti-LRRC15 antibody huM25 prepared in Reference Example 1. Each anti-LRRC15 antibody was intravenously administered to the mice at 1 mg/kg, and blood was collected from the tail vein using a heparinized hematocrit tube 1, 24, 72, 168, 336, and 504 hours later, and plasma was obtained by centrifugation at 4°C and 15000 rpm for 5 minutes. The amount of anti-LRRC15 antibody in plasma was measured using a fully automated immunoassay platform, Gyrolab xP Workstation (GYROS PROTEIN Technologies AB), with biotin-labeled anti-human IgG goat antibody (Southern Biotech, Inc.) as the capture antibody and Alexa fluor 647-labeled anti-human IgG goat antibody (Southern Biotech, Inc.) as the detection antibody. Figure 29 shows a graph showing the plasma antibody concentration and its SD value at each time after administration.

In mouse bodies, antibodies hR024 H02-V1 and hR024 H02-V2 showed higher exposure and sustained pharmacokinetics than the antibody huM25.

<Example 9: Analysis of LRRC15 Binding Site of hR024>

9)-1 Construction of Human LRRC15 Mutant Expression Vector

The full-length human LRRC15 has 17 extracellular domains. Mutant genes obtained by deleting amino acid residues in each domain of the human LRRC15 extracellular domain or by replacing them with other amino acid residues were inserted into the pDisplay vector to prepare 16 types of mutant expression vectors (GenScript Inc.). The wild-type expression vector for the human LRRC15 extracellular domain is referred to as WT, and each mutant expression vector is referred to as:

- Mut 1 (a mutant obtained by deleting one or more amino acid residues within the region consisting of amino acid residues 22 to 75 of SEQ ID NO: 1 or by substituting the one or more amino acid residues with another amino acid residue or other amino acid residues),
- Mut 2 (a mutant obtained by deleting one or more amino acid residues within the region consisting of amino acid residues 78 to 99 of SEQ ID NO: 1 or by substituting the one or more amino acid residues with another amino acid residue or other amino acid residues),
- Mut 3 (a mutant obtained by deleting one or more amino acid residues within the region consisting of amino acid residues 102 to 123 of SEQ ID NO: 1 or by substituting the one or more amino acid residues with another amino acid residue or other amino acid residues),

- Mut 4 (a mutant obtained by deleting one or more amino acid residues within the region consisting of amino acid residues 126 to 149 of SEQ ID NO: 1 or by substituting the one or more amino acid residues with another amino acid residue or other amino acid residues),
- Mut 5 (a mutant obtained by deleting one or more amino acid residues within the region consisting of amino acid residues 150 to 173 of SEQ ID NO: 1 or by substituting the one or more amino acid residues with another amino acid residue or other amino acid residues),
- Mut 6 (a mutant obtained by deleting one or more amino acid residues within the region consisting of amino acid residues 174 to 197 of SEQ ID NO: 1 or by substituting the one or more amino acid residues with another amino acid residue or other amino acid residues),
- Mut 7 (a mutant obtained by deleting one or more amino acid residues within the region consisting of amino acid residues 198 to 221 of SEQ ID NO: 1 or by substituting the one or more amino acid residues with another amino acid residue or other amino acid residues),
- Mut 8 (a mutant obtained by deleting one or more amino acid residues within the region consisting of amino acid residues 222 to 245 of SEQ ID NO: 1 or by substituting the one or more amino acid residues with another amino acid residue or other amino acid residues),
- Mut 9 (a mutant obtained by deleting one or more amino acid residues within the region consisting of amino acid residues 246 to 269 of SEQ ID NO: 1 or by substituting the one or more amino acid residues with another amino acid residue or other amino acid residues),
- Mut 10 (a mutant obtained by deleting one or more amino acid residues within the region consisting of amino acid residues 270 to 293 of SEQ ID NO: 1 or by substituting the one or more amino acid residues with another amino acid residue or other amino acid residues),
- Mut 11 (a mutant obtained by deleting one or more amino acid residues within the region consisting of amino acid residues 294 to 317 of SEQ ID NO: 1 or by substituting the one or more amino acid residues with another amino acid residue or other amino acid residues),

- Mut 12 (a mutant obtained by deleting one or more amino acid residues within the region consisting of amino acid residues 318 to 341 of SEQ ID NO: 1 or by substituting the one or more amino acid residues with another amino acid residue or other amino acid residues),
- Mut 13 (a mutant obtained by deleting one or more amino acid residues within the region consisting of amino acid residues 342 to 365 of SEQ ID NO: 1 or by substituting the one or more amino acid residues with another amino acid residue or other amino acid residues),
- Mut 14 (a mutant obtained by deleting one or more amino acid residues within the region consisting of amino acid

residues 366 to 389 of SEQ ID NO: 1 or by substituting the one or more amino acid residues with another amino acid residue or other amino acid residues),

- Mut 15 (a mutant obtained by deleting one or more amino acid residues within the region consisting of amino acid residues 390 to 422 of SEQ ID NO: 1 or by substituting the one or more amino acid residues with another amino acid residue or other amino acid residues), and
- Mut 16 (a mutant obtained by deleting one or more amino acid residues within the region consisting of amino acid residues 423 to 475 of SEQ ID NO: 1 or by substituting the one or more amino acid residues with another amino acid residue or other amino acid residues).

9)-2 Analysis of LRRC15 Binding Site of hR024 by Flow Cytometry Using Mutant

The human LRRC15 wild-type and respective mutant expression vectors prepared in Example 9)-1 were introduced into HEK293α by the same method as in Example 4)-6-2-1. The binding activity of the three hR024 prepared in Example 4)-5 (clone numbers of H02L01, H02L01-V1, and H02L01-V2), huM25 prepared in Reference Example 1, or a commercially available human IgG control (BioLegend, Inc.) to each mutant-expressing HEK293α was analyzed by flow cytometry by the same method as in Example 4)-6-2-2. The results thereof are shown in Figures 30-1 to 30-3. In the histograms in Figures 30-1 to 30-3, the abscissas indicate the fluorescence intensity of APC, which represents the amount of antibody bound, and the ordinates indicate the cell count; the histograms with half-tone dots indicate cases where a human IgG control is used, and the white-solid histograms indicate cases where a humanized anti-LRRC15 antibody is used. As a result, the three hR024 (H02L01, H02L01-V1, and H02L01-V2) did not bind to Mut1 and Mut2, but bound to the others, and it is considered that at least a part of the epitope of the three hR024 exists within Mut1 and Mut2 (the region consisting of the amino acid residues 22 to 75 of SEQ ID NO: 1 and the region consisting of the amino acid residues 78 to 99 of SEQ ID NO: 1). huM25 did not bind to Mut13 and Mut14, but bound to the others. This result indicated that hR024 and huM25 have different binding sites.

<Example 10: in vivo Antitumor Effect of Antibody-Drug Conjugate>

The antitumor effects of the antibody-drug conjugates were evaluated by using animal models in which cells of a human tumor cell line had been transplanted into immunodeficient mice, or cells of a mouse tumor cell line had been transplanted into immunocompetent mice. 4 to 6 Week-old SCID mice (CB17/Icr-Prkdc[scid]/CrlCrlj, Jackson Laboratory Japan, Inc.), NSG mice (NOD.Cg-Prkdcscid Il2rgtm1Wjl/SzJ, Jackson Laboratory Japan, Inc.), or B6J mice (C57BL/6J, Jackson Laboratory Japan, Inc.) were habituated for 3 days or more under SPF conditions before experimental use. The mice were fed with sterilized solid feed and given sterilized tap water. The major and minor diameters of the transplanted tumors were measured twice a week with an electronic digital caliper (Mitutoyo Corporation), and the estimated tumor volume was calculated using the following formula. Estimated tumor volume ($mm^3$) = 1/2 × major diameter (mm) × [minor diameter (mm)]$^2$

All antibody-drug conjugates were diluted with ABS and administered into the tail vein at doses shown in each Example. In addition, ABS (vehicle) or control hIgG-PBD prepared in Reference Example 4 was administered as the control group, in a similar manner. 5 to 6 Mice were used per group in the experiment.

10)-1 LRRC15 Expression in Mouse Tumor Model

To analyze the LRRC15 expression in mouse tumor models, immunohistochemical staining of LRRC15 was performed. Formalin-fixed paraffin-embedded (FFPE) sections of mouse subcutaneous tumors were prepared, and the FFPE sections were deparaffinized and antigen-retrieval treatment using PT Link (Agilent Technologies, Inc.). The sections were then blocked using a Link48 automated staining device (Agilent Technologies, Inc.) and incubated with rabbit anti-human/mouse LRRC15 antibody, HRP-labeled anti-rabbit secondary antibody (Agilent Technologies, Inc.), and DAB detection reagent (Agilent Technologies, Inc.).

10)-2 Antitumor Effect (HuO9)

Based on publicly known information, LRRC15-positive human osteosarcoma cell line HuO9 (JCRB) was suspended in Matrigel (Corning Incorporated), $5.0 \times 10^6$ cells were subcutaneously transplanted into the right flank of female SCID mice (Day 0), and random grouping was performed on Day 10. On the day of grouping, a vehicle, the antibody-drug conjugate H02L01-V1-PBD or H02L01-V2-PBD prepared in Example 5, or huM25-PBD prepared in Reference Example 6-2 was administered into the tail vein at a dose of 0.3 mg/kg. The results are shown in Figure 31. The abscissa indicates the number of days, and the ordinate indicates estimated tumor volume, and the error bars indicate SEM values. As a result, it was indicated that the two hR024-drug conjugates have antitumor activity higher than that of the vehicle and huM25-PBD.

10)-3 Antitumor Effect (EBC-1)

Human lung carcinoma cell line EBC-1 (JCRB) was suspended in PBS mixed with 50% Matrigel (Corning Incorporated), $1.0 \times 10^6$ cells were subcutaneously transplanted into the right flank of female SCID mice (Day 0), and random grouping was performed on Day 9. From publicly known information, it is known that in this tumor model, carcinoma cells are LRRC15 negative, and cells in the tumor stroma are LRRC15 positive (International Publication No. 2017/095805). On the day of grouping, a vehicle, the antibody-drug conjugate H02L01-V1-PBD prepared in Example 5, or huM25-PBD prepared in Reference Example 6-2 was administered into the tail vein at a dose of 0.3 mg/kg. The results are shown in Figure 32. The abscissa indicates the number of days, and the ordinate indicates estimated tumor volume, and the error bars indicate SEM values. As a result, it was indicated that H02L01-V1-PBD has antitumor activity higher than that of the vehicle and huM25-PBD.

10)-4 Antitumor Effect (SUM190PT)

HER2-positive breast carcinoma cell line SUM190PT (BioIVT, LLC) was suspended in PBS mixed with 50% Matrigel (Corning Incorporated), $4.0 \times 10^6$ cells were subcutaneously transplanted into the right flank of female SCID mice (Day 0), and random grouping was performed on Day 32. From publicly known information, it is known that in this tumor model, carcinoma cells are LRRC15 negative, and cells in the tumor stroma are LRRC15 positive (International Publication No. 2017/095805). On the day of grouping, a vehicle, the antibody-drug conjugate H02L01-V1-PBD prepared in Example 5, or a control hIgG-PBD prepared in Reference Example 4 was administered into the tail vein at a dose of 0.4 mg/kg. The results are shown in Figure 33. The abscissa indicates the number of days, and the ordinate indicates estimated tumor volume, and the error bars indicate SEM values. As a result, it was indicated that H02L01-V1-PBD has antitumor activity higher than that of the vehicle and control hIgG-PBD.

10)-5 Antitumor Effect (HCC1806)

Triple-negative breast carcinoma cell line HCC1806 (ATCC) was suspended in PBS mixed with 50% Matrigel (Corning Incorporated), $1.0 \times 10^6$ cells were subcutaneously transplanted into the right flank of female SCID mice (Day 0), and random grouping was performed on Day 7. From the IHC shown in Example 10)-1, it was confirmed that in this tumor model, carcinoma cells are LRRC15 negative, and cells in the tumor stroma are LRRC15 positive. On the day of grouping, a vehicle, the antibody-drug conjugate H02L01-PBD or H02L01-V1-PBD prepared in Example 5 was administered into the tail vein at a dose of 0.4 mg/kg. The results are shown in Figure 34. The abscissa indicates the number of days, and the ordinate indicates estimated tumor volume, and the error bars indicate SEM values. As a result, it was indicated that the two hR024-drug conjugates have antitumor activity higher than that of the vehicle.

10)-6 Antitumor Effect (Detroit 562)

Head and neck carcinoma cell line Detroit 562 (ATCC) was suspended in PBS mixed with 50% Matrigel (Corning Incorporated), $5.0 \times 10^6$ cells were subcutaneously transplanted into the right flank of female SCID mice (Day 0), and random grouping was performed on Day 10. From the IHC shown in Example 10)-1, it was confirmed that in this tumor model, carcinoma cells are LRRC15 negative, and cells in the tumor stroma are LRRC15 positive. On the day of grouping, a vehicle, the antibody-drug conjugate H02L01-V1-PBD prepared in Example 5, or a control hIgG-PBD prepared in Reference Example 4 was administered into the tail vein at a dose of 0.4 mg/kg. The results are shown in Figure 35. The abscissa indicates the number of days, and the ordinate indicates estimated tumor volume, and the error bars indicate SEM values. As a result, it was indicated that H02L01-V1-PBD has antitumor activity higher than that of the vehicle and control hIgG-PBD.

10)-6 Antitumor Effect (EC-GI-10)

Esophageal carcinoma cell line EC-GI-10 (RIKEN BRC) was suspended in Matrigel (Corning Incorporated), 5.0 $\times 10^6$ cells were subcutaneously transplanted into the right flank of female NSG mice (Day 0), and random grouping was performed on Day 35. From the IHC shown in Example 10)-1, it was confirmed that in this tumor model, carcinoma cells are LRRC15 negative, and cells in the tumor stroma are LRRC15 positive. On the day of grouping, a vehicle, the antibody-drug conjugate H02L01-V1-PBD prepared in Example 5, or a control hIgG-PBD prepared in Reference Example 4 was administered into the tail vein at a dose of 0.4 mg/kg. The results are shown in Figure 36. The abscissa indicates the number of days, and the ordinate indicates estimated tumor volume, and the error bars indicate SEM values. As a result, it was indicated that H02L01-V1-PBD has antitumor activity higher than that of the vehicle and control hIgG-PBD.

10)-7 Antitumor Effect (BxPC-3 Co-Transplanted with Mesenchymal Stem Cell (MSC))

Pancreatic carcinoma cell line BxPC-3 (ATCC) and human adipose tissue-derived mesenchymal stem cells (PromoCell, LLC) were mixed in PBS at a cell number ratio of 1:3, a total of 4.0 $\times 10^6$ cells were subcutaneously transplanted into the right flank of female SCID mice (Day 0), and random grouping was performed on Day 27. From the IHC shown in Example 10)-1, it was found that in this tumor model, carcinoma cells are LRRC15 negative, and cells in the tumor stroma are LRRC15 positive. On the day of grouping, a vehicle, the antibody-drug conjugate H02L01-V1-PBD prepared in Example 5, or a control hIgG-PBD prepared in Reference Example 4 was administered into the tail vein at a dose of 0.4 mg/kg. The results are shown in Figure 37. The abscissa indicates the number of days, and the ordinate indicates estimated tumor volume, and the error bars indicate SEM values. As a result, it was indicated that H02L01-V1-PBD has antitumor activity higher than that of the vehicle and control hIgG-PBD.

10)-8 Antitumor Effect (CMT93)

Mouse colorectal carcinoma cell line CMT93 (ATCC) was suspended in Matrigel (Corning Incorporated), 2.5 $\times 10^6$ cells were subcutaneously transplanted into the right flank of male B6J mice (Day 0), and random grouping was performed on Day 7. From the IHC shown in Example 10)-1, it was confirmed that in this tumor model, carcinoma cells are LRRC15 negative, and cells in the tumor stroma are LRRC15 positive. On the day of grouping, a vehicle, the antibody-drug conjugate H02L01-PBD or H02L01-V1-PBD prepared in Example 5, or a control hIgG-PBD prepared in Reference Example 4 was administered into the tail vein at a dose of 0.4 mg/kg. The results are shown in Figure 38. The abscissa indicates the number of days, and the ordinate indicates estimated tumor volume, and the error bars indicate SEM values. As a result, it was indicated that the two hR024-drug conjugates have antitumor activity higher than that of the vehicle and the control hIgG-PBD.

10)-9 Antitumor Effect (NCI-N87)

Gastric carcinoma cell line NCI-N87 (ATCC) was suspended in PBS, 5.0 $\times 10^6$ cells were subcutaneously transplanted into the right flank of female SCID mice (Day 0), and random grouping was performed on Day 10. From the IHC shown in Example 10)-1, it was confirmed that in this tumor model, carcinoma cells are LRRC15 negative, and cells in the tumor stroma are LRRC15 positive. On the day of grouping, a vehicle, the antibody-drug conjugate H02L01-V1-PBD prepared in Example 5, or a control hIgG-PBD prepared in Reference Example 4 was administered into the tail vein at a dose of 0.4 mg/kg. The results are shown in Figure 39. The abscissa indicates the number of days, and the ordinate indicates estimated tumor volume, and the error bars indicate SEM values. As a result, it was indicated that H02L01-V1-PBD has antitumor activity higher than that of the vehicle.

10)-10 Antitumor Effect (Caki-1)

Gastric carcinoma cell line Caki-1 (ATCC) was suspended in PBS mixed with 50% Matrigel (Corning Incorporated), 6.0 $\times 10^6$ cells were subcutaneously transplanted into the right flank of female SCID mice (Day 0), and random grouping was performed on Day 21. From the IHC shown in Example 10)-1, it was confirmed that in this tumor model, carcinoma cells are LRRC15 negative, and cells in the tumor stroma are LRRC15 positive. On the day of grouping, a vehicle, the antibody-drug conjugate H02L01-V1-PBD prepared in Example 5, or a control hIgG-PBD prepared in Reference Example 4 was administered into the tail vein at a dose of 0.4 mg/kg. The results are shown in Figure 40. The abscissa indicates the number of days, and the ordinate indicates estimated tumor volume, and the error bars indicate SEM values. As a result, it was indicated that H02L01-V1-PBD has antitumor activity higher than that of the vehicle and control hIgG-PBD.

10)-11 Antitumor Effect (U118MG)

Based on publicly known information, LRRC15-positive human glioblastoma cell line U118MG (ATCC) was suspended in Matrigel (Corning Incorporated), $1.0 \times 10^6$ cells were subcutaneously transplanted into the right flank of female SCID mice (Day 0), and random grouping was performed on Day 17. On the day of grouping, a vehicle, the antibody-drug conjugate H02L01-PBD or H02L01-V1-PBD prepared in Example 5, or a control hIgG-PBD prepared in Reference Example 4 was administered into the tail vein at a dose of 0.4 mg/kg. The results are shown in Figure 41. The abscissa indicates the number of days, and the ordinate indicates estimated tumor volume, and the error bars indicate SEM values. As a result, it was indicated that the two hR024-drug conjugates have antitumor activity higher than that of the vehicle and the control hIgG-PBD.

10)-12 Antitumor Effect (HT-1080)

Fibrosarcoma cell line HT-1080 (ATCC) was suspended in PBS, $2.0 \times 10^6$ cells were subcutaneously transplanted into the right flank of female SCID mice (Day 0), and random grouping was performed on Day 7. From the IHC shown in Example 10)-1, it was confirmed that in this tumor model, carcinoma cells are LRRC15 negative, and cells in the tumor stroma are LRRC15 positive. On the day of grouping, a vehicle, the antibody-drug conjugate H02L01-V1-PBD prepared in Example 5, or a control hIgG-PBD prepared in Reference Example 4 was administered into the tail vein at a dose of 0.4 mg/kg. The results are shown in Figure 42. The abscissa indicates the number of days, and the ordinate indicates estimated tumor volume, and the error bars indicate SEM values. As a result, it was indicated that H02L01-V1-PBD has antitumor activity higher than that of the vehicle and control hIgG-PBD.

The anti-LRRC15 antibody with internalization activity and the antibody-drug conjugate containing the antibody were provided from Examples. The antibody-drug conjugate can be used as a therapeutic drug for cancer, or the like.

## Claims

**1.** An antibody or an antigen-binding fragment of the antibody that specifically binds to an epitope at least partially present in a region selected from the group consisting of:

- a region consisting of amino acid residues 22 to 53 of SEQ ID NO: 1;
- a region consisting of amino acid residues 54 to 75 of SEQ ID NO: 1;
- a region consisting of amino acid residues 78 to 99 of SEQ ID NO: 1;
- a region consisting of amino acid residues 102 to 123 of SEQ ID NO: 1;
- a region consisting of amino acid residues 126 to 147 of SEQ ID NO: 1;
- a region consisting of amino acid residues 150 to 171 of SEQ ID NO: 1;
- a region consisting of amino acid residues 174 to 195 of SEQ ID NO: 1;
- a region consisting of amino acid residues 198 to 219 of SEQ ID NO: 1;
- a region consisting of amino acid residues 222 to 243 of SEQ ID NO: 1;
- a region consisting of amino acid residues 246 to 267 of SEQ ID NO: 1;
- a region consisting of amino acid residues 270 to 291 of SEQ ID NO: 1; and
- a region consisting of amino acid residues 294 to 315 of SEQ ID NO: 1.

**2.** The antibody or an antigen-binding fragment of the antibody according to claim 1, wherein the antibody or an antigen-binding fragment of the antibody specifically binds to an epitope at least partially present in a region selected from the group consisting of:

- a region consisting of amino acid residues 22 to 53 of SEQ ID NO: 1;
- a region consisting of amino acid residues 54 to 75 of SEQ ID NO: 1;
- a region consisting of amino acid residues 78 to 99 of SEQ ID NO: 1;
- a region consisting of amino acid residues 102 to 123 of SEQ ID NO: 1;
- a region consisting of amino acid residues 126 to 147 of SEQ ID NO: 1;
- a region consisting of amino acid residues 150 to 171 of SEQ ID NO: 1;
- a region consisting of amino acid residues 174 to 195 of SEQ ID NO: 1; and
- a region consisting of amino acid residues 198 to 219 of SEQ ID NO: 1.

**3.** The antibody or an antigen-binding fragment of the antibody according to claim 1 or 2, wherein the antibody or an antigen-binding fragment of the antibody specifically binds to an epitope at least partially present in a region selected

from the group consisting of:

- a region consisting of amino acid residues 22 to 53 of SEQ ID NO: 1;
- a region consisting of amino acid residues 54 to 75 of SEQ ID NO: 1; and
- a region consisting of amino acid residues 78 to 99 of SEQ ID NO: 1.

**4.** The antibody or an antigen-binding fragment of the antibody according to any one of claims 1 to 3, wherein the antibody or an antigen-binding fragment of the antibody competes for binding to human LRRC15 with at least one antibody consisting of the amino acid sequence of SEQ ID NO: 1, selected from the group consisting of:

(1) an antibody having a light chain consisting of the amino acid sequence set forth in SEQ ID NO: 66 and a heavy chain consisting of the amino acid sequence set forth in SEQ ID NO: 42;
(2) an antibody having a light chain consisting of the amino acid sequence set forth in SEQ ID NO: 66 and a heavy chain consisting of the amino acid sequence set forth in SEQ ID NO: 48;
(3) an antibody having a light chain consisting of the amino acid sequence set forth in SEQ ID NO: 66 and a heavy chain consisting of the amino acid sequence set forth in SEQ ID NO: 54; and
(4) an antibody having a light chain consisting of the amino acid sequence set forth in SEQ ID NO: 60 and a heavy chain consisting of the amino acid sequence set forth in SEQ ID NO: 36.

**5.** An antibody or an antigen-binding fragment of the antibody, comprising CDRL1, CDRL2 and CDRL3, and CDRH1, CDRH2 and CDRH3 selected from the group consisting of the following (1) to (4):

(1) CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and CDRH1 consisting of the amino acid sequence set forth in SEQ ID NO: 38, CDRH2 consisting of the amino acid sequence set forth in SEQ ID NO: 39, and CDRH3 consisting of the amino acid sequence set forth in SEQ ID NO: 40;
(2) CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and CDRH1 consisting of the amino acid sequence set forth in SEQ ID NO: 44, CDRH2 consisting of the amino acid sequence set forth in SEQ ID NO: 45, and CDRH3 consisting of the amino acid sequence set forth in SEQ ID NO: 46;
(3) CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and CDRH1 consisting of the amino acid sequence set forth in SEQ ID NO: 50, CDRH2 consisting of the amino acid sequence set forth in SEQ ID NO: 51, and CDRH3 consisting of the amino acid sequence set forth in SEQ ID NO: 52; and
(4) CDRL1 consisting of the amino acid sequence set forth in SEQ ID NO: 56, CDRL2 consisting of the amino acid sequence of WAS, and CDRL3 consisting of the amino acid sequence set forth in SEQ ID NO: 58, and CDRH1 consisting of the amino acid sequence set forth in SEQ ID NO: 32, CDRH2 consisting of the amino acid sequence set forth in SEQ ID NO: 33, and CDRH3 consisting of the amino acid sequence set forth in SEQ ID NO: 34.

**6.** The antibody or an antigen-binding fragment of the antibody according to any one of claims 1 to 5, having an internalization ability to be taken up into a cell.

**7.** The antibody or an antigen-binding fragment of the antibody according to any one of claims 1 to 6, wherein the antibody or an antigen-binding fragment of the antibody binds to human LRRC15 with a KD of 1 pM to 1000 nM.

**8.** The antibody or an antigen-binding fragment of the antibody according to any one of claims 1 to 7, wherein the antibody or an antigen-binding fragment of the antibody is humanized.

**9.** The antibody or an antigen-binding fragment of the antibody according to any one of claims 1 to 8, comprising a light chain variable region and a heavy chain variable region of one selected from the group consisting of the following (1) to (4):

(1) a light chain variable region consisting of an amino acid sequence having at least 95% or more sequence identity to the amino acid sequence set forth in SEQ ID No: 65, and a heavy chain variable region consisting of an amino acid sequence having at least 95% or more sequence identity to the amino acid sequence set forth in SEQ ID No: 41;
(2) a light chain variable region consisting of an amino acid sequence having at least 95% or more sequence identity to the amino acid sequence set forth in SEQ ID No: 65, and a heavy chain variable region consisting of an

amino acid sequence having at least 95% or more sequence identity to the amino acid sequence set forth in SEQ ID No: 47;

(3) a light chain variable region consisting of an amino acid sequence having at least 95% or more sequence identity to the amino acid sequence set forth in SEQ ID No: 65, and a heavy chain variable region consisting of an amino acid sequence having at least 95% or more sequence identity to the amino acid sequence set forth in SEQ ID No: 53; and

(4) a light chain variable region consisting of an amino acid sequence having at least 95% or more sequence identity to the amino acid sequence set forth in SEQ ID No: 59, and a heavy chain variable region consisting of an amino acid sequence having at least 95% or more sequence identity to the amino acid sequence set forth in SEQ ID No: 35.

**10.** The antibody or an antigen-binding fragment of the antibody according to any one of claims 1 to 9, comprising a light chain variable region and a heavy chain variable region of one selected from the group consisting of the following (1) to (4):

(1) a light chain variable region consisting of the amino acid sequence set forth in SEQ ID NO: 65, and a heavy chain variable region consisting of the amino acid sequence set forth in SEQ ID NO: 41;

(2) a light chain variable region consisting of the amino acid sequence set forth in SEQ ID NO: 65, and a heavy chain variable region consisting of the amino acid sequence set forth in SEQ ID NO: 47;

(3) a light chain variable region consisting of the amino acid sequence set forth in SEQ ID NO: 65, and a heavy chain variable region consisting of the amino acid sequence set forth in SEQ ID NO: 53; and

(4) a light chain variable region consisting of the amino acid sequence set forth in SEQ ID NO: 59, and a heavy chain variable region consisting of the amino acid sequence set forth in SEQ ID NO: 35.

**11.** The antibody or an antigen-binding fragment of the antibody according to any one of claims 1 to 10, comprising a light chain and a heavy chain of one selected from the group consisting of the following (1) to (4):

(1) a light chain consisting of the amino acid sequence of SEQ ID NO: 66, and a heavy chain consisting of the amino acid sequence of SEQ ID NO: 42;

(2) a light chain consisting of the amino acid sequence of SEQ ID NO: 66, and a heavy chain consisting of the amino acid sequence of SEQ ID NO: 48;

(3) a light chain consisting of the amino acid sequence of SEQ ID NO: 66, and a heavy chain consisting of the amino acid sequence of SEQ ID NO: 54; and

(4) a light chain consisting of the amino acid sequence of SEQ ID NO: 60, and a heavy chain consisting of the amino acid sequence of SEQ ID NO: 36.

**12.** The antibody or an antigen-binding fragment of the antibody according to claim 11, comprising the light chain consisting of the amino acid sequence of SEQ ID NO: 66, and the heavy chain consisting of the amino acid sequence of SEQ ID NO: 42.

**13.** The antibody or an antigen-binding fragment of the antibody according to claim 11, comprising the light chain consisting of the amino acid sequence of SEQ ID NO: 66, and the heavy chain consisting of the amino acid sequence of SEQ ID NO: 48.

**14.** The antibody or an antigen-binding fragment of the antibody according to claim 11, comprising the light chain consisting of the amino acid sequence of SEQ ID NO: 66, and the heavy chain consisting of the amino acid sequence of SEQ ID NO: 54.

**15.** The antibody or an antigen-binding fragment of the antibody according to claim 11, comprising the light chain consisting of the amino acid sequence of SEQ ID NO: 60, and the heavy chain consisting of the amino acid sequence of SEQ ID NO: 36.

**16.** The antigen-binding fragment of the antibody according to any one of claims 1 to 15, wherein the antigen-binding fragment is selected from the group consisting of Fab, $F(ab')_2$, Fab', Fv, scFv, VHH, and scFv-containing fusion protein.

**17.** A polynucleotide that encodes the antibody or an antigen-binding fragment of the antibody according to any one of claims 1 to 16.

**18.** The polynucleotide according to claim 17, comprising:

(1) a polynucleotide that encodes a light chain variable region containing CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and a polynucleotide that encodes a heavy chain variable region containing CDRH1 consisting of the amino acid sequence set forth in SEQ ID NO: 38, CDRH2 consisting of the amino acid sequence set forth in SEQ ID NO: 39, and CDRH3 consisting of the amino acid sequence set forth in SEQ ID NO: 40;
(2) a polynucleotide that encodes a light chain variable region containing CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and a polynucleotide that encodes a heavy chain variable region containing CDRH1 consisting of the amino acid sequence set forth in SEQ ID NO: 44, CDRH2 consisting of the amino acid sequence set forth in SEQ ID NO: 45, and CDRH3 consisting of the amino acid sequence set forth in SEQ ID NO: 46;
(3) a polynucleotide that encodes a light chain variable region containing CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and a polynucleotide that encodes a heavy chain variable region containing CDRH1 consisting of the amino acid sequence set forth in SEQ ID NO: 50, CDRH2 consisting of the amino acid sequence set forth in SEQ ID NO: 51, and CDRH3 consisting of the amino acid sequence set forth in SEQ ID NO: 52; or
(4) a polynucleotide that encodes a light chain variable region containing CDRL1 consisting of the amino acid sequence set forth in SEQ ID NO: 56, CDRL2 consisting of the amino acid sequence of WAS, and CDRL3 consisting of the amino acid sequence set forth in SEQ ID NO: 58, and a polynucleotide that encodes a heavy chain variable region containing CDRH1 consisting of the amino acid sequence set forth in SEQ ID NO: 32, CDRH2 consisting of the amino acid sequence set forth in SEQ ID NO: 33, and CDRH3 consisting of the amino acid sequence set forth in SEQ ID NO: 34.

**19.** The polynucleotide according to claim 17 or 18, comprising:

(1) a polynucleotide that encodes a light chain consisting of the amino acid sequence of SEQ ID NO: 66, and a polynucleotide that encodes a heavy chain consisting of the amino acid sequence of SEQ ID NO: 42;
(2) a polynucleotide that encodes a light chain consisting of the amino acid sequence of SEQ ID NO: 66, and a polynucleotide that encodes a heavy chain consisting of the amino acid sequence of SEQ ID NO: 48;
(3) a polynucleotide that encodes a light chain consisting of the amino acid sequence of SEQ ID NO: 66, and a polynucleotide that encodes a heavy chain consisting of the amino acid sequence of SEQ ID NO: 54; or
(4) a polynucleotide that encodes a light chain consisting of the amino acid sequence of SEQ ID NO: 60, and a polynucleotide that encodes a heavy chain consisting of the amino acid sequence of SEQ ID NO: 36.

**20.** An expression vector, comprising the polynucleotide according to any one of claims 17 to 19.

**21.** A host cell transformed with the expression vector according to claim 20.

**22.** The host cell according to claim 21, wherein the host cell is a eukaryotic cell.

**23.** A method for producing the antibody or an antigen-binding fragment of the antibody according to any one of claims 1 to 16, comprising a step of culturing the host cell according to claim 21 or 22, and collecting the antibody or an antigen-binding fragment of the antibody of interest from a culture obtained in the culturing step.

**24.** The antibody or an antigen-binding fragment of the antibody according to any one of claims 1 to 16, wherein the heavy chain or light chain has undergone one or more than one modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, pyroglutamation of N-terminal glutamine or N-terminal glutamic acid, and deletion of one or two amino acids at the carboxyl terminus.

**25.** The antibody or an antigen-binding fragment of the antibody according to claim 24, wherein one or two amino acids are deleted at the carboxyl terminus of one or both of the heavy chains.

**26.** The antibody or an antigen-binding fragment of the antibody according to claim 25, wherein one amino acid is deleted at the carboxyl terminus of each of the two heavy chains.

**27.** The antibody or an antigen-binding fragment of the antibody according to any one of claims 24 to 26, wherein the proline residue at the carboxyl terminus of one or both of the heavy chains is further amidated.

**28.** The antibody according to any one of claims 1 to 16, and 24 to 27, wherein modification of a glycan is regulated or a portion of the amino acid residue in the constant region is substituted, in order to enhance or attenuate antibody-dependent cellular cytotoxicity, to enhance or attenuate antibody-dependent cell-mediated phagocytosis, and/or to enhance or attenuate complementdependent cytotoxicity.

**29.** An antibody-drug conjugate, wherein a drug is bound to the antibody or an antigen-binding fragment of the antibody according to any one of claims 1 to 16, and 24 to 28.

**30.** The antibody-drug conjugate according to claim 29, wherein the drug is an antitumor compound.

**31.** An antibody-drug conjugate, represented by the formula (X):

[Chem. 1]

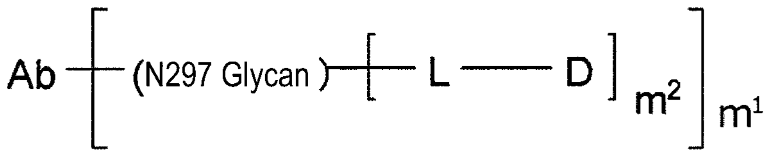
(X)

wherein $m^1$ represents an integer of 1 or 2,

$m^2$ represents an integer of 1 or 2,
Ab represents the antibody or an antigen-binding fragment of the antibody according to any one of claims 1 to 16 and 24 to 28,
L is a linker linking a glycan that binds to N297 (N297 glycan) of Ab to D,
the N297 glycan represents a glycan that can be remodeled,
D is one selected from the following:

[Chem. 2]

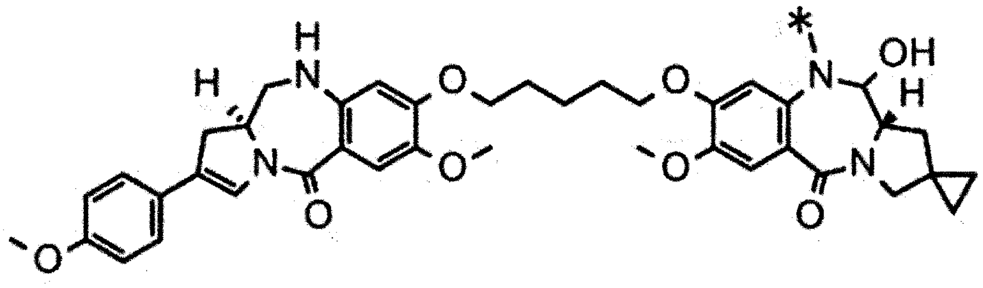

,

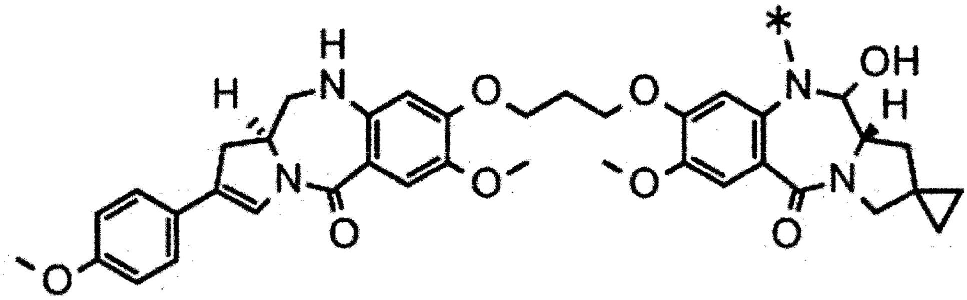

,

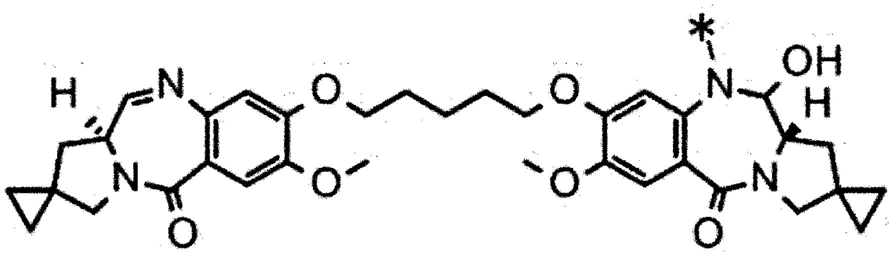

or

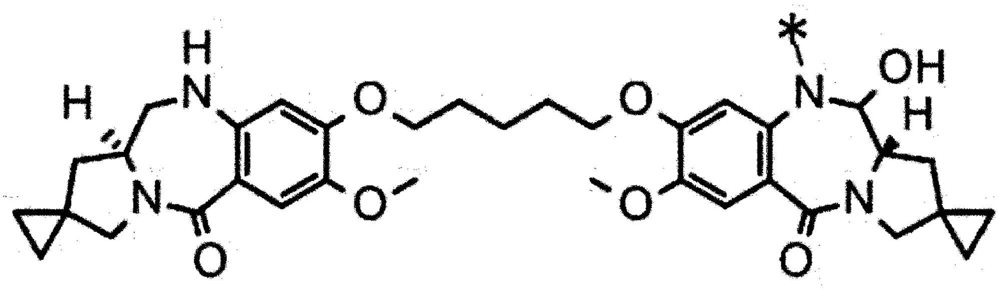

wherein * represents a bond to L.

**32.** The antibody-drug conjugate according to claim 31, wherein:

L is represented by -Lb-La-Lp-NH-B-$CH_2$-O(C=O)-*,
wherein * represents a bond to a nitrogen atom at the N10'-position of the D,
B is 1,4-phenyl group, 2,5-pyridyl group, 3,6-pyridyl group, 2,5-pyrimidyl group, or 2,5-thienyl group,
Lp represents one selected from the following: -GGVA-, -GG-(D-)VA-, -VA-, -GGFG-, -GGPI-, -GGVCit-, - GGVK-, and -GGPL-,
La represents one selected from the following:

$-C(=O)-CH_2CH_2-C(=O)-$,

$-C(=O)-(CH_2CH_2)_2-C(=O)-$,

$-C(=O)-CH_2CH_2-C(=O)-NH-(CH_2CH_2)_2-C(=O)-$,

$-C(=O)-CH_2CH_2-C(=O)-NH-(CH_2CH_2O)_2-CH_2-C(=O)-$,

$-C(=O)-CH_2CH_2-NH-C(=O)-(CH_2CH_2O)_4-CH_2CH_2-C(=O)-$,

$-CH_2-OC(=O)-$,

and,

$-OC(=O)-$,

and
Lb is represented by the following formula:

[Chem. 3]

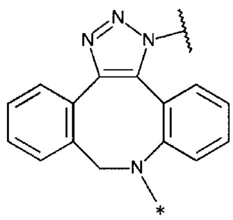

or

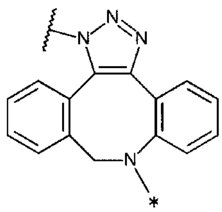

[Chem. 4]

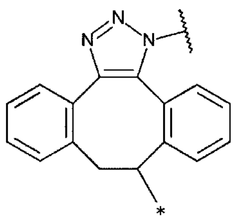

or

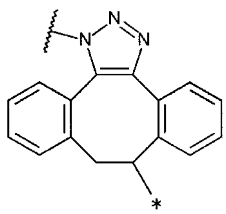

, or

[Chem. 5]

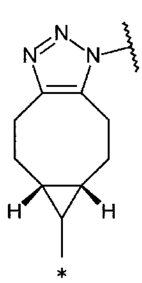

or

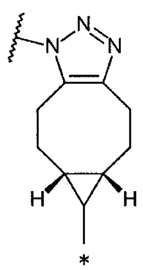

wherein in the structural formulas of Lb shown above, * represents a bond to La, and the wavy lines represent a bond to the N297 glycan.

**33.** The antibody-drug conjugate according to claim 31 or 32, wherein L represents one selected from the group consisting

of the following:

$-Z^1-C(=O)-CH_2CH_2-C(=O)-GGVA-NH-B-CH_2-OC(=O)-$,

$-Z^1-C(=O)-CH_2CH_2-C(=O)-GG-(D-)VA-NH-B-CH_2-OC(=O)-$,

$-Z^1-C(=O)-CH_2CH_2-C(=O)-VA-NH-B-CH_2-OC(=O)-$,

$-Z^1-C(=O)-(CH_2CH_2)_2-C(=O)-VA-NH-B-CH_2-OC(=O)-$,

$-Z^1-C(=O)-CH_2CH_2-C(=O)-GGPI-NH-B-CH_2-OC(=O)-$,

$-Z^l-C(=O)-CH_2CH_2-C(=O)-GGFG-NH-B-CH_2-OC(=O)-$,

$-Z^1-C(=O)-CH_2CH_2-C(=O)-GGVCit-NH-B-CH_2-OC(=O)-$,

$-Z^1-C(=O)-CH_2CH_2-C(=O)-GGVK-NH-B-CH_2-OC(=O)-$,

$-Z^1-C(=O)-CH_2CH_2-C(=O)-GGPL-NH-B-CH_2-OC(=O)-$,

$-Z^1-C(=O)-CH_2CH_2-C(=O)-NH-(CH_2CH_2)_2-C(=O)-VA-NH-B-CH_2-OC(=O)-$,

$-Z^1-C(=O)-CH_2CH_2-C(=O)-NH-(CH_2CH_2O)_2-CH_2-C(=O)-VA-NH-B-CH_2-OC(=O)-$,

$-Z^1-C(=O)-CH_2CH_2-NH-C(=O)-(CH_2CH_2O)_4-CH_2CH_2-C(=O)-VA-NH-B-CH_2-OC(=O)$ -,

$-Z^2-OC(=O)-GGVA-NH-B-CH_2-OC(=O)-$,

and

$-Z^3-CH_2-OC(=O)-GGVA-NH-B-CH_2-OC(=O)-$,

wherein $Z^1$ represents the following structural formula:

[Chem. 6]

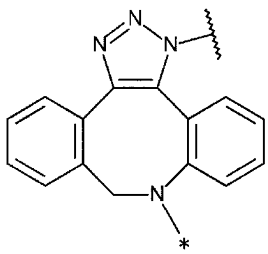

or

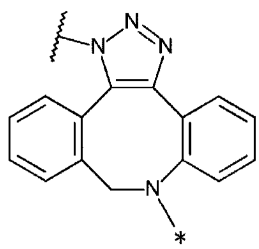

,

$Z^2$ represents the following structural formula:

[Chem. 7]

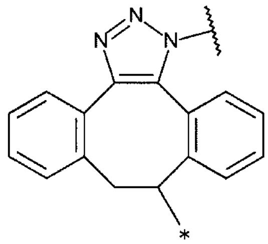

or

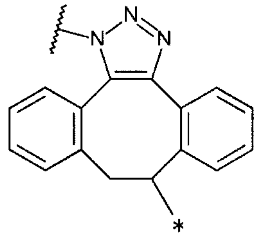

,

$Z^3$ represents the following structural formula:

[Chem. 8]

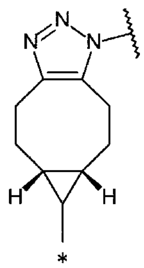

or

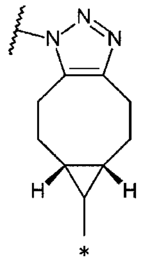

,

wherein in the structural formulas of $Z^1$, $Z^2$, and $Z^3$,
* represents a bond to C(=O), O, or $CH_2$ adjacent to $Z^1$, $Z^2$, or $Z^3$, and the wavy lines represent a bond to the N297 glycan, and
B represents 1,4-phenyl group.

**34.** The antibody-drug conjugate according to any one of claims 31 to 33, wherein L represents one selected from the group consisting of the following:

$-Z^1-C(=O)-CH_2CH_2-C(=O)-GGVA-NH-B-CH_2-OC(=O)-$,

$-Z^1-C(=O)-CH_2CH_2-C(=O)-VA-NH-B-CH_2-OC(=O)-$,

$-Z^1-C(=O)-(CH_2CH_2)_2-C(=O)-VA-NH-B-CH_2-OC(=O)-$,

$-Z^1-C(=O)-CH_2CH_2-C(=O)-GGVCit-NH-B-CH_2-OC(=O)-$,

$-Z^1-C(=O)-CH_2CH_2-C(=O)-NH-(CH_2CH_2)_2-C(=O)-VA-NH-B-CH_2-OC(=O)-$,

$-Z^1-C(=O)-CH_2CH_2-C(=O)-NH-(CH_2CH_2O)_2-CH_2-C(=O)-VA-NH-B-CH_2-OC(=O)-$,

and

$-Z^1-C(=O)-CH_2CH_2-NH-C(=O)-(CH_2CH_2O)_4-CH_2CH_2-C(=O)-VA-NH-B-CH_2-OC(=O)-$,

wherein B is 1,4-phenyl group, and $Z^1$ represents the following structural formula:

[Chem. 9]

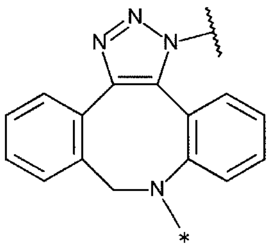

or

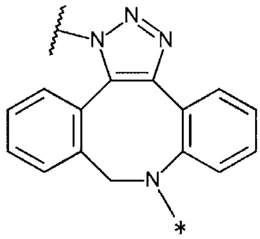

,

wherein in the structural formulas of $Z^1$, * represents a bond to C(=O) adjacent to $Z^1$, and the wavy lines represent a bond to the N297 glycan.

**35.** The antibody-drug conjugate according to any one of claims 31 to 34, wherein the antibody is IgG1, IgG2, or IgG4.

**36.** The antibody-drug conjugate according to any one of claims 31 to 35, wherein the N297 glycan is a remodeled glycan.

**37.** The antibody-drug conjugate according to any one of claims 31 to 36, wherein the N297 glycan is N297-(Fuc)MSG1, N297-(Fuc)MSG2, or a mixture thereof, or N297-(Fuc)SG, having the structures represented by the following formulas:

[Chem. 10]

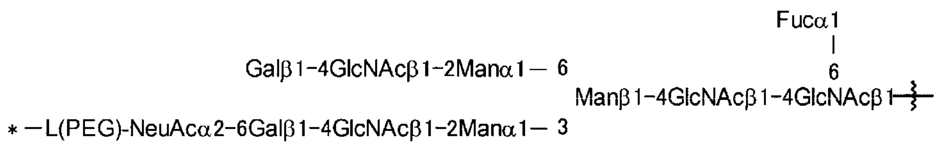

[N297-(Fuc)MSG1]

[Chem. 11]

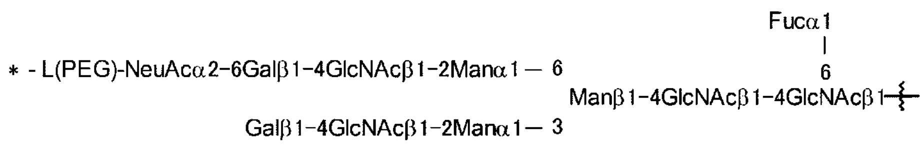

[N297-(Fuc)MSG2]

[Chem. 12]

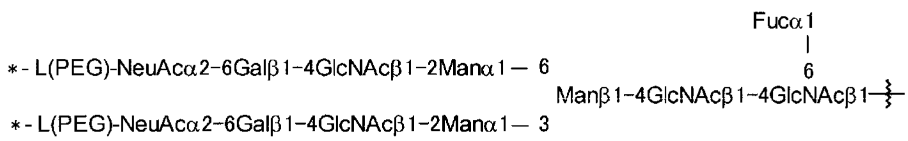

[N297-(Fuc)SG]

wherein the wavy lines represent a bond to Asn297 (N297) of the antibody,
*-L(PEG)- in the N297 glycan represents $*-(CH_2CH_2-O)n^5-CH_2CH_2-NH-$,
wherein, $n^5$ represents an integer of 2 to 5, and it is indicated that the amino group at the right end is bound via amide bond to the carboxylic acid at the 2-position of a sialic acid at the non-reducing terminus of the 1-3 chain or/and 1-6 chain of the β-Man branched chain of the N297 glycan, and * represents a bond to the nitrogen atom at

position 1 or 3 on the triazole ring in each structural formula.

**38.** The antibody-drug conjugate according to claim 37, wherein $n^5$ is 3.

**39.** An antibody-drug conjugate represented by the following formula (XII):

[Chem. 13]

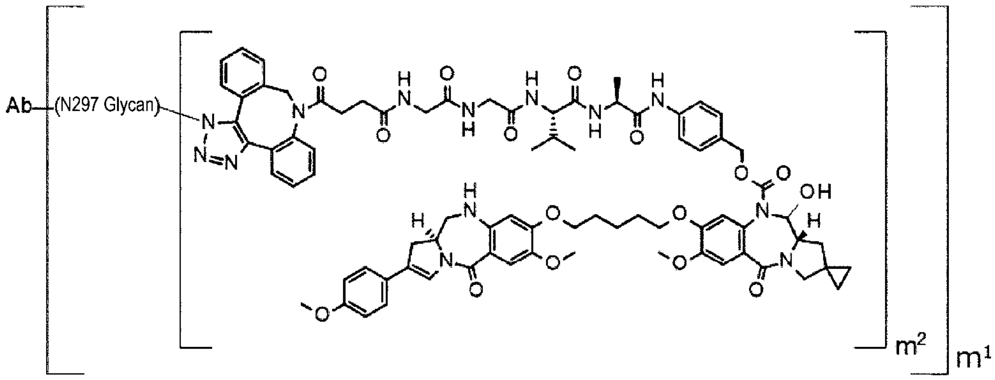

or

(XII)

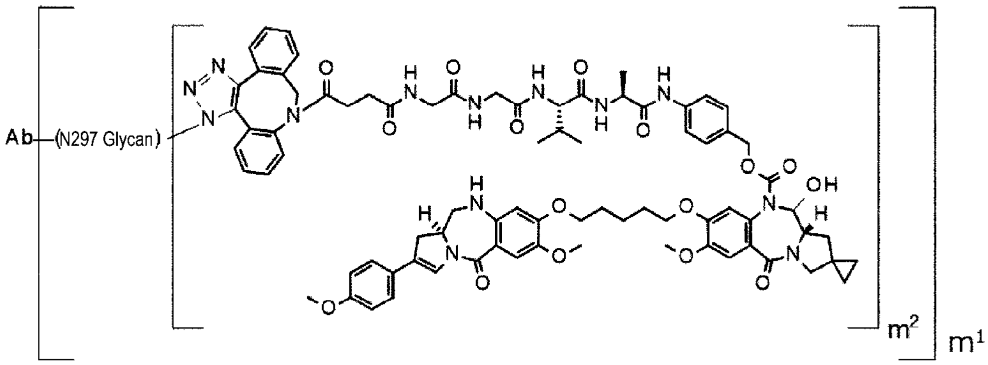

wherein,

wherein $m^1$ represents an integer of 1 or 2 in each structural formula shown above,
$m^2$ represents an integer of 1 or 2,
Ab represents the antibody or an antigen-binding fragment of the antibody according to any one of claims 1 to 16 and 24 to 28,
the N297 glycan represents one of N297-(Fuc)MSG1, N297-(Fuc)MSG2, and a mixture thereof, and N297-(Fuc)SG, having the structures represented by the following formulas:

[Chem. 14]

Fucα1
|
6
Galβ1-4GlcNAcβ1-2Manα1— 6
Manβ1-4GlcNAcβ1-4GlcNAcβ1—
∗—L(PEG)-NeuAcα2-6Galβ1-4GlcNAcβ1-2Manα1— 3

[N297-(Fuc)MSG1]

[Chem. 15]

Fucα1
|
6

*-L(PEG)-NeuAcα2-6Galβ1-4GlcNAcβ1-2Manα1— 6
Manβ1-4GlcNAcβ1-4GlcNAcβ1-
Galβ1-4GlcNAcβ1-2Manα1— 3

[N297-(Fuc)MSG2]

[Chem. 16]

Fucα1
|
6

*-L(PEG)-NeuAcα2-6Galβ1-4GlcNAcβ1-2Manα1— 6
Manβ1-4GlcNAcβ1-4GlcNAcβ1-
*-L(PEG)-NeuAcα2-6Galβ1-4GlcNAcβ1-2Manα1— 3

[N297-(Fuc)SG]

wherein the wavy lines represent a bond to Asn297 (N297) of the antibody,
*-L(PEG)- in the N297 glycan represents *-$(CH_2CH_2\text{-}O)_3$-$CH_2CH_2$-NH-,
wherein it is indicated that the amino group at the right end is bound via amide bond to the carboxylic acid at the 2-position of a sialic acid at the non-reducing terminus of the 1-3 chain or/and 1-6 chain of the β-Man branched chain of the N297 glycan, and * represents a bond to the nitrogen atom at the 1 or 3-position on the triazole ring in each structural formula.

**40.** The antibody-drug conjugate according to claim 39, wherein the compound represented by the formula (XII) is represented by the following formula (XII'):

[Chem. 17]

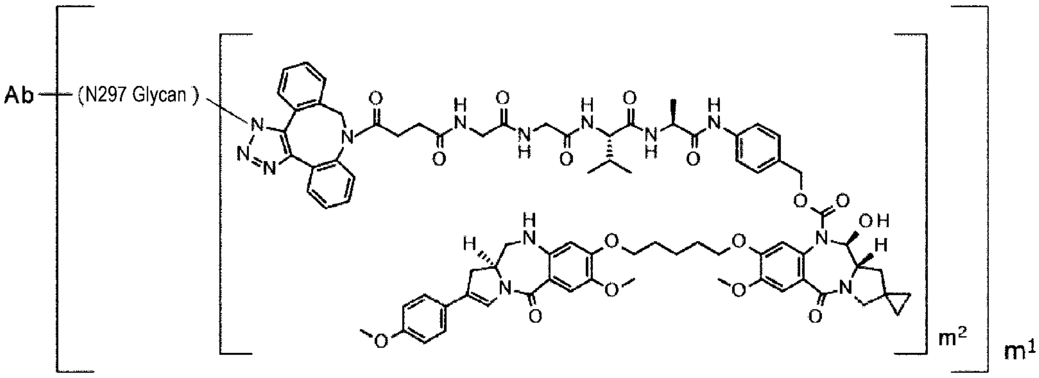

or (XII')

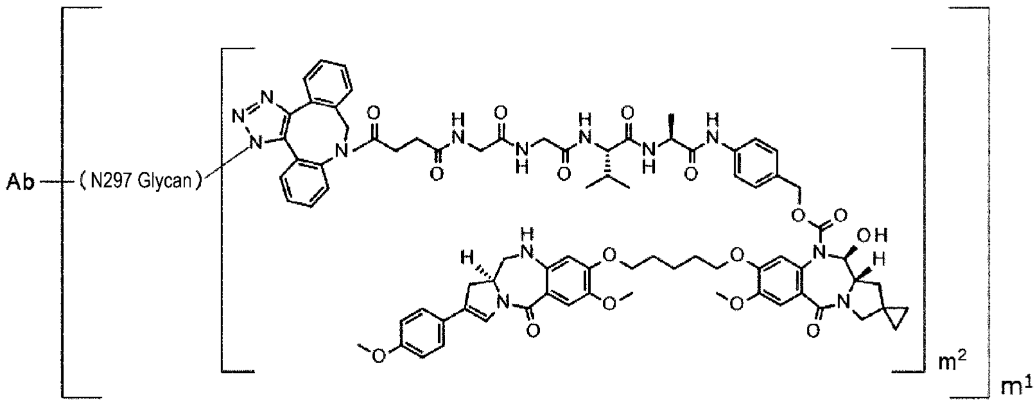

.

**41.** The antibody-drug conjugate according to claim 40, wherein, in the structural formula of the formula (XII'), $m^1$ represents an integer of 2, $m^2$ represents an integer of 1,

the N297 glycan is N297-(Fuc)MSG1 having the structure shown in the following formula:

[Chem. 18]

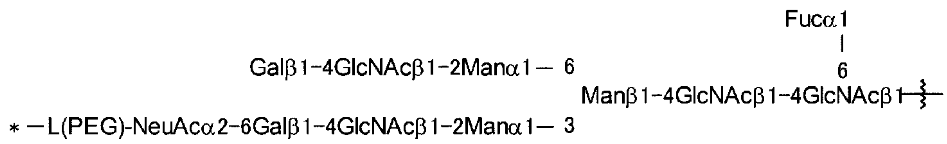

[N297-(Fuc)MSG1]

, and *-L(PEG)- in the N297 glycan represents *-$(CH_2CH_2\text{-}O)_3\text{-}CH_2CH_2$-NH-, wherein it is indicated that the amino group at the right end is bound via amide bond to the carboxylic acid at the 2-position of a sialic acid at the non-reducing terminus of the 1-3 chain of the β-Man branched chain of the N297 glycan, and * represents a bond to the nitrogen atom at the 1 or 3-position on the triazole ring,
Ab represents an antibody comprising CDRL1, CDRL2 and CDRL3, and CDRH1, CDRH2 and CDRH3 selected from the group consisting of:

(1) CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and

CDRH1 consisting of the amino acid sequence set forth in SEQ ID NO: 38, CDRH2 consisting of the amino acid sequence set forth in SEQ ID NO: 39, and CDRH3 consisting of the amino acid sequence set forth in SEQ ID NO: 40;
(2) CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and CDRH1 consisting of the amino acid sequence set forth in SEQ ID NO: 44, CDRH2 consisting of the amino acid sequence set forth in SEQ ID NO: 45, and CDRH3 consisting of the amino acid sequence set forth in SEQ ID NO: 46;
(3) CDRL1 consisting of the amino acid sequence set forth in SEQ ID NO: 62, CDRL2 consisting of the amino acid sequence of WAS, and CDRL3 consisting of the amino acid sequence set forth in SEQ ID NO: 64, and CDRH1 consisting of the amino acid sequence set forth in SEQ ID NO: 50, CDRH2 consisting of the amino acid sequence set forth in SEQ ID NO: 51, and CDRH3 consisting of the amino acid sequence set forth in SEQ ID NO: 52; and
(4) CDRL1 consisting of the amino acid sequence set forth in SEQ ID NO: 56, CDRL2 consisting of the amino acid sequence of WAS, and CDRL3 consisting of the amino acid sequence set forth in SEQ ID NO: 58, and CDRH1 consisting of the amino acid sequence set forth in SEQ ID NO: 32, CDRH2 consisting of the amino acid sequence set forth in SEQ ID NO: 33, and CDRH3 consisting of the amino acid sequence set forth in SEQ ID NO: 34.

**42.** The antibody-drug conjugate according to claim 41, wherein Ab represents an antibody comprising a light chain variable region and a heavy chain variable region of one selected from the group consisting of the following (1) to (4):

(1) a light chain variable region consisting of the amino acid sequence set forth in SEQ ID NO: 65, and a heavy chain variable region consisting of the amino acid sequence set forth in SEQ ID NO: 41;
(2) a light chain variable region consisting of the amino acid sequence set forth in SEQ ID NO: 65, and a heavy chain variable region consisting of the amino acid sequence set forth in SEQ ID NO: 47;
(3) a light chain variable region consisting of the amino acid sequence set forth in SEQ ID NO: 65, and a heavy chain variable region consisting of the amino acid sequence set forth in SEQ ID NO: 53; and
(4) a light chain variable region consisting of the amino acid sequence set forth in SEQ ID NO: 59, and a heavy chain variable region consisting of the amino acid sequence set forth in SEQ ID NO: 35.

**43.** The antibody-drug conjugate according to claim 42, wherein Ab comprises a light chain and a heavy chain of one selected from the group consisting of the following (1) to (5):

(1) a light chain consisting of the amino acid sequence of SEQ ID NO: 66, and a heavy chain consisting of the amino acid sequence of SEQ ID NO: 42;
(2) a light chain consisting of the amino acid sequence of SEQ ID NO: 66, and a heavy chain consisting of the amino acid sequence of SEQ ID NO: 48;
(3) a light chain consisting of the amino acid sequence set forth in SEQ ID NO: 66, and a heavy chain consisting of the amino acid sequence set forth in SEQ ID NO: 54,
(4) a light chain consisting of the amino acid sequence set forth in SEQ ID NO: 60, and a heavy chain consisting of the amino acid sequence set forth in SEQ ID NO: 36; and
(5) the light chain (light chain a) described in one of (1) to (4), and a heavy chain consisting of the amino acid sequence obtained by deleting one or two lysines from the carboxyl terminus of the amino acid sequence of a heavy chain (heavy chain a) combined with the light chain a in (1) to (4).

**44.** An antibody-drug conjugate represented by the following formula (XIII):

[Chem. 19]

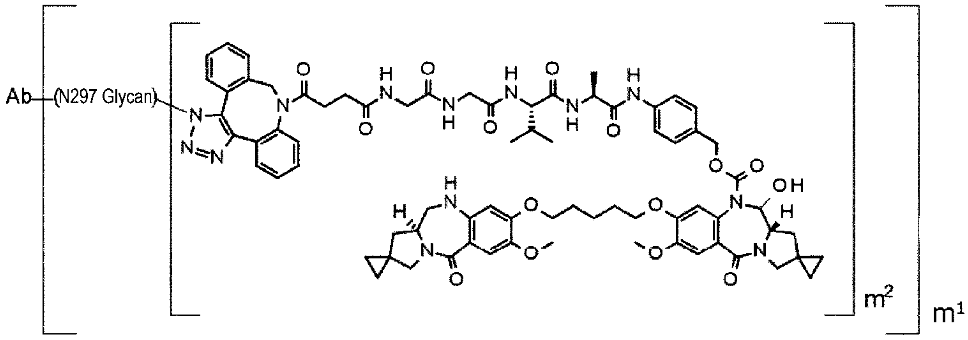

or

(XIII)

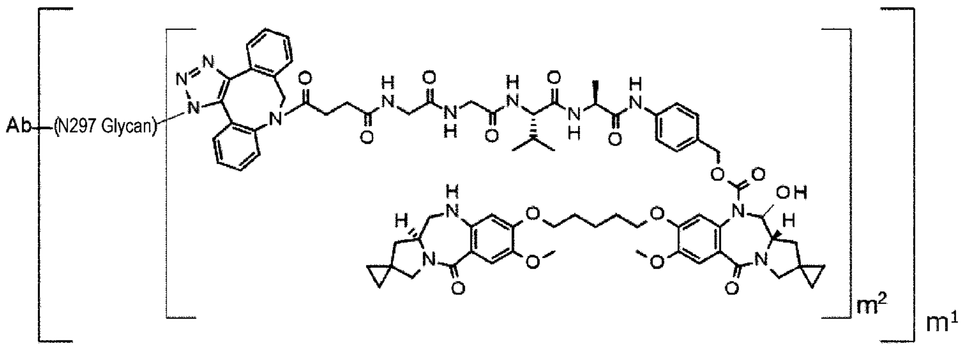

wherein,

in each structural formula shown above, $m^1$ represents an integer of 1 or 2,
$m^2$ represents an integer of 1 or 2,
Ab represents the antibody or an antigen-binding fragment of the antibody according to any one of claims 1 to 16 and 24 to 28, and
the N297 glycan represents one of N297-(Fuc)MSG1, N297-(Fuc)MSG2, and a mixture thereof, and N297-(Fuc) SG, having the structures represented by the following formulas:

[Chem. 20]

```
                                                                   Fucα1
                                                                     |
                        Galβ1-4GlcNAcβ1-2Manα1— 6                    6
                                                  Manβ1-4GlcNAcβ1-4GlcNAcβ1-{-
*—L(PEG)-NeuAcα2-6Galβ1-4GlcNAcβ1-2Manα1— 3
```

[N297-(Fuc)MSG1]

[Chem. 21]

```
                                                                   Fucα1
                                                                     |
*-L(PEG)-NeuAcα2-6Galβ1-4GlcNAcβ1-2Manα1— 6                          6
                                                  Manβ1-4GlcNAcβ1-4GlcNAcβ1-{-
                        Galβ1-4GlcNAcβ1-2Manα1— 3
```

[N297-(Fuc)MSG2]

[Chem. 22]

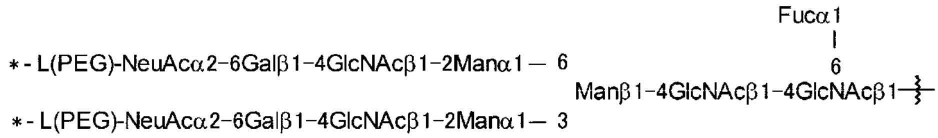

[N297-(Fuc)SG]

wherein the wavy lines represent a bond to Asn297 (N297) of the antibody, and
*-L(PEG)- in the N297 glycan represents *-($CH_2CH_2$-O)$_3$-$CH_2CH_2$-NH-,
wherein it is indicated that the amino group at the right end is bound via amide bond to the carboxylic acid at the 2-position of a sialic acid at the non-reducing terminus of the 1-3 chain or/and 1-6 chain of the β-Man branched chain of the N297 glycan, and * represents a bond to the nitrogen atom at the 1 or 3-position on the triazole ring in each structural formula.

**45.** The antibody-drug conjugate according to claim 44, wherein the compound represented by the formula (XIII) is represented by the following formula (XIII'):

[Chem. 23]

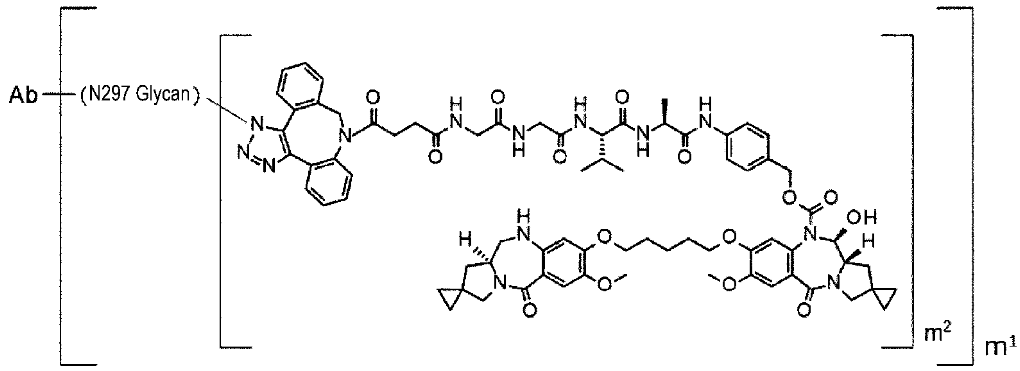

(XIII')

or

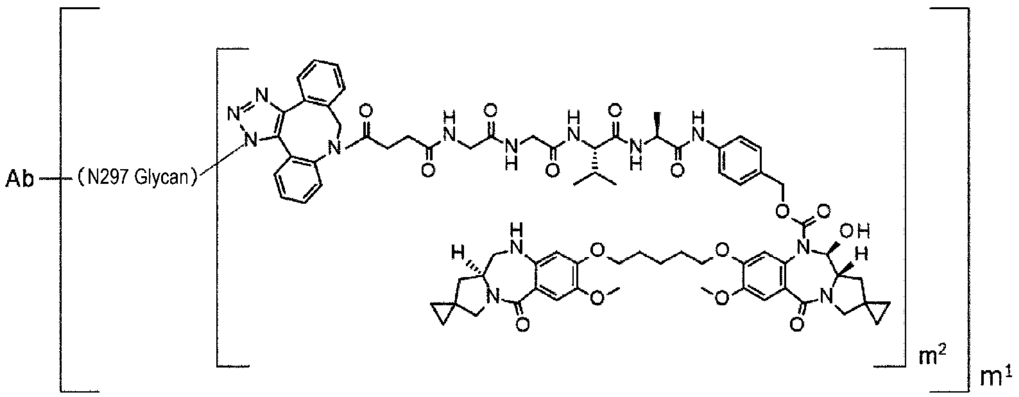

.

**46.** An antibody-drug conjugate represented by the following formula (XIV):

[Chem. 24]

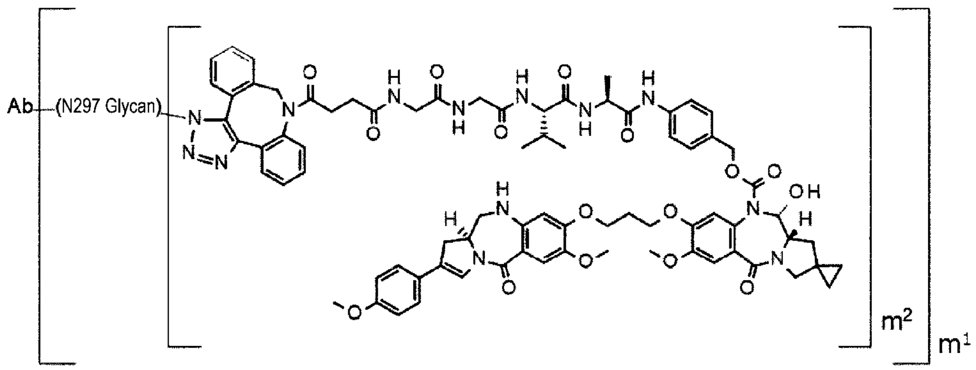

or

(XIV)

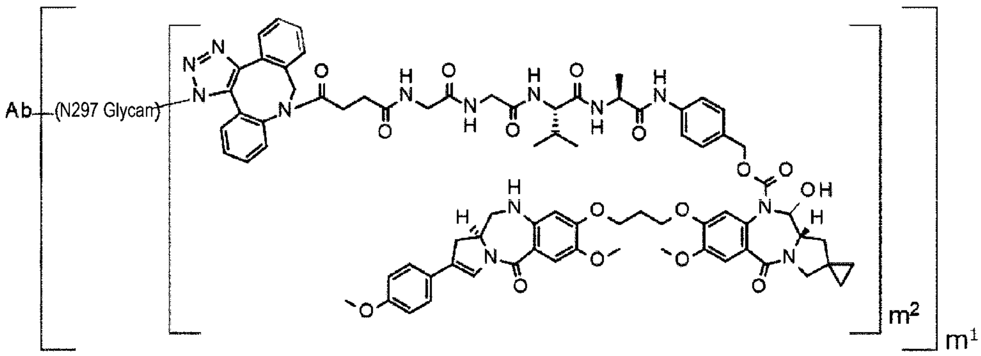

wherein,

in each structural formula shown above, $m^1$ represents an integer of 1 or 2,
$m^2$ represents an integer of 1 or 2,
Ab represents the antibody or an antigen-binding fragment of the antibody according to any one of claims 1 to 16 and 24 to 28, and
the N297 glycan represents one of N297-(Fuc)MSG1, N297-(Fuc)MSG2, and a mixture thereof, and N297-(Fuc)SG, having the structures represented by the following formulas:

[Chem. 25]

Fucα1
|
6
Galβ1-4GlcNAcβ1-2Manα1— 6
Manβ1-4GlcNAcβ1-4GlcNAcβ1—
∗—L(PEG)-NeuAcα2-6Galβ1-4GlcNAcβ1-2Manα1— 3

[N297-(Fuc)MSG1]

[Chem. 26]

Fucα1
|
6
∗-L(PEG)-NeuAcα2-6Galβ1-4GlcNAcβ1-2Manα1— 6
Manβ1-4GlcNAcβ1-4GlcNAcβ1—
Galβ1-4GlcNAcβ1-2Manα1— 3

[N297-(Fuc)MSG2]

[Chem. 27]

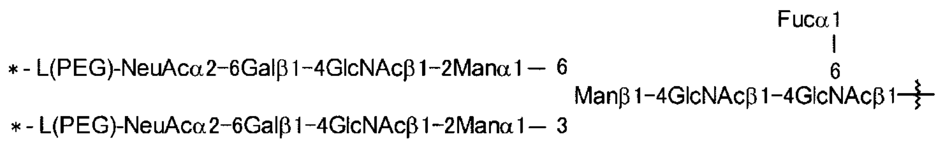

[N297-(Fuc)SG]

wherein the wavy lines represent a bond to Asn297 (N297) of the antibody, and
*-L(PEG)- in the N297 glycan represents *-$(CH_2CH_2\text{-}O)_3\text{-}CH_2CH_2\text{-}NH\text{-}$,
wherein it is indicated that the amino group at the right end is bound via amide bond to the carboxylic acid at the 2-position of a sialic acid at the non-reducing terminus of the 1-3 chain or/and 1-6 chain of the β-Man branched chain of the N297 glycan, and * represents a bond to the nitrogen atom at the 1 or 3-position on the triazole ring in each structural formula.

**47.** The antibody-drug conjugate according to claim 46, wherein the compound represented by the formula (XIV) is represented by the following formula (XIV'):

[Chem. 28]

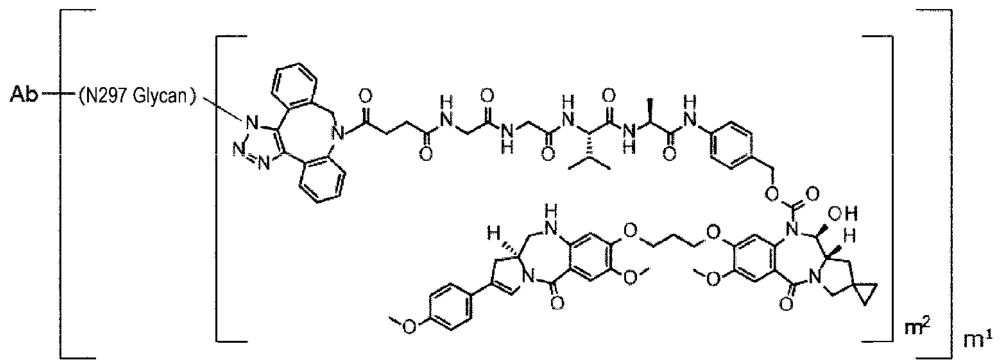

or

(XIV')

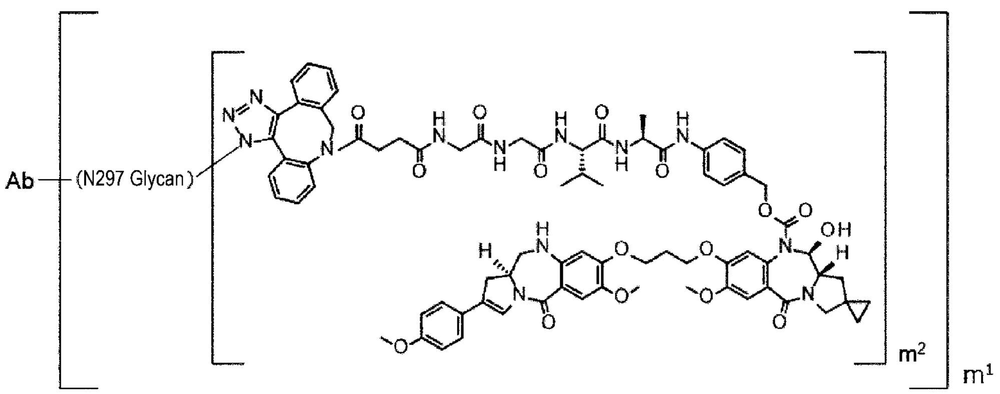

.

**48.** An antibody-drug conjugate represented by the following formula (XV):

[Chem. 29]

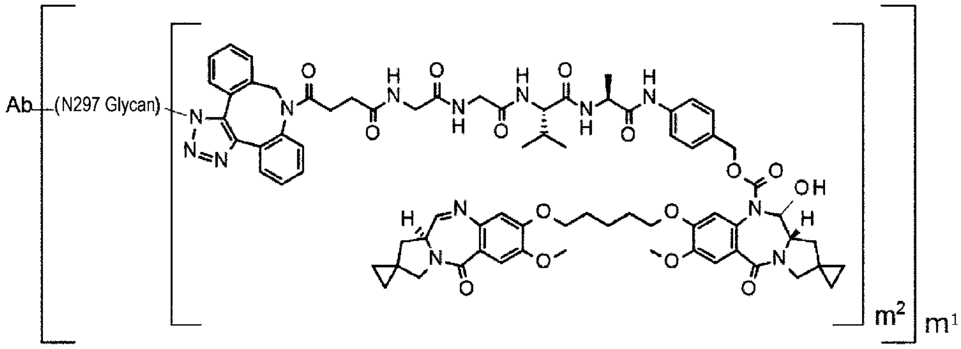

or

(XV)

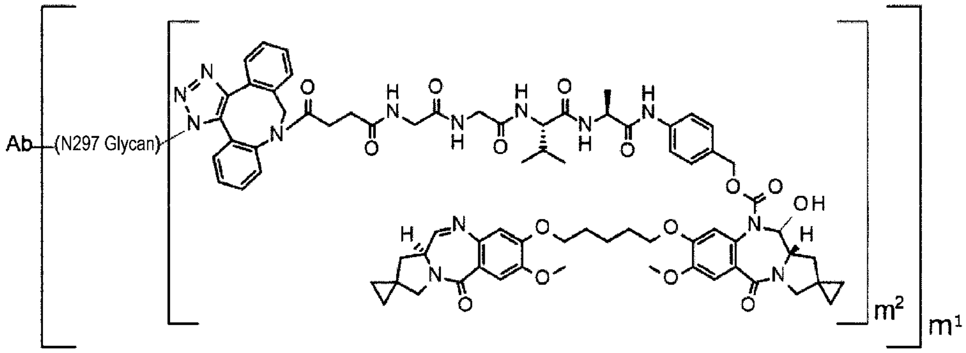

wherein,

in each structural formula shown above, $m^1$ represents an integer of 1 or 2,
$m^2$ represents an integer of 1 or 2,
Ab represents the antibody or an antigen-binding fragment of the antibody according to any one of claims 1 to 16 and 24 to 28, and
the N297 glycan represents one of N297-(Fuc)MSG1, N297-(Fuc)MSG2, and a mixture thereof, and N297-(Fuc)SG, having the structures represented by the following formulas:

[Chem. 30]

Fucα1
|
6
Galβ1-4GlcNAcβ1-2Manα1— 6
Manβ1-4GlcNAcβ1-4GlcNAcβ1—
∗—L(PEG)-NeuAcα2-6Galβ1-4GlcNAcβ1-2Manα1— 3

[N297-(Fuc)MSG1]

[Chem. 31]

Fucα1
|
6
∗ - L(PEG)-NeuAcα2-6Galβ1-4GlcNAcβ1-2Manα1— 6
Manβ1-4GlcNAcβ1-4GlcNAcβ1—
Galβ1-4GlcNAcβ1-2Manα1— 3

[N297-(Fuc)MSG2]

[Chem. 32]

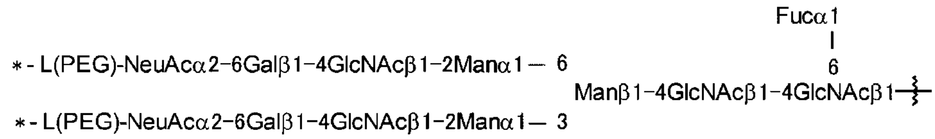

[N297-(Fuc)SG]

wherein the wavy lines represent a bond to Asn297 (N297) of the antibody, and *-L(PEG)- in the N297 glycan represents *-$(CH_2CH_2\text{-}O)_3\text{-}CH_2CH_2\text{-}NH$-, wherein it is indicated that the amino group at the right end is bound via amide bond to the carboxylic acid at the 2-position of a sialic acid at the non-reducing terminus of the 1-3 chain or/and 1-6 chain of the β-Man branched chain of the N297 glycan, and * represents a bond to the nitrogen atom at the 1 or 3-position on the triazole ring in each structural formula.

**49.** The antibody-drug conjugate according to claim 48, wherein the compound represented by the formula (XV) is represented by the following formula (XV'):

[Chem. 33]

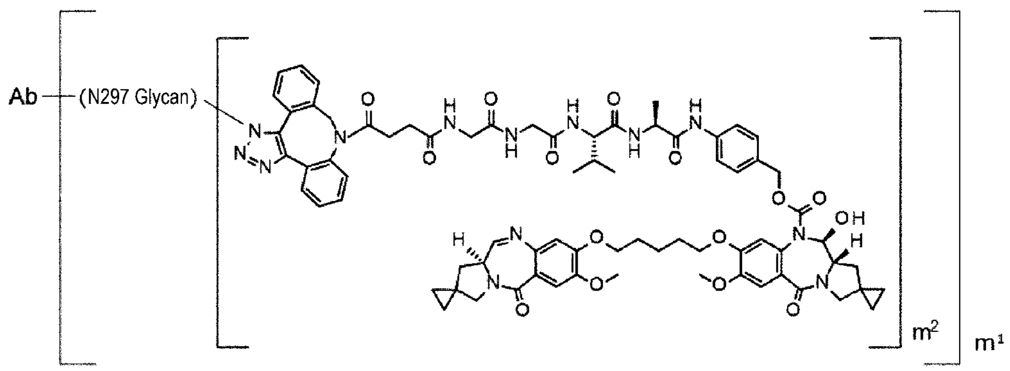

(XV')

or

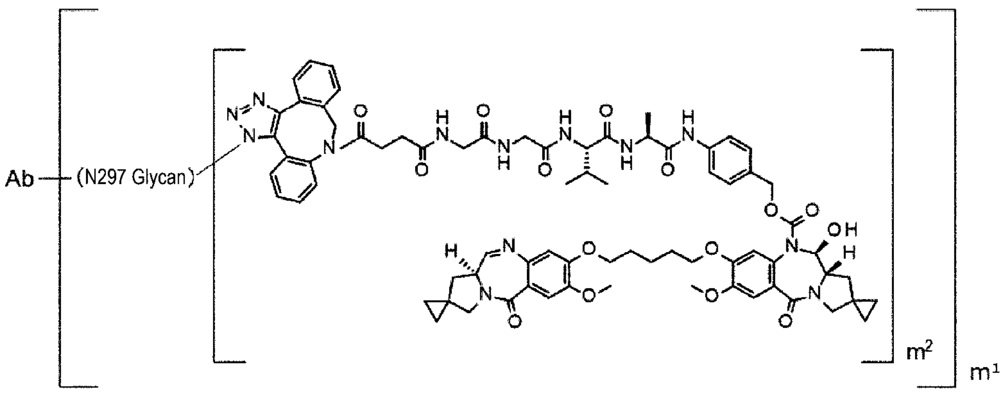

.

**50.** A method for producing a glycan-remodeled antibody, comprising:

i) producing the antibody or an antigen-binding fragment of the antibody according to any one of claims 1 to 16 and 24 to 28,
ii) treating the antibody obtained in i) with hydrolase to produce a (Fucα1,6)GlcNAc-antibody, and
iii)-1 reacting in the presence of glycosyltransferase, the (Fucα1,6)GlcNAc-antibody and a glycan donner

molecule obtained by introducing a PEG linker having an azide group to the carbonyl group of the carboxylic acid at the 2-position of a sialic acid in MSG (9) or SG (10) and oxazolinating the reducing terminus, or
iii)-2 reacting in the presence of glycosyltransferase, the (Fucα1,6)GlcNAc-antibody and a glycan donner molecule obtained by introducing a PEG linker having an azide group to the carbonyl group of the carboxylic acid at the 2-position of a sialic acid in (MSG-)Asn or (SG-)Asn in which an α-amino group is optionally protected and to the carbonyl group of the carboxylic acid in the Asn, allowing hydrolase to act on the resulting molecule, and then oxazolinating the reducing terminus.

**51.** A method for producing a glycan-remodeled antibody, comprising:

i) producing the antibody or an antigen-binding fragment of the antibody according to any one of claims 1 to 16 and 24 to 28,
ii) treating the antibody obtained in i) with hydrolase to produce a (Fucα1,6)GlcNAc-antibody, and
iii) binding a glycan to the (Fucα1,6)GlcNAc-antibody by using two endo-β-N-acetylglucosaminidases.

**52.** A method for producing the antibody-drug conjugate according to any one of claims 31 to 49, comprising reacting the glycan-remodeled antibody obtained by the method according to claim 50 or 51, with a drug linker.

**53.** The antibody-drug conjugate according to any one of claims 31 to 49, wherein the antibody-drug conjugate is produced by the method according to claim 52.

**54.** A pharmaceutical composition, comprising the antibody or an antigen-binding fragment of the antibody according to any one of claims 1 to 16, and 24 to 28, or the antibody-drug conjugate according to any one of claims 29 to 49 and 53.

**55.** The pharmaceutical composition according to claim 54, wherein the pharmaceutical composition is an antitumor drug.

**56.** The pharmaceutical composition according to claim 55, wherein a tumor expresses LRRC15 in a tumor cell and/or a cell in the stroma thereof.

**57.** The pharmaceutical composition according to claim 55 or 56, wherein the tumor is a breast carcinoma, a head and neck carcinoma, a lung carcinoma, a pancreatic carcinoma, a colorectal carcinoma, an esophageal carcinoma, an ovarian carcinoma, a bladder carcinoma, a cholangiocarcinoma, a gastric carcinoma, a mesothelioma, an uterine carcinoma, a thyroid carcinoma, a renal carcinoma, a hepatocarcinoma, a prostate carcinoma, a bone and soft tissue sarcoma, a melanoma, or a glioma.

**58.** A method for treating a tumor, comprising administering a therapeutically effective amount of the antibody or an antigen-binding fragment of the antibody according to any one of claims 1 to 16, and 24 to 28, the antibody-drug conjugate according to any one of claims 29 to 49 and 53, or the pharmaceutical composition according to any one of claims 54 to 57, to a subject in need of tumor treatment.

**59.** The treatment method according to claim 58, wherein the tumor expresses LRRC15 in a tumor cell and/or a cell in the stroma thereof.

**60.** The treatment method according to claim 58 or 59, wherein the tumor is a breast carcinoma, a head and neck carcinoma, a lung carcinoma, a pancreatic carcinoma, a colorectal carcinoma, an esophageal carcinoma, an ovarian carcinoma, a bladder carcinoma, a cholangiocarcinoma, a gastric carcinoma, a mesothelioma, an uterine carcinoma, a thyroid carcinoma, a renal carcinoma, a hepatocarcinoma, a prostate carcinoma, a bone and soft tissue sarcoma, a melanoma, or a glioma.

**61.** The treatment method according to any one of claims 58 to 60, comprising administering to a subject a therapeutically effective amount of the antibody or an antigen-binding fragment of the antibody according to any one of claims 1 to 16, and 24 to 28, the antibody-drug conjugate according to any one of claims 29 to 49 and 53, or the pharmaceutical composition according to any one of claims 54 to 57 and at least one different antitumor drug concurrently, separately, or consecutively.

Fig. 1-1

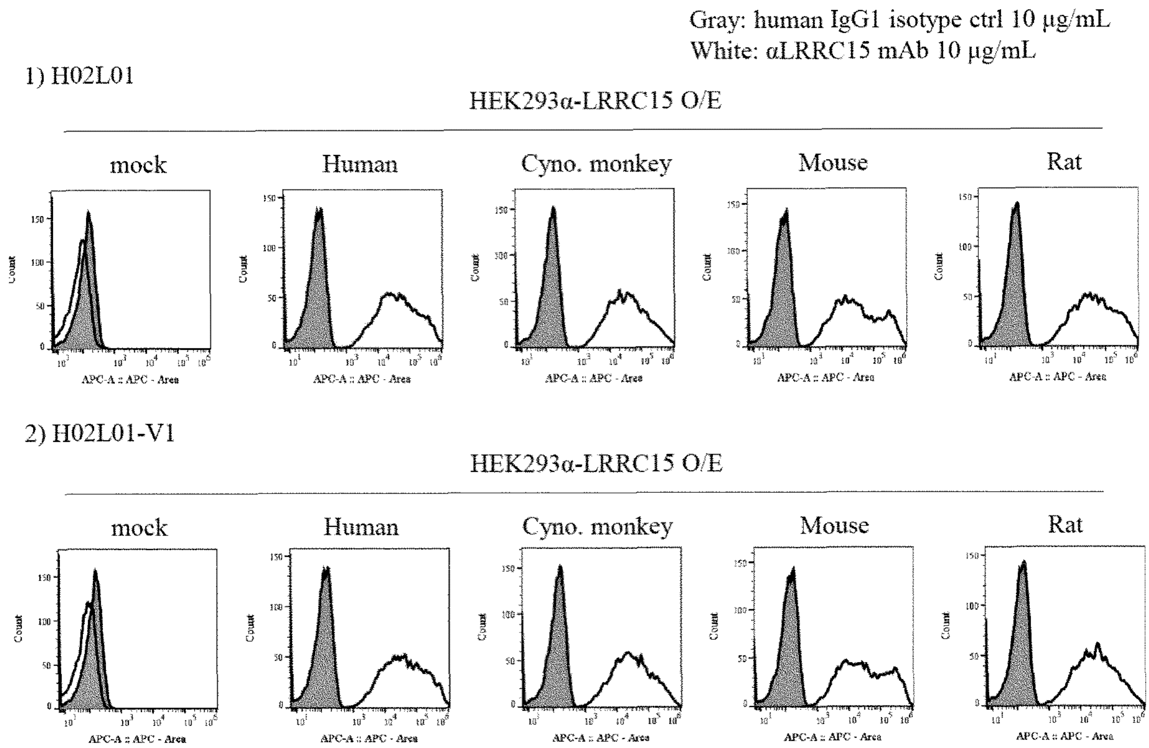
Gray: human IgG1 isotype ctrl 10 μg/mL
White: αLRRC15 mAb 10 μg/mL
1) H02L01
HEK293α-LRRC15 O/E
mock
Human
Cyno. monkey
Mouse
Rat
Count
APC-A :: APC - Area
2) H02L01-V1
HEK293α-LRRC15 O/E
mock
Human
Cyno. monkey
Mouse
Rat
Count
APC-A :: APC - Area

Fig. 1-2

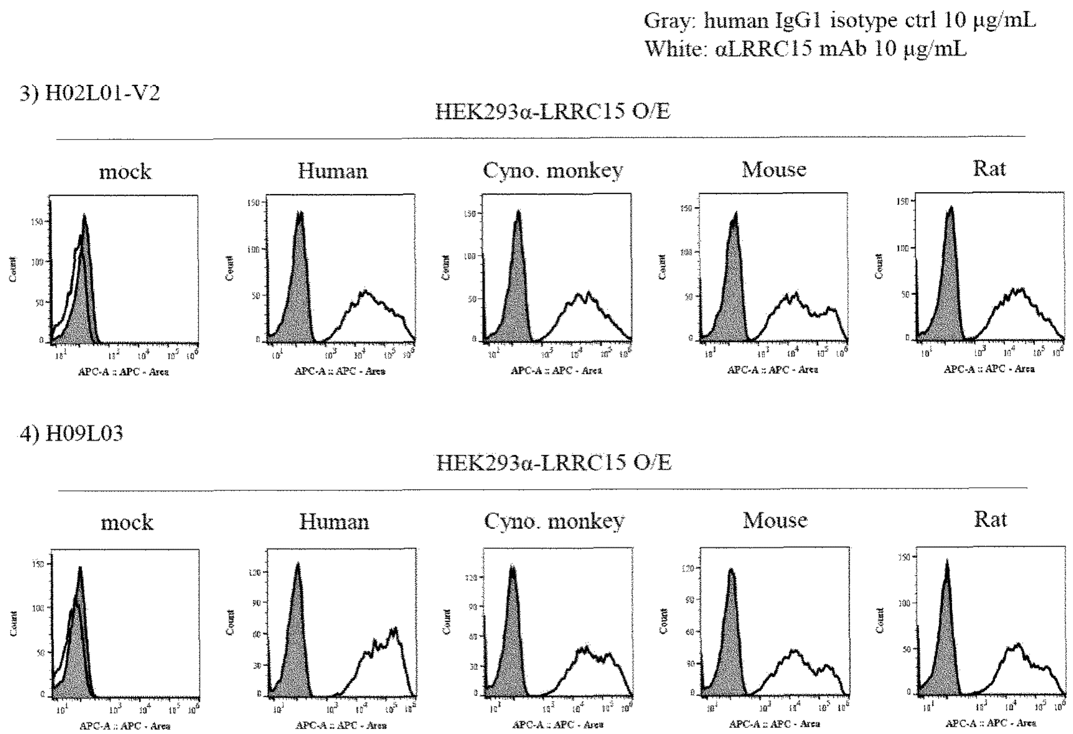
Gray: human IgG1 isotype ctrl 10 μg/mL
White: αLRRC15 mAb 10 μg/mL
3) H02L01-V2
HEK293α-LRRC15 O/E
mock
Human
Cyno. monkey
Mouse
Rat
Count
APC-A :: APC - Area
4) H09L03
HEK293α-LRRC15 O/E
mock
Human
Cyno. monkey
Mouse
Rat
Count
APC-A :: APC - Area

Fig. 2-1

Gray: human IgG1 isotype ctrl 10 μg/mL
White: αLRRC15 mAb 10 μg/mL

1) H02L01

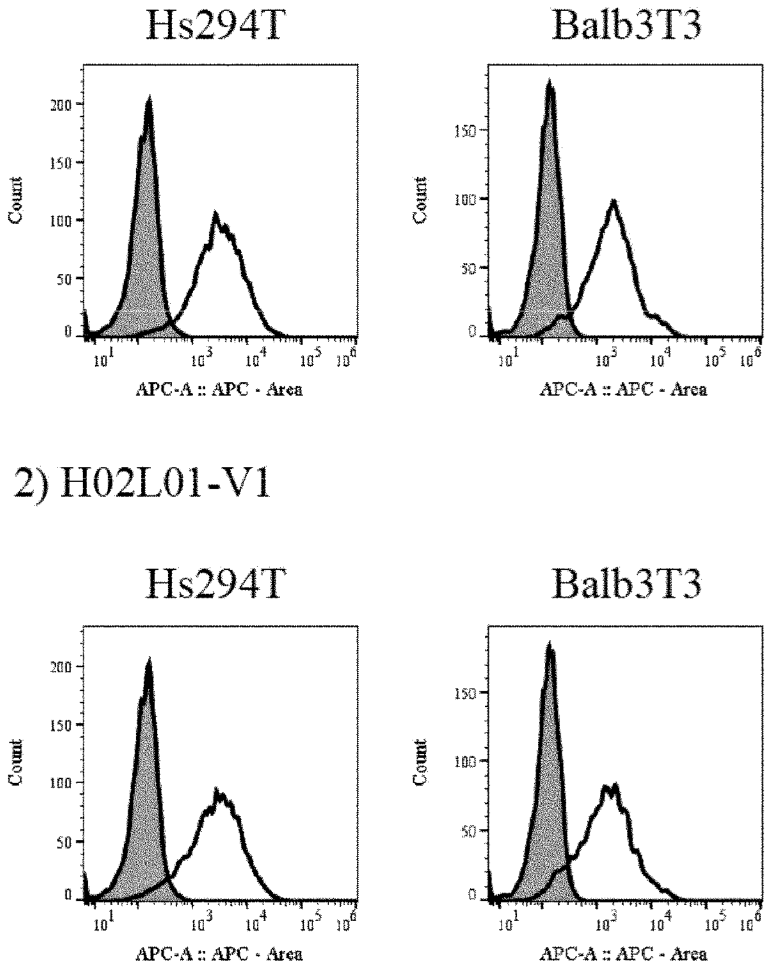

Fig. 2-2

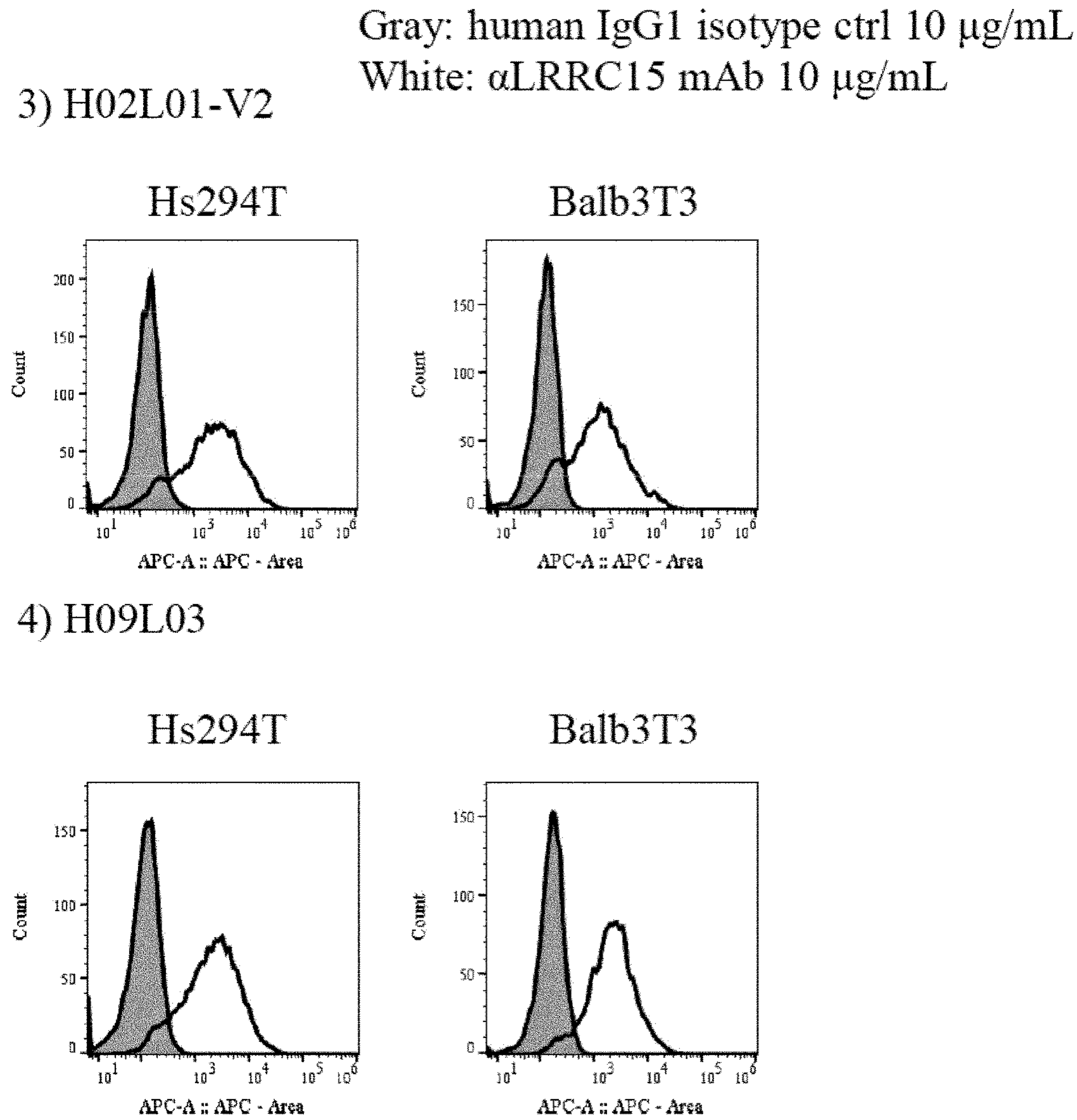
Gray: human IgG1 isotype ctrl 10 μg/mL
White: αLRRC15 mAb 10 μg/mL
3) H02L01-V2
Hs294T
Balb3T3
Count
APC-A :: APC - Area
4) H09L03
Hs294T
Balb3T3
Count
APC-A :: APC - Area

Fig. 3-1

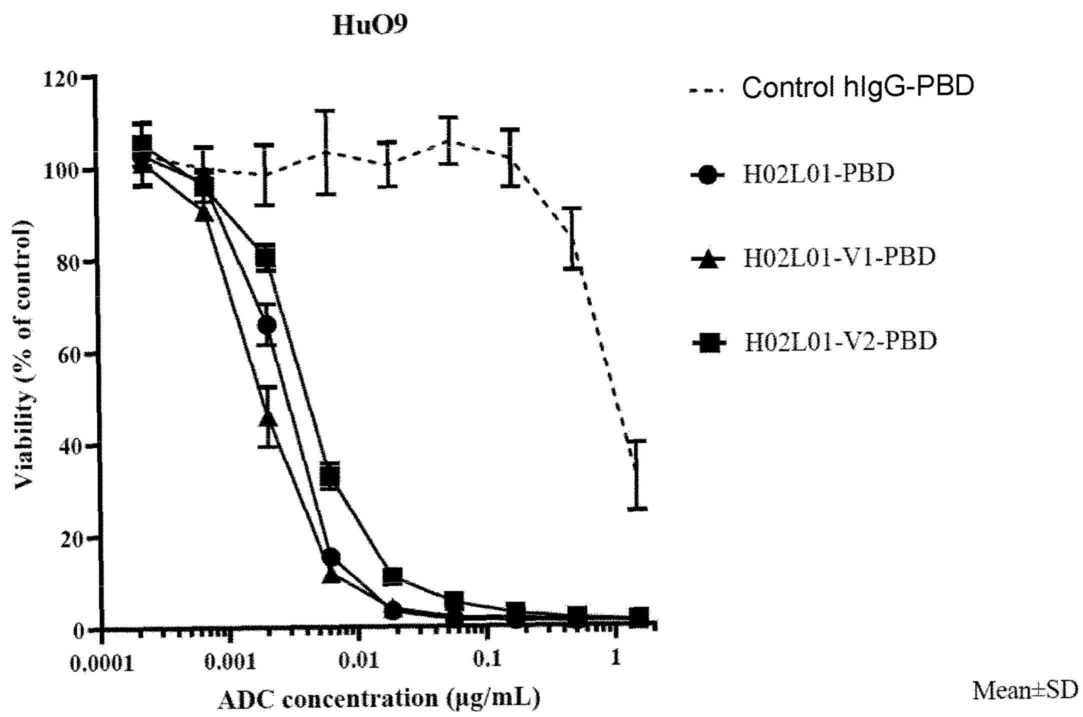
HuO9
Viability (% of control)
120
100
80
60
40
20
0
0.0001
0.001
0.01
0.1
1
ADC concentration (μg/mL)
Control hIgG-PBD
H02L01-PBD
H02L01-V1-PBD
H02L01-V2-PBD
Mean±SD

Fig. 3-2

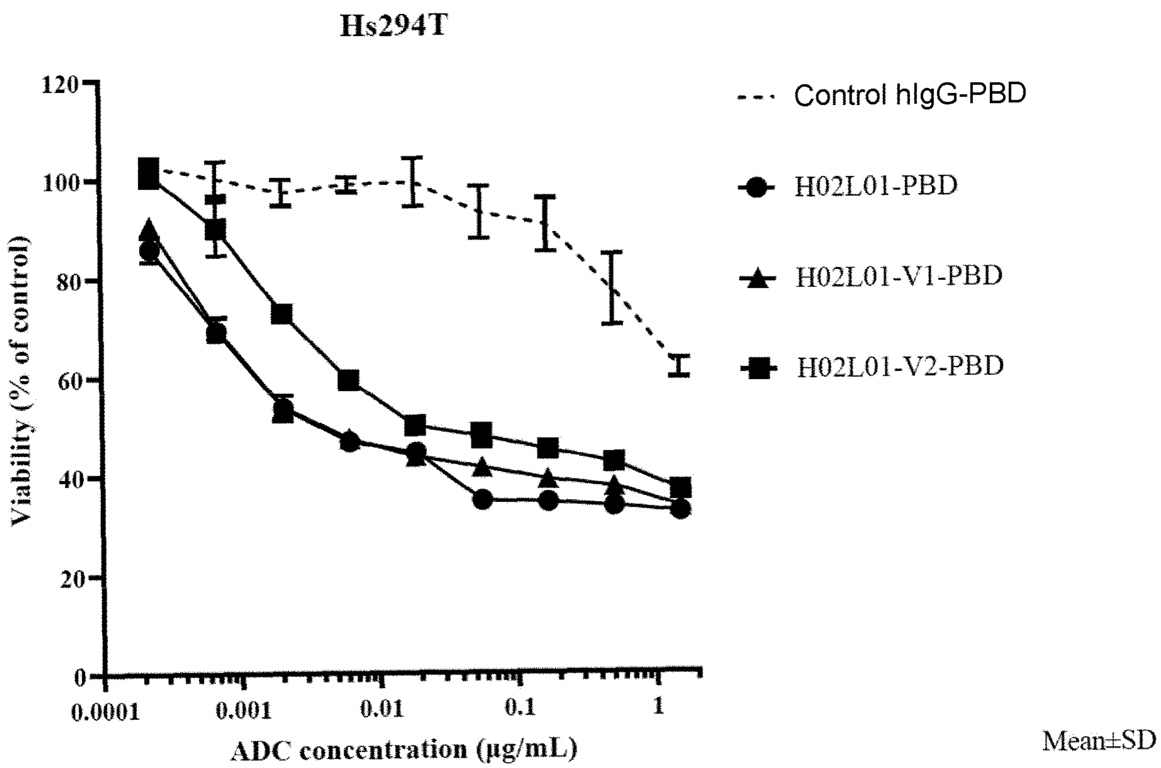
Hs294T
Viability (% of control)
120
100
80
60
40
20
0
0.0001
0.001
0.01
0.1
1
ADC concentration (μg/mL)
Control hIgG-PBD
H02L01-PBD
H02L01-V1-PBD
H02L01-V2-PBD
Mean±SD

Fig. 3-3

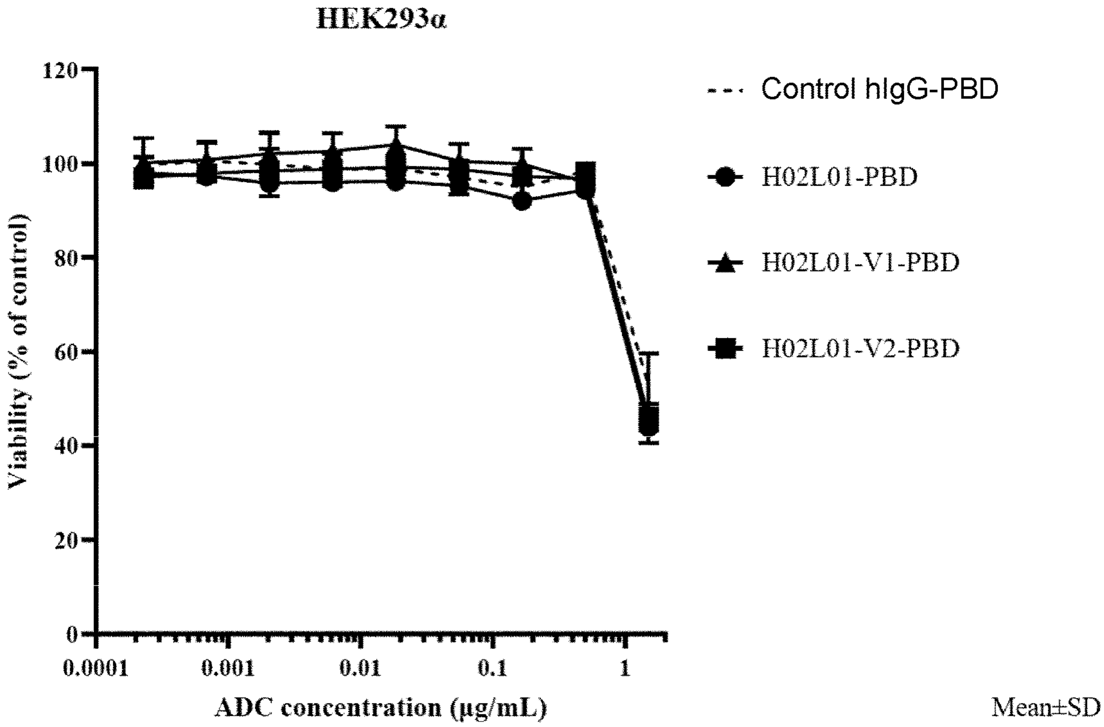
HEK293α
Viability (% of control)
120
100
80
60
40
20
0
0.0001
0.001
0.01
0.1
1
ADC concentration (μg/mL)
Control hIgG-PBD
H02L01-PBD
H02L01-V1-PBD
H02L01-V2-PBD
Mean±SD

Fig. 4

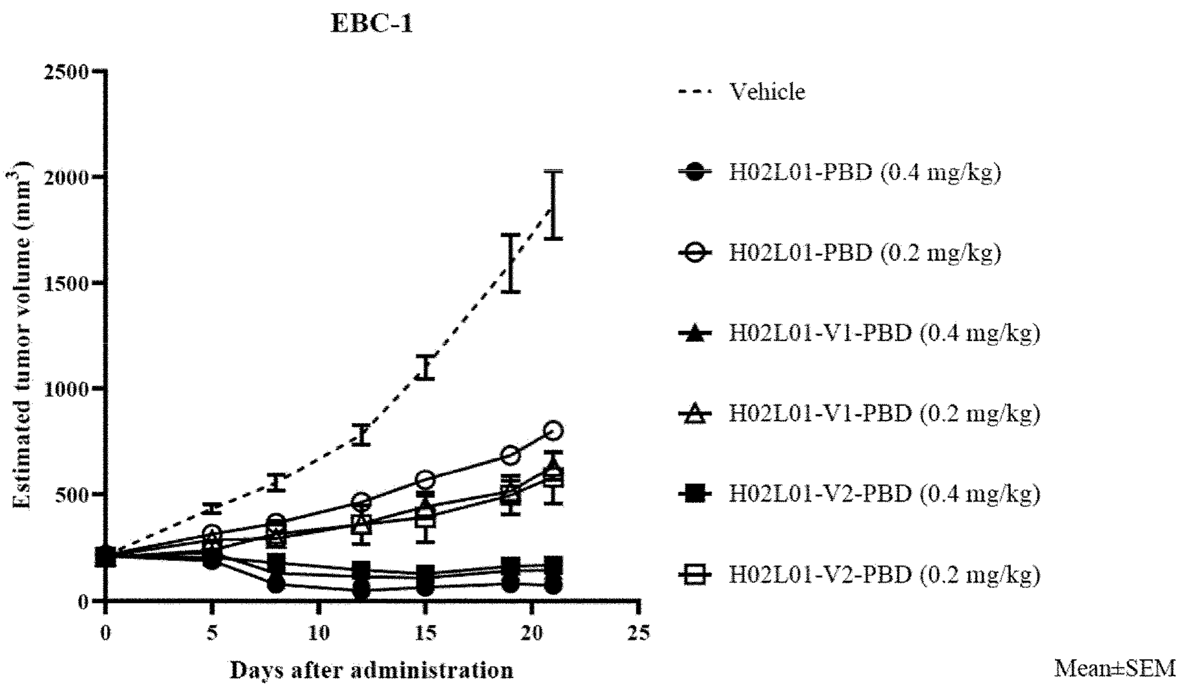
EBC-1
Estimated tumor volume (mm3)
2500
2000
1500
1000
500
0
0
5
10
15
20
25
Days after administration
Vehicle
H02L01-PBD (0.4 mg/kg)
H02L01-PBD (0.2 mg/kg)
H02L01-V1-PBD (0.4 mg/kg)
H02L01-V1-PBD (0.2 mg/kg)
H02L01-V2-PBD (0.4 mg/kg)
H02L01-V2-PBD (0.2 mg/kg)
Mean±SEM

Fig. 5

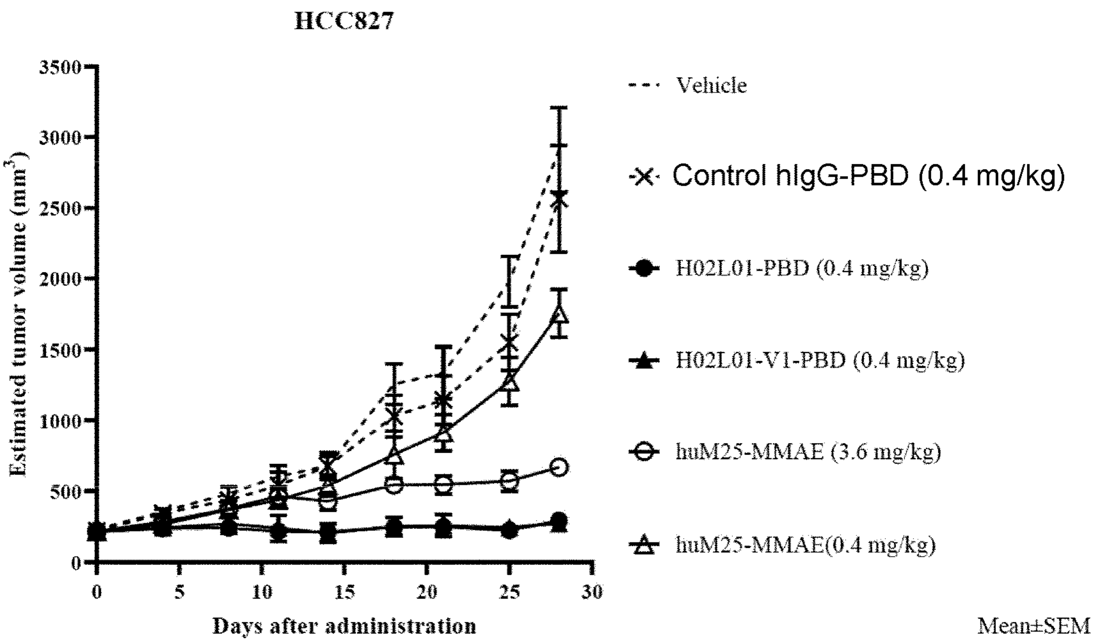
HCC827
Estimated tumor volume (mm3)
3500
3000
2500
2000
1500
1000
500
0
0
5
10
15
20
25
30
Days after administration
Vehicle
Control hIgG-PBD (0.4 mg/kg)
H02L01-PBD (0.4 mg/kg)
H02L01-V1-PBD (0.4 mg/kg)
huM25-MMAE (3.6 mg/kg)
huM25-MMAE(0.4 mg/kg)
Mean±SEM

Fig. 6

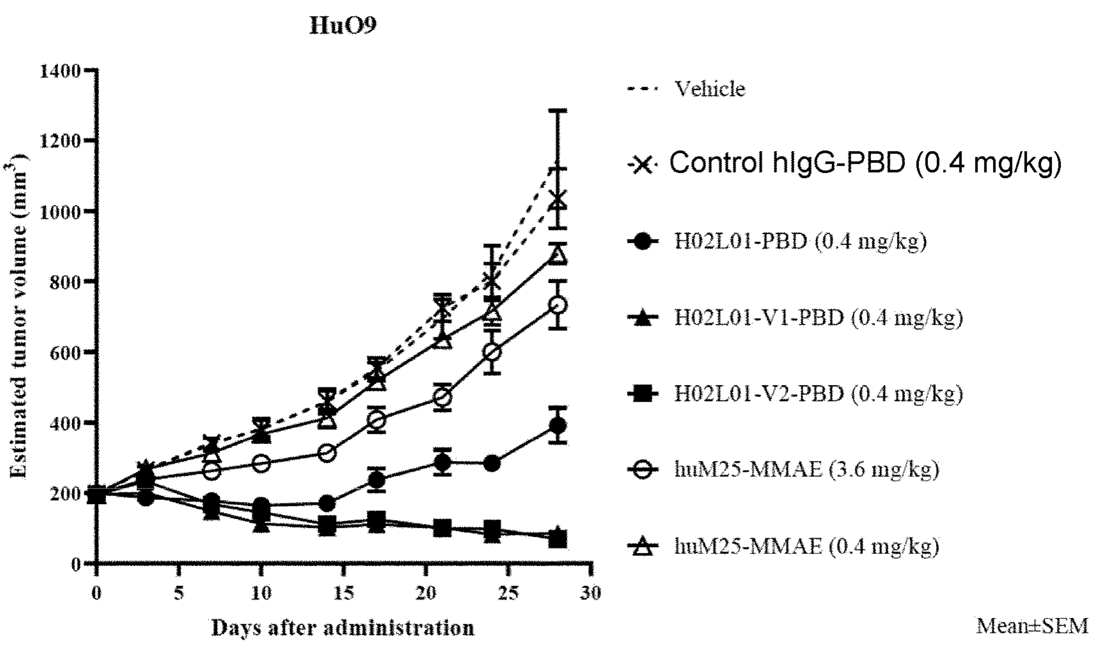
HuO9
Estimated tumor volume (mm3)
1400
1200
1000
800
600
400
200
0
0
5
10
15
20
25
30
Days after administration
Vehicle
Control hIgG-PBD (0.4 mg/kg)
H02L01-PBD (0.4 mg/kg)
H02L01-V1-PBD (0.4 mg/kg)
H02L01-V2-PBD (0.4 mg/kg)
huM25-MMAE (3.6 mg/kg)
huM25-MMAE (0.4 mg/kg)
Mean±SEM

Fig. 7

SEQ ID NO. 1: Amino acid sequence of full-length human LRRC15 isoform b

MPLKHYLLLLVGCQAWGAGLAYHGCPSECTCSRASQVECTGARIVAVPTPLPWNAMSLQILNTHITELNE
SPFLNISALIALRIEKNELSRITPGAFRNLGSLRYLSLANNKLQVLPIGLFQGLDSLESLLLSSNQLLQIQPA
HFSQCSNLKELQLHGNHLEYIPDGAFDHLVGLTKLNLGKNSLTHISPRVFQHLGNLQVLRLYENRLTDIP
MGTFDGLVNLQELALQQNQIGLLSPGLFHNNHNLQRLYLSNNHISQLPPSVFMQLPQLNRLTLFGNSLK
ELSPGIFGPMPNLRELWLYDNHISSLPDNVFSNLRQLQVLILSRNQISFISPGAFNGLTELRELSLHTNAL
QDLDGNVFRMLANLQNISLQNNRLRQLPGNIFANVNGLMAIQLQNNQLENLPLGIFDHLGKLCELRLYD
NPWRCDSDILPLRNWLLLNQPRLGTDTVPVCFSPANVRGQSLIIINVNVAVPSVHVPEVPSYPETPWYPD
TPSYPDTTSVSSTTELTSPVEDYTDLTTIQVTDDRSVWGMTQAQSGLAIAAIVIGIVALACSLAACVGCCCC
KKRSQAVLMQMKAPNEC

SEQ ID NO. 2: Amino acid sequence of extracellular region of human LRRC15 isoform b

YHGCPSECTCSRASQVECTGARIVAVPTPLPWNAMSLQILNTHITELNESPFLNISALIALRIEKNELSRIT
PGAFRNLGSLRYLSLANNKLQVLPIGLFQGLDSLESLLLSSNQLLQIQPAHFSQCSNLKELQLHGNHLEY
IPDGAFDHLVGLTKLNLGKNSLTHISPRVFQHLGNLQVLRLYENRLTDIPMGTFDGLVNLQELALQQNQI
GLLSPGLFHNNHNLQRLYLSNNHISQLPPSVFMQLPQLNRLTLFGNSLKELSPGIFGPMPNLRELWLYD
NHISSLPDNVFSNLRQLQVLILSRNQISFISPGAFNGLTELRELSLHTNALQDLDGNVFRMLANLQNISLQ
NNRLRQLPGNIFANVNGLMAIQLQNNQLENLPLGIFDHLGKLCELRLYDNPWRCDSDILPLRNWLLLNQ
PRLGTDTVPVCFSPANVRGQSLIIINVNVAVPSVHVPEVPSYPETPWYPDTPSYPDTTSVSSTTELTSPVED
YTDLTTIQVTDDRSVWGMTQAQSG

SEQ ID NO. 3: Amino acid sequence of human LRRC15-His protein

MPLKHYLLLLVGCQAWGAGLAYHGCPSECTCSRASQVECTGARIVAVPTPLPWNAMSLQILNTHITELNE
SPFLNISALIALRIEKNELSRITPGAFRNLGSLRYLSLANNKLQVLPIGLFQGLDSLESLLLSSNQLLQIQPA
HFSQCSNLKELQLHGNHLEYIPDGAFDHLVGLTKLNLGKNSLTHISPRVFQHLGNLQVLRLYENRLTDIP
MGTFDGLVNLQELALQQNQIGLLSPGLFHNNHNLQRLYLSNNHISQLPPSVFMQLPQLNRLTLFGNSLK
ELSPGIFGPMPNLRELWLYDNHISSLPDNVFSNLRQLQVLILSRNQISFISPGAFNGLTELRELSLHTNAL
QDLDGNVFRMLANLQNISLQNNRLRQLPGNIFANVNGLMAIQLQNNQLENLPLGIFDHLGKLCELRLYD
NPWRCDSDILPLRNWLLLNQPRLGTDTVPVCFSPANVRGQSLIIINVNVAVPSVHVPEVPSYPETPWYPD
TPSYPDTTSVSSTTELTSPVEDYTDLTTIQVTDDRSVWGMTQAQSGHHHHHH

# Fig. 8

SEQ ID NO. 4: Amino acid sequence of full-length cynomolgus monkey LRRC15

MPLKHYLLLLVGCQAWGAGLAYYGCPSECTCSRASQVECTGARIVAVPTPLPWNAMSLQILNTHITELSE
SPFLNISALIALRIEKNELSHIMPGAFRNLGSLRYLSLANNKLQVLPIGLFQGLDSLESLLLSSNQLVQIQP
AHFSQCSNLKELQLHGNHLEYIPDGAFDHLVGLTKLNLGKNSLTHISPRVFQHLGNLQVLRLYENRLTDI
PMGTFDGLVNLQELALQQNQIGLLSPGLFHNNHNLQRLYLSNNHISQLPPSIFMQLPQLNRLTLFGNSL
KELSPGIFGPMPNLRELWLYDNHITSLPDNVFSNLRQLQVLILSRNQISFISPGAFNGLTELRELSLHTNA
LQDLDGNVFRMLANLQNISLQNNRLRQLPGNIFANVNGLMTIQLQNNQLENLPLGIFDHLGKLCELRLY
DNPWRCDSDILPLRNWLLLNQPRLGTDTVPVCFSPANVRGQSLIIINVNVAVPSVHVPEVPSYPETSWYP
DTSSYPDTTSISSTTELTSPVEDYTDLTTIQVTDDRSVWGMTQAQSGLAIAAIVIGIVALACSLAACIGCCCC
KKRSQAVLMQMKAPNEC

SEQ ID NO. 5: Amino acid sequence of extracellular region of cynomolgus monkey LRRC15

YYGCPSECTCSRASQVECTGARIVAVPTPLPWNAMSLQILNTHITELSESPFLNISALIALRIEKNELSHIM
PGAFRNLGSLRYLSLANNKLQVLPIGLFQGLDSLESLLLSSNQLVQIQPAHFSQCSNLKELQLHGNHLEYI
PDGAFDHLVGLTKLNLGKNSLTHISPRVFQHLGNLQVLRLYENRLTDIPMGTFDGLVNLQELALQQNQIG
LLSPGLFHNNHNLQRLYLSNNHISQLPPSIFMQLPQLNRLTLFGNSLKELSPGIFGPMPNLRELWLYDNH
ITSLPDNVFSNLRQLQVLILSRNQISFISPGAFNGLTELRELSLHTNALQDLDGNVFRMLANLQNISLQNN
RLRQLPGNIFANVNGLMTIQLQNNQLENLPLGIFDHLGKLCELRLYDNPWRCDSDILPLRNWLLLNQPR
LGTDTVPVCFSPANVRGQSLIIINVNVAVPSVHVPEVPSYPETSWYPDTSSYPDTTSISSTTELTSPVEDYT
DLTTIQVTDDRSVWGMTQAQSG

SEQ ID NO. 6: Amino acid sequence of cynomolgus monkey LRRC15-His protein

MPLKHYLLLLVGCQAWGAGLAYYGCPSECTCSRASQVECTGARIVAVPTPLPWNAMSLQILNTHITELSE
SPFLNISALIALRIEKNELSHIMPGAFRNLGSLRYLSLANNKLQVLPIGLFQGLDSLESLLLSSNQLVQIQP
AHFSQCSNLKELQLHGNHLEYIPDGAFDHLVGLTKLNLGKNSLTHISPRVFQHLGNLQVLRLYENRLTDI
PMGTFDGLVNLQELALQQNQIGLLSPGLFHNNHNLQRLYLSNNHISQLPPSIFMQLPQLNRLTLFGNSL
KELSPGIFGPMPNLRELWLYDNHITSLPDNVFSNLRQLQVLILSRNQISFISPGAFNGLTELRELSLHTNA
LQDLDGNVFRMLANLQNISLQNNRLRQLPGNIFANVNGLMTIQLQNNQLENLPLGIFDHLGKLCELRLY
DNPWRCDSDILPLRNWLLLNQPRLGTDTVPVCFSPANVRGQSLIIINVNVAVPSVHVPEVPSYPETSWYP
DTSSYPDTTSISSTTELTSPVEDYTDLTTIQVTDDRSVWGMTQAQSGHHHHHH

Fig. 9

SEQ ID NO. 7: Amino acid sequence of full-length mouse LRRC15

MPLKHYLLLLVSCQAWAAGLAYYGCPSECTCSRASQVECTGAQIVAMPSPLPWNAMSLQILNTHITELPE
DKFLNISALIALKMEKNELANIMPGAFRNLGSLRHLSLANNKLKNLPVRLFQDVNNLETLLLSNNQLVQ
IQPAQFSQFSNLKELQLYGNNLEYIPEGVFDHLVGLTKLNLGNNGFTHLSPRVFQHLGNLQVLRLYENRL
SDIPMGTFDALGNLQELALQENQIGTLSPGLFHNNRNLQRLYLSNNHISHLPPGIFMQLPHLNKLTLFGN
SLKELSPGVFGPMPNLRELWLYNNHITSLPDNAFSHLNQLQVLILSHNQLSYISPGAFNGLTNLRELSLH
TNALQDLDGNVFRSLANLRNVSLQNNRLRQLPGSIFANVNGLMTIQLQNNNLENLPLGIFDHLGNLCEL
RLYDNPWRCDSNILPLHDWLILNRARLGTDTLPVCSSPASVRGQSLVIINVNFPGPSVQGPETPEVSSYPD
TSSYPDSTSISSTTEITRSTDDDYTDLNTIEPIDDRNTWGMTDAQSGLAIAAIVIGIIALACSLAACICCCCC
KKRSQAVLMQMKAPNEC

SEQ ID NO. 8: Amino acid sequence of extracellular region of mouse LRRC15

YYGCPSECTCSRASQVECTGAQIVAMPSPLPWNAMSLQILNTHITELPEDKFLNISALIALKMEKNELANI
MPGAFRNLGSLRHLSLANNKLKNLPVRLFQDVNNLETLLLSNNQLVQIQPAQFSQFSNLKELQLYGNNL
EYIPEGVFDHLVGLTKLNLGNNGFTHLSPRVFQHLGNLQVLRLYENRLSDIPMGTFDALGNLQELALQE
NQIGTLSPGLFHNNRNLQRLYLSNNHISHLPPGIFMQLPHLNKLTLFGNSLKELSPGVFGPMPNLRELW
LYNNHITSLPDNAFSHLNQLQVLILSHNQLSYISPGAFNGLTNLRELSLHTNALQDLDGNVFRSLANLRN
VSLQNNRLRQLPGSIFANVNGLMTIQLQNNNLENLPLGIFDHLGNLCELRLYDNPWRCDSNILPLHDWL
ILNRARLGTDTLPVCSSPASVRGQSLVIINVNFPGPSVQGPETPEVSSYPDTSSYPDSTSISSTTEITRSTDD
DYTDLNTIEPIDDRNTWGMTDAQSG

SEQ ID NO. 9: Amino acid sequence of mouse LRRC15-His protein

MPLKHYLLLLVSCQAWAAGLAYYGCPSECTCSRASQVECTGAQIVAMPSPLPWNAMSLQILNTHITELPE
DKFLNISALIALKMEKNELANIMPGAFRNLGSLRHLSLANNKLKNLPVRLFQDVNNLETLLLSNNQLVQ
IQPAQFSQFSNLKELQLYGNNLEYIPEGVFDHLVGLTKLNLGNNGFTHLSPRVFQHLGNLQVLRLYENRL
SDIPMGTFDALGNLQELALQENQIGTLSPGLFHNNRNLQRLYLSNNHISHLPPGIFMQLPHLNKLTLFGN
SLKELSPGVFGPMPNLRELWLYNNHITSLPDNAFSHLNQLQVLILSHNQLSYISPGAFNGLTNLRELSLH
TNALQDLDGNVFRSLANLRNVSLQNNRLRQLPGSIFANVNGLMTIQLQNNNLENLPLGIFDHLGNLCEL
RLYDNPWRCDSNILPLHDWLILNRARLGTDTLPVCSSPASVRGQSLVIINVNFPGPSVQGPETPEVSSYPD
TSSYPDSTSISSTTEITRSTDDDYTDLNTIEPIDDRNTWGMTDAQSGHHHHHH

# Fig. 10

SEQ ID NO. 10: Amino acid sequence of full-length rat LRRC15

MPLKHYLLLLVGCQAWALGLAYYGCPSECTCSRASQVECTGARIVAMPTPLPWNAMSLQVVNTHITELP
ENLFLNISALIALKMEKNELSTIMPGAFRNLGSLRYLSLANNKLRMLPIRVFQDVNNLESLLLSNNQLVQ
IQPAQFSQFSNLRELQLHGNNLESIPEEAFDHLVGLTKLNLGRNSFTHLSPRLFQHLGNLQVLRLHENRL
SDIPMGTFDALGNLQELALQENQIGTLSPGLFHNNRNLQRLYLSNNHISQLPPGIFMQLPQLNKLTLFGN
SLRELSPGVFGPMPNLRELWLYNNHITSLADNTFSHLNQLQVLILSHNQLTYISPGAFNGLTNLRELSLH
TNALQDLDSNVFRSLANLQNISLQSNRLRQLPGSIFANVNGLTTIQLQNNNLENLPLGIFDHLVNLCELR
LYDNPWRCDSDILPLHNWLLLNRARLGTDTLPVCSSPANVRGQSLVIININFPGPSVQGPETPEVPSYPDT
PSYPDTTSVSSTTEITSAVDDYTDLTTIEATDDRNTWGMTEAQSGLAIAAIVIGIIALACSLAACICCCCKK
RSQAVLMQMKAPNEC

SEQ ID NO. 11: Amino acid sequence of extracellular region of rat LRRC15

YYGCPSECTCSRASQVECTGARIVAMPTPLPWNAMSLQVVNTHITELPENLFLNISALIALKMEKNELSTI
MPGAFRNLGSLRYLSLANNKLRMLPIRVFQDVNNLESLLLSNNQLVQIQPAQFSQFSNLRELQLHGNNL
ESIPEEAFDHLVGLTKLNLGRNSFTHLSPRLFQHLGNLQVLRLHENRLSDIPMGTFDALGNLQELALQE
NQIGTLSPGLFHNNRNLQRLYLSNNHISQLPPGIFMQLPQLNKLTLFGNSLRELSPGVFGPMPNLRELWL
YNNHITSLADNTFSHLNQLQVLILSHNQLTYISPGAFNGLTNLRELSLHTNALQDLDSNVFRSLANLQNI
SLQSNRLRQLPGSIFANVNGLTTIQLQNNNLENLPLGIFDHLVNLCELRLYDNPWRCDSDILPLHNWLLL
NRARLGTDTLPVCSSPANVRGQSLVIININFPGPSVQGPETPEVPSYPDTPSYPDTTSVSSTTEITSAVDDY
TDLTTIEATDDRNTWGMTEAQSG

SEQ ID NO. 12: Amino acid sequence of rat LRRC15-His protein

MPLKHYLLLLVGCQAWALGLAYYGCPSECTCSRASQVECTGARIVAMPTPLPWNAMSLQVVNTHITELP
ENLFLNISALIALKMEKNELSTIMPGAFRNLGSLRYLSLANNKLRMLPIRVFQDVNNLESLLLSNNQLVQ
IQPAQFSQFSNLRELQLHGNNLESIPEEAFDHLVGLTKLNLGRNSFTHLSPRLFQHLGNLQVLRLHENRL
SDIPMGTFDALGNLQELALQENQIGTLSPGLFHNNRNLQRLYLSNNHISQLPPGIFMQLPQLNKLTLFGN
SLRELSPGVFGPMPNLRELWLYNNHITSLADNTFSHLNQLQVLILSHNQLTYISPGAFNGLTNLRELSLH
TNALQDLDSNVFRSLANLQNISLQSNRLRQLPGSIFANVNGLTTIQLQNNNLENLPLGIFDHLVNLCELR
LYDNPWRCDSDILPLHNWLLLNRARLGTDTLPVCSSPANVRGQSLVIININFPGPSVQGPETPEVPSYPDT
PSYPDTTSVSSTTEITSAVDDYTDLTTIEATDDRNTWGMTEAQSGHHHHHH

# Fig. 11

SEQ ID NO. 13: Amino acid sequence of full-length human LRRC15 isoform a

MPLDKAMPLKHYLLLLVGCQAWGAGLAYHGCPSECTCSRASQVECTGARIVAVPTPLPWNAMSLQILNT
HITELNESPFLNISALIALRIEKNELSRITPGAFRNLGSLRYLSLANNKLQVLPIGLFQGLDSLESLLLSSN
QLLQIQPAHFSQCSNLKELQLHGNHLEYIPDGAFDHLVGLTKLNLGKNSLTHISPRVFQHLGNLQVLRLY
ENRLTDIPMGTFDGLVNLQELALQQNQIGLLSPGLFHNNHNLQRLYLSNNHISQLPPSVFMQLPQLNRL
TLFGNSLKELSPGIFGPMPNLRELWLYDNHISSLPDNVFSNLRQLQVLILSRNQISFISPGAFNGLTELRE
LSLHTNALQDLDGNVFRMLANLQNISLQNNRLRQLPGNIFANVNGLMAIQLQNNQLENLPLGIFDHLGK
LCELRLYDNPWRCDSDILPLRNWLLLNQPRLGTDTVPVCFSPANVRGQSLIIINVNVAVPSVHVPEVPSYP
ETPWYPDTPSYPDTTSVSSTTELTSPVEDYTDLTTIQVTDDRSVWGMTQAQSGLAIAAIVIGIVALACSLAA
CVGCCCCKKRSQAVLMQMKAPNEC

SEQ ID NO. 14: Nucleotide sequence of rR024 heavy chain variable region cDNA

GAGGTGCAGCTGGTGGAGTCTAATGGAGCCTTAGTGCAGCCTGGAAGGTCCCTAAAACTCTCCTGTGC
AGCCTCAGGGTTCACCTTCCGTGACTATTACATGGCCTGGGTCCGCCAGGCTCCATCGAAGGGGCTGG
AGTGGGTCGCATCCATTGGTTTTGATGAAAGTCGCACTTACTATCGAGACTCCGTGAAGGGCCGATTCA
CTATCTCCAGAGATGATGTAAAAAGCACCCTATACCTACAACTGGACAGTCTGAGGTCTGAGGACACGG
CCACTTATTACTGTACAAGACATGGGGTTTCTTACTACAGTGGTGACAATTGGTTTGCCTACTGGGGCCA
AGGCGCTCTGGTCATTGTCTCTTCA

SEQ ID NO. 15: Amino acid sequence of rR024 heavy chain variable region cDNA

EVQLVESNGALVQPGRSLKLSCAASGFTFRDYYMAWVRQAPSKGLEWVASIGFDESRTYYRDSVKGRFTI
SRDDVKSTLYLQLDSLRSEDTATYYCTRHGVSYYSGDNWFAYWGQGALVIVSS

SEQ ID NO. 16: Nucleotide sequence of rR024 light chain variable region cDNA

GACATTGTGATGACCCAATCTCCATCCTCTCTGGCTGTGTCAGCAGGAGAGACGGTCATTATAAACTGC
AAGTCCAGTCAGGGTCTTTTATACAGTGGAGACCAAAAGAACTACTTGGCCTGGTACCAGCAGAAACCA
GGGCAGTCTCCTAAACTGCTGATCTACTGGGCATCTACTAGGCAATCTGGTGTCCCTGATCGCTTCATA
GGCAGTGGATCTGGGACAGACTTCACTCTGACCATCAGCAGTGTGCAGGGAGAAGATCTGGCAATTTAT
TACTGTCAGCAGTATTATGATCCTCCGTACACGTTTGGAGCTGGGACCAAGCTGGAACTGAAACGG

SEQ ID NO. 17: Amino acid sequence of rR024 light chain variable region cDNA

DIVMTQSPSSLAVSAGETVIINCKSSQGLLYSGDQKNYLAWYQQKPGQSPKLLIYWASTRQSGVPDRFIGS
GSGTDFTLTISSVQGEDLAIYYCQQYYDPPYTFGAGTKLELKR

# Fig. 12

SEQ ID NO. 18: DNA fragment containing a human light chain signal sequence and a DNA sequence that encodes a human κ chain constant region

gcctccggactctagagccaccATGGTGCTGCAGACCCAGGTGTTCATCTCCCTGCTGCTGTGGATCTCCGGCGCG
TACGGCGATATCGTGATGATTAAACGTACGGTGGCCGCCCCCTCCGTGTTCATCTTCCCCCCCTCCGAC
GAGCAGCTGAAGTCCGGCACCGCCTCCGTGGTGTGCCTGCTGAATAACTTCTACCCCAGAGAGGCCAA
GGTGCAGTGGAAGGTGGACAACGCCCTGCAGTCCGGGAACTCCCAGGAGAGCGTGACCGAGCAGGAC
AGCAAGGACAGCACCTACAGCCTGAGCAGCACCCTGACCCTGAGCAAAGCCGACTACGAGAAGCACAA
GGTGTACGCCTGCGAGGTGACCCACCAGGGCCTGAGCTCCCCCGTCACCAAGAGCTTCAACAGGGGGG
AGTGTtaggggcccgtttaaacgggggaggcta

SEQ ID NO. 19: DNA fragment containing a human heavy chain signal sequence and a DNA sequence that encodes the amino acids of a human IgG1 constant region

gggtctagagccaccATGAAACACCTGTGGTTCTTCCTCCTGCTGGTGGCAGCTCCCAGATGGGTGCTGAGCC
AGGTGCAATTGTGCAGGCGGTTAGCTCAGCCTCCACCAAGGGCCCAAGCGTCTTCCCCCTGGCACCCT
CCTCCAAGAGCACCTCTGGCGGCACAGCCGCCCTGGGCTGCCTGGTCAAGGACTACTTCCCCGAACCC
GTGACCGTGAGCTGGAACTCAGGCGCCCTGACCAGCGGCGTGCACACCTTCCCCGCTGTCCTGCAGTC
CTCAGGACTCTACTCCCTCAGCAGCGTGGTGACCGTGCCCTCCAGCAGCTTGGGCACCCAGACCTACAT
CTGCAACGTGAATCACAAGCCCAGCAACACCAAGGTGGACAAGAAGGTTGAGCCCAAATCTTGTGACA
AAACTCACACATGCCCACCCTGCCCAGCACCTGAAGCCGCGGGGGGACCCTCAGTCTTCCTCTTCCCC
CCAAAACCCAAGGACACCCTCATGATCTCCCGGACCCCTGAGGTCACATGCGTGGTGGTGGACGTGAG
CCACGAAGACCCTGAGGTCAAGTTCAACTGGTACGTGGACGGCGTGGAGGTGCATAATGCCAAGACAA
AGCCCCGGGAGGAGCAGTACAACAGCACGTACCGGGTGGTCAGCGTCCTCACCGTCCTGCACCAGGAC
TGGCTGAATGGCAAGGAGTACAAGTGCAAGGTCTCCAACAAAGCCCTCGCAGCCCCCATCGAGAAAAC
CATCTCCAAAGCCAAAGGCCAGCCCCGGGAACCACAGGTGTACACCCTGCCCCCATCCCGGGAGGAGA
TGACCAAGAACCAGGTCAGCCTGACCTGCCTGGTCAAAGGCTTCTATCCCAGCGACATCGCCGTGGAG
TGGGAGAGCAATGGCCAGCCCGAGAACAACTACAAGACCACCCCTCCCGTGCTGGACTCCGACGGCTC
CTTCTTCCTCTACAGCAAGCTCACCGTGGACAAGAGCAGGTGGCAGCAGGGCAACGTCTTCTCATGCTC
CGTGATGCATGAGGCTCTGCACAACCACTACACCCAGAAGAGCCTCTCCCTGTCTCCCGGCAAAtgagata
tcgggcccgtttaaacggg

# Fig. 13

SEQ ID NO. 20: chR024 CDRH1 amino acid sequence
GFTFRDYY

SEQ ID NO. 21: chR024 CDRH2 amino acid sequence
IGFDESRT

SEQ ID NO. 22: chR024 CDRH3 amino acid sequence
TRHGVSYYSGDNWFAY

SEQ ID NO. 23: chR024 heavy chain variable region amino acid sequence
EVQLVESNGALVQPGRSLKLSCAASGFTFRDYYMAWVRQAPSKGLEWVASIGFDESRTYYRDSVKGRFTI
SRDDVKSTLYLQLDSLRSEDTATYYCTRHGVSYYSGDNWFAYWGQGALVIVSS

SEQ ID NO. 24: chR024 signal sequence and full-length heavy chain amino acid sequence
MKHLWFFLLLVAAPRWVLSEVQLVESNGALVQPGRSLKLSCAASGFTFRDYYMAWVRQAPSKGLEWVAS
IGFDESRTYYRDSVKGRFTISRDDVKSTLYLQLDSLRSEDTATYYCTRHGVSYYSGDNWFAYWGQGALVI
VSSASTKGPSVFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPEAAGGPSVFLFPPKPKDTLMISR
TPEVTCVVVDVSHEDPEVKFNWYVDGVEVHNAKTKPREEQYNSTYRVVSVLTVLHQDWLNGKEYKCK
VSNKALAAPIEKTISKAKGQPREPQVYTLPPSREEMTKNQVSLTCLVKGFYPSDIAVEWESNGQPENNYK
TTPPVLDSDGSFFLYSKLTVDKSRWQQGNVFSCSVMHEALHNHYTQKSLSLSPGK

# Fig. 14

SEQ ID NO. 25: chR024 signal sequence and full-length heavy chain nucleotide sequence
ATGAAGCACCTGTGGTTCTTTCTGCTGCTGGTGGCCGCACCTAGATGGGTCCTGTCTGAAGTGCAGCTG
GTGGAAAGCAATGGCGCCCTGGTTCAGCCTGGCAGAAGCCTGAAACTGAGCTGTGCCGCCAGCGGCTT
CACCTTCAGAGACTACTATATGGCCTGGGTCCGACAGGCCCCTAGCAAAGGACTTGAGTGGGTTGCCA
GCATCGGCTTCGACGAGAGCCGGACCTACTACCGGGATAGCGTGAAGGGCAGATTCACCATCAGCCGG
GACGACGTGAAGTCTACCCTGTACCTGCAGCTGGACAGCCTGAGAAGCGAGGATACCGCCACCTACTA
CTGCACCAGACACGGCGTGTCCTACTACAGCGGCGACAATTGGTTCGCCTATTGGGGACAGGGCGCTC
TGGTCATCGTTAGCTCAGCCTCCACCAAGGGCCCAAGCGTCTTCCCCCTGGCACCCTCCTCCAAGAGCA
CCTCTGGCGGCACAGCCGCCCTGGGCTGCCTGGTCAAGGACTACTTCCCCGAACCCGTGACCGTGAGC
TGGAACTCAGGCGCCCTGACCAGCGGCGTGCACACCTTCCCCGCTGTCCTGCAGTCCTCAGGACTCTA
CTCCCTCAGCAGCGTGGTGACCGTGCCCTCCAGCAGCTTGGGCACCCAGACCTACATCTGCAACGTGA
ATCACAAGCCCAGCAACACCAAGGTGGACAAGAAGGTTGAGCCCAAATCTTGTGACAAAACTCACACAT
GCCCACCCTGCCCAGCACCTGAAGCCGCGGGGGGACCCTCAGTCTTCCTCTTCCCCCCAAAACCCAAG
GACACCCTCATGATCTCCCGGACCCCTGAGGTCACATGCGTGGTGGTGGACGTGAGCCACGAAGACCC
TGAGGTCAAGTTCAACTGGTACGTGGACGGCGTGGAGGTGCATAATGCCAAGACAAAGCCCCGGGAGG
AGCAGTACAACAGCACGTACCGGGTGGTCAGCGTCCTCACCGTCCTGCACCAGGACTGGCTGAATGGC
AAGGAGTACAAGTGCAAGGTCTCCAACAAAGCCCTCGCAGCCCCCATCGAGAAAACCATCTCCAAAGC
CAAAGGCCAGCCCCGGGAACCACAGGTGTACACCCTGCCCCCATCCCGGGAGGAGATGACCAAGAACC
AGGTCAGCCTGACCTGCCTGGTCAAAGGCTTCTATCCCAGCGACATCGCCGTGGAGTGGGAGAGCAAT
GGCCAGCCCGAGAACAACTACAAGACCACCCCTCCCGTGCTGGACTCCGACGGCTCCTTCTTCCTCTAC
AGCAAGCTCACCGTGGACAAGAGCAGGTGGCAGCAGGGCAACGTCTTCTCATGCTCCGTGATGCATGA
GGCTCTGCACAACCACTACACCCAGAAGAGCCTCTCCCTGTCTCCCGGCAAA

# Fig. 15

SEQ ID NO. 26: chR024 CDRL1 amino acid sequence
QGLLYSGDQKNY

SEQ ID NO. 27: chR024 CDRL2 amino acid sequence
WAS

SEQ ID NO. 28: chR024 CDRL3 amino acid sequence
QQYYDPPYT

SEQ ID NO. 29: chR024 light chain variable region amino acid sequence
DIVMTQSPSSLAVSAGETVIINCKSSQGLLYSGDQKNYLAWYQQKPGQSPKLLIYWASTRQSGVPDRFIGS
GSGTDFTLTISSVQGEDLAIYYCQQYYDPPYTFGAGTKLELKR

SEQ ID NO. 30: chR024 signal sequence and full-length light chain amino acid sequence
MVLQTQVFISLLLWISGAYGDIVMTQSPSSLAVSAGETVIINCKSSQGLLYSGDQKNYLAWYQQKPGQSPK
LLIYWASTRQSGVPDRFIGSGSGTDFTLTISSVQGEDLAIYYCQQYYDPPYTFGAGTKLELKRTVAAPSVFI
FPPSDEQLKSGTASVVCLLNNFYPREAKVQWKVDNALQSGNSQESVTEQDSKDSTYSLSSTLTLSKADY
EKHKVYACEVTHQGLSSPVTKSFNRGEC

SEQ ID NO. 31: chR024 signal sequence and full-length light chain nucleotide sequence
ATGGTTCTGCAGACCCAGGTGTTCATCAGCCTGCTGCTGTGGATCAGCGGCGCCTATGGCGACATCGTG
ATGACACAGAGCCCTAGCAGCCTGGCTGTGTCTGCTGGCGAGACAGTGATCATCAACTGCAAGAGCAG
CCAGGGCCTGCTGTACTCCGGCGACCAGAAGAATTACCTGGCCTGGTATCAGCAGAAGCCCGGACAGT
CTCCCAAGCTGCTGATCTACTGGGCCAGCACAAGACAGAGCGGCGTGCCCGATAGATTCATCGGCTCT
GGCAGCGGCACCGACTTCACCCTGACAATCAGTAGCGTGCAGGGCGAAGATCTGGCCATCTACTACTG
CCAGCAGTACTACGACCCTCCTTACACCTTTGGAGCCGGCACCAAGCTGGAACTGAAGCGTACGGTGG
CCGCCCCCTCCGTGTTCATCTTCCCCCCCTCCGACGAGCAGCTGAAGTCCGGCACCGCCTCCGTGGTG
TGCCTGCTGAATAACTTCTACCCCAGAGAGGCCAAGGTGCAGTGGAAGGTGGACAACGCCCTGCAGTC
CGGGAACTCCCAGGAGAGCGTGACCGAGCAGGACAGCAAGGACAGCACCTACAGCCTGAGCAGCACC
CTGACCCTGAGCAAAGCCGACTACGAGAAGCACAAGGTGTACGCCTGCGAGGTGACCCACCAGGGCCT
GAGCTCCCCCGTCACCAAGAGCTTCAACAGGGGGGAGTGT

# Fig. 16

SEQ ID NO. 32: hR024 H09 CDRH1 amino acid sequence
GFTFRDYY

SEQ ID NO. 33: hR024 H09 CDRH2 amino acid sequence
IGFDESRT

SEQ ID NO. 34: hR024 H09 CDRH3 amino acid sequence
TRHGVSYYSGDNWFAY

SEQ ID NO. 35: hR024 H09 heavy chain variable region amino acid sequence
EVQLVESGGGLVQPGGSLRLSCAASGFTFRDYYMAWVRQAPGKGLEWVASIGFDESRTYYADSVKGRFTI
SRDDSKSTLYLQMNSLRAEDTAVYYCTRHGVSYYSGDNWFAYWGQGTLVTVSS

SEQ ID NO. 36: hR024 H09 signal sequence and full-length heavy chain amino acid sequence
MKHLWFFLLLVAAPRWVLSEVQLVESGGGLVQPGGSLRLSCAASGFTFRDYYMAWVRQAPGKGLEWVA
SIGFDESRTYYADSVKGRFTISRDDSKSTLYLQMNSLRAEDTAVYYCTRHGVSYYSGDNWFAYWGQGTLV
TVSSASTKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPEAAGGPSVFLFPPKPKDTLMIS
RTPEVTCVVVDVSHEDPEVKFNWYVDGVEVHNAKTKPREEQYNSTYRVVSVLTVLHQDWLNGKEYKC
KVSNKALAAPIEKTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK
Signal sequence: positions 1 to 19, heavy chain variable region: positions 20 to 142, heavy chain constant region: positions 143 to 472

# Fig. 17

SEQ ID NO. 37: hR024 H09 signal sequence and full-length heavy chain nucleotide sequence
ATGAAGCACCTGTGGTTCTTTCTGCTGCTGGTGGCCGCTCCTAGATGGGTGCTGTCTGAAGTGCAGCTG
GTGGAATCTGGCGGAGGACTGGTTCAACCTGGCGGCTCTCTGAGACTGTCTTGTGCCGCCAGCGGCTT
CACCTTCCGGGATTACTATATGGCCTGGGTCCGACAGGCCCCTGGCAAAGGACTTGAATGGGTTGCCAG
CATCGGCTTCGACGAGAGCAGAACCTACTACGCCGACAGCGTGAAGGGCAGATTCACCATCAGCCGGG
ACGACAGCAAGAGCACCCTGTACCTGCAGATGAACAGCCTGAGAGCCGAGGACACCGCCGTGTACTAC
TGTACAAGACATGGCGTGTCCTACTACAGCGGCGACAATTGGTTCGCCTATTGGGGCCAGGGCACCCTG
GTTACAGTGTCTAGCGCCTCTACAAAGGGCCCCAGCGTTTTCCCACTGGCTCCTAGCAGCAAGTCTACC
AGCGGAGGAACAGCCGCTCTGGGCTGTCTGGTCAAGGACTACTTTCCCGAGCCTGTGACCGTGTCCTG
GAATTCTGGCGCTCTGACAAGCGGCGTGCACACCTTTCCAGCTGTGCTGCAAAGCAGCGGCCTGTACT
CTCTGAGCAGCGTGGTCACAGTGCCAAGCTCTAGCCTGGGCACCCAGACCTACATCTGCAATGTGAACC
ACAAGCCTAGCAACACCAAGGTGGACAAGAAGGTGGAACCCAAGAGCTGCGACAAGACCCACACCTGT
CCTCCATGTCCTGCTCCAGAAGCTGCAGGCGGCCCTTCCGTGTTTCTGTTCCCTCCAAAGCCTAAGGAC
ACCCTGATGATCAGCAGAACCCCTGAAGTGACCTGCGTGGTGGTGGATGTGTCCCACGAGGATCCCGA
AGTGAAGTTCAATTGGTACGTGGACGGCGTGGAAGTGCACAACGCCAAGACCAAGCCTAGAGAGGAAC
AGTACAACAGCACCTACAGAGTGGTGTCCGTGCTGACCGTGCTGCACCAGGATTGGCTGAACGGCAAA
GAGTACAAGTGCAAGGTGTCCAACAAGGCCCTGGCTGCCCCTATCGAGAAAACCATCAGCAAGGCCAA
GGGCCAGCCTAGGGAACCCCAGGTTTACACACTGCCTCCAAGCCGGGAAGAGATGACCAAGAACCAGG
TGTCCCTGACCTGCCTGGTTAAGGGCTTCTACCCCTCCGATATCGCCGTGGAATGGGAGAGCAATGGCC
AGCCAGAGAACAACTACAAGACAACCCCTCCTGTGCTGGACAGCGACGGCTCATTCTTCCTGTACAGCA
AGCTGACAGTGGACAAGTCCAGATGGCAGCAGGGCAACGTGTTCAGCTGCAGCGTGATGCACGAGGCC
CTGCACAACCACTACACCCAGAAGTCCCTGAGCCTGTCTCCTGGCAAA
Signal sequence: positions 1 to 57, heavy chain variable region: positions 58 to 426, heavy chain constant region: positions 427 to 1416

# Fig. 18

SEQ ID NO. 38: hR024 H02 CDRH1 amino acid sequence
GFTFRDYY

SEQ ID NO. 39: hR024 H02 CDRH2 amino acid sequence
IGFDESRT

SEQ ID NO. 40: hR024 H02 CDRH3 amino acid sequence
ARHGVSYYSGDNWFAY

SEQ ID NO. 41: hR024 H02 heavy chain variable region amino acid sequence
EVQLVESGGGLVQPGGSLRLSCAASGFTFRDYYMAWVRQAPGKGLEWVSSIGFDESRTYYADSVKGRFTI
SRDDSKNTLYLQMNSLRAEDTAVYYCARHGVSYYSGDNWFAYWGQGTLVTVSS

SEQ ID NO. 42: hR024 H02 signal sequence and full-length heavy chain amino acid sequence
MKHLWFFLLLVAAPRWVLSEVQLVESGGGLVQPGGSLRLSCAASGFTFRDYYMAWVRQAPGKGLEWVS
SIGFDESRTYYADSVKGRFTISRDDSKNTLYLQMNSLRAEDTAVYYCARHGVSYYSGDNWFAYWGQGTLV
TVSSASTKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPEAAGGPSVFLFPPKPKDTLMIS
RTPEVTCVVVDVSHEDPEVKFNWYVDGVEVHNAKTKPREEQYNSTYRVVSVLTVLHQDWLNGKEYKC
KVSNKALAAPIEKTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK
Signal sequence: positions 1 to 19, heavy chain variable region: positions 20 to 142, heavy chain constant region: positions 143 to 472

# Fig. 19

SEQ ID NO. 43: hR024 H02 signal sequence and full-length heavy chain nucleotide sequence
ATGAAACACCTGTGGTTCTTCCTCCTGCTGGTGGCAGCTCCCAGATGGGTGCTGAGCGAGGTGCAGCT
GGTTGAATCTGGCGGAGGACTGGTTCAGCCTGGCGGATCTCTGAGACTGTCTTGTGCCGCCAGCGGCT
TCACCTTCCGGGATTACTATATGGCCTGGGTCCGACAGGCCCCTGGCAAAGGACTTGAATGGGTGTCCA
GCATCGGCTTCGACGAGAGCAGAACCTACTACGCCGACAGCGTGAAGGGCAGATTCACCATCAGCCGG
GACGACAGCAAGAACACCCTGTACCTGCAGATGAACAGCCTGAGAGCCGAGGACACCGCCGTGTACTA
TTGTGCCAGACATGGCGTGTCCTACTACAGCGGCGACAATTGGTTCGCCTATTGGGGCCAGGGCACCCT
GGTTACAGTGAGCTCAGCCTCCACCAAGGGCCCAAGCGTCTTCCCCCTGGCACCCTCCTCCAAGAGCA
CCTCTGGCGGCACAGCCGCCCTGGGCTGCCTGGTCAAGGACTACTTCCCCGAACCCGTGACCGTGAGC
TGGAACTCAGGCGCCCTGACCAGCGGCGTGCACACCTTCCCCGCTGTCCTGCAGTCCTCAGGACTCTA
CTCCCTCAGCAGCGTGGTGACCGTGCCCTCCAGCAGCTTGGGCACCCAGACCTACATCTGCAACGTGA
ATCACAAGCCCAGCAACACCAAGGTGGACAAGAAGGTTGAGCCCAAATCTTGTGACAAAACTCACACAT
GCCCACCCTGCCCAGCACCTGAAGCCGCGGGGGGACCCTCAGTCTTCCTCTTCCCCCCAAAACCCAAG
GACACCCTCATGATCTCCCGGACCCCTGAGGTCACATGCGTGGTGGTGGACGTGAGCCACGAAGACCC
TGAGGTCAAGTTCAACTGGTACGTGGACGGCGTGGAGGTGCATAATGCCAAGACAAAGCCCCGGGAGG
AGCAGTACAACAGCACGTACCGGGTGGTCAGCGTCCTCACCGTCCTGCACCAGGACTGGCTGAATGGC
AAGGAGTACAAGTGCAAGGTCTCCAACAAAGCCCTCGCAGCCCCCATCGAGAAAACCATCTCCAAAGC
CAAAGGCCAGCCCCGGGAACCACAGGTGTACACCCTGCCCCCATCCCGGGAGGAGATGACCAAGAACC
AGGTCAGCCTGACCTGCCTGGTCAAAGGCTTCTATCCCAGCGACATCGCCGTGGAGTGGGAGAGCAAT
GGCCAGCCCGAGAACAACTACAAGACCACCCCTCCCGTGCTGGACTCCGACGGCTCCTTCTTCCTCTAC
AGCAAGCTCACCGTGGACAAGAGCAGGTGGCAGCAGGGCAACGTCTTCTCATGCTCCGTGATGCATGA
GGCTCTGCACAACCACTACACCCAGAAGAGCCTCTCCCTGTCTCCCGGCAAA
Signal sequence: positions 1 to 57, heavy chain variable region: positions 58 to 426, heavy chain constant region: positions 427 to 1416

# Fig. 20

SEQ ID NO. 44: hR024 H02-V1 CDRH1 amino acid sequence
GFTFRDYY

SEQ ID NO. 45: hR024 H02-V1 CDRH2 amino acid sequence
IGFDESRT

SEQ ID NO. 46: hR024 H02-V1 CDRH3 amino acid sequence
ARHGVSKYSGDNWFAY

SEQ ID NO. 47: hR024 H02-V1 heavy chain variable region amino acid sequence
EVQLVESGGGLVQPGGSLRLSCAASGFTFRDYYMAWVRQAPGKGLEWVSSIGFDESRTYYADSVKGRFTI
SRDDSKNTLYLQMNSLRAEDTAVYYCARHGVSKYSGDNWFAYWGQGTLVTVSS

SEQ ID NO. 48: hR024 H02-V1 signal sequence and full-length heavy chain amino acid sequence
MKHLWFFLLLVAAPRWVLSEVQLVESGGGLVQPGGSLRLSCAASGFTFRDYYMAWVRQAPGKGLEWVS
SIGFDESRTYYADSVKGRFTISRDDSKNTLYLQMNSLRAEDTAVYYCARHGVSKYSGDNWFAYWGQGTL
VTVSSASTKGPSVFPLAPSSKSTSGGTAALGCLVKDYFPEPVTVSWNSGALTSGVHTFPAVLQSSGLYSLS
SVVTVPSSSLGTQTYICNVNHKPSNTKVDKKVEPKSCDKTHTCPPCPAPEAAGGPSVFLFPPKPKDTLMI
SRTPEVTCVVVDVSHEDPEVKFNWYVDGVEVHNAKTKPREEQYNSTYRVVSVLTVLHQDWLNGKEYK
CKVSNKALAAPIEKTISKAKGQPREPQVYTLPPSREEMTKNQVSLTCLVKGFYPSDIAVEWESNGQPENN
YKTTPPVLDSDGSFFLYSKLTVDKSRWQQGNVFSCSVMHEALHNHYTQKSLSLSPGK
Signal sequence: positions 1 to 19, heavy chain variable region: positions 20 to 142, heavy chain constant region: positions 143 to 472

# Fig. 21

SEQ ID NO. 49: hR024 H02-V1 signal sequence and full-length heavy chain nucleotide sequence
ATGAAACACCTGTGGTTCTTCCTCCTGCTGGTGGCAGCTCCCAGATGGGTGCTGAGCGAGGTGCAGCT
GGTTGAATCTGGCGGAGGACTGGTTCAGCCTGGCGGATCTCTGAGACTGTCTTGTGCCGCCAGCGGCT
TCACCTTCCGGGATTACTATATGGCCTGGGTCCGACAGGCCCCTGGCAAAGGACTTGAATGGGTGTCCA
GCATCGGCTTCGACGAGAGCAGAACCTACTACGCCGACAGCGTGAAGGGCAGATTCACCATCAGCCGG
GACGACAGCAAGAACACCCTGTACCTGCAGATGAACAGCCTGAGAGCCGAGGACACCGCCGTGTACTA
TTGTGCCAGACATGGCGTGTCCAAATACAGCGGCGACAATTGGTTCGCCTATTGGGGCCAGGGCACCC
TGGTTACAGTGAGCTCAGCCTCCACCAAGGGCCCAAGCGTCTTCCCCCTGGCACCCTCCTCCAAGAGC
ACCTCTGGCGGCACAGCCGCCCTGGGCTGCCTGGTCAAGGACTACTTCCCCGAACCCGTGACCGTGAG
CTGGAACTCAGGCGCCCTGACCAGCGGCGTGCACACCTTCCCCGCTGTCCTGCAGTCCTCAGGACTCT
ACTCCCTCAGCAGCGTGGTGACCGTGCCCTCCAGCAGCTTGGGCACCCAGACCTACATCTGCAACGTG
AATCACAAGCCCAGCAACACCAAGGTGGACAAGAAGGTTGAGCCCAAATCTTGTGACAAAACTCACAC
ATGCCCACCCTGCCCAGCACCTGAAGCCGCGGGGGGACCCTCAGTCTTCCTCTTCCCCCCAAAACCCA
AGGACACCCTCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAAGCCCCGGGA
GGAGCAGTACAACAGCACGTACCGGGTGGTCAGCGTCCTCACCGTCCTGCACCAGGACTGGCTGAATG
GCAAGGAGTACAAGTGCAAGGTCTCCAACAAAGCCCTCGCAGCCCCCATCGAGAAAACCATCTCCAAA
GCCAAAGGCCAGCCCCGGGAACCACAGGTGTACACCCTGCCCCCATCCCGGGAGGAGATGACCAAGAA
CCAGGTCAGCCTGACCTGCCTGGTCAAAGGCTTCTATCCCAGCGACATCGCCGTGGAGTGGGAGAGCA
ATGGCCAGCCCGAGAACAACTACAAGACCACCCCTCCCGTGCTGGACTCCGACGGCTCCTTCTTCCTCT
ACAGCAAGCTCACCGTGGACAAGAGCAGGTGGCAGCAGGGCAACGTCTTCTCATGCTCCGTGATGCAT
GAGGCTCTGCACAACCACTACACCCAGAAGAGCCTCTCCCTGTCTCCCGGCAAA
Signal sequence: positions 1 to 57, heavy chain variable region: positions 58 to 426, heavy chain constant region: positions 427 to 1416

# Fig. 22

SEQ ID NO. 50: hR024 H02-V2 CDRH1 amino acid sequence
GFTFRDYY

SEQ ID NO. 51: hR024 H02-V2 CDRH2 amino acid sequence
IGFDESRT

SEQ ID NO. 52: hR024 H02-V2 CDRH3 amino acid sequence
ARHGVSRYSGDNWFAY

SEQ ID NO. 53: hR024 H02-V2 heavy chain variable region amino acid sequence
EVQLVESGGGLVQPGGSLRLSCAASGFTFRDYYMAWVRQAPGKGLEWVSSIGFDESRTYYADSVKGRFTI
SRDDSKNTLYLQMNSLRAEDTAVYYCARHGVSRYSGDNWFAYWGQGTLVTVSS

SEQ ID NO. 54: hR024 H02-V2 signal sequence and full-length heavy chain amino acid sequence
MKHLWFFLLLVAAPRWVLSEVQLVESGGGLVQPGGSLRLSCAASGFTFRDYYMAWVRQAPGKGLEWVS
SIGFDESRTYYADSVKGRFTISRDDSKNTLYLQMNSLRAEDTAVYYCARHGVSRYSGDNWFAYWGQGTLV
TVSSASTKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPEAAGGPSVFLFPPKPKDTLMIS
RTPEVTCVVVDVSHEDPEVKFNWYVDGVEVHNAKTKPREEQYNSTYRVVSVLTVLHQDWLNGKEYKC
KVSNKALAAPIEKTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK
Signal sequence: positions 1 to 19, heavy chain variable region: positions 20 to 142, heavy chain constant region: positions 143 to 472

# Fig. 23

SEQ ID NO. 55: hR024 H02-V2 signal sequence and full-length heavy chain nucleotide sequence
ATGAAACACCTGTGGTTCTTCCTCCTGCTGGTGGCAGCTCCCAGATGGGTGCTGAGCGAGGTGCAGCT
GGTTGAATCTGGCGGAGGACTGGTTCAGCCTGGCGGATCTCTGAGACTGTCTTGTGCCGCCAGCGGCT
TCACCTTCCGGGATTACTATATGGCCTGGGTCCGACAGGCCCCTGGCAAAGGACTTGAATGGGTGTCCA
GCATCGGCTTCGACGAGAGCAGAACCTACTACGCCGACAGCGTGAAGGGCAGATTCACCATCAGCCGG
GACGACAGCAAGAACACCCTGTACCTGCAGATGAACAGCCTGAGAGCCGAGGACACCGCCGTGTACTA
TTGTGCCAGACATGGCGTGTCCAGATACAGCGGCGACAATTGGTTCGCCTATTGGGGCCAGGGCACCC
TGGTTACAGTGAGCTCAGCCTCCACCAAGGGCCCAAGCGTCTTCCCCCTGGCACCCTCCTCCAAGAGC
ACCTCTGGCGGCACAGCCGCCCTGGGCTGCCTGGTCAAGGACTACTTCCCCGAACCCGTGACCGTGAG
CTGGAACTCAGGCGCCCTGACCAGCGGCGTGCACACCTTCCCCGCTGTCCTGCAGTCCTCAGGACTCT
ACTCCCTCAGCAGCGTGGTGACCGTGCCCTCCAGCAGCTTGGGCACCCAGACCTACATCTGCAACGTG
AATCACAAGCCCAGCAACACCAAGGTGGACAAGAAGGTTGAGCCCAAATCTTGTGACAAAACTCACAC
ATGCCCACCCTGCCCAGCACCTGAAGCCGCGGGGGGACCCTCAGTCTTCCTCTTCCCCCCAAAACCCA
AGGACACCCTCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAAGCCCCGGGA
GGAGCAGTACAACAGCACGTACCGGGTGGTCAGCGTCCTCACCGTCCTGCACCAGGACTGGCTGAATG
GCAAGGAGTACAAGTGCAAGGTCTCCAACAAAGCCCTCGCAGCCCCCATCGAGAAAACCATCTCCAAA
GCCAAAGGCCAGCCCCGGGAACCACAGGTGTACACCCTGCCCCCATCCCGGGAGGAGATGACCAAGAA
CCAGGTCAGCCTGACCTGCCTGGTCAAAGGCTTCTATCCCAGCGACATCGCCGTGGAGTGGGAGAGCA
ATGGCCAGCCCGAGAACAACTACAAGACCACCCCTCCCGTGCTGGACTCCGACGGCTCCTTCTTCCTCT
ACAGCAAGCTCACCGTGGACAAGAGCAGGTGGCAGCAGGGCAACGTCTTCTCATGCTCCGTGATGCAT
GAGGCTCTGCACAACCACTACACCCAGAAGAGCCTCTCCCTGTCTCCCGGCAAA
Signal sequence: positions 1 to 57, heavy chain variable region: positions 58 to 426, heavy chain constant region: positions 427 to 1416

# Fig. 24

SEQ ID NO. 56: hR024 L03 CDRL1 amino acid sequence
QGLLYSGDQKNY

SEQ ID NO. 57: hR024 L03 CDRL2 amino acid sequence
WAS

SEQ ID NO. 58: hR024 L03 CDRL3 amino acid sequence
QQYYDPPYT

SEQ ID NO. 59: hR024 L03 light chain variable region amino acid sequence
DIQMTQSPSSLSASVGDRVTITCKSSQGLLYSGDQKNYLAWYQQKPGKSPKLLIYWASTRQSGVPDRFSG
SGSGTDFTLTISSLQPEDFATYYCQQYYDPPYTFGQGTKVEIKR

SEQ ID NO. 60: hR024 L03 signal sequence and full-length light chain amino acid sequence
MVLQTQVFISLLLWISGAYGDIQMTQSPSSLSASVGDRVTITCKSSQGLLYSGDQKNYLAWYQQKPGKSP
KLLIYWASTRQSGVPDRFSGSGSGTDFTLTISSLQPEDFATYYCQQYYDPPYTFGQGTKVEIKRTVAAPSV
FIFPPSDEQLKSGTASVVCLLNNFYPREAKVQWKVDNALQSGNSQESVTEQDSKDSTYSLSSTLTLSKAD
YEKHKVYACEVTHQGLSSPVTKSFNRGEC
Signal sequence: positions 1 to 20, heavy chain variable region: positions 21 to 134, heavy chain constant region: positions 135 to 240

SEQ ID NO. 61: hR024 L03 signal sequence and full-length light chain nucleotide sequence
ATGGTGCTGCAGACCCAGGTGTTCATCTCCCTGCTGCTGTGGATCTCCGGCGCGTACGGCGACATCCA
GATGACACAGAGCCCTAGCAGCCTGTCTGCCAGCGTGGGAGACAGAGTGACCATCACATGCAAGAGCA
GCCAGGGCCTGCTGTACTCCGGCGACCAGAAGAATTACCTGGCCTGGTATCAGCAGAAGCCCGGCAAG
TCTCCCAAGCTGCTGATCTACTGGGCCAGCACAAGACAGAGCGGCGTGCCCGATAGATTTTCTGGCAG
CGGCTCTGGCACCGACTTCACCCTGACCATATCTAGCCTGCAGCCTGAGGACTTCGCCACCTACTACTG
CCAGCAGTACTACGACCCTCCTTACACCTTTGGCCAGGGCACCAAGGTGGAAATCAAGCGTACGGTGG
CCGCCCCCTCCGTGTTCATCTTCCCCCCCTCCGACGAGCAGCTGAAGTCCGGCACCGCCTCCGTGGTG
TGCCTGCTGAATAACTTCTACCCCAGAGAGGCCAAGGTGCAGTGGAAGGTGGACAACGCCCTGCAGTC
CGGGAACTCCCAGGAGAGCGTGACCGAGCAGGACAGCAAGGACAGCACCTACAGCCTGAGCAGCACC
CTGACCCTGAGCAAAGCCGACTACGAGAAGCACAAGGTGTACGCCTGCGAGGTGACCCACCAGGGCCT
GAGCTCCCCCGTCACCAAGAGCTTCAACAGGGGGGAGTGT
Signal sequence: positions 1 to 60, heavy chain variable region: positions 61 to 402, heavy chain constant region: positions 403 to 720

# Fig. 25

SEQ ID NO. 62: hR024 L01 CDRL1 amino acid sequence
QGLLYSGDQKNY

SEQ ID NO. 63: hR024 L01 CDRL2 amino acid sequence
WAS

SEQ ID NO. 64: hR024 L01 CDRL3 amino acid sequence
QQYYDPPYT

SEQ ID NO. 65: hR024 L01 light chain variable region amino acid sequence
DIQMTQSPSSLSASVGDRVTITCKSSQGLLYSGDQKNYLAWYQQKPGKAPKLLIYWASTRQSGVPSRFSG
SGSGTDFTLTISSLQPEDFATYYCQQYYDPPYTFGQGTKVEIKR

SEQ ID NO. 66: hR024 L01 signal sequence and full-length light chain amino acid sequence
MVLQTQVFISLLLWISGAYGDIQMTQSPSSLSASVGDRVTITCKSSQGLLYSGDQKNYLAWYQQKPGKAP
KLLIYWASTRQSGVPSRFSGSGSGTDFTLTISSLQPEDFATYYCQQYYDPPYTFGQGTKVEIKRTVAAPSVF
IFPPSDEQLKSGTASVVCLLNNFYPREAKVQWKVDNALQSGNSQESVTEQDSKDSTYSLSSTLTLSKADY
EKHKVYACEVTHQGLSSPVTKSFNRGEC
Signal sequence: positions 1 to 20, heavy chain variable region: positions 21 to 134, heavy chain constant region: positions 135 to 240

SEQ ID NO. 67: hR024 L01 signal sequence and full-length light chain nucleotide sequence
ATGGTGCTGCAGACCCAGGTGTTCATCTCCCTGCTGCTGTGGATCTCCGGCGCGTACGGCGACATCCA
GATGACACAGAGCCCTAGCAGCCTGTCTGCCAGCGTGGGAGACAGAGTGACCATCACATGCAAGAGCA
GCCAGGGCCTGCTGTACTCCGGCGACCAGAAGAATTACCTGGCCTGGTATCAGCAGAAGCCCGGCAAG
GCTCCTAAGCTGCTGATCTACTGGGCCAGCACAAGACAGAGCGGCGTGCCAAGCAGATTTTCTGGCAG
CGGCTCTGGCACCGACTTCACCCTGACCATATCTAGCCTGCAGCCTGAGGACTTCGCCACCTACTACTG
CCAGCAGTACTACGACCCTCCTTACACCTTTGGCCAGGGCACCAAGGTGGAAATCAAGCGTACGGTGG
CCGCCCCCTCCGTGTTCATCTTCCCCCCCTCCGACGAGCAGCTGAAGTCCGGCACCGCCTCCGTGGTG
TGCCTGCTGAATAACTTCTACCCCAGAGAGGCCAAGGTGCAGTGGAAGGTGGACAACGCCCTGCAGTC
CGGGAACTCCCAGGAGAGCGTGACCGAGCAGGACAGCAAGGACAGCACCTACAGCCTGAGCAGCACC
CTGACCCTGAGCAAAGCCGACTACGAGAAGCACAAGGTGTACGCCTGCGAGGTGACCCACCAGGGCCT
GAGCTCCCCCGTCACCAAGAGCTTCAACAGGGGGGAGTGT
Signal sequence: positions 1 to 60, heavy chain variable region: positions 61 to 402, heavy chain constant region: positions 403 to 720

# Fig. 26

SEQ ID NO. 68: huM25 signal sequence and full-length heavy chain amino acid sequence

MKHLWFFLLLVAAPRWVLSEVQLVQSGAEVKKPGASVKVSCKASGYKFSSYWIEWVKQAPGQGLEWIG
EILPGSDTTNYNEKFKDRATFTSDTSINTAYMELSRLRSDDTAVYYCARDRGNYRAWFGYWGQGTLVTV
SSASTKGPSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTLMISRT
PEVTCVVVDVSHEDPEVKFNWYVDGVEVHNAKTKPREEQYNSTYRVVSVLTVLHQDWLNGKEYKCKV
SNKALPAPIEKTISKAKGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK

Signal sequence: positions 1 to 19, heavy chain variable region: positions 20 to 139, heavy chain constant region: positions 140 to 469

SEQ ID NO. 69: huM25 signal sequence and full-length heavy chain nucleotide sequence

ATGAAACACCTGTGGTTCTTCCTCCTGCTGGTGGCAGCTCCCAGATGGGTGCTGAGCGAAGTTCAGCT
GGTTCAGTCTGGCGCCGAAGTGAAGAAACCTGGCGCCTCTGTGAAGGTGTCCTGCAAGGCCAGCGGCT
ACAAGTTCAGCAGCTACTGGATCGAGTGGGTCAAGCAGGCCCCTGGACAAGGCCTGGAATGGATCGGA
GAGATCCTGCCTGGCAGCGACACCACCAACTACAACGAGAAGTTCAAGGACCGGGCCACCTTCACCTC
CGACACCTCTATCAACACCGCCTACATGGAACTGAGCCGGCTGAGATCCGATGACACCGCCGTGTACT
ACTGCGCCAGAGACAGAGGCAACTACAGAGCTTGGTTTGGCTACTGGGGCCAGGGCACACTGGTCACA
GTTAGCTCAGCCTCCACCAAGGGCCCAAGCGTCTTCCCCCTGGCACCCTCCTCCAAGAGCACCTCTGG
CGGCACAGCCGCCCTGGGCTGCCTGGTCAAGGACTACTTCCCCGAACCCGTGACCGTGAGCTGGAACT
CAGGCGCCCTGACCAGCGGCGTGCACACCTTCCCCGCTGTCCTGCAGTCCTCAGGACTCTACTCCCTC
AGCAGCGTGGTGACCGTGCCCTCCAGCAGCTTGGGCACCCAGACCTACATCTGCAACGTGAATCACAA
GCCCAGCAACACCAAGGTGGACAAGAAGGTTGAGCCCAAATCTTGTGACAAAACTCACACATGCCCAC
CCTGCCCAGCACCTGAACTCCTGGGGGGACCCTCAGTCTTCCTCTTCCCCCCAAAACCCAAGGACACC
CTCATGATCTCCCGGACCCCTGAGGTCACATGCGTGGTGGTGGACGTGAGCCACGAAGACCCTGAGGT
CAAGTTCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGGCAAGGA
GTACAAGTGCAAGGTCTCCAACAAAGCCCTCCCAGCCCCCATCGAGAAAACCATCTCCAAAGCCAAAG
GCCAGCCCCGGGAACCACAGGTGTACACCCTGCCCCCATCCCGGGAGGAGATGACCAAGAACCAGGTC
AGCCTGACCTGCCTGGTCAAAGGCTTCTATCCCAGCGACATCGCCGTGGAGTGGGAGAGCAATGGCCA
GCCCGAGAACAACTACAAGACCACCCCTCCCGTGCTGGACTCCGACGGCTCCTTCTTCCTCTACAGCAA
GCTCACCGTGGACAAGAGCAGGTGGCAGCAGGGCAACGTCTTCTCATGCTCCGTGATGCATGAGGCTC
TGCACAACCACTACACCCAGAAGAGCCTCTCCCTGTCTCCCGGCAAA

Signal sequence: positions 1 to 57, heavy chain variable region: positions 58 to 417, heavy chain constant region: positions 418 to 1407

# Fig. 27

SEQ ID NO. 70: huM25 signal sequence and full-length light chain amino acid sequence

MVLQTQVFISLLLWISGAYGDIQMTQSPSSLSASVGDRVTITCRASQDISNYLNWYQQKPGGAVKFLIYYT
SRLHSGVPSRFSGSGSGTDYTLTISSLQPEDFATYFCQQGEALPWTFGGGTKVEIKRTVAAPSVFIFPPSD
EQLKSGTASVVCLLNNFYPREAKVQWKVDNALQSGNSQESVTEQDSKDSTYSLSSTLTLSKADYEKHKV
YACEVTHQGLSSPVTKSFNRGEC

Signal sequence: positions 1 to 20, heavy chain variable region: positions 21 to 128, heavy chain constant region: positions 129 to 234

SEQ ID NO. 71: huM25 signal sequence and full-length light chain nucleotide sequence

ATGGTGCTGCAGACCCAGGTGTTCATCTCCCTGCTGCTGTGGATCTCCGGCGCGTACGGCGATATCCA
GATGACACAGAGCCCTAGCAGCCTGTCTGCCAGCGTGGGAGACAGAGTGACCATCACCTGTAGAGCCA
GCCAGGACATCAGCAACTACCTGAACTGGTATCAGCAGAAACCCGGCGGAGCCGTGAAGTTCCTGATC
TACTACACCAGCAGACTGCACAGCGGCGTGCCCAGCAGATTTTCTGGCTCTGGCAGCGGCACCGACTA
CACCCTGACCATATCTAGCCTGCAGCCTGAGGACTTCGCTACCTACTTCTGCCAGCAAGGCGAAGCCCT
GCCTTGGACATTTGGCGGCGGAACAAAGGTGGAAATCAAGCGTACGGTGGCCGCCCCCTCCGTGTTCA
TCTTCCCCCCCTCCGACGAGCAGCTGAAGTCCGGCACCGCCTCCGTGGTGTGCCTGCTGAATAACTTCT
ACCCCAGAGAGGCCAAGGTGCAGTGGAAGGTGGACAACGCCCTGCAGTCCGGGAACTCCCAGGAGAG
CGTGACCGAGCAGGACAGCAAGGACAGCACCTACAGCCTGAGCAGCACCCTGACCCTGAGCAAAGCC
GACTACGAGAAGCACAAGGTGTACGCCTGCGAGGTGACCCACCAGGGCCTGAGCTCCCCCGTCACCAA
GAGCTTCAACAGGGGGGAGTGT

Signal sequence: positions 1 to 60, heavy chain variable region: positions 61 to 384, heavy chain constant region: positions 385 to 702

# Fig. 28

SEQ ID NO. 72: Control hIgG signal sequence and full-length heavy chain amino acid sequence

MKHLWFFLLLVAAPRWVLSQVQLVQSGAEVKKPGASVKVSCKASGYTFTSYWINWVRQAPGQGLEWM
GNIYPGSSSINYNEKFKSRVTITADTSTSTAYMELSSLRSEDTAVYYCARTIYNYGSSGYNYAMDYWGQG
TLVTVSSASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPEAAGGPSVFLFPPKPKDTL
MISRTPEVTCVVVDVSHEDPEVKFNWYVDGVEVHNAKTKPREEQYNSTYRVVSVLTVLHQDWLNGKEY
KCKVSNKALAAPIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSPGK

Signal sequence: positions 1 to 19, heavy chain variable region: positions 20 to 144, heavy chain constant region: positions 145 to 474

SEQ ID NO. 73: Control hIgG signal sequence and full-length light chain amino acid sequence

MVLQTQVFISLLLWISGAYGDIVMTQSPDSLAVSLGERATINCKASENVGNSVSWYQQKPGQPPKLLIYG
ASNRYTGVPDRFSGSGSGTDFTLTISSLQAEDVAVYYCGQSYSYPYTFGQGTKVEIKRTVAAPSVFIFPPS
DEQLKSGTASVVCLLNNFYPREAKVQWKVDNALQSGNSQESVTEQDSKDSTYSLSSTLTLSKADYEKHK
VYACEVTHQGLSSPVTKSFNRGEC

Signal sequence: positions 1 to 20, heavy chain variable region: positions 21 to 128, heavy chain constant region: positions 129 to 234

Fig. 29

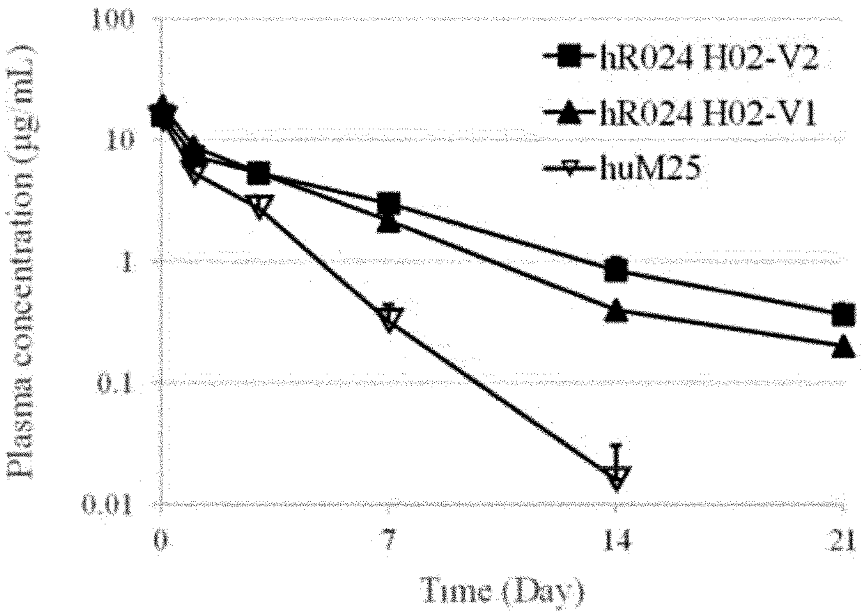
100
10
1
0.1
0.01
Plasma concentration (µg/mL)
hR024 H02-V2
hR024 H02-V1
huM25
0
7
14
21
Time (Day)

Fig. 30-1

1) H02L01

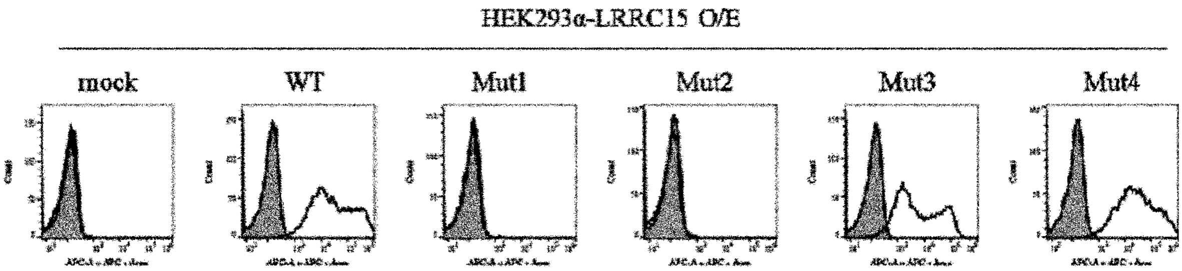


2) H02L01-V1

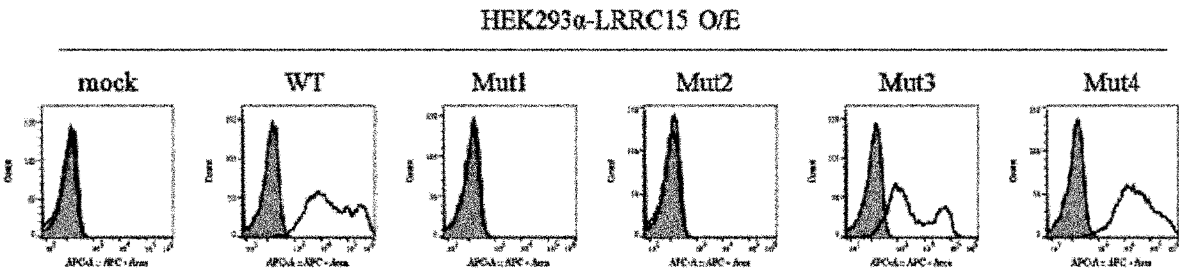


3) H02L01-V2

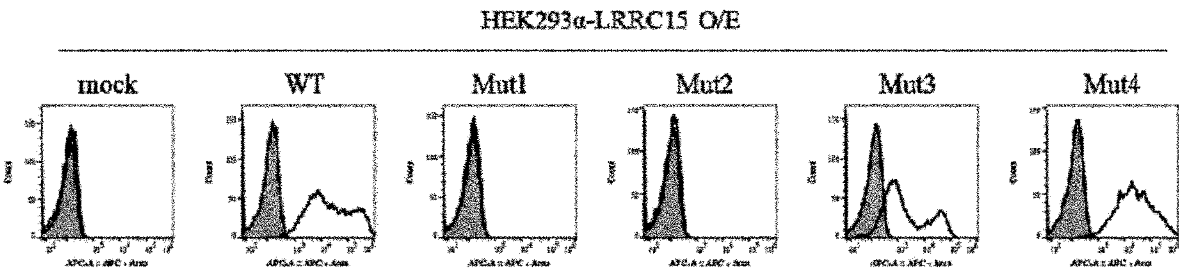


4) huM25

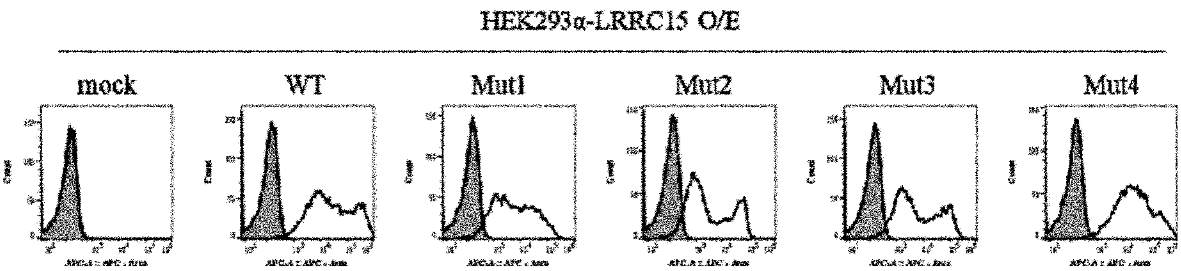

Fig. 30-2

1) H02L01

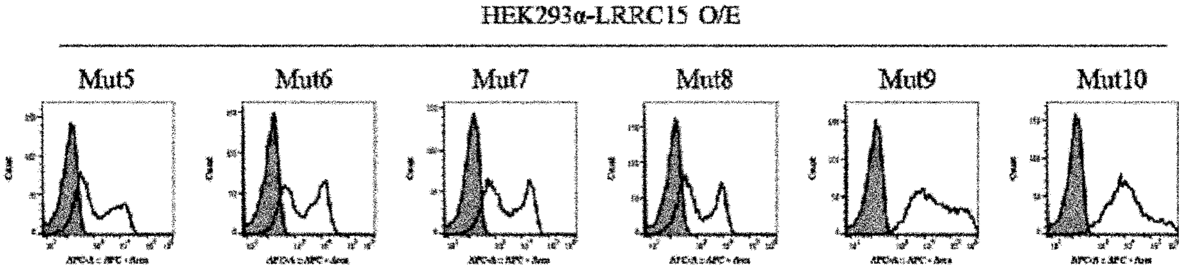


2) H02L01-V1

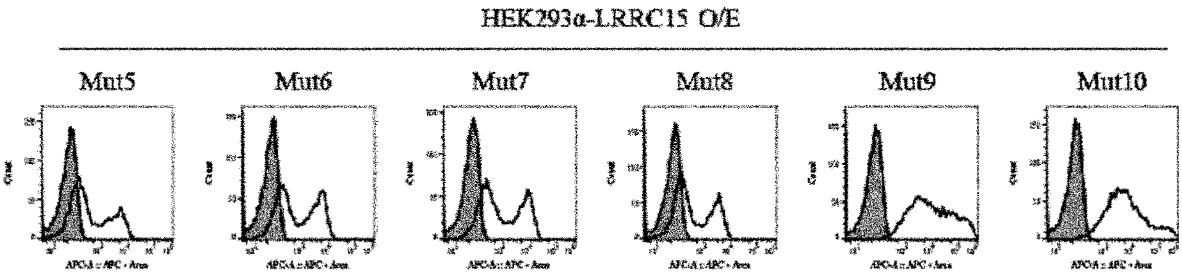


3) H02L01-V2

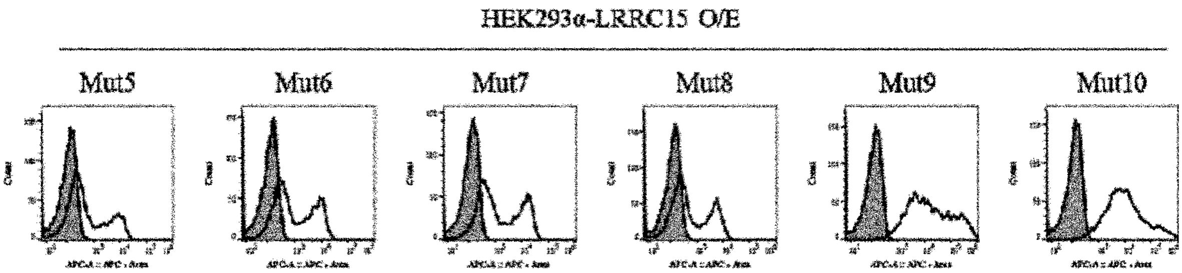


4) huM25

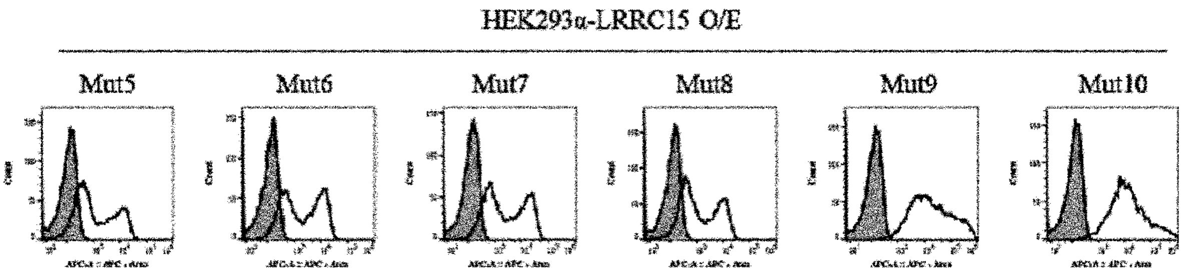

Fig. 30-3

1) H02L01

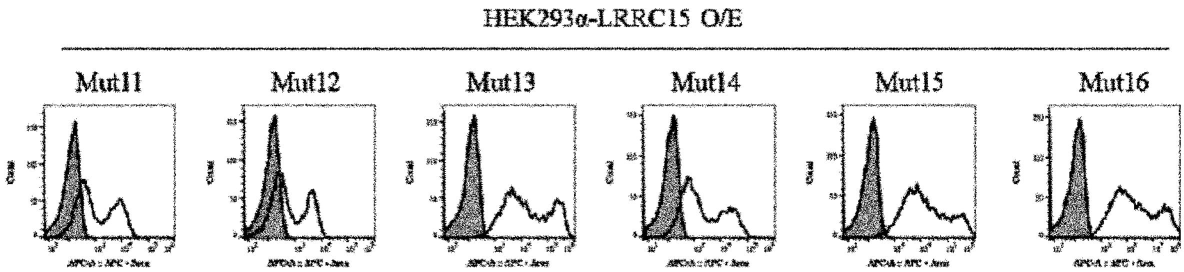


2) H02L01-V1

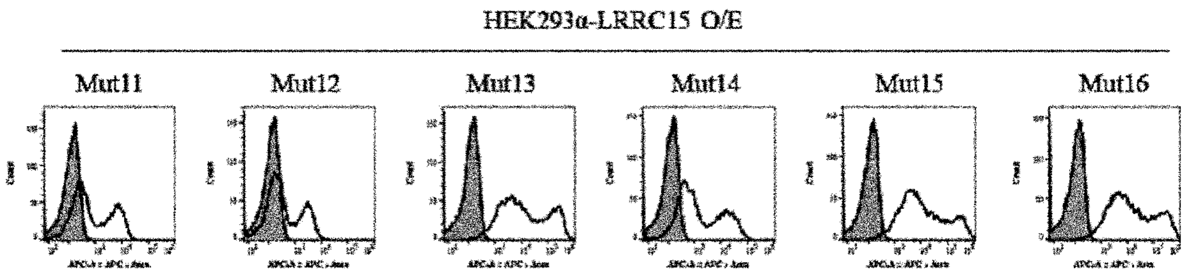


3) H02L01-V2

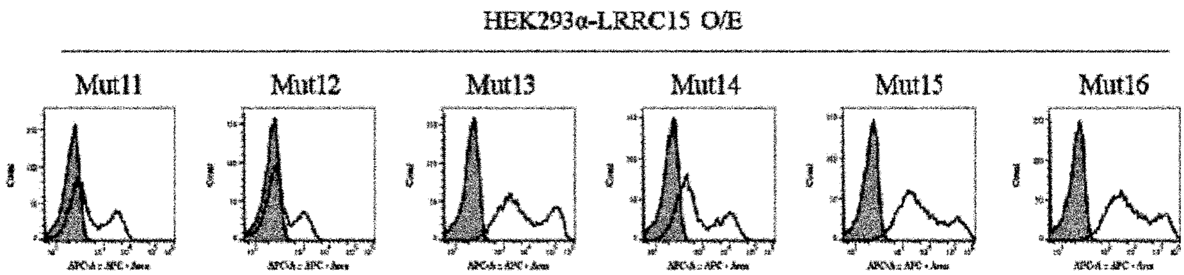


4) huM25

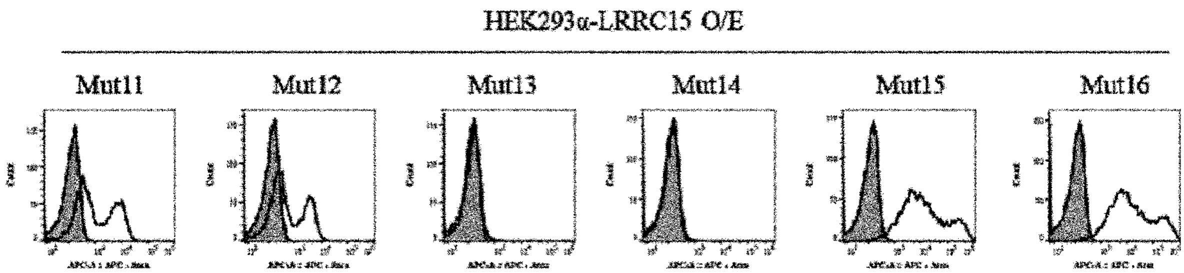

Fig. 31

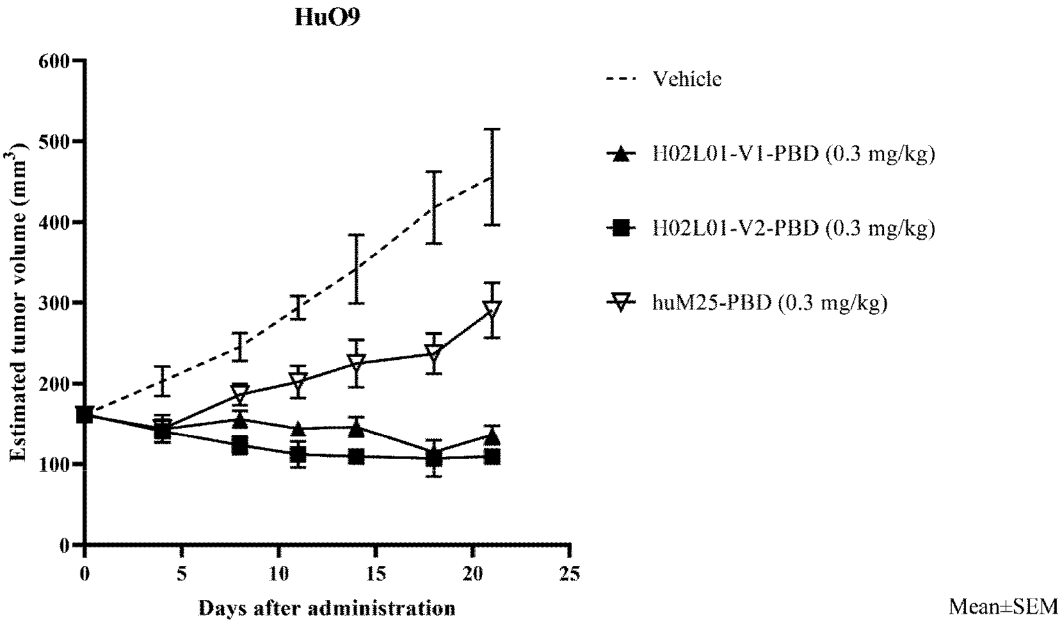
HuO9
Estimated tumor volume (mm3)
600
500
400
300
200
100
0
0
5
10
15
20
25
Days after administration
Vehicle
H02L01-V1-PBD (0.3 mg/kg)
H02L01-V2-PBD (0.3 mg/kg)
huM25-PBD (0.3 mg/kg)
Mean±SEM

Fig. 32

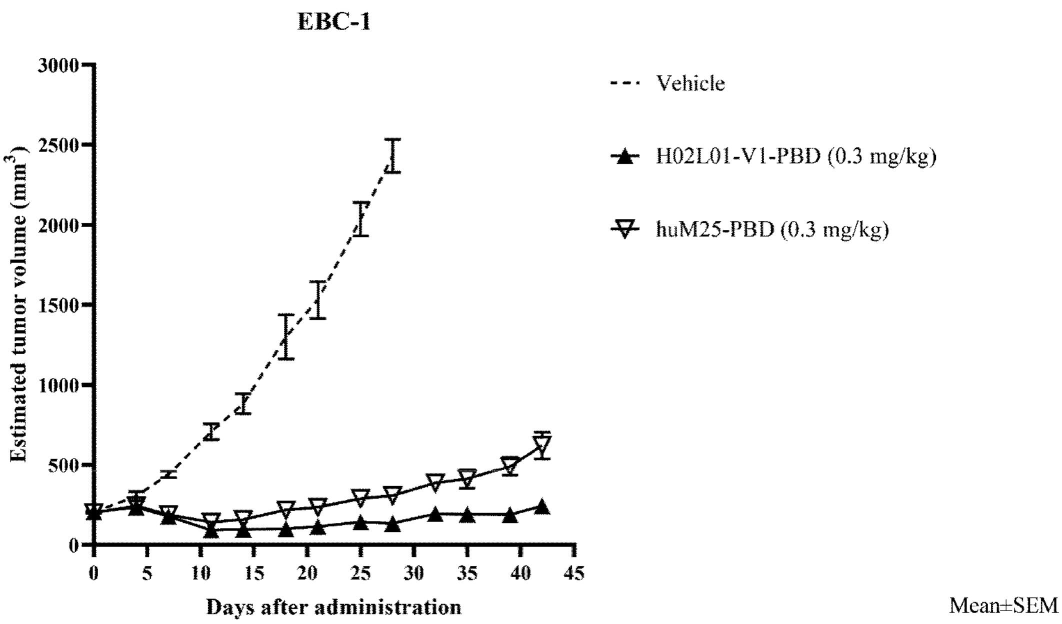
EBC-1
Estimated tumor volume (mm3)
3000
2500
2000
1500
1000
500
0
0
5
10
15
20
25
30
35
40
45
Days after administration
Vehicle
H02L01-V1-PBD (0.3 mg/kg)
huM25-PBD (0.3 mg/kg)
Mean±SEM

Fig. 33

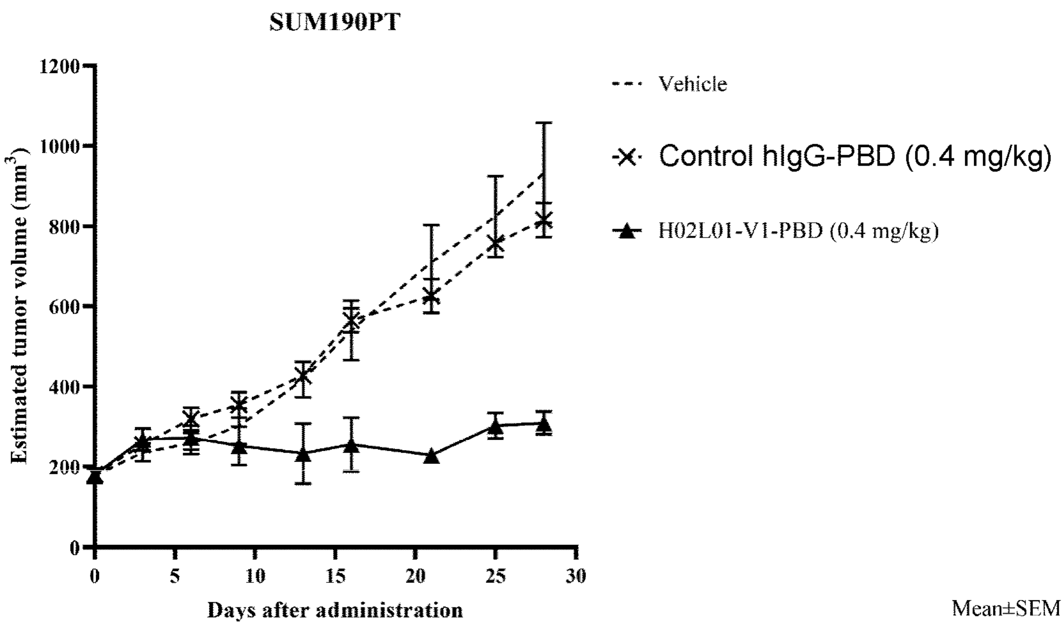
SUM190PT
Estimated tumor volume (mm³)
1200
1000
800
600
400
200
0
0
5
10
15
20
25
30
Days after administration
Vehicle
Control hIgG-PBD (0.4 mg/kg)
H02L01-V1-PBD (0.4 mg/kg)
Mean±SEM

Fig. 34

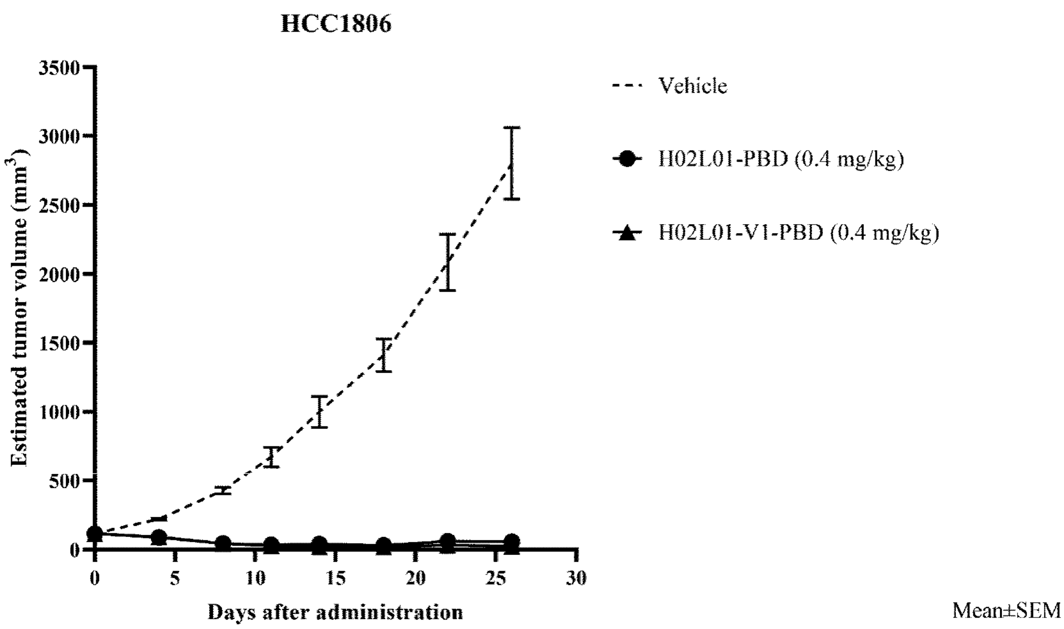
HCC1806
Estimated tumor volume (mm³)
3500
3000
2500
2000
1500
1000
500
0
0
5
10
15
20
25
30
Days after administration
Vehicle
H02L01-PBD (0.4 mg/kg)
H02L01-V1-PBD (0.4 mg/kg)
Mean±SEM

Fig. 35

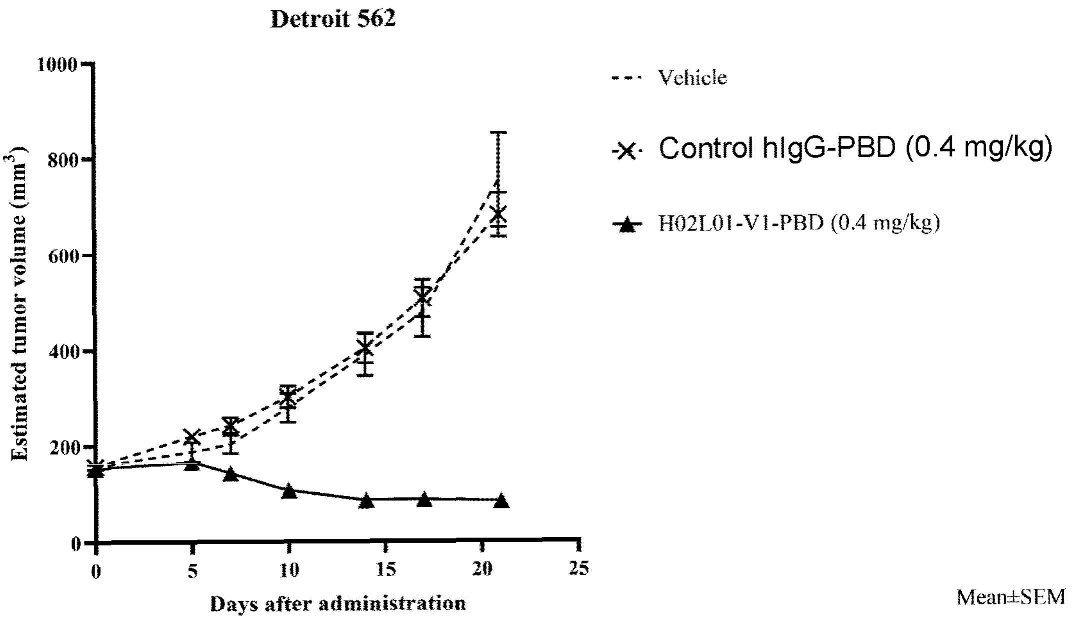
Detroit 562
Estimated tumor volume (mm³)
1000
800
600
400
200
0
0
5
10
15
20
25
Days after administration
--- Vehicle
-×- Control hIgG-PBD (0.4 mg/kg)
-▲- H02L01-V1-PBD (0.4 mg/kg)
Mean±SEM

Fig. 36

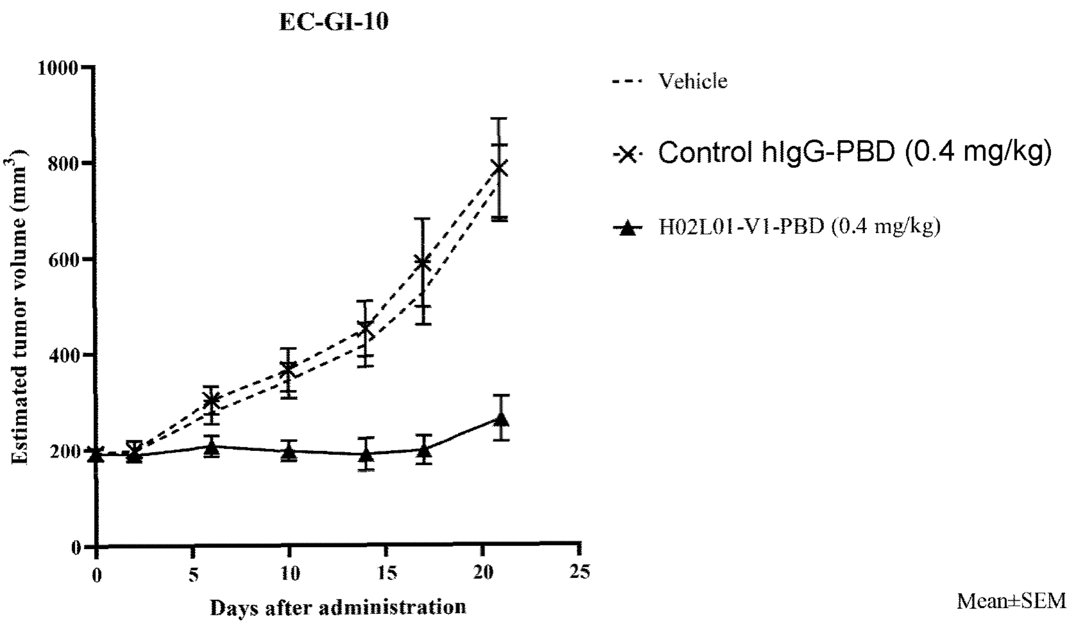
EC-GI-10
Estimated tumor volume (mm³)
1000
800
600
400
200
0
0
5
10
15
20
25
Days after administration
--- Vehicle
-×- Control hIgG-PBD (0.4 mg/kg)
-▲- H02L01-V1-PBD (0.4 mg/kg)
Mean±SEM

Fig. 37

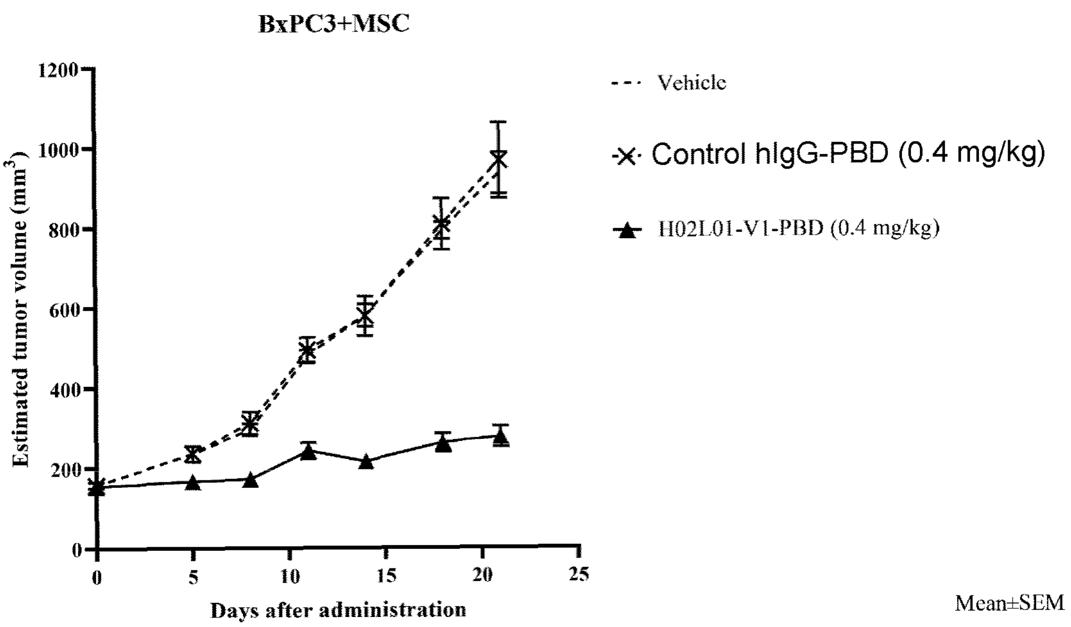
BxPC3+MSC
Estimated tumor volume (mm3)
1200
1000
800
600
400
200
0
0
5
10
15
20
25
Days after administration
Vehicle
Control hIgG-PBD (0.4 mg/kg)
H02L01-V1-PBD (0.4 mg/kg)
Mean±SEM

Fig. 38

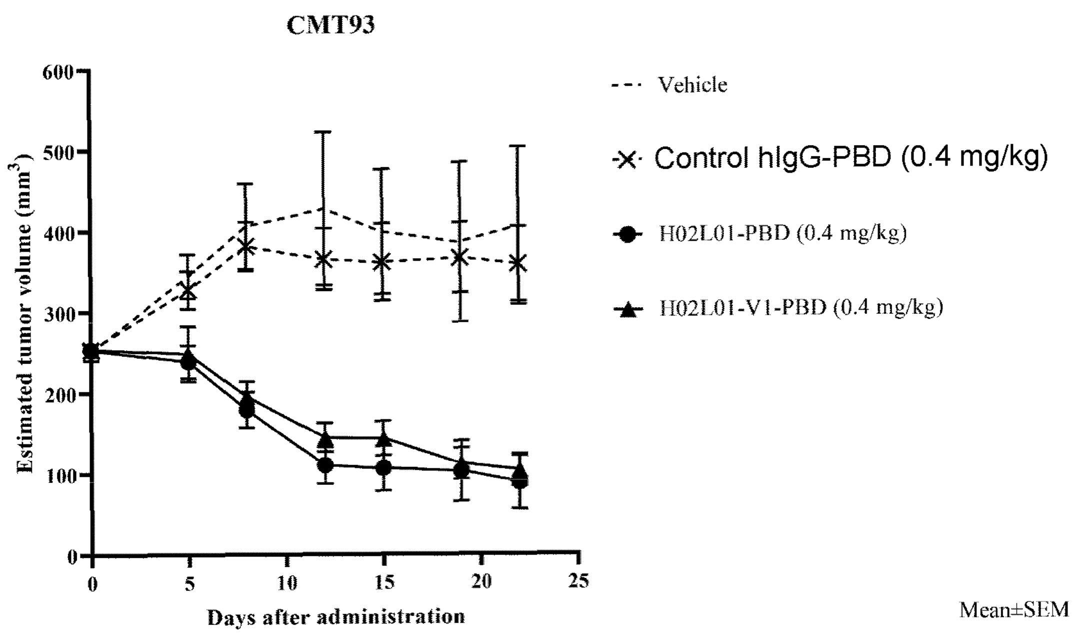
CMT93
Estimated tumor volume (mm3)
600
500
400
300
200
100
0
0
5
10
15
20
25
Days after administration
Vehicle
Control hIgG-PBD (0.4 mg/kg)
H02L01-PBD (0.4 mg/kg)
H02L01-V1-PBD (0.4 mg/kg)
Mean±SEM

Fig. 39

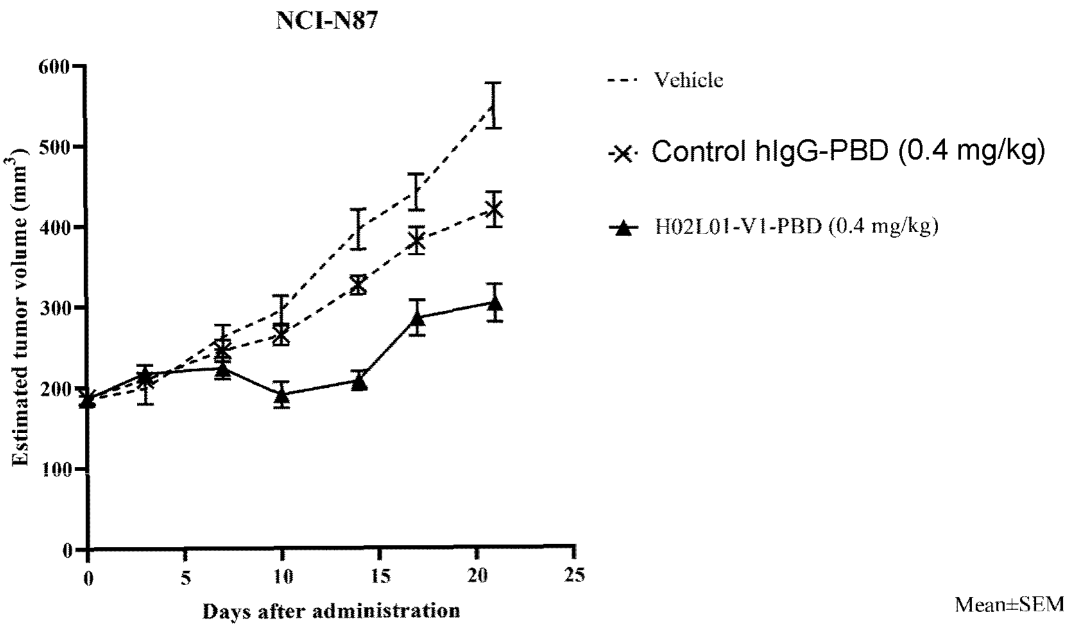
NCI-N87
Estimated tumor volume (mm3)
600
500
400
300
200
100
0
0
5
10
15
20
25
Days after administration
Vehicle
Control hIgG-PBD (0.4 mg/kg)
H02L01-V1-PBD (0.4 mg/kg)
Mean±SEM

Fig. 40

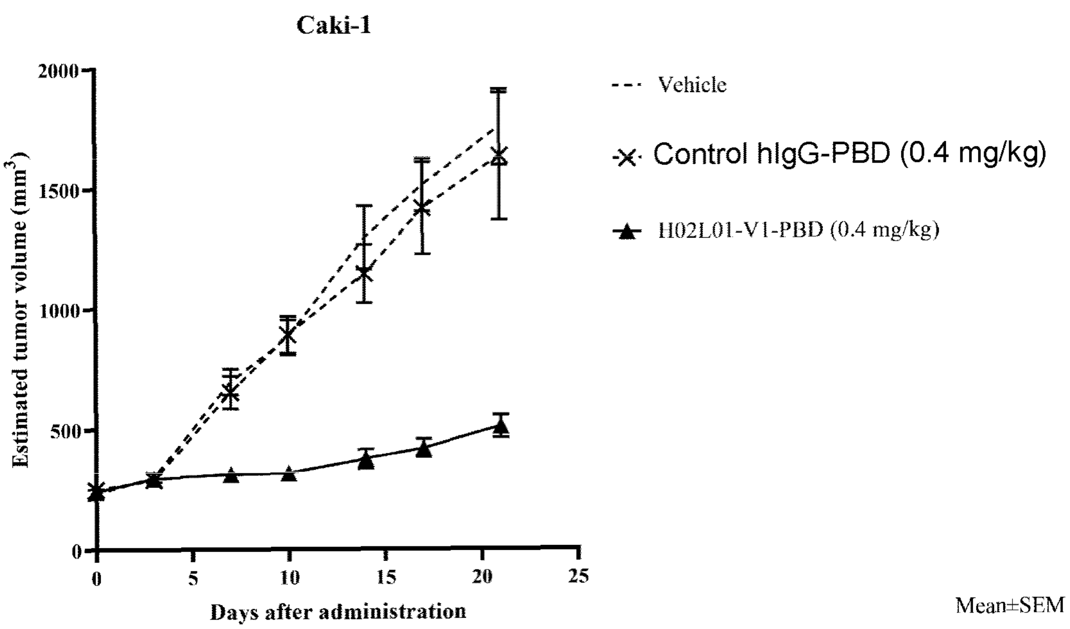
Caki-1
Estimated tumor volume (mm3)
2000
1500
1000
500
0
0
5
10
15
20
25
Days after administration
Vehicle
Control hIgG-PBD (0.4 mg/kg)
H02L01-V1-PBD (0.4 mg/kg)
Mean±SEM

Fig. 41

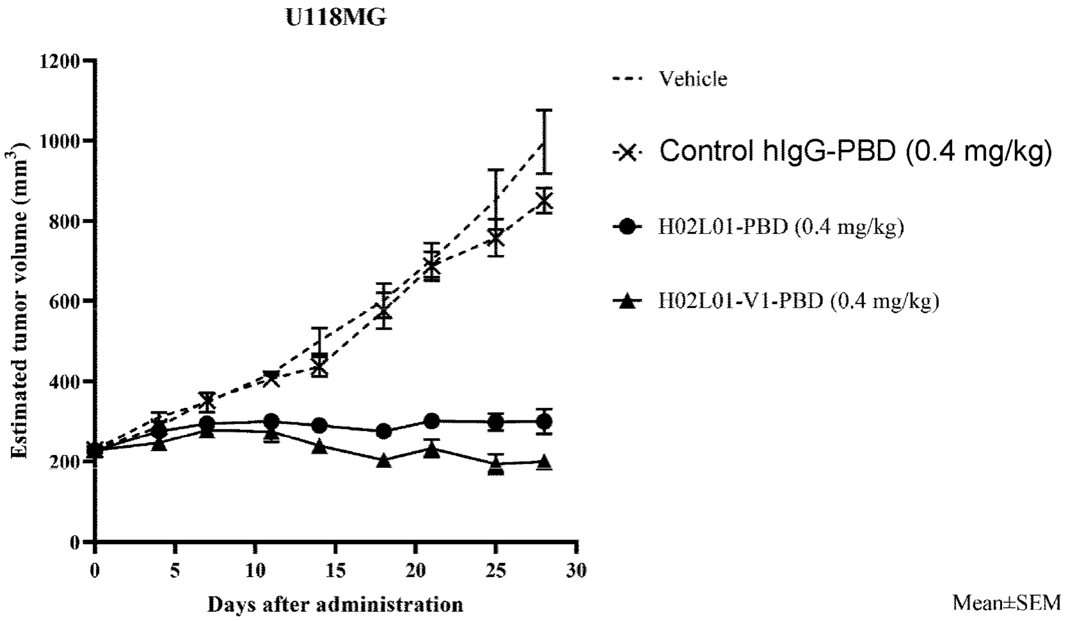
U118MG
Estimated tumor volume (mm$^3$)
1200
1000
800
600
400
200
0
0
5
10
15
20
25
30
Days after administration
Vehicle
Control hIgG-PBD (0.4 mg/kg)
H02L01-PBD (0.4 mg/kg)
H02L01-V1-PBD (0.4 mg/kg)
Mean±SEM

Fig. 42

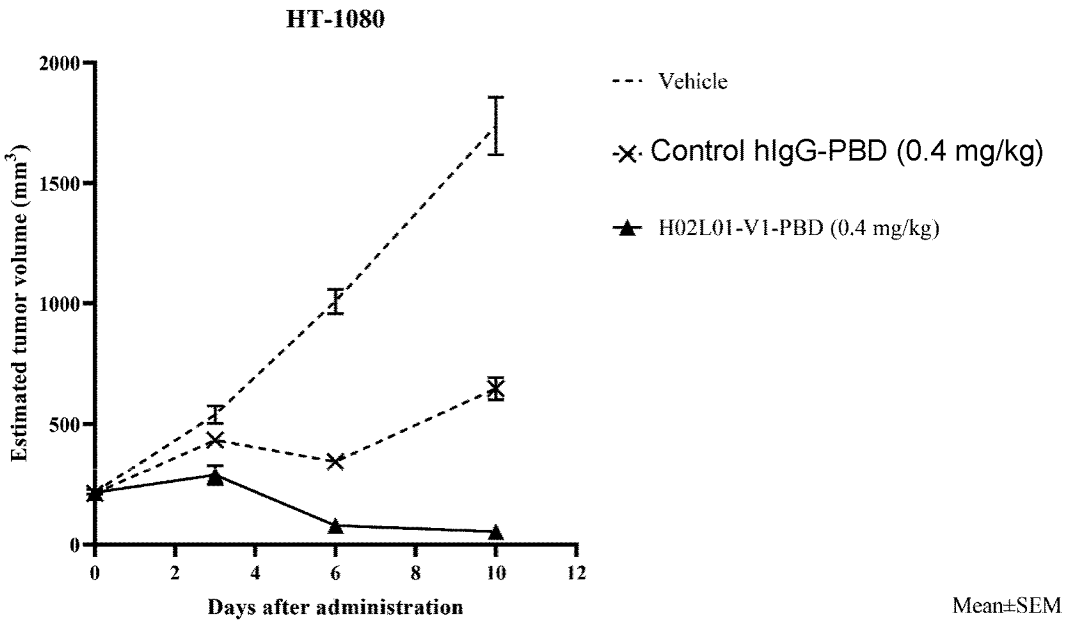
HT-1080
Estimated tumor volume (mm$^3$)
2000
1500
1000
500
0
0
2
4
6
8
10
12
Days after administration
Vehicle
Control hIgG-PBD (0.4 mg/kg)
H02L01-V1-PBD (0.4 mg/kg)
Mean±SEM

## INTERNATIONAL SEARCH REPORT

International application No.
**PCT/JP2024/002363**

### A. CLASSIFICATION OF SUBJECT MATTER

***C12N 15/13***(2006.01)i; ***A61K 31/5517***(2006.01)i; ***A61K 39/395***(2006.01)i; ***A61K 47/55***(2017.01)i; ***A61K 47/65***(2017.01)i; ***A61K 47/68***(2017.01)i; ***A61P 1/02***(2006.01)i; ***A61P 1/04***(2006.01)i; ***A61P 1/16***(2006.01)i; ***A61P 1/18***(2006.01)i; ***A61P 11/00***(2006.01)i; ***A61P 11/02***(2006.01)i; ***A61P 11/04***(2006.01)i; ***A61P 13/08***(2006.01)i; ***A61P 13/10***(2006.01)i; ***A61P 13/12***(2006.01)i; ***A61P 15/00***(2006.01)i; ***A61P 17/00***(2006.01)i; ***A61P 19/08***(2006.01)i; ***A61P 25/00***(2006.01)i; ***A61P 35/00***(2006.01)i; ***A61P 43/00***(2006.01)i; ***C07K 16/28***(2006.01)i; ***C07K 16/46***(2006.01)i; ***C12N 1/15***(2006.01)i; ***C12N 1/19***(2006.01)i; ***C12N 1/21***(2006.01)i; ***C12N 5/10***(2006.01)i; ***C12N 15/62***(2006.01)i; ***C12P 21/08***(2006.01)i

FI: C12N15/13 ZNA; C07K16/28; C12N1/15; C12N1/19; C12N1/21; C12N5/10; C07K16/46; C12N15/62 Z; A61K39/395 N; A61K39/395 L; A61K31/5517; A61P1/02; A61P1/04; A61P1/16; A61P1/18; A61P11/00; A61P11/02; A61P11/04; A61P13/08; A61P13/10; A61P13/12; A61P15/00; A61P17/00; A61P19/08; A61P25/00; A61P35/00; A61P43/00 111; A61K47/68; A61K47/55; A61K47/65; C12P21/08

According to International Patent Classification (IPC) or to both national classification and IPC

### B. FIELDS SEARCHED

Minimum documentation searched (classification system followed by classification symbols)

C12N15/00-15/90; C07K16/00-16/46; C12P21/08 ;A61K31/5517; A61K39/395

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | WO 2022/157094 A2 (BAYER AKTIENGESELLSCHAFT, BAYER AS) 28 July 2022 (2022-07-28)<br>claims 1, 5, 15-18, 21-23, 26, example 1 | 1-30, 54-61 |
| Y | | 31-53 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 April 2024** | **16 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/002363**

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2021/202642 A2 (THE REGENTS OF THE UNIVERSITY OF CALIFORNIA) 07 October 2021 (2021-10-07)<br>claims 12-17, 52-62, 68-70, example 1 | 1-30, 54-61<br>31-53 |
| X<br>Y | PURCELL J. W. et al. LRRC 15 Is a Novel Mesenchymal Protein and Stromal Target for Antibody-Drug Conjugates. CANCER RESEARCH. 15 May 2018, vol. 78, no. 14, pp. 4059-4072<br>abstract, p. 4063, left column, 2nd paragraph | 1-30, 54-61<br>31-53 |
| X<br>Y | JP 2019-501124 A (ABBVIE INC., ABBVIE BIOTHERAPEUTICS INC.) 17 January 2019 (2019-01-17)<br>abstract, paragraph [0030], examples 1, 3 | 1-30, 54-61<br>31-53 |
| Y | WO 2020/100954 A1 (DAIICHI SANKYO CO., LTD.) 22 May 2020 (2020-05-22)<br>claims 1-28 | 31-50, 52, 53 |
| Y | WO 2020/196474 A1 (DAIICHI SANKYO CO., LTD.) 01 October 2020 (2020-10-01)<br>claims 1-15 | 31-50, 52, 53 |
| Y | WO 2020/196475 A1 (DAIICHI SANKYO CO., LTD.) 01 October 2020 (2020-10-01)<br>claims 1-25 | 31-50, 52, 53 |
| Y | WO 2022/211075 A1 (MICROBIOPHARM JAPAN CO., LTD.) 06 October 2022 (2022-10-06)<br>paragraphs [0003], [0004] | 51 |

Form PCT/ISA/210 (second sheet) (July 2022)

**INTERNATIONAL SEARCH REPORT**

International application No.
**PCT/JP2024/002363**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/002363**

| Patent document cited in search report | Publication date (day/month/year) | Patent family member(s) | Publication date (day/month/year) |
|---|---|---|---|
| WO 2022/157094 A2 | 28 July 2022 | EP 4281477 A2<br>CN 116745323 A<br>KR 10-2023-0135107 A | |
| WO 2021/202642 A2 | 07 October 2021 | JP 2023-520392 A<br>US 2023/0133775 A1<br>EP 4125991 A2<br>CN 115379848 A<br>KR 10-2022-0161395 A | |
| JP 2019-501124 A | 17 January 2019 | US 2017/0151344 A1<br>abstract, paragraph [0029], examples 1, 3<br>WO 2017/095808 A1<br>EP 3383910 A1 | |
| WO 2020/100954 A1 | 22 May 2020 | US 2022/0016257 A1<br>claims 1-28<br>EP 3882349 A1<br>CN 113166764 A<br>KR 10-2021-0091711 A | |
| WO 2020/196474 A1 | 01 October 2020 | US 2022/0168440 A1<br>claims 1-15<br>EP 3950061 A1<br>CN 113631229 A<br>KR 10-2021-0143237 A | |
| WO 2020/196475 A1 | 01 October 2020 | US 2022/0177601 A1<br>claims 1-25<br>EP 3949987 A1<br>CN 113631190 A<br>KR 10-2021-0141630 A | |
| WO 2022/211075 A1 | 06 October 2022 | EP 4316527 A1<br>paragraphs [0003], [0004] | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017095805 A
- WO 2017095808 A
- WO 9007861 A
- WO 9201047 A
- WO 9220791 A
- WO 9306213 A
- WO 9311236 A
- WO 9319172 A
- WO 9501438 A
- WO 9515388 A
- WO 199954342 A
- WO 200061739 A
- WO 200231140 A
- WO 2007133855 A
- US 2016361436 A
- WO 20110278681 A
- WO 2013120066 A
- WO 2022050300 A
- WO 2023167238 A
- WO 2017010559 A
- WO 2018101454 A
- WO 2018212136 A
- WO 2019065964 A
- WO 2020196474 A


### Non-patent literature cited in the description


- *Cancer Res*, 2018, vol. 78 (14), 4059-4072
- *Cell Death and Differentiation*, 2008, vol. 15, 751-761
- *Molecular Biology of the Cell*, December 2004, vol. 15, 5268-5282
- *Bio Techniques*, January 2000, vol. 28, 162-165
- **LEFRANC et al.** Dev Comparat Immunol. 2003, vol. 27, 55-77
- **KABAT et al.** Sequences of Proteins of Immunological Interest. Public Health Service, National Institutes of Health, 1991
- **AL-LAZIKANI et al.** *J. Mol. Biol*, 1997, vol. 273, 927-948
- **LARKIN MA** ; **BLACKSHIELDS G** ; **BROWN NP** ; **CHENNA R** ; **MCGETTIGAN PA** ; **MCWILLIAM H** ; **VALENTIN F** ; **WALLACE IM** ; **WILM A** ; **LOPEZ R**. Clustal W and Clustal X version 2.0. *Bioinformatics*, 2007, vol. 23 (21), 2947-2948
- **KOHLER** ; **MILSTEIN**. *Nature*, 1975, vol. 256, 495-497
- Monoclonal Antibodies. Plenum Press, 1980, 365-367
- **AIHARA H** ; **MIYAZAKI J.** *Nat Biotechnol.*, September 1998, vol. 16 (9), 867-70
- **MIR LM** ; **BUREAU MF** ; **GEHL J** ; **RANGARA R** ; **ROUY D** ; **CAILLAUD JM** ; **DELAERE P** ; **BRANELLEC D** ; **SCHWARTZ B** ; **SCHERMAN D.** *Proc Natl Acad Sci USA.*, 13 April 1999, vol. 96 (8), 4262-7
- **MCMAHON JM1** ; **SIGNORI E** ; **WELLS KE** ; **FAZIO VM** ; **WELLS DJ.** *Gene Ther.*, August 2001, vol. 8 (16), 1264-70
- *Proc. Natl. Acad. Sci. U.S.A.*, 1984, vol. 81, 6851-6855
- *Nature*, 1986, vol. 321, 522-525
- **TOMIZUKA, K. et al.** *Nature Genetics*, 1997, vol. 16, 133-143
- **KUROIWA, Y. et al.** *Nucl. Acids Res.*, 1998, vol. 26, 3447-3448
- **YOSHIDA, H. et al.** Animal Cell Technology: Basic and Applied Aspects. Kluwer Academic Publishers, 1999, vol. 10, 69-73
- **TOMIZUKA, K. et al.** *Proc. Natl. Acad. Sci. USA*, 2000, vol. 97, 722-727
- **WORMSTONE, I. M. et al.** *Investigative Ophthalmology & Visual Science.*, 2002, vol. 43 (7), 2301-2308
- **CARMEN, S. et al.** *Briefings in Functional Genomics and Proteomics*, 2002, vol. 1 (2), 189-203
- **SIRIWARDENA, D. et al.** *Ophthalmology*, 2002, vol. 109 (3), 427-431
- *Nature Biotechnology*, 2005, vol. 23 (9), 1105-1116
- *Annu. Rev. Immunol*, 1994, vol. 12, 433-455
- **GLUZMAN, Y.** *Cell*, 1981, vol. 23, 175-182
- **URLAUB, G.** ; **CHASIN, L. A.** *Proc. Natl. Acad. Sci. U.S.A.*, 1980, vol. 77, 4126-4220
- *Journal of Chromatography A*, 1995, vol. 705, 129-134
- *Analytical Biochemistry*, 2007, vol. 360, 75-83

- *Proc. Natl. Acad. Sci. U.S.A.*, 15 May 1969, vol. 63, 78-85
- Strategies for Protein Purification and Characterization: A Laboratory Course Manual. Cold Spring Harbor Laboratory Press, 1996
- Antibodies: A Laboratory Manual.. Cold Spring Harbor Laboratory, 1988
- *ACS Chemical Biology*, 2012, vol. 7, 110
- *ACS Medicinal Chemistry Letters*, 2016, vol. 7, 1005
- *Bioconjugate Chemistry*, 2015, vol. 26, 2233
- *Angew. Chem. Int. Ed.*, 2016, vol. 55, 2361-2367
- *Biotechnol. Prog.*, 2012, vol. 28, 608-622
- **SANGLIER-CIANFERANI, S.** *Anal. Chem.*, 2013, vol. 85, 715-736
- **EDELMAN et al.** *Proc. Natl. Acad. Sci. U.S.A.*, 15 May 1969, vol. 63, 78-85
- *J. Org Chem.*, 2009, vol. 74 (5), 2210-2212
- *Helv. Chim. Acta*, 2012, vol. 95, 1928-1936
- **YAMAMOTO K et al.** *Biochem Biophys Res Commun.*, 1994, vol. 203, 244-252
- **UMEKAWA M et al.** *J. Biol Chem.*, 2021, vol. 285, 511-521
- **ESHIMA Y et al.** *PLoS One.*, 2015, vol. 10, e0132859
- **MURAKAMI S et al.** *Glycobiology.*, 2013, vol. 23, 736-744
- *J. AM. CHEM. SOC.*, 2004, vol. 126, 15046-15047
- *Protein Science*, 1995, vol. 4, 2411-2423
- **SAMBROOK, J.** ; **FRITSCH, E.F.** ; **MANIATIS, T.** Molecular Cloning. Cold SpringHarbor Laboratory Press, 1989
- *Methods in Enzymology*, 1991, vol. 203, 121-153
- *Proc. Natl. Acad. Sci. USA*, 1989, vol. 86, 10029-10033
- **KABAT et al.** Sequences of Proteins of Immunological Interest. Public Health Service National Institutes of Health, 1991
- **QUEEN et al.** *Proc. Natl. Acad. Sci. USA*, 1989, vol. 86, 10029-10033
- *CHEMICAL ABSTRACTS*, 646502-53-6